# EUROPEAN PATENT APPLICATION

(11) **EP 2 982 684 A1**
(43) Date of publication of application: **10.02.2016**
(21) Application number: 15184135.0
(22) Date of filing: 23.09.2011
(51) Int. Cl.: C07K 14/37, C07K 14/415, C07K 14/435, A61K 39/35

(54) **Peptides deriving from Alternaria Alternata major allergen Alt a 1**

(30) Priority: 24.09.2010 US 385992 P
(62) Divisional of application: 11766930.9
(71) Applicant: Diaz-Torres, Maria R., 38360 El Sauzal, Tenerife (ES); Dunn-Coleman, Nigel Stuart, 38360 El Sauzal, Tenerife (ES); Miller, Brian S., Newberry, FL 32669 (US)
(72) Inventor: Diaz-Torres, Maria R., 38360 El Sauzal, Tenerife (ES); Dunn-Coleman, Nigel Stuart, 38360 El Sauzal, Tenerife (ES); Miller, Brian S., Newberry, FL 32669 (US)
(74) Representative: Clegg, Richard Ian

(57) **Abstract**

The application discloses peptides capable of preventing or treating fungal disease, including fungal allergy disease.

SEQUENCE LISTING

<110>  Alergenetica SL
       Dunn-Coleman, Nigel S
       Diaz-Torres, Maria R
       Miller, Brian

<120>  Peptides

<130>  RIC/6779854

<140>  PCT/EP2011/066610
<141>  2011-09-23

<150>  US 61/385,992
<151>  2010-09-24

<160>  1163

<170>  PatentIn version 3.3

<210>  1
<211>  9
<212>  PRT
<213>  Alternaria alternata

<400>  1

Phe Thr Thr Ile Ala Ser Leu Phe Ala
1               5


<210>  2
<211>  9
<212>  PRT
<213>  Alternaria alternata

<400>  2

Met Gln Phe Thr Thr Ile Ala Ser Leu
1               5


<210>  3
<211>  9
<212>  PRT
<213>  Alternaria alternata

<400>  3

Ile Ala Ser Leu Phe Ala Ala Ala Gly
1               5


<210>  4
<211>  9
<212>  PRT
<213>  Alternaria alternata

<400>  4

Leu Ala Ala Ala Ala Pro Leu Glu Ser
1               5

<210> 5
<211> 9
<212> PRT
<213> Alternaria alternata

<400> 5

Trp Lys Ile Ser Glu Phe Tyr Gly Arg
1               5

<210> 6
<211> 9
<212> PRT
<213> Alternaria alternata

<400> 6

Tyr Tyr Asn Ser Leu Gly Phe Asn Ile
1               5

<210> 7
<211> 9
<212> PRT
<213> Alternaria alternata

<400> 7

Leu Gly Phe Asn Ile Lys Ala Thr Asn
1               5

<210> 8
<211> 9
<212> PRT
<213> Alternaria alternata

<400> 8

Phe Asn Ile Lys Ala Thr Asn Gly Gly
1               5

<210> 9
<211> 9
<212> PRT
<213> Alternaria alternata

<400> 9

Ile Lys Ala Thr Asn Gly Gly Thr Leu
1               5

<210> 10
<211> 9
<212> PRT
<213> Alternaria alternata

<400> 10

Ile Thr Tyr Val Ala Thr Ala Thr Leu
1               5


<210>  11
<211>  9
<212>  PRT
<213>  Alternaria alternata

<400>  11

Tyr Val Ala Thr Ala Thr Leu Pro Asn
1               5


<210>  12
<211>  9
<212>  PRT
<213>  Alternaria alternata

<400>  12

Leu Pro Asn Tyr Cys Arg Ala Gly Gly
1               5


<210>  13
<211>  9
<212>  PRT
<213>  Artificial sequence

<220>
<223>  Synthetic sequence: Peptide derivative

<400>  13

Leu Pro Asn Tyr Val Arg Ala Gly Gly
1               5


<210>  14
<211>  9
<212>  PRT
<213>  Alternaria alternata

<400>  14

Phe Val Cys Gln Gly Val Ala Asp Ala
1               5


<210>  15
<211>  9
<212>  PRT
<213>  Artificial sequence

<220>
<223>  Synthetic sequence: Peptide derivative

<400>  15

Phe Val Val Gln Gly Val Ala Asp Ala

1               5


<210>  16
<211>  9
<212>  PRT
<213>  Alternaria alternata

<400>  16

Tyr Ile Thr Leu Val Thr Leu Pro Lys
1               5


<210>  17
<211>  9
<212>  PRT
<213>  Alternaria alternata

<400>  17

Ile Thr Leu Val Thr Leu Pro Lys Ser
1               5


<210>  18
<211>  9
<212>  PRT
<213>  Alternaria alternata

<400>  18

Leu Phe Ala Ala Ala Gly Leu Ala Ala
1               5


<210>  19
<211>  9
<212>  PRT
<213>  Alternaria alternata

<400>  19

Phe Ala Ala Ala Gly Leu Ala Ala Ala
1               5


<210>  20
<211>  9
<212>  PRT
<213>  Alternaria alternata

<400>  20

Tyr Asn Ser Leu Gly Phe Asn Ile Lys
1               5


<210>  21
<211>  9
<212>  PRT
<213>  Alternaria alternata

<400>  21

Tyr Cys Arg Ala Gly Gly Asn Gly Pro
1               5


<210>  22
<211>  9
<212>  PRT
<213>  Artificial sequence

<220>
<223>  Synthetic sequence: Peptide derivative

<400>  22

Tyr Val Arg Ala Gly Gly Asn Gly Pro
1               5


<210>  23
<211>  9
<212>  PRT
<213>  Alternaria alternata

<400>  23

Leu Asp Phe Thr Cys Ser Ala Gln Ala
1               5


<210>  24
<211>  9
<212>  PRT
<213>  Artificial sequence

<220>
<223>  Synthetic sequence: Peptide derivative

<400>  24

Leu Asp Phe Thr Val Ser Ala Gln Ala
1               5


<210>  25
<211>  9
<212>  PRT
<213>  Alternaria alternata

<400>  25

Trp Tyr Ser Cys Gly Glu Asn Ser Phe
1               5


<210>  26
<211>  9
<212>  PRT
<213>  Artificial sequence

<220>
<223>  Synthetic sequence: Peptide derivative

<400>  26

Trp Tyr Ser Val Gly Glu Asn Ser Phe
1               5


<210>  27
<211>  9
<212>  PRT
<213>  Alternaria alternata

<400>  27

Val Ser Asp Asp Ile Thr Tyr Val Ala
1               5


<210>  28
<211>  9
<212>  PRT
<213>  Alternaria alternata

<400>  28

Leu Val Asn His Phe Thr Asn Glu Phe
1               5


<210>  29
<211>  9
<212>  PRT
<213>  Alternaria alternata

<400>  29

Ile Leu Leu Leu Asp Val Ala Pro Leu
1               5


<210>  30
<211>  9
<212>  PRT
<213>  Alternaria alternata

<400>  30

Leu Arg Ile Ile Asn Glu Pro Thr Ala
1               5


<210>  31
<211>  9
<212>  PRT
<213>  Alternaria alternata

<400>  31

Leu Arg Arg Leu Arg Thr Ala Cys Glu
1               5


<210>  32

<211>  9
<212>  PRT
<213>  Alternaria alternata

<400>  32

Ile Gln Gly Phe Pro Thr Ile Lys Leu
1               5


<210>  33
<211>  9
<212>  PRT
<213>  Alternaria alternata

<400>  33

Val Val Gly Leu Arg Ser Gly Gln Ile
1               5


<210>  34
<211>  9
<212>  PRT
<213>  Alternaria alternata

<400>  34

Phe Val Ser Thr Gly Thr Leu Gly Gly
1               5


<210>  35
<211>  9
<212>  PRT
<213>  Alternaria alternata

<400>  35

Val Val Ile Val Thr Gly Ala Ser Gly
1               5


<210>  36
<211>  9
<212>  PRT
<213>  Alternaria alternata

<400>  36

Leu Val Ile Thr Ser Ser Met Ser Gly
1               5


<210>  37
<211>  9
<212>  PRT
<213>  Artificial sequence

<220>
<223>  Synthetic sequence: Peptide derivative

<400>  37

Leu Val Ile Thr Ser Ser Leu Ser Gly
1               5


<210>  38
<211>  9
<212>  PRT
<213>  Alternaria alternata

<400>  38

Ile Val Leu Leu Ser Leu Cys Gln Leu
1               5


<210>  39
<211>  9
<212>  PRT
<213>  Artificial sequence

<220>
<223>  Synthetic sequence: Peptide derivative

<400>  39

Ile Val Leu Leu Ser Leu Val Gln Leu
1               5


<210>  40
<211>  9
<212>  PRT
<213>  Alternaria alternata

<400>  40

Phe Arg Asp Asp Arg Ile Glu Ile Ile
1               5


<210>  41
<211>  9
<212>  PRT
<213>  Alternaria alternata

<400>  41

Val Ala Met Asn Pro Val Asn Thr Val
1               5


<210>  42
<211>  9
<212>  PRT
<213>  Artificial sequence

<220>
<223>  Synthetic sequence: Peptide derivative

<400>  42

Val Ala Leu Asn Pro Val Asn Thr Val

1               5


<210>  43
<211>  9
<212>  PRT
<213>  Alternaria alternata

<400>  43

Leu Val Thr Ile Ala Lys Val Asp Ala
1               5


<210>  44
<211>  9
<212>  PRT
<213>  Alternaria alternata

<400>  44

Met Lys His Leu Ala Ala Tyr Leu Leu
1               5


<210>  45
<211>  9
<212>  PRT
<213>  Artificial sequence

<220>
<223>  Synthetic sequence: Peptide derivative

<400>  45

Leu Lys His Leu Ala Ala Tyr Leu Leu
1               5


<210>  46
<211>  9
<212>  PRT
<213>  Alternaria alternata

<400>  46

Tyr Gln Lys Leu Lys Ala Leu Ala Lys
1               5


<210>  47
<211>  9
<212>  PRT
<213>  Alternaria alternata

<400>  47

Leu Lys Ser Cys Asn Ala Leu Leu Leu
1               5


<210>  48
<211>  9

<212>  PRT
<213>  Artificial sequence

<220>
<223>  Synthetic sequence: Peptide derivative

<400>  48

Leu Lys Ser Val Asn Ala Leu Leu Leu
1               5


<210>  49
<211>  9
<212>  PRT
<213>  Alternaria alternata

<400>  49

Trp Gly Val Met Val Ser His Arg Ser
1               5


<210>  50
<211>  9
<212>  PRT
<213>  Artificial sequence

<220>
<223>  Synthetic sequence: Peptide derivative

<400>  50

Trp Gly Val Leu Val Ser His Arg Ser
1               5


<210>  51
<211>  9
<212>  PRT
<213>  Alternaria alternata

<400>  51

Gly Tyr Lys Thr Ala Phe Ser Met Leu
1               5


<210>  52
<211>  9
<212>  PRT
<213>  Artificial sequence

<220>
<223>  Synthetic sequence: Peptide derivative

<400>  52

Gly Tyr Lys Thr Ala Phe Ser Leu Leu
1               5


<210>  53

<211>  9
<212>  PRT
<213>  Alternaria alternata

<400>  53

Phe Val Pro Gln Asp Ile Gln Lys Leu
1               5


<210>  54
<211>  9
<212>  PRT
<213>  Alternaria alternata

<400>  54

Tyr Val Tyr Phe Ala Ser Asp Ala Ser
1               5


<210>  55
<211>  9
<212>  PRT
<213>  Alternaria alternata

<400>  55

Tyr Phe Ile Glu Pro Thr Ile Phe Ser
1               5


<210>  56
<211>  9
<212>  PRT
<213>  Alternaria alternata

<400>  56

Tyr Ile Gln Thr Lys Thr Val Ser Ile
1               5


<210>  57
<211>  9
<212>  PRT
<213>  Alternaria alternata

<400>  57

Ile Arg Leu Gly Asp Val Leu Phe Gly
1               5


<210>  58
<211>  9
<212>  PRT
<213>  Alternaria alternata

<400>  58

Trp Val Asn Ser Tyr Asn Thr Leu His
1               5

<210>  59
<211>  11
<212>  PRT
<213>  Artificial sequence

<220>
<223>  Synthetic sequence: Peptide derivative

<400>  59

Leu Gln Phe Thr Thr Ile Ala Ser Leu Phe Ala
1               5                   10


<210>  60
<211>  10
<212>  PRT
<213>  Alternaria alternata

<400>  60

Gln Phe Thr Thr Ile Ala Ser Leu Phe Ala
1               5                   10


<210>  61
<211>  13
<212>  PRT
<213>  Alternaria alternata

<400>  61

Phe Thr Thr Ile Ala Ser Leu Phe Ala Ala Ala Gly Leu
1               5                   10


<210>  62
<211>  12
<212>  PRT
<213>  Alternaria alternata

<400>  62

Phe Thr Thr Ile Ala Ser Leu Phe Ala Ala Ala Gly
1               5                   10


<210>  63
<211>  11
<212>  PRT
<213>  Alternaria alternata

<400>  63

Phe Thr Thr Ile Ala Ser Leu Phe Ala Ala Ala
1               5                   10


<210>  64
<211>  10
<212>  PRT

<213>  Alternaria alternata

<400>  64

Phe Thr Thr Ile Ala Ser Leu Phe Ala Ala
1               5                   10


<210>  65
<211>  15
<212>  PRT
<213>  Artificial sequence

<220>
<223>  Synthetic sequence: Peptide derivative

<400>  65

Leu Gln Phe Thr Thr Ile Ala Ser Leu Phe Ala Ala Ala Gly Leu
1               5                   10                  15


<210>  66
<211>  14
<212>  PRT
<213>  Alternaria alternata

<400>  66

Gln Phe Thr Thr Ile Ala Ser Leu Phe Ala Ala Ala Gly Leu
1               5                   10


<210>  67
<211>  13
<212>  PRT
<213>  Alternaria alternata

<400>  67

Gln Phe Thr Thr Ile Ala Ser Leu Phe Ala Ala Ala Gly
1               5                   10


<210>  68
<211>  12
<212>  PRT
<213>  Alternaria alternata

<400>  68

Gln Phe Thr Thr Ile Ala Ser Leu Phe Ala Ala Ala
1               5                   10


<210>  69
<211>  11
<212>  PRT
<213>  Alternaria alternata

<400>  69

Gln Phe Thr Thr Ile Ala Ser Leu Phe Ala Ala

1               5                   10


<210>  70
<211>  14
<212>  PRT
<213>  Artificial sequence

<220>
<223>  Synthetic sequence: Peptide derivative

<400>  70

Leu Gln Phe Thr Thr Ile Ala Ser Leu Phe Ala Ala Ala Gly
1               5                   10


<210>  71
<211>  13
<212>  PRT
<213>  Alternaria alternata

<400>  71

Gln Phe Thr Thr Ile Ala Ser Leu Phe Ala Ala Ala Gly
1               5                   10


<210>  72
<211>  13
<212>  PRT
<213>  Artificial sequence

<220>
<223>  Synthetic sequence: Peptide derivative

<400>  72

Leu Gln Phe Thr Thr Ile Ala Ser Leu Phe Ala Ala Ala
1               5                   10


<210>  73
<211>  12
<212>  PRT
<213>  Alternaria alternata

<400>  73

Gln Phe Thr Thr Ile Ala Ser Leu Phe Ala Ala Ala
1               5                   10


<210>  74
<211>  12
<212>  PRT
<213>  Artificial sequence

<220>
<223>  Synthetic sequence: Peptide derivative

<400>  74

Leu Gln Phe Thr Thr Ile Ala Ser Leu Phe Ala Ala
1               5                   10

<210>  75
<211>  11
<212>  PRT
<213>  Alternaria alternata

<400>  75

Gln Phe Thr Thr Ile Ala Ser Leu Phe Ala Ala
1               5                   10

<210>  76
<211>  11
<212>  PRT
<213>  Alternaria alternata

<400>  76

Met Gln Phe Thr Thr Ile Ala Ser Leu Phe Ala
1               5                   10

<210>  77
<211>  15
<212>  PRT
<213>  Alternaria alternata

<400>  77

Met Gln Phe Thr Thr Ile Ala Ser Leu Phe Ala Ala Ala Gly Leu
1               5                   10                  15

<210>  78
<211>  14
<212>  PRT
<213>  Alternaria alternata

<400>  78

Met Gln Phe Thr Thr Ile Ala Ser Leu Phe Ala Ala Ala Gly
1               5                   10

<210>  79
<211>  13
<212>  PRT
<213>  Alternaria alternata

<400>  79

Met Gln Phe Thr Thr Ile Ala Ser Leu Phe Ala Ala Ala
1               5                   10

<210>  80
<211>  12
<212>  PRT
<213>  Alternaria alternata

<400>  80

Met Gln Phe Thr Thr Ile Ala Ser Leu Phe Ala Ala
1               5                   10


<210>  81
<211>  15
<212>  PRT
<213>  Alternaria alternata

<400>  81

Met Gln Phe Thr Thr Ile Ala Ser Leu Phe Ala Ala Ala Gly Leu
1               5                   10                  15


<210>  82
<211>  14
<212>  PRT
<213>  Alternaria alternata

<400>  82

Met Gln Phe Thr Thr Ile Ala Ser Leu Phe Ala Ala Ala Gly
1               5                   10


<210>  83
<211>  13
<212>  PRT
<213>  Alternaria alternata

<400>  83

Met Gln Phe Thr Thr Ile Ala Ser Leu Phe Ala Ala Ala
1               5                   10


<210>  84
<211>  12
<212>  PRT
<213>  Alternaria alternata

<400>  84

Met Gln Phe Thr Thr Ile Ala Ser Leu Phe Ala Ala
1               5                   10


<210>  85
<211>  11
<212>  PRT
<213>  Alternaria alternata

<400>  85

Met Gln Phe Thr Thr Ile Ala Ser Leu Phe Ala
1               5                   10


<210>  86

<211>  10
<212>  PRT
<213>  Alternaria alternata

<400>  86

Met Gln Phe Thr Thr Ile Ala Ser Leu Phe
1               5                   10


<210>  87
<211>  15
<212>  PRT
<213>  Artificial sequence

<220>
<223>  Synthetic sequence: Peptide derivative

<400>  87

Leu Gln Phe Thr Thr Ile Ala Ser Leu Phe Ala Ala Ala Gly Leu
1               5                   10                  15


<210>  88
<211>  14
<212>  PRT
<213>  Artificial sequence

<220>
<223>  Synthetic sequence: Peptide derivative

<400>  88

Leu Gln Phe Thr Thr Ile Ala Ser Leu Phe Ala Ala Ala Gly
1               5                   10


<210>  89
<211>  13
<212>  PRT
<213>  Artificial sequence

<220>
<223>  Synthetic sequence: Peptide derivative

<400>  89

Leu Gln Phe Thr Thr Ile Ala Ser Leu Phe Ala Ala Ala
1               5                   10


<210>  90
<211>  12
<212>  PRT
<213>  Artificial sequence

<220>
<223>  Synthetic sequence: Peptide derivative

<400>  90

Leu Gln Phe Thr Thr Ile Ala Ser Leu Phe Ala Ala

1               5                   10


<210>  91
<211>  11
<212>  PRT
<213>  Artificial sequence

<220>
<223>  Synthetic sequence: Peptide derivative

<400>  91

Leu Gln Phe Thr Thr Ile Ala Ser Leu Phe Ala
1               5                   10


<210>  92
<211>  10
<212>  PRT
<213>  Artificial sequence

<220>
<223>  Synthetic sequence: Peptide derivative

<400>  92

Leu Gln Phe Thr Thr Ile Ala Ser Leu Phe
1               5                   10


<210>  93
<211>  15
<212>  PRT
<213>  Artificial sequence

<220>
<223>  Synthetic sequence: Peptide derivative

<400>  93

Leu Gln Phe Thr Thr Ile Ala Ser Leu Phe Ala Ala Ala Gly Leu
1               5                   10                  15


<210>  94
<211>  14
<212>  PRT
<213>  Artificial sequence

<220>
<223>  Synthetic sequence: Peptide derivative

<400>  94

Leu Gln Phe Thr Thr Ile Ala Ser Leu Phe Ala Ala Ala Gly
1               5                   10


<210>  95
<211>  13
<212>  PRT
<213>  Alternaria alternata

<400>  95

Gln Phe Thr Thr Ile Ala Ser Leu Phe Ala Ala Ala Gly
1               5                   10


<210>  96
<211>  12
<212>  PRT
<213>  Alternaria alternata

<400>  96

Phe Thr Thr Ile Ala Ser Leu Phe Ala Ala Ala Gly
1               5                   10


<210>  97
<211>  11
<212>  PRT
<213>  Alternaria alternata

<400>  97

Thr Thr Ile Ala Ser Leu Phe Ala Ala Ala Gly
1               5                   10


<210>  98
<211>  10
<212>  PRT
<213>  Alternaria alternata

<400>  98

Thr Ile Ala Ser Leu Phe Ala Ala Ala Gly
1               5                   10


<210>  99
<211>  14
<212>  PRT
<213>  Alternaria alternata

<400>  99

Gln Phe Thr Thr Ile Ala Ser Leu Phe Ala Ala Ala Gly Leu
1               5                   10


<210>  100
<211>  13
<212>  PRT
<213>  Alternaria alternata

<400>  100

Phe Thr Thr Ile Ala Ser Leu Phe Ala Ala Ala Gly Leu
1               5                   10


<210>  101

<211>  12
<212>  PRT
<213>  Alternaria alternata

<400>  101

Thr Thr Ile Ala Ser Leu Phe Ala Ala Ala Gly Leu
1               5                   10


<210>  102
<211>  11
<212>  PRT
<213>  Alternaria alternata

<400>  102

Thr Ile Ala Ser Leu Phe Ala Ala Ala Gly Leu
1               5                   10


<210>  103
<211>  10
<212>  PRT
<213>  Alternaria alternata

<400>  103

Ile Ala Ser Leu Phe Ala Ala Ala Gly Leu
1               5                   10


<210>  104
<211>  15
<212>  PRT
<213>  Alternaria alternata

<400>  104

Met Gln Phe Thr Thr Ile Ala Ser Leu Phe Ala Ala Ala Gly Leu
1               5                   10                  15


<210>  105
<211>  14
<212>  PRT
<213>  Alternaria alternata

<400>  105

Met Gln Phe Thr Thr Ile Ala Ser Leu Phe Ala Ala Ala Gly
1               5                   10


<210>  106
<211>  12
<212>  PRT
<213>  Alternaria alternata

<400>  106

Ala Ala Gly Leu Ala Ala Ala Ala Pro Leu Glu Ser
1               5                   10

<210>  107
<211>  11
<212>  PRT
<213>  Alternaria alternata

<400>  107

Ala Gly Leu Ala Ala Ala Ala Pro Leu Glu Ser
1               5                   10


<210>  108
<211>  10
<212>  PRT
<213>  Alternaria alternata

<400>  108

Gly Leu Ala Ala Ala Ala Pro Leu Glu Ser
1               5                   10


<210>  109
<211>  12
<212>  PRT
<213>  Alternaria alternata

<400>  109

Leu Ala Ala Ala Ala Pro Leu Glu Ser Arg Gln Asp
1               5                   10


<210>  110
<211>  11
<212>  PRT
<213>  Alternaria alternata

<400>  110

Leu Ala Ala Ala Ala Pro Leu Glu Ser Arg Gln
1               5                   10


<210>  111
<211>  10
<212>  PRT
<213>  Alternaria alternata

<400>  111

Leu Ala Ala Ala Ala Pro Leu Glu Ser Arg
1               5                   10


<210>  112
<211>  15
<212>  PRT
<213>  Alternaria alternata

<400>  112

Ala Ala Gly Leu Ala Ala Ala Ala Pro Leu Glu Ser Arg Gln Asp
1               5                   10                  15


<210>  113
<211>  14
<212>  PRT
<213>  Alternaria alternata

<400>  113

Ala Gly Leu Ala Ala Ala Ala Pro Leu Glu Ser Arg Gln Asp
1               5                   10


<210>  114
<211>  13
<212>  PRT
<213>  Alternaria alternata

<400>  114

Gly Leu Ala Ala Ala Ala Pro Leu Glu Ser Arg Gln Asp
1               5                   10


<210>  115
<211>  14
<212>  PRT
<213>  Alternaria alternata

<400>  115

Ala Ala Gly Leu Ala Ala Ala Ala Pro Leu Glu Ser Arg Gln
1               5                   10


<210>  116
<211>  13
<212>  PRT
<213>  Alternaria alternata

<400>  116

Ala Gly Leu Ala Ala Ala Ala Pro Leu Glu Ser Arg Gln
1               5                   10


<210>  117
<211>  12
<212>  PRT
<213>  Alternaria alternata

<400>  117

Gly Leu Ala Ala Ala Ala Pro Leu Glu Ser Arg Gln
1               5                   10


<210>  118
<211>  13
<212>  PRT

<213>  Alternaria alternata

<400>  118

Ala Ala Gly Leu Ala Ala Ala Ala Pro Leu Glu Ser Arg
1               5                   10


<210>  119
<211>  12
<212>  PRT
<213>  Alternaria alternata

<400>  119

Ala Gly Leu Ala Ala Ala Ala Pro Leu Glu Ser Arg
1               5                   10


<210>  120
<211>  11
<212>  PRT
<213>  Alternaria alternata

<400>  120

Gly Leu Ala Ala Ala Ala Pro Leu Glu Ser Arg
1               5                   10


<210>  121
<211>  12
<212>  PRT
<213>  Alternaria alternata

<400>  121

Asp Tyr Val Trp Lys Ile Ser Glu Phe Tyr Gly Arg
1               5                   10


<210>  122
<211>  11
<212>  PRT
<213>  Alternaria alternata

<400>  122

Tyr Val Trp Lys Ile Ser Glu Phe Tyr Gly Arg
1               5                   10


<210>  123
<211>  10
<212>  PRT
<213>  Alternaria alternata

<400>  123

Val Trp Lys Ile Ser Glu Phe Tyr Gly Arg
1               5                   10

<210>  124
<211>  12
<212>  PRT
<213>  Alternaria alternata

<400>  124

Trp Lys Ile Ser Glu Phe Tyr Gly Arg Lys Pro Glu
1               5                   10


<210>  125
<211>  11
<212>  PRT
<213>  Alternaria alternata

<400>  125

Trp Lys Ile Ser Glu Phe Tyr Gly Arg Lys Pro
1               5                   10


<210>  126
<211>  10
<212>  PRT
<213>  Alternaria alternata

<400>  126

Trp Lys Ile Ser Glu Phe Tyr Gly Arg Lys
1               5                   10


<210>  127
<211>  15
<212>  PRT
<213>  Alternaria alternata

<400>  127

Asp Tyr Val Trp Lys Ile Ser Glu Phe Tyr Gly Arg Lys Pro Glu
1               5                   10                  15


<210>  128
<211>  14
<212>  PRT
<213>  Alternaria alternata

<400>  128

Asp Tyr Val Trp Lys Ile Ser Glu Phe Tyr Gly Arg Lys Pro
1               5                   10


<210>  129
<211>  13
<212>  PRT
<213>  Alternaria alternata

<400>  129

Asp Tyr Val Trp Lys Ile Ser Glu Phe Tyr Gly Arg Lys

1               5                   10


<210>  130
<211>  14
<212>  PRT
<213>  Alternaria alternata

<400>  130

Tyr Val Trp Lys Ile Ser Glu Phe Tyr Gly Arg Lys Pro Glu
1               5                   10


<210>  131
<211>  13
<212>  PRT
<213>  Alternaria alternata

<400>  131

Tyr Val Trp Lys Ile Ser Glu Phe Tyr Gly Arg Lys Pro
1               5                   10


<210>  132
<211>  12
<212>  PRT
<213>  Alternaria alternata

<400>  132

Tyr Val Trp Lys Ile Ser Glu Phe Tyr Gly Arg Lys
1               5                   10


<210>  133
<211>  13
<212>  PRT
<213>  Alternaria alternata

<400>  133

Val Trp Lys Ile Ser Glu Phe Tyr Gly Arg Lys Pro Glu
1               5                   10


<210>  134
<211>  12
<212>  PRT
<213>  Alternaria alternata

<400>  134

Val Trp Lys Ile Ser Glu Phe Tyr Gly Arg Lys Pro
1               5                   10


<210>  135
<211>  11
<212>  PRT
<213>  Alternaria alternata

<400>  135

Val Trp Lys Ile Ser Glu Phe Tyr Gly Arg Lys
1               5                   10


<210>  136
<211>  12
<212>  PRT
<213>  Alternaria alternata

<400>  136

Glu Gly Thr Tyr Tyr Asn Ser Leu Gly Phe Asn Ile
1               5                   10


<210>  137
<211>  11
<212>  PRT
<213>  Alternaria alternata

<400>  137

Gly Thr Tyr Tyr Asn Ser Leu Gly Phe Asn Ile
1               5                   10


<210>  138
<211>  10
<212>  PRT
<213>  Alternaria alternata

<400>  138

Thr Tyr Tyr Asn Ser Leu Gly Phe Asn Ile
1               5                   10


<210>  139
<211>  12
<212>  PRT
<213>  Alternaria alternata

<400>  139

Tyr Tyr Asn Ser Leu Gly Phe Asn Ile Lys Ala Thr
1               5                   10


<210>  140
<211>  11
<212>  PRT
<213>  Alternaria alternata

<400>  140

Tyr Tyr Asn Ser Leu Gly Phe Asn Ile Lys Ala
1               5                   10


<210>  141
<211>  10

<212>  PRT
<213>  Alternaria alternata

<400>  141

Tyr Tyr Asn Ser Leu Gly Phe Asn Ile Lys
1               5                   10


<210>  142
<211>  15
<212>  PRT
<213>  Alternaria alternata

<400>  142

Glu Gly Thr Tyr Tyr Asn Ser Leu Gly Phe Asn Ile Lys Ala Thr
1               5                   10                  15


<210>  143
<211>  14
<212>  PRT
<213>  Alternaria alternata

<400>  143

Glu Gly Thr Tyr Tyr Asn Ser Leu Gly Phe Asn Ile Lys Ala
1               5                   10


<210>  144
<211>  13
<212>  PRT
<213>  Alternaria alternata

<400>  144

Glu Gly Thr Tyr Tyr Asn Ser Leu Gly Phe Asn Ile Lys
1               5                   10


<210>  145
<211>  14
<212>  PRT
<213>  Alternaria alternata

<400>  145

Gly Thr Tyr Tyr Asn Ser Leu Gly Phe Asn Ile Lys Ala Thr
1               5                   10


<210>  146
<211>  13
<212>  PRT
<213>  Alternaria alternata

<400>  146

Gly Thr Tyr Tyr Asn Ser Leu Gly Phe Asn Ile Lys Ala
1               5                   10

<210>  147
<211>  12
<212>  PRT
<213>  Alternaria alternata

<400>  147

Gly Thr Tyr Tyr Asn Ser Leu Gly Phe Asn Ile Lys
1               5                   10


<210>  148
<211>  13
<212>  PRT
<213>  Alternaria alternata

<400>  148

Thr Tyr Tyr Asn Ser Leu Gly Phe Asn Ile Lys Ala Thr
1               5                   10


<210>  149
<211>  12
<212>  PRT
<213>  Alternaria alternata

<400>  149

Thr Tyr Tyr Asn Ser Leu Gly Phe Asn Ile Lys Ala
1               5                   10


<210>  150
<211>  11
<212>  PRT
<213>  Alternaria alternata

<400>  150

Thr Tyr Tyr Asn Ser Leu Gly Phe Asn Ile Lys
1               5                   10


<210>  151
<211>  12
<212>  PRT
<213>  Alternaria alternata

<400>  151

Tyr Asn Ser Leu Gly Phe Asn Ile Lys Ala Thr Asn
1               5                   10


<210>  152
<211>  11
<212>  PRT
<213>  Alternaria alternata

<400>  152

Asn Ser Leu Gly Phe Asn Ile Lys Ala Thr Asn
1               5                   10

<210>  153
<211>  10
<212>  PRT
<213>  Alternaria alternata

<400>  153

Ser Leu Gly Phe Asn Ile Lys Ala Thr Asn
1               5                   10

<210>  154
<211>  12
<212>  PRT
<213>  Alternaria alternata

<400>  154

Leu Gly Phe Asn Ile Lys Ala Thr Asn Gly Gly Thr
1               5                   10

<210>  155
<211>  11
<212>  PRT
<213>  Alternaria alternata

<400>  155

Leu Gly Phe Asn Ile Lys Ala Thr Asn Gly Gly
1               5                   10

<210>  156
<211>  10
<212>  PRT
<213>  Alternaria alternata

<400>  156

Leu Gly Phe Asn Ile Lys Ala Thr Asn Gly
1               5                   10

<210>  157
<211>  15
<212>  PRT
<213>  Alternaria alternata

<400>  157

Tyr Asn Ser Leu Gly Phe Asn Ile Lys Ala Thr Asn Gly Gly Thr
1               5                   10                  15

<210>  158
<211>  14
<212>  PRT
<213>  Alternaria alternata

<400>  158

Tyr Asn Ser Leu Gly Phe Asn Ile Lys Ala Thr Asn Gly Gly
1               5                   10


<210>  159
<211>  13
<212>  PRT
<213>  Alternaria alternata

<400>  159

Tyr Asn Ser Leu Gly Phe Asn Ile Lys Ala Thr Asn Gly
1               5                   10


<210>  160
<211>  14
<212>  PRT
<213>  Alternaria alternata

<400>  160

Asn Ser Leu Gly Phe Asn Ile Lys Ala Thr Asn Gly Gly Thr
1               5                   10


<210>  161
<211>  13
<212>  PRT
<213>  Alternaria alternata

<400>  161

Asn Ser Leu Gly Phe Asn Ile Lys Ala Thr Asn Gly Gly
1               5                   10


<210>  162
<211>  12
<212>  PRT
<213>  Alternaria alternata

<400>  162

Asn Ser Leu Gly Phe Asn Ile Lys Ala Thr Asn Gly
1               5                   10


<210>  163
<211>  13
<212>  PRT
<213>  Alternaria alternata

<400>  163

Ser Leu Gly Phe Asn Ile Lys Ala Thr Asn Gly Gly Thr
1               5                   10


<210>  164

<211>  12
<212>  PRT
<213>  Alternaria alternata

<400>  164

Ser Leu Gly Phe Asn Ile Lys Ala Thr Asn Gly Gly
1               5                   10


<210>  165
<211>  11
<212>  PRT
<213>  Alternaria alternata

<400>  165

Ser Leu Gly Phe Asn Ile Lys Ala Thr Asn Gly
1               5                   10


<210>  166
<211>  12
<212>  PRT
<213>  Alternaria alternata

<400>  166

Ser Leu Gly Phe Asn Ile Lys Ala Thr Asn Gly Gly
1               5                   10


<210>  167
<211>  11
<212>  PRT
<213>  Alternaria alternata

<400>  167

Leu Gly Phe Asn Ile Lys Ala Thr Asn Gly Gly
1               5                   10


<210>  168
<211>  10
<212>  PRT
<213>  Alternaria alternata

<400>  168

Gly Phe Asn Ile Lys Ala Thr Asn Gly Gly
1               5                   10


<210>  169
<211>  12
<212>  PRT
<213>  Alternaria alternata

<400>  169

Phe Asn Ile Lys Ala Thr Asn Gly Gly Thr Leu Asp
1               5                   10

<210>  170
<211>  11
<212>  PRT
<213>  Alternaria alternata

<400>  170

Phe Asn Ile Lys Ala Thr Asn Gly Gly Thr Leu
1               5                   10


<210>  171
<211>  10
<212>  PRT
<213>  Alternaria alternata

<400>  171

Phe Asn Ile Lys Ala Thr Asn Gly Gly Thr
1               5                   10


<210>  172
<211>  15
<212>  PRT
<213>  Alternaria alternata

<400>  172

Ser Leu Gly Phe Asn Ile Lys Ala Thr Asn Gly Gly Thr Leu Asp
1               5                   10                  15


<210>  173
<211>  14
<212>  PRT
<213>  Alternaria alternata

<400>  173

Ser Leu Gly Phe Asn Ile Lys Ala Thr Asn Gly Gly Thr Leu
1               5                   10


<210>  174
<211>  13
<212>  PRT
<213>  Alternaria alternata

<400>  174

Ser Leu Gly Phe Asn Ile Lys Ala Thr Asn Gly Gly Thr
1               5                   10


<210>  175
<211>  14
<212>  PRT
<213>  Alternaria alternata

<400>  175

Leu Gly Phe Asn Ile Lys Ala Thr Asn Gly Gly Thr Leu Asp
1               5                   10

<210>  176
<211>  13
<212>  PRT
<213>  Alternaria alternata

<400>  176

Leu Gly Phe Asn Ile Lys Ala Thr Asn Gly Gly Thr Leu
1               5                   10

<210>  177
<211>  12
<212>  PRT
<213>  Alternaria alternata

<400>  177

Leu Gly Phe Asn Ile Lys Ala Thr Asn Gly Gly Thr
1               5                   10

<210>  178
<211>  13
<212>  PRT
<213>  Alternaria alternata

<400>  178

Gly Phe Asn Ile Lys Ala Thr Asn Gly Gly Thr Leu Asp
1               5                   10

<210>  179
<211>  12
<212>  PRT
<213>  Alternaria alternata

<400>  179

Gly Phe Asn Ile Lys Ala Thr Asn Gly Gly Thr Leu
1               5                   10

<210>  180
<211>  11
<212>  PRT
<213>  Alternaria alternata

<400>  180

Gly Phe Asn Ile Lys Ala Thr Asn Gly Gly Thr
1               5                   10

<210>  181
<211>  12
<212>  PRT

<213>  Alternaria alternata

<400>  181

Gly Phe Asn Ile Lys Ala Thr Asn Gly Gly Thr Leu
1               5                   10


<210>  182
<211>  11
<212>  PRT
<213>  Alternaria alternata

<400>  182

Phe Asn Ile Lys Ala Thr Asn Gly Gly Thr Leu
1               5                   10


<210>  183
<211>  10
<212>  PRT
<213>  Alternaria alternata

<400>  183

Asn Ile Lys Ala Thr Asn Gly Gly Thr Leu
1               5                   10


<210>  184
<211>  12
<212>  PRT
<213>  Alternaria alternata

<400>  184

Ile Lys Ala Thr Asn Gly Gly Thr Leu Asp Phe Thr
1               5                   10


<210>  185
<211>  11
<212>  PRT
<213>  Alternaria alternata

<400>  185

Ile Lys Ala Thr Asn Gly Gly Thr Leu Asp Phe
1               5                   10


<210>  186
<211>  10
<212>  PRT
<213>  Alternaria alternata

<400>  186

Ile Lys Ala Thr Asn Gly Gly Thr Leu Asp
1               5                   10

<210>  187
<211>  15
<212>  PRT
<213>  Alternaria alternata

<400>  187

Gly Phe Asn Ile Lys Ala Thr Asn Gly Gly Thr Leu Asp Phe Thr
1               5                   10                  15


<210>  188
<211>  14
<212>  PRT
<213>  Alternaria alternata

<400>  188

Gly Phe Asn Ile Lys Ala Thr Asn Gly Gly Thr Leu Asp Phe
1               5                   10


<210>  189
<211>  13
<212>  PRT
<213>  Alternaria alternata

<400>  189

Gly Phe Asn Ile Lys Ala Thr Asn Gly Gly Thr Leu Asp
1               5                   10


<210>  190
<211>  14
<212>  PRT
<213>  Alternaria alternata

<400>  190

Phe Asn Ile Lys Ala Thr Asn Gly Gly Thr Leu Asp Phe Thr
1               5                   10


<210>  191
<211>  13
<212>  PRT
<213>  Alternaria alternata

<400>  191

Phe Asn Ile Lys Ala Thr Asn Gly Gly Thr Leu Asp Phe
1               5                   10


<210>  192
<211>  12
<212>  PRT
<213>  Alternaria alternata

<400>  192

Phe Asn Ile Lys Ala Thr Asn Gly Gly Thr Leu Asp

1               5                   10


<210>  193
<211>  13
<212>  PRT
<213>  Alternaria alternata

<400>  193

Asn Ile Lys Ala Thr Asn Gly Gly Thr Leu Asp Phe Thr
1               5                   10


<210>  194
<211>  12
<212>  PRT
<213>  Alternaria alternata

<400>  194

Asn Ile Lys Ala Thr Asn Gly Gly Thr Leu Asp Phe
1               5                   10


<210>  195
<211>  11
<212>  PRT
<213>  Alternaria alternata

<400>  195

Asn Ile Lys Ala Thr Asn Gly Gly Thr Leu Asp
1               5                   10


<210>  196
<211>  12
<212>  PRT
<213>  Alternaria alternata

<400>  196

Ser Asp Asp Ile Thr Tyr Val Ala Thr Ala Thr Leu
1               5                   10


<210>  197
<211>  11
<212>  PRT
<213>  Alternaria alternata

<400>  197

Asp Asp Ile Thr Tyr Val Ala Thr Ala Thr Leu
1               5                   10


<210>  198
<211>  10
<212>  PRT
<213>  Alternaria alternata

<400>  198

Asp Ile Thr Tyr Val Ala Thr Ala Thr Leu
1               5                   10


<210>  199
<211>  12
<212>  PRT
<213>  Alternaria alternata

<400>  199

Ile Thr Tyr Val Ala Thr Ala Thr Leu Pro Asn Tyr
1               5                   10


<210>  200
<211>  11
<212>  PRT
<213>  Alternaria alternata

<400>  200

Ile Thr Tyr Val Ala Thr Ala Thr Leu Pro Asn
1               5                   10


<210>  201
<211>  10
<212>  PRT
<213>  Alternaria alternata

<400>  201

Ile Thr Tyr Val Ala Thr Ala Thr Leu Pro
1               5                   10


<210>  202
<211>  15
<212>  PRT
<213>  Alternaria alternata

<400>  202

Ser Asp Asp Ile Thr Tyr Val Ala Thr Ala Thr Leu Pro Asn Tyr
1               5                   10                  15


<210>  203
<211>  14
<212>  PRT
<213>  Alternaria alternata

<400>  203

Ser Asp Asp Ile Thr Tyr Val Ala Thr Ala Thr Leu Pro Asn
1               5                   10


<210>  204
<211>  13

<212>  PRT
<213>  Alternaria alternata

<400>  204

Ser Asp Asp Ile Thr Tyr Val Ala Thr Ala Thr Leu Pro
1               5                   10


<210>  205
<211>  14
<212>  PRT
<213>  Alternaria alternata

<400>  205

Asp Asp Ile Thr Tyr Val Ala Thr Ala Thr Leu Pro Asn Tyr
1               5                   10


<210>  206
<211>  13
<212>  PRT
<213>  Alternaria alternata

<400>  206

Asp Asp Ile Thr Tyr Val Ala Thr Ala Thr Leu Pro Asn
1               5                   10


<210>  207
<211>  12
<212>  PRT
<213>  Alternaria alternata

<400>  207

Asp Asp Ile Thr Tyr Val Ala Thr Ala Thr Leu Pro
1               5                   10


<210>  208
<211>  13
<212>  PRT
<213>  Alternaria alternata

<400>  208

Asp Ile Thr Tyr Val Ala Thr Ala Thr Leu Pro Asn Tyr
1               5                   10


<210>  209
<211>  12
<212>  PRT
<213>  Alternaria alternata

<400>  209

Asp Ile Thr Tyr Val Ala Thr Ala Thr Leu Pro Asn
1               5                   10

<210>  210
<211>  11
<212>  PRT
<213>  Alternaria alternata

<400>  210

Asp Ile Thr Tyr Val Ala Thr Ala Thr Leu Pro
1               5                   10


<210>  211
<211>  12
<212>  PRT
<213>  Alternaria alternata

<400>  211

Asp Ile Thr Tyr Val Ala Thr Ala Thr Leu Pro Asn
1               5                   10


<210>  212
<211>  11
<212>  PRT
<213>  Alternaria alternata

<400>  212

Ile Thr Tyr Val Ala Thr Ala Thr Leu Pro Asn
1               5                   10


<210>  213
<211>  10
<212>  PRT
<213>  Alternaria alternata

<400>  213

Thr Tyr Val Ala Thr Ala Thr Leu Pro Asn
1               5                   10


<210>  214
<211>  12
<212>  PRT
<213>  Artificial sequence

<220>
<223>  Synthetic sequence: Peptide derivative

<400>  214

Tyr Val Ala Thr Ala Thr Leu Pro Asn Tyr Val Arg
1               5                   10


<210>  215
<211>  11
<212>  PRT
<213>  Artificial sequence

<220>
<223>  Synthetic sequence: Peptide derivative

<400>  215

Tyr Val Ala Thr Ala Thr Leu Pro Asn Tyr Val
1               5                   10


<210>  216
<211>  10
<212>  PRT
<213>  Alternaria alternata

<400>  216

Tyr Val Ala Thr Ala Thr Leu Pro Asn Tyr
1               5                   10


<210>  217
<211>  15
<212>  PRT
<213>  Artificial sequence

<220>
<223>  Synthetic sequence: Peptide derivative

<400>  217

Asp Ile Thr Tyr Val Ala Thr Ala Thr Leu Pro Asn Tyr Val Arg
1               5                   10                  15


<210>  218
<211>  14
<212>  PRT
<213>  Artificial sequence

<220>
<223>  Synthetic sequence: Peptide derivative

<400>  218

Asp Ile Thr Tyr Val Ala Thr Ala Thr Leu Pro Asn Tyr Val
1               5                   10


<210>  219
<211>  13
<212>  PRT
<213>  Alternaria alternata

<400>  219

Asp Ile Thr Tyr Val Ala Thr Ala Thr Leu Pro Asn Tyr
1               5                   10


<210>  220
<211>  14
<212>  PRT

<213>  Artificial sequence

<220>
<223>  Synthetic sequence: Peptide derivative

<400>  220

Ile Thr Tyr Val Ala Thr Ala Thr Leu Pro Asn Tyr Val Arg
1               5                   10


<210>  221
<211>  13
<212>  PRT
<213>  Artificial sequence

<220>
<223>  Synthetic sequence: Peptide derivative

<400>  221

Ile Thr Tyr Val Ala Thr Ala Thr Leu Pro Asn Tyr Val
1               5                   10


<210>  222
<211>  12
<212>  PRT
<213>  Alternaria alternata

<400>  222

Ile Thr Tyr Val Ala Thr Ala Thr Leu Pro Asn Tyr
1               5                   10


<210>  223
<211>  13
<212>  PRT
<213>  Artificial sequence

<220>
<223>  Synthetic sequence: Peptide derivative

<400>  223

Thr Tyr Val Ala Thr Ala Thr Leu Pro Asn Tyr Val Arg
1               5                   10


<210>  224
<211>  12
<212>  PRT
<213>  Artificial sequence

<220>
<223>  Synthetic sequence: Peptide derivative

<400>  224

Thr Tyr Val Ala Thr Ala Thr Leu Pro Asn Tyr Val
1               5                   10

<210>  225
<211>  11
<212>  PRT
<213>  Alternaria alternata

<400>  225

Thr Tyr Val Ala Thr Ala Thr Leu Pro Asn Tyr
1               5                   10


<210>  226
<211>  12
<212>  PRT
<213>  Alternaria alternata

<400>  226

Tyr Val Ala Thr Ala Thr Leu Pro Asn Tyr Cys Arg
1               5                   10


<210>  227
<211>  11
<212>  PRT
<213>  Alternaria alternata

<400>  227

Tyr Val Ala Thr Ala Thr Leu Pro Asn Tyr Cys
1               5                   10


<210>  228
<211>  15
<212>  PRT
<213>  Alternaria alternata

<400>  228

Asp Ile Thr Tyr Val Ala Thr Ala Thr Leu Pro Asn Tyr Cys Arg
1               5                   10                  15


<210>  229
<211>  14
<212>  PRT
<213>  Alternaria alternata

<400>  229

Asp Ile Thr Tyr Val Ala Thr Ala Thr Leu Pro Asn Tyr Cys
1               5                   10


<210>  230
<211>  14
<212>  PRT
<213>  Alternaria alternata

<400>  230

Ile Thr Tyr Val Ala Thr Ala Thr Leu Pro Asn Tyr Cys Arg
1               5                   10

<210>  231
<211>  13
<212>  PRT
<213>  Alternaria alternata

<400>  231

Ile Thr Tyr Val Ala Thr Ala Thr Leu Pro Asn Tyr Cys
1               5                   10

<210>  232
<211>  13
<212>  PRT
<213>  Alternaria alternata

<400>  232

Thr Tyr Val Ala Thr Ala Thr Leu Pro Asn Tyr Cys Arg
1               5                   10

<210>  233
<211>  12
<212>  PRT
<213>  Alternaria alternata

<400>  233

Thr Tyr Val Ala Thr Ala Thr Leu Pro Asn Tyr Cys
1               5                   10

<210>  234
<211>  12
<212>  PRT
<213>  Alternaria alternata

<400>  234

Thr Ala Thr Leu Pro Asn Tyr Cys Arg Ala Gly Gly
1               5                   10

<210>  235
<211>  11
<212>  PRT
<213>  Alternaria alternata

<400>  235

Ala Thr Leu Pro Asn Tyr Cys Arg Ala Gly Gly
1               5                   10

<210>  236
<211>  10
<212>  PRT
<213>  Alternaria alternata

<400>  236

Thr Leu Pro Asn Tyr Cys Arg Ala Gly Gly
1               5                   10


<210>  237
<211>  12
<212>  PRT
<213>  Alternaria alternata

<400>  237

Leu Pro Asn Tyr Cys Arg Ala Gly Gly Asn Gly Pro
1               5                   10


<210>  238
<211>  11
<212>  PRT
<213>  Alternaria alternata

<400>  238

Leu Pro Asn Tyr Cys Arg Ala Gly Gly Asn Gly
1               5                   10


<210>  239
<211>  10
<212>  PRT
<213>  Alternaria alternata

<400>  239

Leu Pro Asn Tyr Cys Arg Ala Gly Gly Asn
1               5                   10


<210>  240
<211>  15
<212>  PRT
<213>  Alternaria alternata

<400>  240

Thr Ala Thr Leu Pro Asn Tyr Cys Arg Ala Gly Gly Asn Gly Pro
1               5                   10                  15


<210>  241
<211>  14
<212>  PRT
<213>  Alternaria alternata

<400>  241

Thr Ala Thr Leu Pro Asn Tyr Cys Arg Ala Gly Gly Asn Gly
1               5                   10


<210>  242

<211>  13
<212>  PRT
<213>  Alternaria alternata

<400>  242

Thr Ala Thr Leu Pro Asn Tyr Cys Arg Ala Gly Gly Asn
1               5                   10


<210>  243
<211>  14
<212>  PRT
<213>  Alternaria alternata

<400>  243

Ala Thr Leu Pro Asn Tyr Cys Arg Ala Gly Gly Asn Gly Pro
1               5                   10


<210>  244
<211>  13
<212>  PRT
<213>  Alternaria alternata

<400>  244

Ala Thr Leu Pro Asn Tyr Cys Arg Ala Gly Gly Asn Gly
1               5                   10


<210>  245
<211>  12
<212>  PRT
<213>  Alternaria alternata

<400>  245

Ala Thr Leu Pro Asn Tyr Cys Arg Ala Gly Gly Asn
1               5                   10


<210>  246
<211>  13
<212>  PRT
<213>  Alternaria alternata

<400>  246

Thr Leu Pro Asn Tyr Cys Arg Ala Gly Gly Asn Gly Pro
1               5                   10


<210>  247
<211>  12
<212>  PRT
<213>  Alternaria alternata

<400>  247

Thr Leu Pro Asn Tyr Cys Arg Ala Gly Gly Asn Gly
1               5                   10

<210>  248
<211>  11
<212>  PRT
<213>  Alternaria alternata

<400>  248

Thr Leu Pro Asn Tyr Cys Arg Ala Gly Gly Asn
1               5                   10


<210>  249
<211>  12
<212>  PRT
<213>  Artificial sequence

<220>
<223>  Synthetic sequence: Peptide derivative

<400>  249

Thr Ala Thr Leu Pro Asn Tyr Val Arg Ala Gly Gly
1               5                   10


<210>  250
<211>  11
<212>  PRT
<213>  Artificial sequence

<220>
<223>  Synthetic sequence: Peptide derivative

<400>  250

Ala Thr Leu Pro Asn Tyr Val Arg Ala Gly Gly
1               5                   10


<210>  251
<211>  10
<212>  PRT
<213>  Artificial sequence

<220>
<223>  Synthetic sequence: Peptide derivative

<400>  251

Thr Leu Pro Asn Tyr Val Arg Ala Gly Gly
1               5                   10


<210>  252
<211>  12
<212>  PRT
<213>  Artificial sequence

<220>
<223>  Synthetic sequence: Peptide derivative

<400>  252

Leu Pro Asn Tyr Val Arg Ala Gly Gly Asn Gly Pro
1               5                   10


<210>  253
<211>  11
<212>  PRT
<213>  Artificial sequence

<220>
<223>  Synthetic sequence: Peptide derivative

<400>  253

Leu Pro Asn Tyr Val Arg Ala Gly Gly Asn Gly
1               5                   10


<210>  254
<211>  10
<212>  PRT
<213>  Artificial sequence

<220>
<223>  Synthetic sequence: Peptide derivative

<400>  254

Leu Pro Asn Tyr Val Arg Ala Gly Gly Asn
1               5                   10


<210>  255
<211>  15
<212>  PRT
<213>  Artificial sequence

<220>
<223>  Synthetic sequence: Peptide derivative

<400>  255

Thr Ala Thr Leu Pro Asn Tyr Val Arg Ala Gly Gly Asn Gly Pro
1               5                   10                  15


<210>  256
<211>  14
<212>  PRT
<213>  Artificial sequence

<220>
<223>  Synthetic sequence: Peptide derivative

<400>  256

Thr Ala Thr Leu Pro Asn Tyr Val Arg Ala Gly Gly Asn Gly
1               5                   10


<210>  257

<211>  13
<212>  PRT
<213>  Artificial sequence

<220>
<223>  Synthetic sequence: Peptide derivative

<400>  257

Thr Ala Thr Leu Pro Asn Tyr Val Arg Ala Gly Gly Asn
1               5                   10


<210>  258
<211>  14
<212>  PRT
<213>  Artificial sequence

<220>
<223>  Synthetic sequence: Peptide derivative

<400>  258

Ala Thr Leu Pro Asn Tyr Val Arg Ala Gly Gly Asn Gly Pro
1               5                   10


<210>  259
<211>  13
<212>  PRT
<213>  Artificial sequence

<220>
<223>  Synthetic sequence: Peptide derivative

<400>  259

Ala Thr Leu Pro Asn Tyr Val Arg Ala Gly Gly Asn Gly
1               5                   10


<210>  260
<211>  12
<212>  PRT
<213>  Artificial sequence

<220>
<223>  Synthetic sequence: Peptide derivative

<400>  260

Ala Thr Leu Pro Asn Tyr Val Arg Ala Gly Gly Asn
1               5                   10


<210>  261
<211>  13
<212>  PRT
<213>  Artificial sequence

<220>
<223>  Synthetic sequence: Peptide derivative

<400>  261

Thr Leu Pro Asn Tyr Val Arg Ala Gly Gly Asn Gly Pro
1               5                   10


<210>  262
<211>  12
<212>  PRT
<213>  Artificial sequence

<220>
<223>  Synthetic sequence: Peptide derivative

<400>  262

Thr Leu Pro Asn Tyr Val Arg Ala Gly Gly Asn Gly
1               5                   10


<210>  263
<211>  11
<212>  PRT
<213>  Artificial sequence

<220>
<223>  Synthetic sequence: Peptide derivative

<400>  263

Thr Leu Pro Asn Tyr Val Arg Ala Gly Gly Asn
1               5                   10


<210>  264
<211>  12
<212>  PRT
<213>  Alternaria alternata

<400>  264

Pro Lys Asp Phe Val Cys Gln Gly Val Ala Asp Ala
1               5                   10


<210>  265
<211>  11
<212>  PRT
<213>  Alternaria alternata

<400>  265

Lys Asp Phe Val Cys Gln Gly Val Ala Asp Ala
1               5                   10


<210>  266
<211>  10
<212>  PRT
<213>  Alternaria alternata

<400>  266

Asp Phe Val Cys Gln Gly Val Ala Asp Ala
1               5                   10

<210>  267
<211>  12
<212>  PRT
<213>  Alternaria alternata

<400>  267

Phe Val Cys Gln Gly Val Ala Asp Ala Tyr Ile Thr
1               5                   10

<210>  268
<211>  11
<212>  PRT
<213>  Alternaria alternata

<400>  268

Phe Val Cys Gln Gly Val Ala Asp Ala Tyr Ile
1               5                   10

<210>  269
<211>  10
<212>  PRT
<213>  Alternaria alternata

<400>  269

Phe Val Cys Gln Gly Val Ala Asp Ala Tyr
1               5                   10

<210>  270
<211>  15
<212>  PRT
<213>  Alternaria alternata

<400>  270

Pro Lys Asp Phe Val Cys Gln Gly Val Ala Asp Ala Tyr Ile Thr
1               5                   10                  15

<210>  271
<211>  14
<212>  PRT
<213>  Alternaria alternata

<400>  271

Pro Lys Asp Phe Val Cys Gln Gly Val Ala Asp Ala Tyr Ile
1               5                   10

<210>  272
<211>  13
<212>  PRT
<213>  Alternaria alternata

<400>  272

Pro Lys Asp Phe Val Cys Gln Gly Val Ala Asp Ala Tyr
1               5                   10


<210>  273
<211>  14
<212>  PRT
<213>  Alternaria alternata

<400>  273

Lys Asp Phe Val Cys Gln Gly Val Ala Asp Ala Tyr Ile Thr
1               5                   10


<210>  274
<211>  13
<212>  PRT
<213>  Alternaria alternata

<400>  274

Lys Asp Phe Val Cys Gln Gly Val Ala Asp Ala Tyr Ile
1               5                   10


<210>  275
<211>  12
<212>  PRT
<213>  Alternaria alternata

<400>  275

Lys Asp Phe Val Cys Gln Gly Val Ala Asp Ala Tyr
1               5                   10


<210>  276
<211>  13
<212>  PRT
<213>  Alternaria alternata

<400>  276

Asp Phe Val Cys Gln Gly Val Ala Asp Ala Tyr Ile Thr
1               5                   10


<210>  277
<211>  12
<212>  PRT
<213>  Alternaria alternata

<400>  277

Asp Phe Val Cys Gln Gly Val Ala Asp Ala Tyr Ile
1               5                   10


<210>  278

<211>  11
<212>  PRT
<213>  Alternaria alternata

<400>  278

Asp Phe Val Cys Gln Gly Val Ala Asp Ala Tyr
1               5                   10


<210>  279
<211>  12
<212>  PRT
<213>  Artificial sequence

<220>
<223>  Synthetic sequence: Peptide derivative

<400>  279

Pro Lys Asp Phe Val Val Gln Gly Val Ala Asp Ala
1               5                   10


<210>  280
<211>  11
<212>  PRT
<213>  Artificial sequence

<220>
<223>  Synthetic sequence: Peptide derivative

<400>  280

Lys Asp Phe Val Val Gln Gly Val Ala Asp Ala
1               5                   10


<210>  281
<211>  10
<212>  PRT
<213>  Artificial sequence

<220>
<223>  Synthetic sequence: Peptide derivative

<400>  281

Asp Phe Val Val Gln Gly Val Ala Asp Ala
1               5                   10


<210>  282
<211>  12
<212>  PRT
<213>  Artificial sequence

<220>
<223>  Synthetic sequence: Peptide derivative

<400>  282

Phe Val Val Gln Gly Val Ala Asp Ala Tyr Ile Thr

1                5                   10


<210>  283
<211>  11
<212>  PRT
<213>  Artificial sequence

<220>
<223>  Synthetic sequence: Peptide derivative

<400>  283

Phe Val Val Gln Gly Val Ala Asp Ala Tyr Ile
1               5                   10


<210>  284
<211>  10
<212>  PRT
<213>  Artificial sequence

<220>
<223>  Synthetic sequence: Peptide derivative

<400>  284

Phe Val Val Gln Gly Val Ala Asp Ala Tyr
1               5                   10


<210>  285
<211>  15
<212>  PRT
<213>  Artificial sequence

<220>
<223>  Synthetic sequence: Peptide derivative

<400>  285

Pro Lys Asp Phe Val Val Gln Gly Val Ala Asp Ala Tyr Ile Thr
1               5                   10                  15


<210>  286
<211>  14
<212>  PRT
<213>  Artificial sequence

<220>
<223>  Synthetic sequence: Peptide derivative

<400>  286

Pro Lys Asp Phe Val Val Gln Gly Val Ala Asp Ala Tyr Ile
1               5                   10


<210>  287
<211>  13
<212>  PRT
<213>  Artificial sequence

<220>
<223>  Synthetic sequence: Peptide derivative

<400>  287

Pro Lys Asp Phe Val Val Gln Gly Val Ala Asp Ala Tyr
1               5                   10


<210>  288
<211>  14
<212>  PRT
<213>  Artificial sequence

<220>
<223>  Synthetic sequence: Peptide derivative

<400>  288

Lys Asp Phe Val Val Gln Gly Val Ala Asp Ala Tyr Ile Thr
1               5                   10


<210>  289
<211>  13
<212>  PRT
<213>  Artificial sequence

<220>
<223>  Synthetic sequence: Peptide derivative

<400>  289

Lys Asp Phe Val Val Gln Gly Val Ala Asp Ala Tyr Ile
1               5                   10


<210>  290
<211>  12
<212>  PRT
<213>  Artificial sequence

<220>
<223>  Synthetic sequence: Peptide derivative

<400>  290

Lys Asp Phe Val Val Gln Gly Val Ala Asp Ala Tyr
1               5                   10


<210>  291
<211>  13
<212>  PRT
<213>  Artificial sequence

<220>
<223>  Synthetic sequence: Peptide derivative

<400>  291

Asp Phe Val Val Gln Gly Val Ala Asp Ala Tyr Ile Thr

1               5                   10


<210>  292
<211>  12
<212>  PRT
<213>  Artificial sequence

<220>
<223>  Synthetic sequence: Peptide derivative

<400>  292

Asp Phe Val Val Gln Gly Val Ala Asp Ala Tyr Ile
1               5                   10


<210>  293
<211>  11
<212>  PRT
<213>  Artificial sequence

<220>
<223>  Synthetic sequence: Peptide derivative

<400>  293

Asp Phe Val Val Gln Gly Val Ala Asp Ala Tyr
1               5                   10


<210>  294
<211>  13
<212>  PRT
<213>  Alternaria alternata

<400>  294

Val Ala Asp Ala Tyr Ile Thr Leu Val Thr Leu Pro Lys
1               5                   10


<210>  295
<211>  12
<212>  PRT
<213>  Alternaria alternata

<400>  295

Ala Asp Ala Tyr Ile Thr Leu Val Thr Leu Pro Lys
1               5                   10


<210>  296
<211>  11
<212>  PRT
<213>  Alternaria alternata

<400>  296

Asp Ala Tyr Ile Thr Leu Val Thr Leu Pro Lys
1               5                   10

<210>  297
<211>  10
<212>  PRT
<213>  Alternaria alternata

<400>  297

Ala Tyr Ile Thr Leu Val Thr Leu Pro Lys
1               5                   10


<210>  298
<211>  11
<212>  PRT
<213>  Alternaria alternata

<400>  298

Tyr Ile Thr Leu Val Thr Leu Pro Lys Ser Ser
1               5                   10


<210>  299
<211>  10
<212>  PRT
<213>  Alternaria alternata

<400>  299

Tyr Ile Thr Leu Val Thr Leu Pro Lys Ser
1               5                   10


<210>  300
<211>  15
<212>  PRT
<213>  Alternaria alternata

<400>  300

Val Ala Asp Ala Tyr Ile Thr Leu Val Thr Leu Pro Lys Ser Ser
1               5                   10                  15


<210>  301
<211>  14
<212>  PRT
<213>  Alternaria alternata

<400>  301

Val Ala Asp Ala Tyr Ile Thr Leu Val Thr Leu Pro Lys Ser
1               5                   10


<210>  302
<211>  14
<212>  PRT
<213>  Alternaria alternata

<400>  302

Ala Asp Ala Tyr Ile Thr Leu Val Thr Leu Pro Lys Ser Ser
1               5                   10

<210>  303
<211>  13
<212>  PRT
<213>  Alternaria alternata

<400>  303

Ala Asp Ala Tyr Ile Thr Leu Val Thr Leu Pro Lys Ser
1               5                   10

<210>  304
<211>  13
<212>  PRT
<213>  Alternaria alternata

<400>  304

Asp Ala Tyr Ile Thr Leu Val Thr Leu Pro Lys Ser Ser
1               5                   10

<210>  305
<211>  12
<212>  PRT
<213>  Alternaria alternata

<400>  305

Asp Ala Tyr Ile Thr Leu Val Thr Leu Pro Lys Ser
1               5                   10

<210>  306
<211>  12
<212>  PRT
<213>  Alternaria alternata

<400>  306

Ala Tyr Ile Thr Leu Val Thr Leu Pro Lys Ser Ser
1               5                   10

<210>  307
<211>  11
<212>  PRT
<213>  Alternaria alternata

<400>  307

Ala Tyr Ile Thr Leu Val Thr Leu Pro Lys Ser
1               5                   10

<210>  308
<211>  14
<212>  PRT
<213>  Alternaria alternata

<400>  308

Val Ala Asp Ala Tyr Ile Thr Leu Val Thr Leu Pro Lys Ser
1               5                   10


<210>  309
<211>  13
<212>  PRT
<213>  Alternaria alternata

<400>  309

Ala Asp Ala Tyr Ile Thr Leu Val Thr Leu Pro Lys Ser
1               5                   10


<210>  310
<211>  12
<212>  PRT
<213>  Alternaria alternata

<400>  310

Asp Ala Tyr Ile Thr Leu Val Thr Leu Pro Lys Ser
1               5                   10


<210>  311
<211>  11
<212>  PRT
<213>  Alternaria alternata

<400>  311

Ala Tyr Ile Thr Leu Val Thr Leu Pro Lys Ser
1               5                   10


<210>  312
<211>  10
<212>  PRT
<213>  Alternaria alternata

<400>  312

Tyr Ile Thr Leu Val Thr Leu Pro Lys Ser
1               5                   10


<210>  313
<211>  15
<212>  PRT
<213>  Alternaria alternata

<400>  313

Val Ala Asp Ala Tyr Ile Thr Leu Val Thr Leu Pro Lys Ser Ser
1               5                   10                  15


<210>  314

<211>  14
<212>  PRT
<213>  Alternaria alternata

<400>  314

Ala Asp Ala Tyr Ile Thr Leu Val Thr Leu Pro Lys Ser Ser
1               5                   10


<210>  315
<211>  13
<212>  PRT
<213>  Alternaria alternata

<400>  315

Asp Ala Tyr Ile Thr Leu Val Thr Leu Pro Lys Ser Ser
1               5                   10


<210>  316
<211>  12
<212>  PRT
<213>  Alternaria alternata

<400>  316

Ala Tyr Ile Thr Leu Val Thr Leu Pro Lys Ser Ser
1               5                   10


<210>  317
<211>  11
<212>  PRT
<213>  Alternaria alternata

<400>  317

Tyr Ile Thr Leu Val Thr Leu Pro Lys Ser Ser
1               5                   10


<210>  318
<211>  12
<212>  PRT
<213>  Alternaria alternata

<400>  318

Ile Ala Ser Leu Phe Ala Ala Ala Gly Leu Ala Ala
1               5                   10


<210>  319
<211>  11
<212>  PRT
<213>  Alternaria alternata

<400>  319

Ala Ser Leu Phe Ala Ala Ala Gly Leu Ala Ala
1               5                   10

<210>  320
<211>  10
<212>  PRT
<213>  Alternaria alternata

<400>  320

Ser Leu Phe Ala Ala Ala Gly Leu Ala Ala
1               5                   10


<210>  321
<211>  12
<212>  PRT
<213>  Alternaria alternata

<400>  321

Leu Phe Ala Ala Ala Gly Leu Ala Ala Ala Ala Pro
1               5                   10


<210>  322
<211>  11
<212>  PRT
<213>  Alternaria alternata

<400>  322

Leu Phe Ala Ala Ala Gly Leu Ala Ala Ala Ala
1               5                   10


<210>  323
<211>  10
<212>  PRT
<213>  Alternaria alternata

<400>  323

Leu Phe Ala Ala Ala Gly Leu Ala Ala Ala
1               5                   10


<210>  324
<211>  15
<212>  PRT
<213>  Alternaria alternata

<400>  324

Ile Ala Ser Leu Phe Ala Ala Ala Gly Leu Ala Ala Ala Ala Pro
1               5                   10                  15


<210>  325
<211>  14
<212>  PRT
<213>  Alternaria alternata

<400>  325

Ile Ala Ser Leu Phe Ala Ala Ala Gly Leu Ala Ala Ala Ala
1               5                   10


<210>  326
<211>  13
<212>  PRT
<213>  Alternaria alternata

<400>  326

Ile Ala Ser Leu Phe Ala Ala Ala Gly Leu Ala Ala Ala
1               5                   10


<210>  327
<211>  14
<212>  PRT
<213>  Alternaria alternata

<400>  327

Ala Ser Leu Phe Ala Ala Ala Gly Leu Ala Ala Ala Ala Pro
1               5                   10


<210>  328
<211>  13
<212>  PRT
<213>  Alternaria alternata

<400>  328

Ala Ser Leu Phe Ala Ala Ala Gly Leu Ala Ala Ala Ala
1               5                   10


<210>  329
<211>  12
<212>  PRT
<213>  Alternaria alternata

<400>  329

Ala Ser Leu Phe Ala Ala Ala Gly Leu Ala Ala Ala
1               5                   10


<210>  330
<211>  13
<212>  PRT
<213>  Alternaria alternata

<400>  330

Ser Leu Phe Ala Ala Ala Gly Leu Ala Ala Ala Ala Pro
1               5                   10


<210>  331
<211>  12
<212>  PRT

<213>  Alternaria alternata

<400>  331

Ser Leu Phe Ala Ala Ala Gly Leu Ala Ala Ala Ala
1               5                   10


<210>  332
<211>  11
<212>  PRT
<213>  Alternaria alternata

<400>  332

Ser Leu Phe Ala Ala Ala Gly Leu Ala Ala Ala
1               5                   10


<210>  333
<211>  12
<212>  PRT
<213>  Alternaria alternata

<400>  333

Ala Ser Leu Phe Ala Ala Ala Gly Leu Ala Ala Ala
1               5                   10


<210>  334
<211>  11
<212>  PRT
<213>  Alternaria alternata

<400>  334

Ser Leu Phe Ala Ala Ala Gly Leu Ala Ala Ala
1               5                   10


<210>  335
<211>  10
<212>  PRT
<213>  Alternaria alternata

<400>  335

Leu Phe Ala Ala Ala Gly Leu Ala Ala Ala
1               5                   10


<210>  336
<211>  12
<212>  PRT
<213>  Alternaria alternata

<400>  336

Phe Ala Ala Ala Gly Leu Ala Ala Ala Ala Pro Leu
1               5                   10

<210>  337
<211>  11
<212>  PRT
<213>  Alternaria alternata

<400>  337

Phe Ala Ala Ala Gly Leu Ala Ala Ala Ala Pro
1               5                   10


<210>  338
<211>  10
<212>  PRT
<213>  Alternaria alternata

<400>  338

Phe Ala Ala Ala Gly Leu Ala Ala Ala Ala
1               5                   10


<210>  339
<211>  15
<212>  PRT
<213>  Alternaria alternata

<400>  339

Ala Ser Leu Phe Ala Ala Ala Gly Leu Ala Ala Ala Ala Pro Leu
1               5                   10                  15


<210>  340
<211>  14
<212>  PRT
<213>  Alternaria alternata

<400>  340

Ala Ser Leu Phe Ala Ala Ala Gly Leu Ala Ala Ala Ala Pro
1               5                   10


<210>  341
<211>  13
<212>  PRT
<213>  Alternaria alternata

<400>  341

Ala Ser Leu Phe Ala Ala Ala Gly Leu Ala Ala Ala Ala
1               5                   10


<210>  342
<211>  14
<212>  PRT
<213>  Alternaria alternata

<400>  342

Ser Leu Phe Ala Ala Ala Gly Leu Ala Ala Ala Ala Pro Leu

1                   5                   10


<210>  343
<211>  13
<212>  PRT
<213>  Alternaria alternata

<400>  343

Ser Leu Phe Ala Ala Ala Gly Leu Ala Ala Ala Ala Pro
1               5                   10


<210>  344
<211>  12
<212>  PRT
<213>  Alternaria alternata

<400>  344

Ser Leu Phe Ala Ala Ala Gly Leu Ala Ala Ala Ala
1               5                   10


<210>  345
<211>  13
<212>  PRT
<213>  Alternaria alternata

<400>  345

Leu Phe Ala Ala Ala Gly Leu Ala Ala Ala Ala Pro Leu
1               5                   10


<210>  346
<211>  12
<212>  PRT
<213>  Alternaria alternata

<400>  346

Leu Phe Ala Ala Ala Gly Leu Ala Ala Ala Ala Pro
1               5                   10


<210>  347
<211>  11
<212>  PRT
<213>  Alternaria alternata

<400>  347

Leu Phe Ala Ala Ala Gly Leu Ala Ala Ala Ala
1               5                   10


<210>  348
<211>  12
<212>  PRT
<213>  Alternaria alternata

<400>  348

Gly Thr Tyr Tyr Asn Ser Leu Gly Phe Asn Ile Lys
1               5                   10


<210>  349
<211>  11
<212>  PRT
<213>  Alternaria alternata

<400>  349

Thr Tyr Tyr Asn Ser Leu Gly Phe Asn Ile Lys
1               5                   10


<210>  350
<211>  10
<212>  PRT
<213>  Alternaria alternata

<400>  350

Tyr Tyr Asn Ser Leu Gly Phe Asn Ile Lys
1               5                   10


<210>  351
<211>  12
<212>  PRT
<213>  Alternaria alternata

<400>  351

Tyr Asn Ser Leu Gly Phe Asn Ile Lys Ala Thr Asn
1               5                   10


<210>  352
<211>  11
<212>  PRT
<213>  Alternaria alternata

<400>  352

Tyr Asn Ser Leu Gly Phe Asn Ile Lys Ala Thr
1               5                   10


<210>  353
<211>  10
<212>  PRT
<213>  Alternaria alternata

<400>  353

Tyr Asn Ser Leu Gly Phe Asn Ile Lys Ala
1               5                   10


<210>  354
<211>  15

<212>  PRT
<213>  Alternaria alternata

<400>  354

Gly Thr Tyr Tyr Asn Ser Leu Gly Phe Asn Ile Lys Ala Thr Asn
1               5                   10                  15


<210>  355
<211>  14
<212>  PRT
<213>  Alternaria alternata

<400>  355

Gly Thr Tyr Tyr Asn Ser Leu Gly Phe Asn Ile Lys Ala Thr
1               5                   10


<210>  356
<211>  13
<212>  PRT
<213>  Alternaria alternata

<400>  356

Gly Thr Tyr Tyr Asn Ser Leu Gly Phe Asn Ile Lys Ala
1               5                   10


<210>  357
<211>  14
<212>  PRT
<213>  Alternaria alternata

<400>  357

Thr Tyr Tyr Asn Ser Leu Gly Phe Asn Ile Lys Ala Thr Asn
1               5                   10


<210>  358
<211>  13
<212>  PRT
<213>  Alternaria alternata

<400>  358

Thr Tyr Tyr Asn Ser Leu Gly Phe Asn Ile Lys Ala Thr
1               5                   10


<210>  359
<211>  12
<212>  PRT
<213>  Alternaria alternata

<400>  359

Thr Tyr Tyr Asn Ser Leu Gly Phe Asn Ile Lys Ala
1               5                   10

<210>  360
<211>  13
<212>  PRT
<213>  Alternaria alternata

<400>  360

Tyr Tyr Asn Ser Leu Gly Phe Asn Ile Lys Ala Thr Asn
1               5                   10


<210>  361
<211>  12
<212>  PRT
<213>  Alternaria alternata

<400>  361

Tyr Tyr Asn Ser Leu Gly Phe Asn Ile Lys Ala Thr
1               5                   10


<210>  362
<211>  11
<212>  PRT
<213>  Alternaria alternata

<400>  362

Tyr Tyr Asn Ser Leu Gly Phe Asn Ile Lys Ala
1               5                   10


<210>  363
<211>  12
<212>  PRT
<213>  Alternaria alternata

<400>  363

Leu Pro Asn Tyr Cys Arg Ala Gly Gly Asn Gly Pro
1               5                   10


<210>  364
<211>  11
<212>  PRT
<213>  Alternaria alternata

<400>  364

Pro Asn Tyr Cys Arg Ala Gly Gly Asn Gly Pro
1               5                   10


<210>  365
<211>  10
<212>  PRT
<213>  Alternaria alternata

<400>  365

Asn Tyr Cys Arg Ala Gly Gly Asn Gly Pro
1               5                   10

<210>  366
<211>  12
<212>  PRT
<213>  Alternaria alternata

<400>  366

Tyr Cys Arg Ala Gly Gly Asn Gly Pro Lys Asp Phe
1               5                   10

<210>  367
<211>  11
<212>  PRT
<213>  Alternaria alternata

<400>  367

Tyr Cys Arg Ala Gly Gly Asn Gly Pro Lys Asp
1               5                   10

<210>  368
<211>  10
<212>  PRT
<213>  Alternaria alternata

<400>  368

Tyr Cys Arg Ala Gly Gly Asn Gly Pro Lys
1               5                   10

<210>  369
<211>  15
<212>  PRT
<213>  Alternaria alternata

<400>  369

Leu Pro Asn Tyr Cys Arg Ala Gly Gly Asn Gly Pro Lys Asp Phe
1               5                   10                  15

<210>  370
<211>  14
<212>  PRT
<213>  Alternaria alternata

<400>  370

Leu Pro Asn Tyr Cys Arg Ala Gly Gly Asn Gly Pro Lys Asp
1               5                   10

<210>  371
<211>  13
<212>  PRT
<213>  Alternaria alternata

<400>  371

Leu Pro Asn Tyr Cys Arg Ala Gly Gly Asn Gly Pro Lys
1               5                   10


<210>  372
<211>  14
<212>  PRT
<213>  Alternaria alternata

<400>  372

Pro Asn Tyr Cys Arg Ala Gly Gly Asn Gly Pro Lys Asp Phe
1               5                   10


<210>  373
<211>  13
<212>  PRT
<213>  Alternaria alternata

<400>  373

Pro Asn Tyr Cys Arg Ala Gly Gly Asn Gly Pro Lys Asp
1               5                   10


<210>  374
<211>  12
<212>  PRT
<213>  Alternaria alternata

<400>  374

Pro Asn Tyr Cys Arg Ala Gly Gly Asn Gly Pro Lys
1               5                   10


<210>  375
<211>  13
<212>  PRT
<213>  Alternaria alternata

<400>  375

Asn Tyr Cys Arg Ala Gly Gly Asn Gly Pro Lys Asp Phe
1               5                   10


<210>  376
<211>  12
<212>  PRT
<213>  Alternaria alternata

<400>  376

Asn Tyr Cys Arg Ala Gly Gly Asn Gly Pro Lys Asp
1               5                   10


<210>  377

<211>  11
<212>  PRT
<213>  Alternaria alternata

<400>  377

Asn Tyr Cys Arg Ala Gly Gly Asn Gly Pro Lys
1               5                   10


<210>  378
<211>  12
<212>  PRT
<213>  Artificial sequence

<220>
<223>  Synthetic sequence: Peptide derivative

<400>  378

Leu Pro Asn Tyr Val Arg Ala Gly Gly Asn Gly Pro
1               5                   10


<210>  379
<211>  11
<212>  PRT
<213>  Artificial sequence

<220>
<223>  Synthetic sequence: Peptide derivative

<400>  379

Pro Asn Tyr Val Arg Ala Gly Gly Asn Gly Pro
1               5                   10


<210>  380
<211>  10
<212>  PRT
<213>  Artificial sequence

<220>
<223>  Synthetic sequence: Peptide derivative

<400>  380

Asn Tyr Val Arg Ala Gly Gly Asn Gly Pro
1               5                   10


<210>  381
<211>  12
<212>  PRT
<213>  Artificial sequence

<220>
<223>  Synthetic sequence: Peptide derivative

<400>  381

Tyr Val Arg Ala Gly Gly Asn Gly Pro Lys Asp Phe

1               5                   10


<210>  382
<211>  11
<212>  PRT
<213>  Artificial sequence

<220>
<223>  Synthetic sequence: Peptide derivative

<400>  382

Tyr Val Arg Ala Gly Gly Asn Gly Pro Lys Asp
1               5                   10


<210>  383
<211>  10
<212>  PRT
<213>  Artificial sequence

<220>
<223>  Synthetic sequence: Peptide derivative

<400>  383

Tyr Val Arg Ala Gly Gly Asn Gly Pro Lys
1               5                   10


<210>  384
<211>  15
<212>  PRT
<213>  Artificial sequence

<220>
<223>  Synthetic sequence: Peptide derivative

<400>  384

Leu Pro Asn Tyr Val Arg Ala Gly Gly Asn Gly Pro Lys Asp Phe
1               5                   10                  15


<210>  385
<211>  14
<212>  PRT
<213>  Artificial sequence

<220>
<223>  Synthetic sequence: Peptide derivative

<400>  385

Leu Pro Asn Tyr Val Arg Ala Gly Gly Asn Gly Pro Lys Asp
1               5                   10


<210>  386
<211>  13
<212>  PRT
<213>  Artificial sequence

<220>
<223>  Synthetic sequence: Peptide derivative

<400>  386

Leu Pro Asn Tyr Val Arg Ala Gly Gly Asn Gly Pro Lys
1               5                   10


<210>  387
<211>  14
<212>  PRT
<213>  Artificial sequence

<220>
<223>  Synthetic sequence: Peptide derivative

<400>  387

Pro Asn Tyr Val Arg Ala Gly Gly Asn Gly Pro Lys Asp Phe
1               5                   10


<210>  388
<211>  13
<212>  PRT
<213>  Artificial sequence

<220>
<223>  Synthetic sequence: Peptide derivative

<400>  388

Pro Asn Tyr Val Arg Ala Gly Gly Asn Gly Pro Lys Asp
1               5                   10


<210>  389
<211>  12
<212>  PRT
<213>  Artificial sequence

<220>
<223>  Synthetic sequence: Peptide derivative

<400>  389

Pro Asn Tyr Val Arg Ala Gly Gly Asn Gly Pro Lys
1               5                   10


<210>  390
<211>  13
<212>  PRT
<213>  Artificial sequence

<220>
<223>  Synthetic sequence: Peptide derivative

<400>  390

Asn Tyr Val Arg Ala Gly Gly Asn Gly Pro Lys Asp Phe

1                   5                   10


<210>  391
<211>  12
<212>  PRT
<213>  Artificial sequence

<220>
<223>  Synthetic sequence: Peptide derivative

<400>  391

Asn Tyr Val Arg Ala Gly Gly Asn Gly Pro Lys Asp
1               5                   10


<210>  392
<211>  11
<212>  PRT
<213>  Artificial sequence

<220>
<223>  Synthetic sequence: Peptide derivative

<400>  392

Asn Tyr Val Arg Ala Gly Gly Asn Gly Pro Lys
1               5                   10


<210>  393
<211>  12
<212>  PRT
<213>  Alternaria alternata

<400>  393

Gly Gly Thr Leu Asp Phe Thr Cys Ser Ala Gln Ala
1               5                   10


<210>  394
<211>  12
<212>  PRT
<213>  Alternaria alternata

<400>  394

Gly Thr Leu Asp Phe Thr Val Cys Ser Ala Gln Ala
1               5                   10


<210>  395
<211>  10
<212>  PRT
<213>  Alternaria alternata

<400>  395

Thr Leu Asp Phe Thr Cys Ser Ala Gln Ala
1               5                   10

<210>  396
<211>  12
<212>  PRT
<213>  Alternaria alternata

<400>  396

Leu Asp Phe Thr Cys Ser Ala Gln Ala Asp Lys Leu
1               5                   10


<210>  397
<211>  11
<212>  PRT
<213>  Alternaria alternata

<400>  397

Leu Asp Phe Thr Cys Ser Ala Gln Ala Asp Lys
1               5                   10


<210>  398
<211>  10
<212>  PRT
<213>  Alternaria alternata

<400>  398

Leu Asp Phe Thr Cys Ser Ala Gln Ala Asp
1               5                   10


<210>  399
<211>  15
<212>  PRT
<213>  Alternaria alternata

<400>  399

Gly Gly Thr Leu Asp Phe Thr Cys Ser Ala Gln Ala Asp Lys Leu
1               5                   10                  15


<210>  400
<211>  14
<212>  PRT
<213>  Alternaria alternata

<400>  400

Gly Gly Thr Leu Asp Phe Thr Cys Ser Ala Gln Ala Asp Lys
1               5                   10


<210>  401
<211>  13
<212>  PRT
<213>  Alternaria alternata

<400>  401

Gly Gly Thr Leu Asp Phe Thr Cys Ser Ala Gln Ala Asp
1               5                   10

<210>  402
<211>  14
<212>  PRT
<213>  Alternaria alternata

<400>  402

Gly Thr Leu Asp Phe Thr Cys Ser Ala Gln Ala Asp Lys Leu
1               5                   10

<210>  403
<211>  13
<212>  PRT
<213>  Alternaria alternata

<400>  403

Gly Thr Leu Asp Phe Thr Cys Ser Ala Gln Ala Asp Lys
1               5                   10

<210>  404
<211>  12
<212>  PRT
<213>  Alternaria alternata

<400>  404

Gly Thr Leu Asp Phe Thr Cys Ser Ala Gln Ala Asp
1               5                   10

<210>  405
<211>  13
<212>  PRT
<213>  Alternaria alternata

<400>  405

Thr Leu Asp Phe Thr Cys Ser Ala Gln Ala Asp Lys Leu
1               5                   10

<210>  406
<211>  12
<212>  PRT
<213>  Alternaria alternata

<400>  406

Thr Leu Asp Phe Thr Cys Ser Ala Gln Ala Asp Lys
1               5                   10

<210>  407
<211>  11
<212>  PRT
<213>  Alternaria alternata

<400>  407

Thr Leu Asp Phe Thr Cys Ser Ala Gln Ala Asp
1               5                   10


<210>  408
<211>  12
<212>  PRT
<213>  Artificial sequence

<220>
<223>  Synthetic sequence: Peptide derivative

<400>  408

Gly Gly Thr Leu Asp Phe Thr Val Ser Ala Gln Ala
1               5                   10


<210>  409
<211>  11
<212>  PRT
<213>  Artificial sequence

<220>
<223>  Synthetic sequence: Peptide derivative

<400>  409

Gly Thr Leu Asp Phe Thr Val Ser Ala Gln Ala
1               5                   10


<210>  410
<211>  10
<212>  PRT
<213>  Artificial sequence

<220>
<223>  Synthetic sequence: Peptide derivative

<400>  410

Thr Leu Asp Phe Thr Val Ser Ala Gln Ala
1               5                   10


<210>  411
<211>  12
<212>  PRT
<213>  Artificial sequence

<220>
<223>  Synthetic sequence: Peptide derivative

<400>  411

Leu Asp Phe Thr Val Ser Ala Gln Ala Asp Lys Leu
1               5                   10

<210>  412
<211>  11
<212>  PRT
<213>  Artificial sequence

<220>
<223>  Synthetic sequence: Peptide derivative

<400>  412

Leu Asp Phe Thr Val Ser Ala Gln Ala Asp Lys
1               5                   10


<210>  413
<211>  10
<212>  PRT
<213>  Artificial sequence

<220>
<223>  Synthetic sequence: Peptide derivative

<400>  413

Leu Asp Phe Thr Val Ser Ala Gln Ala Asp
1               5                   10


<210>  414
<211>  15
<212>  PRT
<213>  Artificial sequence

<220>
<223>  Synthetic sequence: Peptide derivative

<400>  414

Gly Gly Thr Leu Asp Phe Thr Val Ser Ala Gln Ala Asp Lys Leu
1               5                   10                  15


<210>  415
<211>  14
<212>  PRT
<213>  Artificial sequence

<220>
<223>  Synthetic sequence: Peptide derivative

<400>  415

Gly Gly Thr Leu Asp Phe Thr Val Ser Ala Gln Ala Asp Lys
1               5                   10


<210>  416
<211>  13
<212>  PRT
<213>  Artificial sequence

<220>
<223>  Synthetic sequence: Peptide derivative

<400>  416

Gly Gly Thr Leu Asp Phe Thr Val Ser Ala Gln Ala Asp
1               5                   10


<210>  417
<211>  14
<212>  PRT
<213>  Artificial sequence

<220>
<223>  Synthetic sequence: Peptide derivative

<400>  417

Gly Thr Leu Asp Phe Thr Val Ser Ala Gln Ala Asp Lys Leu
1               5                   10


<210>  418
<211>  13
<212>  PRT
<213>  Artificial sequence

<220>
<223>  Synthetic sequence: Peptide derivative

<400>  418

Gly Thr Leu Asp Phe Thr Val Ser Ala Gln Ala Asp Lys
1               5                   10


<210>  419
<211>  12
<212>  PRT
<213>  Artificial sequence

<220>
<223>  Synthetic sequence: Peptide derivative

<400>  419

Gly Thr Leu Asp Phe Thr Val Ser Ala Gln Ala Asp
1               5                   10


<210>  420
<211>  13
<212>  PRT
<213>  Artificial sequence

<220>
<223>  Synthetic sequence: Peptide derivative

<400>  420

Thr Leu Asp Phe Thr Val Ser Ala Gln Ala Asp Lys Leu
1               5                   10

<210>  421
<211>  12
<212>  PRT
<213>  Artificial sequence

<220>
<223>  Synthetic sequence: Peptide derivative

<400>  421

Thr Leu Asp Phe Thr Val Ser Ala Gln Ala Asp Lys
1               5                   10


<210>  422
<211>  11
<212>  PRT
<213>  Artificial sequence

<220>
<223>  Synthetic sequence: Peptide derivative

<400>  422

Thr Leu Asp Phe Thr Val Ser Ala Gln Ala Asp
1               5                   10


<210>  423
<211>  12
<212>  PRT
<213>  Alternaria alternata

<400>  423

Asp His Lys Trp Tyr Ser Cys Gly Glu Asn Ser Phe
1               5                   10


<210>  424
<211>  11
<212>  PRT
<213>  Alternaria alternata

<400>  424

His Lys Trp Tyr Ser Cys Gly Glu Asn Ser Phe
1               5                   10


<210>  425
<211>  10
<212>  PRT
<213>  Alternaria alternata

<400>  425

Lys Trp Tyr Ser Cys Gly Glu Asn Ser Phe
1               5                   10


<210>  426
<211>  12

<212>  PRT
<213>  Alternaria alternata

<400>  426

Trp Tyr Ser Cys Gly Glu Asn Ser Phe Leu Asp Phe
1               5                   10


<210>  427
<211>  11
<212>  PRT
<213>  Alternaria alternata

<400>  427

Trp Tyr Ser Cys Gly Glu Asn Ser Phe Leu Asp
1               5                   10


<210>  428
<211>  10
<212>  PRT
<213>  Alternaria alternata

<400>  428

Trp Tyr Ser Cys Gly Glu Asn Ser Phe Leu
1               5                   10


<210>  429
<211>  15
<212>  PRT
<213>  Alternaria alternata

<400>  429

Asp His Lys Trp Tyr Ser Cys Gly Glu Asn Ser Phe Leu Asp Phe
1               5                   10                  15


<210>  430
<211>  14
<212>  PRT
<213>  Alternaria alternata

<400>  430

Asp His Lys Trp Tyr Ser Cys Gly Glu Asn Ser Phe Leu Asp
1               5                   10


<210>  431
<211>  13
<212>  PRT
<213>  Alternaria alternata

<400>  431

Asp His Lys Trp Tyr Ser Cys Gly Glu Asn Ser Phe Leu
1               5                   10

<210>  432
<211>  14
<212>  PRT
<213>  Alternaria alternata

<400>  432

His Lys Trp Tyr Ser Cys Gly Glu Asn Ser Phe Leu Asp Phe
1               5                   10


<210>  433
<211>  13
<212>  PRT
<213>  Alternaria alternata

<400>  433

His Lys Trp Tyr Ser Cys Gly Glu Asn Ser Phe Leu Asp
1               5                   10


<210>  434
<211>  12
<212>  PRT
<213>  Alternaria alternata

<400>  434

His Lys Trp Tyr Ser Cys Gly Glu Asn Ser Phe Leu
1               5                   10


<210>  435
<211>  13
<212>  PRT
<213>  Alternaria alternata

<400>  435

Lys Trp Tyr Ser Cys Gly Glu Asn Ser Phe Leu Asp Phe
1               5                   10


<210>  436
<211>  12
<212>  PRT
<213>  Alternaria alternata

<400>  436

Lys Trp Tyr Ser Cys Gly Glu Asn Ser Phe Leu Asp
1               5                   10


<210>  437
<211>  11
<212>  PRT
<213>  Alternaria alternata

<400>  437

Lys Trp Tyr Ser Cys Gly Glu Asn Ser Phe Leu
1               5                   10

<210>  438
<211>  12
<212>  PRT
<213>  Artificial sequence

<220>
<223>  Synthetic sequence: Peptide derivative

<400>  438

Asp His Lys Trp Tyr Ser Val Gly Glu Asn Ser Phe
1               5                   10

<210>  439
<211>  11
<212>  PRT
<213>  Artificial sequence

<220>
<223>  Synthetic sequence: Peptide derivative

<400>  439

His Lys Trp Tyr Ser Val Gly Glu Asn Ser Phe
1               5                   10

<210>  440
<211>  10
<212>  PRT
<213>  Artificial sequence

<220>
<223>  Synthetic sequence: Peptide derivative

<400>  440

Lys Trp Tyr Ser Val Gly Glu Asn Ser Phe
1               5                   10

<210>  441
<211>  12
<212>  PRT
<213>  Artificial sequence

<220>
<223>  Synthetic sequence: Peptide derivative

<400>  441

Trp Tyr Ser Val Gly Glu Asn Ser Phe Leu Asp Phe
1               5                   10

<210>  442
<211>  11
<212>  PRT

<213>  Artificial sequence

<220>
<223>  Synthetic sequence: Peptide derivative

<400>  442

Trp Tyr Ser Val Gly Glu Asn Ser Phe Leu Asp
1               5                   10


<210>  443
<211>  10
<212>  PRT
<213>  Artificial sequence

<220>
<223>  Synthetic sequence: Peptide derivative

<400>  443

Trp Tyr Ser Val Gly Glu Asn Ser Phe Leu
1               5                   10


<210>  444
<211>  15
<212>  PRT
<213>  Artificial sequence

<220>
<223>  Synthetic sequence: Peptide derivative

<400>  444

Asp His Lys Trp Tyr Ser Val Gly Glu Asn Ser Phe Leu Asp Phe
1               5                   10                  15


<210>  445
<211>  14
<212>  PRT
<213>  Artificial sequence

<220>
<223>  Synthetic sequence: Peptide derivative

<400>  445

Asp His Lys Trp Tyr Ser Val Gly Glu Asn Ser Phe Leu Asp
1               5                   10


<210>  446
<211>  13
<212>  PRT
<213>  Artificial sequence

<220>
<223>  Synthetic sequence: Peptide derivative

<400>  446

Asp His Lys Trp Tyr Ser Val Gly Glu Asn Ser Phe Leu
1               5                   10

<210>  447
<211>  14
<212>  PRT
<213>  Artificial sequence

<220>
<223>  Synthetic sequence: Peptide derivative

<400>  447

His Lys Trp Tyr Ser Val Gly Glu Asn Ser Phe Leu Asp Phe
1               5                   10

<210>  448
<211>  13
<212>  PRT
<213>  Artificial sequence

<220>
<223>  Synthetic sequence: Peptide derivative

<400>  448

His Lys Trp Tyr Ser Val Gly Glu Asn Ser Phe Leu Asp
1               5                   10

<210>  449
<211>  12
<212>  PRT
<213>  Artificial sequence

<220>
<223>  Synthetic sequence: Peptide derivative

<400>  449

His Lys Trp Tyr Ser Val Gly Glu Asn Ser Phe Leu
1               5                   10

<210>  450
<211>  13
<212>  PRT
<213>  Artificial sequence

<220>
<223>  Synthetic sequence: Peptide derivative

<400>  450

Lys Trp Tyr Ser Val Gly Glu Asn Ser Phe Leu Asp Phe
1               5                   10

<210>  451
<211>  12
<212>  PRT

<213>  Artificial sequence

<220>
<223>  Synthetic sequence: Peptide derivative

<400>  451

Lys Trp Tyr Ser Val Gly Glu Asn Ser Phe Leu Asp
1               5                   10


<210>  452
<211>  11
<212>  PRT
<213>  Artificial sequence

<220>
<223>  Synthetic sequence: Peptide derivative

<400>  452

Lys Trp Tyr Ser Val Gly Glu Asn Ser Phe Leu
1               5                   10


<210>  453
<211>  12
<212>  PRT
<213>  Alternaria alternata

<400>  453

Lys Gln Lys Val Ser Asp Asp Ile Thr Tyr Val Ala
1               5                   10


<210>  454
<211>  11
<212>  PRT
<213>  Alternaria alternata

<400>  454

Gln Lys Val Ser Asp Asp Ile Thr Tyr Val Ala
1               5                   10


<210>  455
<211>  10
<212>  PRT
<213>  Alternaria alternata

<400>  455

Lys Val Ser Asp Asp Ile Thr Tyr Val Ala
1               5                   10


<210>  456
<211>  12
<212>  PRT
<213>  Alternaria alternata

<400>  456

Val Ser Asp Asp Ile Thr Tyr Val Ala Thr Ala Thr
1               5                   10


<210>  457
<211>  11
<212>  PRT
<213>  Alternaria alternata

<400>  457

Val Ser Asp Asp Ile Thr Tyr Val Ala Thr Ala
1               5                   10


<210>  458
<211>  10
<212>  PRT
<213>  Alternaria alternata

<400>  458

Val Ser Asp Asp Ile Thr Tyr Val Ala Thr
1               5                   10


<210>  459
<211>  15
<212>  PRT
<213>  Alternaria alternata

<400>  459

Lys Gln Lys Val Ser Asp Asp Ile Thr Tyr Val Ala Thr Ala Thr
1               5                   10                  15


<210>  460
<211>  14
<212>  PRT
<213>  Alternaria alternata

<400>  460

Lys Gln Lys Val Ser Asp Asp Ile Thr Tyr Val Ala Thr Ala
1               5                   10


<210>  461
<211>  13
<212>  PRT
<213>  Alternaria alternata

<400>  461

Lys Gln Lys Val Ser Asp Asp Ile Thr Tyr Val Ala Thr
1               5                   10


<210>  462
<211>  14

<212>  PRT
<213>  Alternaria alternata

<400>  462

Gln Lys Val Ser Asp Asp Ile Thr Tyr Val Ala Thr Ala Thr
1               5                   10


<210>  463
<211>  13
<212>  PRT
<213>  Alternaria alternata

<400>  463

Gln Lys Val Ser Asp Asp Ile Thr Tyr Val Ala Thr Ala
1               5                   10


<210>  464
<211>  12
<212>  PRT
<213>  Alternaria alternata

<400>  464

Gln Lys Val Ser Asp Asp Ile Thr Tyr Val Ala Thr
1               5                   10


<210>  465
<211>  13
<212>  PRT
<213>  Alternaria alternata

<400>  465

Lys Val Ser Asp Asp Ile Thr Tyr Val Ala Thr Ala Thr
1               5                   10


<210>  466
<211>  12
<212>  PRT
<213>  Alternaria alternata

<400>  466

Lys Val Ser Asp Asp Ile Thr Tyr Val Ala Thr Ala
1               5                   10


<210>  467
<211>  11
<212>  PRT
<213>  Alternaria alternata

<400>  467

Lys Val Ser Asp Asp Ile Thr Tyr Val Ala Thr
1               5                   10

<210>  468
<211>  12
<212>  PRT
<213>  Alternaria alternata

<400>  468

Asp Asn Arg Leu Val Asn His Phe Thr Asn Glu Phe
1               5                   10


<210>  469
<211>  11
<212>  PRT
<213>  Alternaria alternata

<400>  469

Asn Arg Leu Val Asn His Phe Thr Asn Glu Phe
1               5                   10


<210>  470
<211>  10
<212>  PRT
<213>  Alternaria alternata

<400>  470

Arg Leu Val Asn His Phe Thr Asn Glu Phe
1               5                   10


<210>  471
<211>  12
<212>  PRT
<213>  Alternaria alternata

<400>  471

Leu Val Asn His Phe Thr Asn Glu Phe Lys Arg Lys
1               5                   10


<210>  472
<211>  11
<212>  PRT
<213>  Alternaria alternata

<400>  472

Leu Val Asn His Phe Thr Asn Glu Phe Lys Arg
1               5                   10


<210>  473
<211>  10
<212>  PRT
<213>  Alternaria alternata

<400>  473

Leu Val Asn His Phe Thr Asn Glu Phe Lys
1               5                   10


<210>  474
<211>  15
<212>  PRT
<213>  Alternaria alternata

<400>  474

Asp Asn Arg Leu Val Asn His Phe Thr Asn Glu Phe Lys Arg Lys
1               5                   10                  15


<210>  475
<211>  14
<212>  PRT
<213>  Alternaria alternata

<400>  475

Asp Asn Arg Leu Val Asn His Phe Thr Asn Glu Phe Lys Arg
1               5                   10


<210>  476
<211>  13
<212>  PRT
<213>  Alternaria alternata

<400>  476

Asp Asn Arg Leu Val Asn His Phe Thr Asn Glu Phe Lys
1               5                   10


<210>  477
<211>  14
<212>  PRT
<213>  Alternaria alternata

<400>  477

Asn Arg Leu Val Asn His Phe Thr Asn Glu Phe Lys Arg Lys
1               5                   10


<210>  478
<211>  13
<212>  PRT
<213>  Alternaria alternata

<400>  478

Asn Arg Leu Val Asn His Phe Thr Asn Glu Phe Lys Arg
1               5                   10


<210>  479
<211>  12
<212>  PRT
<213>  Alternaria alternata

<400>  479

Asn Arg Leu Val Asn His Phe Thr Asn Glu Phe Lys
1               5                   10


<210>  480
<211>  13
<212>  PRT
<213>  Alternaria alternata

<400>  480

Arg Leu Val Asn His Phe Thr Asn Glu Phe Lys Arg Lys
1               5                   10


<210>  481
<211>  12
<212>  PRT
<213>  Alternaria alternata

<400>  481

Arg Leu Val Asn His Phe Thr Asn Glu Phe Lys Arg
1               5                   10


<210>  482
<211>  11
<212>  PRT
<213>  Alternaria alternata

<400>  482

Arg Leu Val Asn His Phe Thr Asn Glu Phe Lys
1               5                   10


<210>  483
<211>  12
<212>  PRT
<213>  Alternaria alternata

<400>  483

Thr Ser Glu Ile Leu Leu Leu Asp Val Ala Pro Leu
1               5                   10


<210>  484
<211>  11
<212>  PRT
<213>  Alternaria alternata

<400>  484

Ser Glu Ile Leu Leu Leu Asp Val Ala Pro Leu
1               5                   10


<210>  485

<211>  10
<212>  PRT
<213>  Alternaria alternata

<400>  485

Glu Ile Leu Leu Leu Asp Val Ala Pro Leu
1               5                   10


<210>  486
<211>  12
<212>  PRT
<213>  Alternaria alternata

<400>  486

Ile Leu Leu Leu Asp Val Ala Pro Leu Ser Ile Gly
1               5                   10


<210>  487
<211>  11
<212>  PRT
<213>  Alternaria alternata

<400>  487

Ile Leu Leu Leu Asp Val Ala Pro Leu Ser Ile
1               5                   10


<210>  488
<211>  10
<212>  PRT
<213>  Alternaria alternata

<400>  488

Ile Leu Leu Leu Asp Val Ala Pro Leu Ser
1               5                   10


<210>  489
<211>  15
<212>  PRT
<213>  Alternaria alternata

<400>  489

Thr Ser Glu Ile Leu Leu Leu Asp Val Ala Pro Leu Ser Ile Gly
1               5                   10                  15


<210>  490
<211>  14
<212>  PRT
<213>  Alternaria alternata

<400>  490

Thr Ser Glu Ile Leu Leu Leu Asp Val Ala Pro Leu Ser Ile
1               5                   10

<210>  491
<211>  13
<212>  PRT
<213>  Alternaria alternata

<400>  491

Thr Ser Glu Ile Leu Leu Leu Asp Val Ala Pro Leu Ser
1               5                   10


<210>  492
<211>  14
<212>  PRT
<213>  Alternaria alternata

<400>  492

Ser Glu Ile Leu Leu Leu Asp Val Ala Pro Leu Ser Ile Gly
1               5                   10


<210>  493
<211>  13
<212>  PRT
<213>  Alternaria alternata

<400>  493

Ser Glu Ile Leu Leu Leu Asp Val Ala Pro Leu Ser Ile
1               5                   10


<210>  494
<211>  12
<212>  PRT
<213>  Alternaria alternata

<400>  494

Ser Glu Ile Leu Leu Leu Asp Val Ala Pro Leu Ser
1               5                   10


<210>  495
<211>  13
<212>  PRT
<213>  Alternaria alternata

<400>  495

Glu Ile Leu Leu Leu Asp Val Ala Pro Leu Ser Ile Gly
1               5                   10


<210>  496
<211>  12
<212>  PRT
<213>  Alternaria alternata

<400>  496

Glu Ile Leu Leu Leu Asp Val Ala Pro Leu Ser Ile
1               5                   10

<210>  497
<211>  11
<212>  PRT
<213>  Alternaria alternata

<400>  497

Glu Ile Leu Leu Leu Asp Val Ala Pro Leu Ser
1               5                   10

<210>  498
<211>  12
<212>  PRT
<213>  Alternaria alternata

<400>  498

Leu Asn Val Leu Arg Ile Ile Asn Glu Pro Thr Ala
1               5                   10

<210>  499
<211>  11
<212>  PRT
<213>  Alternaria alternata

<400>  499

Asn Val Leu Arg Ile Ile Asn Glu Pro Thr Ala
1               5                   10

<210>  500
<211>  10
<212>  PRT
<213>  Alternaria alternata

<400>  500

Val Leu Arg Ile Ile Asn Glu Pro Thr Ala
1               5                   10

<210>  501
<211>  12
<212>  PRT
<213>  Alternaria alternata

<400>  501

Leu Arg Ile Ile Asn Glu Pro Thr Ala Ala Ala Ile
1               5                   10

<210>  502
<211>  11
<212>  PRT

<213>  Alternaria alternata

<400>  502

Leu Arg Ile Ile Asn Glu Pro Thr Ala Ala Ala
1               5                   10


<210>  503
<211>  10
<212>  PRT
<213>  Alternaria alternata

<400>  503

Leu Arg Ile Ile Asn Glu Pro Thr Ala Ala
1               5                   10


<210>  504
<211>  15
<212>  PRT
<213>  Alternaria alternata

<400>  504

Leu Asn Val Leu Arg Ile Ile Asn Glu Pro Thr Ala Ala Ala Ile
1               5                   10                  15


<210>  505
<211>  14
<212>  PRT
<213>  Alternaria alternata

<400>  505

Leu Asn Val Leu Arg Ile Ile Asn Glu Pro Thr Ala Ala Ala
1               5                   10


<210>  506
<211>  13
<212>  PRT
<213>  Alternaria alternata

<400>  506

Leu Asn Val Leu Arg Ile Ile Asn Glu Pro Thr Ala Ala
1               5                   10


<210>  507
<211>  14
<212>  PRT
<213>  Alternaria alternata

<400>  507

Asn Val Leu Arg Ile Ile Asn Glu Pro Thr Ala Ala Ala Ile
1               5                   10

<210>  508
<211>  13
<212>  PRT
<213>  Alternaria alternata

<400>  508

Asn Val Leu Arg Ile Ile Asn Glu Pro Thr Ala Ala Ala
1               5                   10


<210>  509
<211>  12
<212>  PRT
<213>  Alternaria alternata

<400>  509

Asn Val Leu Arg Ile Ile Asn Glu Pro Thr Ala Ala
1               5                   10


<210>  510
<211>  13
<212>  PRT
<213>  Alternaria alternata

<400>  510

Val Leu Arg Ile Ile Asn Glu Pro Thr Ala Ala Ala Ile
1               5                   10


<210>  511
<211>  12
<212>  PRT
<213>  Alternaria alternata

<400>  511

Val Leu Arg Ile Ile Asn Glu Pro Thr Ala Ala Ala
1               5                   10


<210>  512
<211>  11
<212>  PRT
<213>  Alternaria alternata

<400>  512

Val Leu Arg Ile Ile Asn Glu Pro Thr Ala Ala
1               5                   10


<210>  513
<211>  12
<212>  PRT
<213>  Alternaria alternata

<400>  513

Ala Arg Ala Leu Arg Arg Leu Arg Thr Ala Cys Glu

1                   5                   10


<210>  514
<211>  11
<212>  PRT
<213>  Alternaria alternata

<400>  514

Arg Ala Leu Arg Arg Leu Arg Thr Ala Cys Glu
1               5                   10


<210>  515
<211>  10
<212>  PRT
<213>  Alternaria alternata

<400>  515

Ala Leu Arg Arg Leu Arg Thr Ala Cys Glu
1               5                   10


<210>  516
<211>  12
<212>  PRT
<213>  Alternaria alternata

<400>  516

Leu Arg Arg Leu Arg Thr Ala Cys Glu Arg Ala Lys
1               5                   10


<210>  517
<211>  11
<212>  PRT
<213>  Alternaria alternata

<400>  517

Leu Arg Arg Leu Arg Thr Ala Cys Glu Arg Ala
1               5                   10


<210>  518
<211>  10
<212>  PRT
<213>  Alternaria alternata

<400>  518

Leu Arg Arg Leu Arg Thr Ala Cys Glu Arg
1               5                   10


<210>  519
<211>  15
<212>  PRT
<213>  Alternaria alternata

<400>  519

Ala Arg Ala Leu Arg Arg Leu Arg Thr Ala Cys Glu Arg Ala Lys
1               5                   10                  15


<210>  520
<211>  14
<212>  PRT
<213>  Alternaria alternata

<400>  520

Ala Arg Ala Leu Arg Arg Leu Arg Thr Ala Cys Glu Arg Ala
1               5                   10


<210>  521
<211>  13
<212>  PRT
<213>  Alternaria alternata

<400>  521

Ala Arg Ala Leu Arg Arg Leu Arg Thr Ala Cys Glu Arg
1               5                   10


<210>  522
<211>  14
<212>  PRT
<213>  Alternaria alternata

<400>  522

Arg Ala Leu Arg Arg Leu Arg Thr Ala Cys Glu Arg Ala Lys
1               5                   10


<210>  523
<211>  13
<212>  PRT
<213>  Alternaria alternata

<400>  523

Arg Ala Leu Arg Arg Leu Arg Thr Ala Cys Glu Arg Ala
1               5                   10


<210>  524
<211>  12
<212>  PRT
<213>  Alternaria alternata

<400>  524

Arg Ala Leu Arg Arg Leu Arg Thr Ala Cys Glu Arg
1               5                   10


<210>  525
<211>  13

<212>  PRT
<213>  Alternaria alternata

<400>  525

Ala Leu Arg Arg Leu Arg Thr Ala Cys Glu Arg Ala Lys
1               5                   10


<210>  526
<211>  12
<212>  PRT
<213>  Alternaria alternata

<400>  526

Ala Leu Arg Arg Leu Arg Thr Ala Cys Glu Arg Ala
1               5                   10


<210>  527
<211>  11
<212>  PRT
<213>  Alternaria alternata

<400>  527

Ala Leu Arg Arg Leu Arg Thr Ala Cys Glu Arg
1               5                   10


<210>  528
<211>  12
<212>  PRT
<213>  Alternaria alternata

<400>  528

Pro Asp Glu Ile Gln Gly Phe Pro Thr Ile Lys Leu
1               5                   10


<210>  529
<211>  11
<212>  PRT
<213>  Alternaria alternata

<400>  529

Asp Glu Ile Gln Gly Phe Pro Thr Ile Lys Leu
1               5                   10


<210>  530
<211>  10
<212>  PRT
<213>  Alternaria alternata

<400>  530

Glu Ile Gln Gly Phe Pro Thr Ile Lys Leu
1               5                   10

<210>  531
<211>  12
<212>  PRT
<213>  Alternaria alternata

<400>  531

Ile Gln Gly Phe Pro Thr Ile Lys Leu Phe Pro Ala
1               5                   10


<210>  532
<211>  11
<212>  PRT
<213>  Alternaria alternata

<400>  532

Ile Gln Gly Phe Pro Thr Ile Lys Leu Phe Pro
1               5                   10


<210>  533
<211>  10
<212>  PRT
<213>  Alternaria alternata

<400>  533

Ile Gln Gly Phe Pro Thr Ile Lys Leu Phe
1               5                   10


<210>  534
<211>  15
<212>  PRT
<213>  Alternaria alternata

<400>  534

Pro Asp Glu Ile Gln Gly Phe Pro Thr Ile Lys Leu Phe Pro Ala
1               5                   10                  15


<210>  535
<211>  14
<212>  PRT
<213>  Alternaria alternata

<400>  535

Pro Asp Glu Ile Gln Gly Phe Pro Thr Ile Lys Leu Phe Pro
1               5                   10


<210>  536
<211>  13
<212>  PRT
<213>  Alternaria alternata

<400>  536

Pro Asp Glu Ile Gln Gly Phe Pro Thr Ile Lys Leu Phe
1               5                   10


<210>  537
<211>  14
<212>  PRT
<213>  Alternaria alternata

<400>  537

Asp Glu Ile Gln Gly Phe Pro Thr Ile Lys Leu Phe Pro Ala
1               5                   10


<210>  538
<211>  13
<212>  PRT
<213>  Alternaria alternata

<400>  538

Asp Glu Ile Gln Gly Phe Pro Thr Ile Lys Leu Phe Pro
1               5                   10


<210>  539
<211>  12
<212>  PRT
<213>  Alternaria alternata

<400>  539

Asp Glu Ile Gln Gly Phe Pro Thr Ile Lys Leu Phe
1               5                   10


<210>  540
<211>  13
<212>  PRT
<213>  Alternaria alternata

<400>  540

Glu Ile Gln Gly Phe Pro Thr Ile Lys Leu Phe Pro Ala
1               5                   10


<210>  541
<211>  12
<212>  PRT
<213>  Alternaria alternata

<400>  541

Glu Ile Gln Gly Phe Pro Thr Ile Lys Leu Phe Pro
1               5                   10


<210>  542
<211>  11
<212>  PRT
<213>  Alternaria alternata

<400>  542

Glu Ile Gln Gly Phe Pro Thr Ile Lys Leu Phe
1               5                   10


<210>  543
<211>  12
<212>  PRT
<213>  Alternaria alternata

<400>  543

Ala Asp Ile Val Val Gly Leu Arg Ser Gly Gln Ile
1               5                   10


<210>  544
<211>  11
<212>  PRT
<213>  Alternaria alternata

<400>  544

Asp Ile Val Val Gly Leu Arg Ser Gly Gln Ile
1               5                   10


<210>  545
<211>  10
<212>  PRT
<213>  Alternaria alternata

<400>  545

Ile Val Val Gly Leu Arg Ser Gly Gln Ile
1               5                   10


<210>  546
<211>  12
<212>  PRT
<213>  Alternaria alternata

<400>  546

Val Val Gly Leu Arg Ser Gly Gln Ile Lys Thr Gly
1               5                   10


<210>  547
<211>  11
<212>  PRT
<213>  Alternaria alternata

<400>  547

Val Val Gly Leu Arg Ser Gly Gln Ile Lys Thr
1               5                   10


<210>  548

<211>  10
<212>  PRT
<213>  Alternaria alternata

<400>  548

Val Val Gly Leu Arg Ser Gly Gln Ile Lys
1               5                   10


<210>  549
<211>  15
<212>  PRT
<213>  Alternaria alternata

<400>  549

Ala Asp Ile Val Val Gly Leu Arg Ser Gly Gln Ile Lys Thr Gly
1               5                   10                  15


<210>  550
<211>  14
<212>  PRT
<213>  Alternaria alternata

<400>  550

Ala Asp Ile Val Val Gly Leu Arg Ser Gly Gln Ile Lys Thr
1               5                   10


<210>  551
<211>  13
<212>  PRT
<213>  Alternaria alternata

<400>  551

Ala Asp Ile Val Val Gly Leu Arg Ser Gly Gln Ile Lys
1               5                   10


<210>  552
<211>  14
<212>  PRT
<213>  Alternaria alternata

<400>  552

Asp Ile Val Val Gly Leu Arg Ser Gly Gln Ile Lys Thr Gly
1               5                   10


<210>  553
<211>  13
<212>  PRT
<213>  Alternaria alternata

<400>  553

Asp Ile Val Val Gly Leu Arg Ser Gly Gln Ile Lys Thr
1               5                   10

<210>  554
<211>  12
<212>  PRT
<213>  Alternaria alternata

<400>  554

Asp Ile Val Val Gly Leu Arg Ser Gly Gln Ile Lys
1               5                   10


<210>  555
<211>  13
<212>  PRT
<213>  Alternaria alternata

<400>  555

Ile Val Val Gly Leu Arg Ser Gly Gln Ile Lys Thr Gly
1               5                   10


<210>  556
<211>  12
<212>  PRT
<213>  Alternaria alternata

<400>  556

Ile Val Val Gly Leu Arg Ser Gly Gln Ile Lys Thr
1               5                   10


<210>  557
<211>  11
<212>  PRT
<213>  Alternaria alternata

<400>  557

Ile Val Val Gly Leu Arg Ser Gly Gln Ile Lys
1               5                   10


<210>  558
<211>  12
<212>  PRT
<213>  Alternaria alternata

<400>  558

Ala Gly Val Phe Val Ser Thr Gly Thr Leu Gly Gly
1               5                   10


<210>  559
<211>  11
<212>  PRT
<213>  Alternaria alternata

<400>  559

Gly Val Phe Val Ser Thr Gly Thr Leu Gly Gly
1               5                   10

<210>  560
<211>  10
<212>  PRT
<213>  Alternaria alternata

<400>  560

Val Phe Val Ser Thr Gly Thr Leu Gly Gly
1               5                   10

<210>  561
<211>  12
<212>  PRT
<213>  Alternaria alternata

<400>  561

Phe Val Ser Thr Gly Thr Leu Gly Gly Gly Gln Glu
1               5                   10

<210>  562
<211>  11
<212>  PRT
<213>  Alternaria alternata

<400>  562

Phe Val Ser Thr Gly Thr Leu Gly Gly Gly Gln
1               5                   10

<210>  563
<211>  10
<212>  PRT
<213>  Alternaria alternata

<400>  563

Phe Val Ser Thr Gly Thr Leu Gly Gly Gly
1               5                   10

<210>  564
<211>  15
<212>  PRT
<213>  Alternaria alternata

<400>  564

Ala Gly Val Phe Val Ser Thr Gly Thr Leu Gly Gly Gly Gln Glu
1               5                   10                  15

<210>  565
<211>  14
<212>  PRT

<213>  Alternaria alternata

<400>  565

Ala Gly Val Phe Val Ser Thr Gly Thr Leu Gly Gly Gly Gln
1               5                   10


<210>  566
<211>  13
<212>  PRT
<213>  Alternaria alternata

<400>  566

Ala Gly Val Phe Val Ser Thr Gly Thr Leu Gly Gly Gly
1               5                   10


<210>  567
<211>  14
<212>  PRT
<213>  Alternaria alternata

<400>  567

Gly Val Phe Val Ser Thr Gly Thr Leu Gly Gly Gly Gln Glu
1               5                   10


<210>  568
<211>  13
<212>  PRT
<213>  Alternaria alternata

<400>  568

Gly Val Phe Val Ser Thr Gly Thr Leu Gly Gly Gly Gln
1               5                   10


<210>  569
<211>  12
<212>  PRT
<213>  Alternaria alternata

<400>  569

Gly Val Phe Val Ser Thr Gly Thr Leu Gly Gly Gly
1               5                   10


<210>  570
<211>  13
<212>  PRT
<213>  Alternaria alternata

<400>  570

Val Phe Val Ser Thr Gly Thr Leu Gly Gly Gly Gln Glu
1               5                   10

<210>  571
<211>  12
<212>  PRT
<213>  Alternaria alternata

<400>  571

Val Phe Val Ser Thr Gly Thr Leu Gly Gly Gly Gln
1               5                   10


<210>  572
<211>  11
<212>  PRT
<213>  Alternaria alternata

<400>  572

Val Phe Val Ser Thr Gly Thr Leu Gly Gly Gly
1               5                   10


<210>  573
<211>  12
<212>  PRT
<213>  Alternaria alternata

<400>  573

Lys Gly Lys Val Val Ile Val Thr Gly Ala Ser Gly
1               5                   10


<210>  574
<211>  11
<212>  PRT
<213>  Alternaria alternata

<400>  574

Gly Lys Val Val Ile Val Thr Gly Ala Ser Gly
1               5                   10


<210>  575
<211>  10
<212>  PRT
<213>  Alternaria alternata

<400>  575

Lys Val Val Ile Val Thr Gly Ala Ser Gly
1               5                   10


<210>  576
<211>  12
<212>  PRT
<213>  Alternaria alternata

<400>  576

Val Val Ile Val Thr Gly Ala Ser Gly Pro Thr Gly

1                   5                   10


<210>  577
<211>  11
<212>  PRT
<213>  Alternaria alternata

<400>  577

Val Val Ile Val Thr Gly Ala Ser Gly Pro Thr
1               5                   10


<210>  578
<211>  10
<212>  PRT
<213>  Alternaria alternata

<400>  578

Val Val Ile Val Thr Gly Ala Ser Gly Pro
1               5                   10


<210>  579
<211>  15
<212>  PRT
<213>  Alternaria alternata

<400>  579

Lys Gly Lys Val Val Ile Val Thr Gly Ala Ser Gly Pro Thr Gly
1               5                   10                  15


<210>  580
<211>  14
<212>  PRT
<213>  Alternaria alternata

<400>  580

Lys Gly Lys Val Val Ile Val Thr Gly Ala Ser Gly Pro Thr
1               5                   10


<210>  581
<211>  13
<212>  PRT
<213>  Alternaria alternata

<400>  581

Lys Gly Lys Val Val Ile Val Thr Gly Ala Ser Gly Pro
1               5                   10


<210>  582
<211>  14
<212>  PRT
<213>  Alternaria alternata

<400>  582

Gly Lys Val Val Ile Val Thr Gly Ala Ser Gly Pro Thr Gly
1               5                   10


<210>  583
<211>  13
<212>  PRT
<213>  Alternaria alternata

<400>  583

Gly Lys Val Val Ile Val Thr Gly Ala Ser Gly Pro Thr
1               5                   10


<210>  584
<211>  12
<212>  PRT
<213>  Alternaria alternata

<400>  584

Gly Lys Val Val Ile Val Thr Gly Ala Ser Gly Pro
1               5                   10


<210>  585
<211>  13
<212>  PRT
<213>  Alternaria alternata

<400>  585

Lys Val Val Ile Val Thr Gly Ala Ser Gly Pro Thr Gly
1               5                   10


<210>  586
<211>  12
<212>  PRT
<213>  Alternaria alternata

<400>  586

Lys Val Val Ile Val Thr Gly Ala Ser Gly Pro Thr
1               5                   10


<210>  587
<211>  11
<212>  PRT
<213>  Alternaria alternata

<400>  587

Lys Val Val Ile Val Thr Gly Ala Ser Gly Pro
1               5                   10


<210>  588
<211>  12

<212>  PRT
<213>  Alternaria alternata

<400>  588

Thr Gly Ser Leu Val Ile Thr Ser Ser Met Ser Gly
1               5                   10


<210>  589
<211>  11
<212>  PRT
<213>  Alternaria alternata

<400>  589

Gly Ser Leu Val Ile Thr Ser Ser Met Ser Gly
1               5                   10


<210>  590
<211>  10
<212>  PRT
<213>  Alternaria alternata

<400>  590

Ser Leu Val Ile Thr Ser Ser Met Ser Gly
1               5                   10


<210>  591
<211>  12
<212>  PRT
<213>  Alternaria alternata

<400>  591

Leu Val Ile Thr Ser Ser Met Ser Gly His Ile Ala
1               5                   10


<210>  592
<211>  11
<212>  PRT
<213>  Alternaria alternata

<400>  592

Leu Val Ile Thr Ser Ser Met Ser Gly His Ile
1               5                   10


<210>  593
<211>  10
<212>  PRT
<213>  Alternaria alternata

<400>  593

Leu Val Ile Thr Ser Ser Met Ser Gly His
1               5                   10

<210>  594
<211>  15
<212>  PRT
<213>  Alternaria alternata

<400>  594

Thr Gly Ser Leu Val Ile Thr Ser Ser Met Ser Gly His Ile Ala
1               5                   10                  15


<210>  595
<211>  14
<212>  PRT
<213>  Alternaria alternata

<400>  595

Thr Gly Ser Leu Val Ile Thr Ser Ser Met Ser Gly His Ile
1               5                   10


<210>  596
<211>  13
<212>  PRT
<213>  Alternaria alternata

<400>  596

Thr Gly Ser Leu Val Ile Thr Ser Ser Met Ser Gly His
1               5                   10


<210>  597
<211>  14
<212>  PRT
<213>  Alternaria alternata

<400>  597

Gly Ser Leu Val Ile Thr Ser Ser Met Ser Gly His Ile Ala
1               5                   10


<210>  598
<211>  13
<212>  PRT
<213>  Alternaria alternata

<400>  598

Gly Ser Leu Val Ile Thr Ser Ser Met Ser Gly His Ile
1               5                   10


<210>  599
<211>  12
<212>  PRT
<213>  Alternaria alternata

<400>  599

Gly Ser Leu Val Ile Thr Ser Ser Met Ser Gly His
1               5                   10

<210>  600
<211>  13
<212>  PRT
<213>  Alternaria alternata

<400>  600

Ser Leu Val Ile Thr Ser Ser Met Ser Gly His Ile Ala
1               5                   10

<210>  601
<211>  12
<212>  PRT
<213>  Alternaria alternata

<400>  601

Ser Leu Val Ile Thr Ser Ser Met Ser Gly His Ile
1               5                   10

<210>  602
<211>  11
<212>  PRT
<213>  Alternaria alternata

<400>  602

Ser Leu Val Ile Thr Ser Ser Met Ser Gly His
1               5                   10

<210>  603
<211>  12
<212>  PRT
<213>  Artificial sequence

<220>
<223>  Synthetic sequence: Peptide derivative

<400>  603

Thr Gly Ser Leu Val Ile Thr Ser Ser Leu Ser Gly
1               5                   10

<210>  604
<211>  11
<212>  PRT
<213>  Artificial sequence

<220>
<223>  Synthetic sequence: Peptide derivative

<400>  604

Gly Ser Leu Val Ile Thr Ser Ser Leu Ser Gly
1               5                   10

<210>  605
<211>  10
<212>  PRT
<213>  Artificial sequence

<220>
<223>  Synthetic sequence: Peptide derivative

<400>  605

Ser Leu Val Ile Thr Ser Ser Leu Ser Gly
1               5                   10


<210>  606
<211>  12
<212>  PRT
<213>  Artificial sequence

<220>
<223>  Synthetic sequence: Peptide derivative

<400>  606

Leu Val Ile Thr Ser Ser Leu Ser Gly His Ile Ala
1               5                   10


<210>  607
<211>  11
<212>  PRT
<213>  Artificial sequence

<220>
<223>  Synthetic sequence: Peptide derivative

<400>  607

Leu Val Ile Thr Ser Ser Leu Ser Gly His Ile
1               5                   10


<210>  608
<211>  10
<212>  PRT
<213>  Artificial sequence

<220>
<223>  Synthetic sequence: Peptide derivative

<400>  608

Leu Val Ile Thr Ser Ser Leu Ser Gly His
1               5                   10


<210>  609
<211>  15
<212>  PRT
<213>  Artificial sequence

<220>
<223>  Synthetic sequence: Peptide derivative

<400>  609

Thr Gly Ser Leu Val Ile Thr Ser Ser Leu Ser Gly His Ile Ala
1               5                   10                  15


<210>  610
<211>  14
<212>  PRT
<213>  Artificial sequence

<220>
<223>  Synthetic sequence: Peptide derivative

<400>  610

Thr Gly Ser Leu Val Ile Thr Ser Ser Leu Ser Gly His Ile
1               5                   10


<210>  611
<211>  13
<212>  PRT
<213>  Artificial sequence

<220>
<223>  Synthetic sequence: Peptide derivative

<400>  611

Thr Gly Ser Leu Val Ile Thr Ser Ser Leu Ser Gly His
1               5                   10


<210>  612
<211>  14
<212>  PRT
<213>  Artificial sequence

<220>
<223>  Synthetic sequence: Peptide derivative

<400>  612

Gly Ser Leu Val Ile Thr Ser Ser Leu Ser Gly His Ile Ala
1               5                   10


<210>  613
<211>  13
<212>  PRT
<213>  Artificial sequence

<220>
<223>  Synthetic sequence: Peptide derivative

<400>  613

Gly Ser Leu Val Ile Thr Ser Ser Leu Ser Gly His Ile
1               5                   10

<210>  614
<211>  12
<212>  PRT
<213>  Artificial sequence

<220>
<223>  Synthetic sequence: Peptide derivative

<400>  614

Gly Ser Leu Val Ile Thr Ser Ser Leu Ser Gly His
1               5                   10


<210>  615
<211>  13
<212>  PRT
<213>  Artificial sequence

<220>
<223>  Synthetic sequence: Peptide derivative

<400>  615

Ser Leu Val Ile Thr Ser Ser Leu Ser Gly His Ile Ala
1               5                   10


<210>  616
<211>  12
<212>  PRT
<213>  Artificial sequence

<220>
<223>  Synthetic sequence: Peptide derivative

<400>  616

Ser Leu Val Ile Thr Ser Ser Leu Ser Gly His Ile
1               5                   10


<210>  617
<211>  11
<212>  PRT
<213>  Artificial sequence

<220>
<223>  Synthetic sequence: Peptide derivative

<400>  617

Ser Leu Val Ile Thr Ser Ser Leu Ser Gly His
1               5                   10


<210>  618
<211>  12
<212>  PRT
<213>  Alternaria alternata

<400>  618

Val Trp Ala Ile Val Leu Leu Ser Leu Cys Gln Leu
1               5                   10


<210>  619
<211>  11
<212>  PRT
<213>  Alternaria alternata

<400>  619

Trp Ala Ile Val Leu Leu Ser Leu Cys Gln Leu
1               5                   10


<210>  620
<211>  10
<212>  PRT
<213>  Alternaria alternata

<400>  620

Ala Ile Val Leu Leu Ser Leu Cys Gln Leu
1               5                   10


<210>  621
<211>  12
<212>  PRT
<213>  Alternaria alternata

<400>  621

Ile Val Leu Leu Ser Leu Cys Gln Leu Thr Thr Pro
1               5                   10


<210>  622
<211>  11
<212>  PRT
<213>  Alternaria alternata

<400>  622

Ile Val Leu Leu Ser Leu Cys Gln Leu Thr Thr
1               5                   10


<210>  623
<211>  10
<212>  PRT
<213>  Alternaria alternata

<400>  623

Ile Val Leu Leu Ser Leu Cys Gln Leu Thr
1               5                   10


<210>  624
<211>  15

<212>  PRT
<213>  Alternaria alternata

<400>  624

Val Trp Ala Ile Val Leu Leu Ser Leu Cys Gln Leu Thr Thr Pro
1               5                   10                  15


<210>  625
<211>  14
<212>  PRT
<213>  Alternaria alternata

<400>  625

Val Trp Ala Ile Val Leu Leu Ser Leu Cys Gln Leu Thr Thr
1               5                   10


<210>  626
<211>  13
<212>  PRT
<213>  Alternaria alternata

<400>  626

Val Trp Ala Ile Val Leu Leu Ser Leu Cys Gln Leu Thr
1               5                   10


<210>  627
<211>  14
<212>  PRT
<213>  Alternaria alternata

<400>  627

Trp Ala Ile Val Leu Leu Ser Leu Cys Gln Leu Thr Thr Pro
1               5                   10


<210>  628
<211>  13
<212>  PRT
<213>  Alternaria alternata

<400>  628

Trp Ala Ile Val Leu Leu Ser Leu Cys Gln Leu Thr Thr
1               5                   10


<210>  629
<211>  12
<212>  PRT
<213>  Alternaria alternata

<400>  629

Trp Ala Ile Val Leu Leu Ser Leu Cys Gln Leu Thr
1               5                   10

<210>  630
<211>  13
<212>  PRT
<213>  Alternaria alternata

<400>  630

Ala Ile Val Leu Leu Ser Leu Cys Gln Leu Thr Thr Pro
1               5                   10


<210>  631
<211>  12
<212>  PRT
<213>  Alternaria alternata

<400>  631

Ala Ile Val Leu Leu Ser Leu Cys Gln Leu Thr Thr
1               5                   10


<210>  632
<211>  11
<212>  PRT
<213>  Alternaria alternata

<400>  632

Ala Ile Val Leu Leu Ser Leu Cys Gln Leu Thr
1               5                   10


<210>  633
<211>  12
<212>  PRT
<213>  Artificial sequence

<220>
<223>  Synthetic sequence: Peptide derivative

<400>  633

Val Trp Ala Ile Val Leu Leu Ser Leu Val Gln Leu
1               5                   10


<210>  634
<211>  11
<212>  PRT
<213>  Artificial sequence

<220>
<223>  Synthetic sequence: Peptide derivative

<400>  634

Trp Ala Ile Val Leu Leu Ser Leu Val Gln Leu
1               5                   10


<210>  635

<211>  10
<212>  PRT
<213>  Artificial sequence

<220>
<223>  Synthetic sequence: Peptide derivative

<400>  635

Ala Ile Val Leu Leu Ser Leu Val Gln Leu
1               5                   10


<210>  636
<211>  12
<212>  PRT
<213>  Artificial sequence

<220>
<223>  Synthetic sequence: Peptide derivative

<400>  636

Ile Val Leu Leu Ser Leu Val Gln Leu Thr Thr Pro
1               5                   10


<210>  637
<211>  11
<212>  PRT
<213>  Artificial sequence

<220>
<223>  Synthetic sequence: Peptide derivative

<400>  637

Ile Val Leu Leu Ser Leu Val Gln Leu Thr Thr
1               5                   10


<210>  638
<211>  10
<212>  PRT
<213>  Artificial sequence

<220>
<223>  Synthetic sequence: Peptide derivative

<400>  638

Ile Val Leu Leu Ser Leu Val Gln Leu Thr
1               5                   10


<210>  639
<211>  15
<212>  PRT
<213>  Artificial sequence

<220>
<223>  Synthetic sequence: Peptide derivative

<400>  639

Val Trp Ala Ile Val Leu Leu Ser Leu Val Gln Leu Thr Thr Pro
1               5                   10                  15


<210>  640
<211>  14
<212>  PRT
<213>  Artificial sequence

<220>
<223>  Synthetic sequence: Peptide derivative

<400>  640

Val Trp Ala Ile Val Leu Leu Ser Leu Val Gln Leu Thr Thr
1               5                   10


<210>  641
<211>  13
<212>  PRT
<213>  Artificial sequence

<220>
<223>  Synthetic sequence: Peptide derivative

<400>  641

Val Trp Ala Ile Val Leu Leu Ser Leu Val Gln Leu Thr
1               5                   10


<210>  642
<211>  14
<212>  PRT
<213>  Artificial sequence

<220>
<223>  Synthetic sequence: Peptide derivative

<400>  642

Trp Ala Ile Val Leu Leu Ser Leu Val Gln Leu Thr Thr Pro
1               5                   10


<210>  643
<211>  13
<212>  PRT
<213>  Artificial sequence

<220>
<223>  Synthetic sequence: Peptide derivative

<400>  643

Trp Ala Ile Val Leu Leu Ser Leu Val Gln Leu Thr Thr
1               5                   10


<210>  644

<211>  12
<212>  PRT
<213>  Artificial sequence

<220>
<223>  Synthetic sequence: Peptide derivative

<400>  644

Trp Ala Ile Val Leu Leu Ser Leu Val Gln Leu Thr
1               5                   10


<210>  645
<211>  13
<212>  PRT
<213>  Artificial sequence

<220>
<223>  Synthetic sequence: Peptide derivative

<400>  645

Ala Ile Val Leu Leu Ser Leu Val Gln Leu Thr Thr Pro
1               5                   10


<210>  646
<211>  12
<212>  PRT
<213>  Artificial sequence

<220>
<223>  Synthetic sequence: Peptide derivative

<400>  646

Ala Ile Val Leu Leu Ser Leu Val Gln Leu Thr Thr
1               5                   10


<210>  647
<211>  11
<212>  PRT
<213>  Artificial sequence

<220>
<223>  Synthetic sequence: Peptide derivative

<400>  647

Ala Ile Val Leu Leu Ser Leu Val Gln Leu Thr
1               5                   10


<210>  648
<211>  12
<212>  PRT
<213>  Alternaria alternata

<400>  648

Val Gly Ile Phe Arg Asp Asp Arg Ile Glu Ile Ile

1               5                   10


<210>  649
<211>  11
<212>  PRT
<213>  Alternaria alternata

<400>  649

Gly Ile Phe Arg Asp Asp Arg Ile Glu Ile Ile
1               5                   10


<210>  650
<211>  10
<212>  PRT
<213>  Alternaria alternata

<400>  650

Ile Phe Arg Asp Asp Arg Ile Glu Ile Ile
1               5                   10


<210>  651
<211>  12
<212>  PRT
<213>  Alternaria alternata

<400>  651

Phe Arg Asp Asp Arg Ile Glu Ile Ile Ala Asn Asp
1               5                   10


<210>  652
<211>  11
<212>  PRT
<213>  Alternaria alternata

<400>  652

Phe Arg Asp Asp Arg Ile Glu Ile Ile Ala Asn
1               5                   10


<210>  653
<211>  10
<212>  PRT
<213>  Alternaria alternata

<400>  653

Phe Arg Asp Asp Arg Ile Glu Ile Ile Ala
1               5                   10


<210>  654
<211>  15
<212>  PRT
<213>  Alternaria alternata

<400>  654

Val Gly Ile Phe Arg Asp Asp Arg Ile Glu Ile Ile Ala Asn Asp
1               5                   10                  15


<210>  655
<211>  14
<212>  PRT
<213>  Alternaria alternata

<400>  655

Val Gly Ile Phe Arg Asp Asp Arg Ile Glu Ile Ile Ala Asn
1               5                   10


<210>  656
<211>  13
<212>  PRT
<213>  Alternaria alternata

<400>  656

Val Gly Ile Phe Arg Asp Asp Arg Ile Glu Ile Ile Ala
1               5                   10


<210>  657
<211>  14
<212>  PRT
<213>  Alternaria alternata

<400>  657

Gly Ile Phe Arg Asp Asp Arg Ile Glu Ile Ile Ala Asn Asp
1               5                   10


<210>  658
<211>  13
<212>  PRT
<213>  Alternaria alternata

<400>  658

Gly Ile Phe Arg Asp Asp Arg Ile Glu Ile Ile Ala Asn
1               5                   10


<210>  659
<211>  12
<212>  PRT
<213>  Alternaria alternata

<400>  659

Gly Ile Phe Arg Asp Asp Arg Ile Glu Ile Ile Ala
1               5                   10


<210>  660
<211>  13

<212>  PRT
<213>  Alternaria alternata

<400>  660

Ile Phe Arg Asp Asp Arg Ile Glu Ile Ile Ala Asn Asp
1               5                   10


<210>  661
<211>  12
<212>  PRT
<213>  Alternaria alternata

<400>  661

Ile Phe Arg Asp Asp Arg Ile Glu Ile Ile Ala Asn
1               5                   10


<210>  662
<211>  11
<212>  PRT
<213>  Alternaria alternata

<400>  662

Ile Phe Arg Asp Asp Arg Ile Glu Ile Ile Ala
1               5                   10


<210>  663

<400>  663
000

<210>  664

<400>  664
000

<210>  665

<400>  665
000

<210>  666

<400>  666
000

<210>  667
<211>  12
<212>  PRT
<213>  Alternaria alternata

<400>  667

Lys Asn Gln Val Ala Met Asn Pro Val Asn Thr Val
1               5                   10


<210>  668

<211>  11
<212>  PRT
<213>  Alternaria alternata

<400>  668

Asn Gln Val Ala Met Asn Pro Val Asn Thr Val
1               5                   10


<210>  669
<211>  10
<212>  PRT
<213>  Alternaria alternata

<400>  669

Gln Val Ala Met Asn Pro Val Asn Thr Val
1               5                   10


<210>  670
<211>  12
<212>  PRT
<213>  Alternaria alternata

<400>  670

Val Ala Met Asn Pro Val Asn Thr Val Phe Asp Ala
1               5                   10


<210>  671
<211>  11
<212>  PRT
<213>  Alternaria alternata

<400>  671

Val Ala Met Asn Pro Val Asn Thr Val Phe Asp
1               5                   10


<210>  672
<211>  10
<212>  PRT
<213>  Alternaria alternata

<400>  672

Val Ala Met Asn Pro Val Asn Thr Val Phe
1               5                   10


<210>  673
<211>  15
<212>  PRT
<213>  Alternaria alternata

<400>  673

Lys Asn Gln Val Ala Met Asn Pro Val Asn Thr Val Phe Asp Ala
1               5                   10                  15

<210>  674
<211>  14
<212>  PRT
<213>  Alternaria alternata

<400>  674

Lys Asn Gln Val Ala Met Asn Pro Val Asn Thr Val Phe Asp
1               5                   10


<210>  675
<211>  13
<212>  PRT
<213>  Alternaria alternata

<400>  675

Lys Asn Gln Val Ala Met Asn Pro Val Asn Thr Val Phe
1               5                   10


<210>  676
<211>  14
<212>  PRT
<213>  Alternaria alternata

<400>  676

Asn Gln Val Ala Met Asn Pro Val Asn Thr Val Phe Asp Ala
1               5                   10


<210>  677
<211>  13
<212>  PRT
<213>  Alternaria alternata

<400>  677

Asn Gln Val Ala Met Asn Pro Val Asn Thr Val Phe Asp
1               5                   10


<210>  678
<211>  12
<212>  PRT
<213>  Alternaria alternata

<400>  678

Asn Gln Val Ala Met Asn Pro Val Asn Thr Val Phe
1               5                   10


<210>  679
<211>  13
<212>  PRT
<213>  Alternaria alternata

<400>  679

Gln Val Ala Met Asn Pro Val Asn Thr Val Phe Asp Ala
1               5                   10

<210>  680
<211>  12
<212>  PRT
<213>  Alternaria alternata

<400>  680

Gln Val Ala Met Asn Pro Val Asn Thr Val Phe Asp
1               5                   10

<210>  681
<211>  11
<212>  PRT
<213>  Alternaria alternata

<400>  681

Gln Val Ala Met Asn Pro Val Asn Thr Val Phe
1               5                   10

<210>  682
<211>  12
<212>  PRT
<213>  Artificial sequence

<220>
<223>  Synthetic sequence: Peptide derivative

<400>  682

Lys Asn Gln Val Ala Leu Asn Pro Val Asn Thr Val
1               5                   10

<210>  683
<211>  11
<212>  PRT
<213>  Artificial sequence

<220>
<223>  Synthetic sequence: Peptide derivative

<400>  683

Asn Gln Val Ala Leu Asn Pro Val Asn Thr Val
1               5                   10

<210>  684
<211>  10
<212>  PRT
<213>  Artificial sequence

<220>
<223>  Synthetic sequence: Peptide derivative

<400>  684

Gln Val Ala Leu Asn Pro Val Asn Thr Val
1               5                   10


<210>  685
<211>  12
<212>  PRT
<213>  Artificial sequence

<220>
<223>  Synthetic sequence: Peptide derivative

<400>  685

Val Ala Leu Asn Pro Val Asn Thr Val Phe Asp Ala
1               5                   10


<210>  686
<211>  11
<212>  PRT
<213>  Artificial sequence

<220>
<223>  Synthetic sequence: Peptide derivative

<400>  686

Val Ala Leu Asn Pro Val Asn Thr Val Phe Asp
1               5                   10


<210>  687
<211>  10
<212>  PRT
<213>  Artificial sequence

<220>
<223>  Synthetic sequence: Peptide derivative

<400>  687

Val Ala Leu Asn Pro Val Asn Thr Val Phe
1               5                   10


<210>  688
<211>  15
<212>  PRT
<213>  Artificial sequence

<220>
<223>  Synthetic sequence: Peptide derivative

<400>  688

Lys Asn Gln Val Ala Leu Asn Pro Val Asn Thr Val Phe Asp Ala
1               5                   10                  15


<210>  689

<211>  14
<212>  PRT
<213>  Artificial sequence

<220>
<223>  Synthetic sequence: Peptide derivative

<400>  689

Lys Asn Gln Val Ala Leu Asn Pro Val Asn Thr Val Phe Asp
1               5                   10


<210>  690
<211>  13
<212>  PRT
<213>  Artificial sequence

<220>
<223>  Synthetic sequence: Peptide derivative

<400>  690

Lys Asn Gln Val Ala Leu Asn Pro Val Asn Thr Val Phe
1               5                   10


<210>  691
<211>  14
<212>  PRT
<213>  Artificial sequence

<220>
<223>  Synthetic sequence: Peptide derivative

<400>  691

Asn Gln Val Ala Leu Asn Pro Val Asn Thr Val Phe Asp Ala
1               5                   10


<210>  692
<211>  13
<212>  PRT
<213>  Artificial sequence

<220>
<223>  Synthetic sequence: Peptide derivative

<400>  692

Asn Gln Val Ala Leu Asn Pro Val Asn Thr Val Phe Asp
1               5                   10


<210>  693
<211>  12
<212>  PRT
<213>  Artificial sequence

<220>
<223>  Synthetic sequence: Peptide derivative

<400>  693

Asn Gln Val Ala Leu Asn Pro Val Asn Thr Val Phe
1               5                   10


<210>  694
<211>  13
<212>  PRT
<213>  Artificial sequence

<220>
<223>  Synthetic sequence: Peptide derivative

<400>  694

Gln Val Ala Leu Asn Pro Val Asn Thr Val Phe Asp Ala
1               5                   10


<210>  695
<211>  12
<212>  PRT
<213>  Artificial sequence

<220>
<223>  Synthetic sequence: Peptide derivative

<400>  695

Gln Val Ala Leu Asn Pro Val Asn Thr Val Phe Asp
1               5                   10


<210>  696
<211>  11
<212>  PRT
<213>  Artificial sequence

<220>
<223>  Synthetic sequence: Peptide derivative

<400>  696

Gln Val Ala Leu Asn Pro Val Asn Thr Val Phe
1               5                   10


<210>  697
<211>  12
<212>  PRT
<213>  Alternaria alternata

<400>  697

Leu Ser Lys Leu Val Thr Ile Ala Lys Val Asp Ala
1               5                   10


<210>  698
<211>  11
<212>  PRT
<213>  Alternaria alternata

<400>  698

Ser Lys Leu Val Thr Ile Ala Lys Val Asp Ala
1               5                   10

<210>  699
<211>  10
<212>  PRT
<213>  Alternaria alternata

<400>  699

Lys Leu Val Thr Ile Ala Lys Val Asp Ala
1               5                   10

<210>  700
<211>  12
<212>  PRT
<213>  Alternaria alternata

<400>  700

Leu Val Thr Ile Ala Lys Val Asp Ala Thr Leu Asn
1               5                   10

<210>  701
<211>  11
<212>  PRT
<213>  Alternaria alternata

<400>  701

Leu Val Thr Ile Ala Lys Val Asp Ala Thr Leu
1               5                   10

<210>  702
<211>  10
<212>  PRT
<213>  Alternaria alternata

<400>  702

Leu Val Thr Ile Ala Lys Val Asp Ala Thr
1               5                   10

<210>  703
<211>  15
<212>  PRT
<213>  Alternaria alternata

<400>  703

Leu Ser Lys Leu Val Thr Ile Ala Lys Val Asp Ala Thr Leu Asn
1               5                   10                  15

<210>  704

<211>  14
<212>  PRT
<213>  Alternaria alternata

<400>  704

Leu Ser Lys Leu Val Thr Ile Ala Lys Val Asp Ala Thr Leu
1               5                   10


<210>  705
<211>  13
<212>  PRT
<213>  Alternaria alternata

<400>  705

Leu Ser Lys Leu Val Thr Ile Ala Lys Val Asp Ala Thr
1               5                   10


<210>  706
<211>  14
<212>  PRT
<213>  Alternaria alternata

<400>  706

Ser Lys Leu Val Thr Ile Ala Lys Val Asp Ala Thr Leu Asn
1               5                   10


<210>  707
<211>  13
<212>  PRT
<213>  Alternaria alternata

<400>  707

Ser Lys Leu Val Thr Ile Ala Lys Val Asp Ala Thr Leu
1               5                   10


<210>  708
<211>  12
<212>  PRT
<213>  Alternaria alternata

<400>  708

Ser Lys Leu Val Thr Ile Ala Lys Val Asp Ala Thr
1               5                   10


<210>  709
<211>  13
<212>  PRT
<213>  Alternaria alternata

<400>  709

Lys Leu Val Thr Ile Ala Lys Val Asp Ala Thr Leu Asn
1               5                   10

<210>  710
<211>  12
<212>  PRT
<213>  Alternaria alternata

<400>  710

Lys Leu Val Thr Ile Ala Lys Val Asp Ala Thr Leu
1               5                   10


<210>  711
<211>  11
<212>  PRT
<213>  Alternaria alternata

<400>  711

Lys Leu Val Thr Ile Ala Lys Val Asp Ala Thr
1               5                   10


<210>  712
<211>  15
<212>  PRT
<213>  Alternaria alternata

<400>  712

Met Lys His Leu Ala Ala Tyr Leu Leu Leu Gly Leu Gly Gly Asn
1               5                   10                  15


<210>  713
<211>  14
<212>  PRT
<213>  Alternaria alternata

<400>  713

Met Lys His Leu Ala Ala Tyr Leu Leu Leu Gly Leu Gly Gly
1               5                   10


<210>  714
<211>  13
<212>  PRT
<213>  Alternaria alternata

<400>  714

Met Lys His Leu Ala Ala Tyr Leu Leu Leu Gly Leu Gly
1               5                   10


<210>  715
<211>  12
<212>  PRT
<213>  Alternaria alternata

<400>  715

Met Lys His Leu Ala Ala Tyr Leu Leu Leu Gly Leu
1               5                   10

<210>  716
<211>  11
<212>  PRT
<213>  Alternaria alternata

<400>  716

Met Lys His Leu Ala Ala Tyr Leu Leu Leu Gly
1               5                   10

<210>  717
<211>  10
<212>  PRT
<213>  Alternaria alternata

<400>  717

Met Lys His Leu Ala Ala Tyr Leu Leu Leu
1               5                   10

<210>  718
<211>  15
<212>  PRT
<213>  Artificial sequence

<220>
<223>  Synthetic sequence: Peptide derivative

<400>  718

Leu Lys His Leu Ala Ala Tyr Leu Leu Leu Gly Leu Gly Gly Asn
1               5                   10                  15

<210>  719
<211>  14
<212>  PRT
<213>  Artificial sequence

<220>
<223>  Synthetic sequence: Peptide derivative

<400>  719

Leu Lys His Leu Ala Ala Tyr Leu Leu Leu Gly Leu Gly Gly
1               5                   10

<210>  720
<211>  13
<212>  PRT
<213>  Artificial sequence

<220>
<223>  Synthetic sequence: Peptide derivative

<400>  720

Leu Lys His Leu Ala Ala Tyr Leu Leu Leu Gly Leu Gly
1               5                   10


<210>  721
<211>  12
<212>  PRT
<213>  Artificial sequence

<220>
<223>  Synthetic sequence: Peptide derivative

<400>  721

Leu Lys His Leu Ala Ala Tyr Leu Leu Leu Gly Leu
1               5                   10


<210>  722
<211>  11
<212>  PRT
<213>  Artificial sequence

<220>
<223>  Synthetic sequence: Peptide derivative

<400>  722

Leu Lys His Leu Ala Ala Tyr Leu Leu Leu Gly
1               5                   10


<210>  723
<211>  10
<212>  PRT
<213>  Artificial sequence

<220>
<223>  Synthetic sequence: Peptide derivative

<400>  723

Leu Lys His Leu Ala Ala Tyr Leu Leu Leu
1               5                   10


<210>  724
<211>  12
<212>  PRT
<213>  Alternaria alternata

<400>  724

Ala Glu Val Tyr Gln Lys Leu Lys Ala Leu Ala Lys
1               5                   10


<210>  725
<211>  11
<212>  PRT
<213>  Alternaria alternata

<400>  725

Glu Val Tyr Gln Lys Leu Lys Ala Leu Ala Lys
1               5                   10


<210>  726
<211>  10
<212>  PRT
<213>  Alternaria alternata

<400>  726

Val Tyr Gln Lys Leu Lys Ala Leu Ala Lys
1               5                   10


<210>  727
<211>  12
<212>  PRT
<213>  Alternaria alternata

<400>  727

Tyr Gln Lys Leu Lys Ala Leu Ala Lys Lys Thr Tyr
1               5                   10


<210>  728
<211>  11
<212>  PRT
<213>  Alternaria alternata

<400>  728

Tyr Gln Lys Leu Lys Ala Leu Ala Lys Lys Thr
1               5                   10


<210>  729
<211>  10
<212>  PRT
<213>  Alternaria alternata

<400>  729

Tyr Gln Lys Leu Lys Ala Leu Ala Lys Lys
1               5                   10


<210>  730
<211>  15
<212>  PRT
<213>  Alternaria alternata

<400>  730

Ala Glu Val Tyr Gln Lys Leu Lys Ala Leu Ala Lys Lys Thr Tyr
1               5                   10                  15


<210>  731

<211>  14
<212>  PRT
<213>  Alternaria alternata

<400>  731

Ala Glu Val Tyr Gln Lys Leu Lys Ala Leu Ala Lys Lys Thr
1               5                   10


<210>  732
<211>  13
<212>  PRT
<213>  Alternaria alternata

<400>  732

Ala Glu Val Tyr Gln Lys Leu Lys Ala Leu Ala Lys Lys
1               5                   10


<210>  733
<211>  14
<212>  PRT
<213>  Alternaria alternata

<400>  733

Glu Val Tyr Gln Lys Leu Lys Ala Leu Ala Lys Lys Thr Tyr
1               5                   10


<210>  734
<211>  13
<212>  PRT
<213>  Alternaria alternata

<400>  734

Glu Val Tyr Gln Lys Leu Lys Ala Leu Ala Lys Lys Thr
1               5                   10


<210>  735
<211>  12
<212>  PRT
<213>  Alternaria alternata

<400>  735

Glu Val Tyr Gln Lys Leu Lys Ala Leu Ala Lys Lys
1               5                   10


<210>  736
<211>  13
<212>  PRT
<213>  Alternaria alternata

<400>  736

Val Tyr Gln Lys Leu Lys Ala Leu Ala Lys Lys Thr Tyr
1               5                   10

<210>  737
<211>  12
<212>  PRT
<213>  Alternaria alternata

<400>  737

Val Tyr Gln Lys Leu Lys Ala Leu Ala Lys Lys Thr
1               5                   10


<210>  738
<211>  11
<212>  PRT
<213>  Alternaria alternata

<400>  738

Val Tyr Gln Lys Leu Lys Ala Leu Ala Lys Lys
1               5                   10


<210>  739
<211>  12
<212>  PRT
<213>  Alternaria alternata

<400>  739

Ala Ile Glu Leu Lys Ser Cys Asn Ala Leu Leu Leu
1               5                   10


<210>  740
<211>  11
<212>  PRT
<213>  Alternaria alternata

<400>  740

Ile Glu Leu Lys Ser Cys Asn Ala Leu Leu Leu
1               5                   10


<210>  741
<211>  10
<212>  PRT
<213>  Alternaria alternata

<400>  741

Glu Leu Lys Ser Cys Asn Ala Leu Leu Leu
1               5                   10


<210>  742
<211>  12
<212>  PRT
<213>  Alternaria alternata

<400>  742

Leu Lys Ser Cys Asn Ala Leu Leu Leu Lys Val Asn
1               5                   10

<210>  743
<211>  11
<212>  PRT
<213>  Alternaria alternata

<400>  743

Leu Lys Ser Cys Asn Ala Leu Leu Leu Lys Val
1               5                   10

<210>  744
<211>  10
<212>  PRT
<213>  Alternaria alternata

<400>  744

Leu Lys Ser Cys Asn Ala Leu Leu Leu Lys
1               5                   10

<210>  745
<211>  15
<212>  PRT
<213>  Alternaria alternata

<400>  745

Ala Ile Glu Leu Lys Ser Cys Asn Ala Leu Leu Leu Lys Val Asn
1               5                   10                  15

<210>  746
<211>  14
<212>  PRT
<213>  Alternaria alternata

<400>  746

Ala Ile Glu Leu Lys Ser Cys Asn Ala Leu Leu Leu Lys Val
1               5                   10

<210>  747
<211>  13
<212>  PRT
<213>  Alternaria alternata

<400>  747

Ala Ile Glu Leu Lys Ser Cys Asn Ala Leu Leu Leu Lys
1               5                   10

<210>  748
<211>  14
<212>  PRT

<213>  Alternaria alternata

<400>  748

Ile Glu Leu Lys Ser Cys Asn Ala Leu Leu Leu Lys Val Asn
1               5                   10


<210>  749
<211>  13
<212>  PRT
<213>  Alternaria alternata

<400>  749

Ile Glu Leu Lys Ser Cys Asn Ala Leu Leu Leu Lys Val
1               5                   10


<210>  750
<211>  12
<212>  PRT
<213>  Alternaria alternata

<400>  750

Ile Glu Leu Lys Ser Cys Asn Ala Leu Leu Leu Lys
1               5                   10


<210>  751
<211>  13
<212>  PRT
<213>  Alternaria alternata

<400>  751

Glu Leu Lys Ser Cys Asn Ala Leu Leu Leu Lys Val Asn
1               5                   10


<210>  752
<211>  12
<212>  PRT
<213>  Alternaria alternata

<400>  752

Glu Leu Lys Ser Cys Asn Ala Leu Leu Leu Lys Val
1               5                   10


<210>  753
<211>  11
<212>  PRT
<213>  Alternaria alternata

<400>  753

Glu Leu Lys Ser Cys Asn Ala Leu Leu Leu Lys
1               5                   10

<210>  754
<211>  12
<212>  PRT
<213>  Artificial sequence

<220>
<223>  Synthetic sequence: Peptide derivative

<400>  754

Ala Ile Glu Leu Lys Ser Val Asn Ala Leu Leu Leu
1               5                   10


<210>  755
<211>  11
<212>  PRT
<213>  Artificial sequence

<220>
<223>  Synthetic sequence: Peptide derivative

<400>  755

Ile Glu Leu Lys Ser Val Asn Ala Leu Leu Leu
1               5                   10


<210>  756
<211>  10
<212>  PRT
<213>  Artificial sequence

<220>
<223>  Synthetic sequence: Peptide derivative

<400>  756

Glu Leu Lys Ser Val Asn Ala Leu Leu Leu
1               5                   10


<210>  757
<211>  12
<212>  PRT
<213>  Artificial sequence

<220>
<223>  Synthetic sequence: Peptide derivative

<400>  757

Leu Lys Ser Val Asn Ala Leu Leu Leu Lys Val Asn
1               5                   10


<210>  758
<211>  11
<212>  PRT
<213>  Artificial sequence

<220>
<223>  Synthetic sequence: Peptide derivative

<400>  758

Leu Lys Ser Val Asn Ala Leu Leu Leu Lys Val
1               5                   10


<210>  759
<211>  10
<212>  PRT
<213>  Artificial sequence

<220>
<223>  Synthetic sequence: Peptide derivative

<400>  759

Leu Lys Ser Val Asn Ala Leu Leu Leu Lys
1               5                   10


<210>  760
<211>  15
<212>  PRT
<213>  Artificial sequence

<220>
<223>  Synthetic sequence: Peptide derivative

<400>  760

Ala Ile Glu Leu Lys Ser Val Asn Ala Leu Leu Leu Lys Val Asn
1               5                   10                  15


<210>  761
<211>  14
<212>  PRT
<213>  Artificial sequence

<220>
<223>  Synthetic sequence: Peptide derivative

<400>  761

Ala Ile Glu Leu Lys Ser Val Asn Ala Leu Leu Leu Lys Val
1               5                   10


<210>  762
<211>  13
<212>  PRT
<213>  Artificial sequence

<220>
<223>  Synthetic sequence: Peptide derivative

<400>  762

Ala Ile Glu Leu Lys Ser Val Asn Ala Leu Leu Leu Lys
1               5                   10

<210>  763
<211>  14
<212>  PRT
<213>  Artificial sequence

<220>
<223>  Synthetic sequence: Peptide derivative

<400>  763

Ile Glu Leu Lys Ser Val Asn Ala Leu Leu Leu Lys Val Asn
1               5                   10


<210>  764
<211>  13
<212>  PRT
<213>  Artificial sequence

<220>
<223>  Synthetic sequence: Peptide derivative

<400>  764

Ile Glu Leu Lys Ser Val Asn Ala Leu Leu Leu Lys Val
1               5                   10


<210>  765
<211>  12
<212>  PRT
<213>  Artificial sequence

<220>
<223>  Synthetic sequence: Peptide derivative

<400>  765

Ile Glu Leu Lys Ser Val Asn Ala Leu Leu Leu Lys
1               5                   10


<210>  766
<211>  13
<212>  PRT
<213>  Artificial sequence

<220>
<223>  Synthetic sequence: Peptide derivative

<400>  766

Glu Leu Lys Ser Val Asn Ala Leu Leu Leu Lys Val Asn
1               5                   10


<210>  767
<211>  12
<212>  PRT
<213>  Artificial sequence

<220>
<223>  Synthetic sequence: Peptide derivative

<400>  767

Glu Leu Lys Ser Val Asn Ala Leu Leu Leu Lys Val
1               5                   10


<210>  768
<211>  11
<212>  PRT
<213>  Artificial sequence

<220>
<223>  Synthetic sequence: Peptide derivative

<400>  768

Glu Leu Lys Ser Val Asn Ala Leu Leu Leu Lys
1               5                   10


<210>  769
<211>  12
<212>  PRT
<213>  Alternaria alternata

<400>  769

Gly Ala Gly Trp Gly Val Met Val Ser His Arg Ser
1               5                   10


<210>  770
<211>  11
<212>  PRT
<213>  Alternaria alternata

<400>  770

Ala Gly Trp Gly Val Met Val Ser His Arg Ser
1               5                   10


<210>  771
<211>  10
<212>  PRT
<213>  Alternaria alternata

<400>  771

Gly Trp Gly Val Met Val Ser His Arg Ser
1               5                   10


<210>  772
<211>  12
<212>  PRT
<213>  Alternaria alternata

<400>  772

Trp Gly Val Met Val Ser His Arg Ser Gly Glu Thr
1               5                   10

<210>  773
<211>  11
<212>  PRT
<213>  Alternaria alternata

<400>  773

Trp Gly Val Met Val Ser His Arg Ser Gly Glu
1               5                   10


<210>  774
<211>  10
<212>  PRT
<213>  Alternaria alternata

<400>  774

Trp Gly Val Met Val Ser His Arg Ser Gly
1               5                   10


<210>  775
<211>  15
<212>  PRT
<213>  Alternaria alternata

<400>  775

Gly Ala Gly Trp Gly Val Met Val Ser His Arg Ser Gly Glu Thr
1               5                   10                  15


<210>  776
<211>  14
<212>  PRT
<213>  Alternaria alternata

<400>  776

Gly Ala Gly Trp Gly Val Met Val Ser His Arg Ser Gly Glu
1               5                   10


<210>  777
<211>  13
<212>  PRT
<213>  Alternaria alternata

<400>  777

Gly Ala Gly Trp Gly Val Met Val Ser His Arg Ser Gly
1               5                   10


<210>  778
<211>  14
<212>  PRT
<213>  Alternaria alternata

<400>  778

Ala Gly Trp Gly Val Met Val Ser His Arg Ser Gly Glu Thr
1               5                   10

<210>  779
<211>  13
<212>  PRT
<213>  Alternaria alternata

<400>  779

Ala Gly Trp Gly Val Met Val Ser His Arg Ser Gly Glu
1               5                   10

<210>  780
<211>  12
<212>  PRT
<213>  Alternaria alternata

<400>  780

Ala Gly Trp Gly Val Met Val Ser His Arg Ser Gly
1               5                   10

<210>  781
<211>  13
<212>  PRT
<213>  Alternaria alternata

<400>  781

Gly Trp Gly Val Met Val Ser His Arg Ser Gly Glu Thr
1               5                   10

<210>  782
<211>  12
<212>  PRT
<213>  Alternaria alternata

<400>  782

Gly Trp Gly Val Met Val Ser His Arg Ser Gly Glu
1               5                   10

<210>  783
<211>  11
<212>  PRT
<213>  Alternaria alternata

<400>  783

Gly Trp Gly Val Met Val Ser His Arg Ser Gly
1               5                   10

<210>  784
<211>  12
<212>  PRT

<213>  Artificial sequence

<220>
<223>  Synthetic sequence: Peptide derivative

<400>  784

Gly Ala Gly Trp Gly Val Leu Val Ser His Arg Ser
1               5                   10


<210>  785
<211>  11
<212>  PRT
<213>  Artificial sequence

<220>
<223>  Synthetic sequence: Peptide derivative

<400>  785

Ala Gly Trp Gly Val Leu Val Ser His Arg Ser
1               5                   10


<210>  786
<211>  10
<212>  PRT
<213>  Artificial sequence

<220>
<223>  Synthetic sequence: Peptide derivative

<400>  786

Gly Trp Gly Val Leu Val Ser His Arg Ser
1               5                   10


<210>  787
<211>  12
<212>  PRT
<213>  Artificial sequence

<220>
<223>  Synthetic sequence: Peptide derivative

<400>  787

Trp Gly Val Leu Val Ser His Arg Ser Gly Glu Thr
1               5                   10


<210>  788
<211>  11
<212>  PRT
<213>  Artificial sequence

<220>
<223>  Synthetic sequence: Peptide derivative

<400>  788

Trp Gly Val Leu Val Ser His Arg Ser Gly Glu
1               5                   10

<210>  789
<211>  10
<212>  PRT
<213>  Artificial sequence

<220>
<223>  Synthetic sequence: Peptide derivative

<400>  789

Trp Gly Val Leu Val Ser His Arg Ser Gly
1               5                   10

<210>  790
<211>  15
<212>  PRT
<213>  Artificial sequence

<220>
<223>  Synthetic sequence: Peptide derivative

<400>  790

Gly Ala Gly Trp Gly Val Leu Val Ser His Arg Ser Gly Glu Thr
1               5                   10                  15

<210>  791
<211>  14
<212>  PRT
<213>  Artificial sequence

<220>
<223>  Synthetic sequence: Peptide derivative

<400>  791

Gly Ala Gly Trp Gly Val Leu Val Ser His Arg Ser Gly Glu
1               5                   10

<210>  792
<211>  13
<212>  PRT
<213>  Artificial sequence

<220>
<223>  Synthetic sequence: Peptide derivative

<400>  792

Gly Ala Gly Trp Gly Val Leu Val Ser His Arg Ser Gly
1               5                   10

<210>  793
<211>  14
<212>  PRT

<213>  Artificial sequence

<220>
<223>  Synthetic sequence: Peptide derivative

<400>  793

Ala Gly Trp Gly Val Leu Val Ser His Arg Ser Gly Glu Thr
1               5                   10


<210>  794
<211>  13
<212>  PRT
<213>  Artificial sequence

<220>
<223>  Synthetic sequence: Peptide derivative

<400>  794

Ala Gly Trp Gly Val Leu Val Ser His Arg Ser Gly Glu
1               5                   10


<210>  795
<211>  12
<212>  PRT
<213>  Artificial sequence

<220>
<223>  Synthetic sequence: Peptide derivative

<400>  795

Ala Gly Trp Gly Val Leu Val Ser His Arg Ser Gly
1               5                   10


<210>  796
<211>  13
<212>  PRT
<213>  Artificial sequence

<220>
<223>  Synthetic sequence: Peptide derivative

<400>  796

Gly Trp Gly Val Leu Val Ser His Arg Ser Gly Glu Thr
1               5                   10


<210>  797
<211>  12
<212>  PRT
<213>  Artificial sequence

<220>
<223>  Synthetic sequence: Peptide derivative

<400>  797

Gly Trp Gly Val Leu Val Ser His Arg Ser Gly Glu
1               5                   10

<210>  798
<211>  11
<212>  PRT
<213>  Artificial sequence

<220>
<223>  Synthetic sequence: Peptide derivative

<400>  798

Gly Trp Gly Val Leu Val Ser His Arg Ser Gly
1               5                   10

<210>  799
<211>  12
<212>  PRT
<213>  Alternaria alternata

<400>  799

Val Pro Leu Gly Tyr Lys Thr Ala Phe Ser Met Leu
1               5                   10

<210>  800
<211>  11
<212>  PRT
<213>  Alternaria alternata

<400>  800

Pro Leu Gly Tyr Lys Thr Ala Phe Ser Met Leu
1               5                   10

<210>  801
<211>  10
<212>  PRT
<213>  Alternaria alternata

<400>  801

Leu Gly Tyr Lys Thr Ala Phe Ser Met Leu
1               5                   10

<210>  802
<211>  12
<212>  PRT
<213>  Alternaria alternata

<400>  802

Gly Tyr Lys Thr Ala Phe Ser Met Leu Ala Asn Leu
1               5                   10

<210>  803

<211>  11
<212>  PRT
<213>  Alternaria alternata

<400>  803

Gly Tyr Lys Thr Ala Phe Ser Met Leu Ala Asn
1               5                   10


<210>  804
<211>  10
<212>  PRT
<213>  Alternaria alternata

<400>  804

Gly Tyr Lys Thr Ala Phe Ser Met Leu Ala
1               5                   10


<210>  805
<211>  15
<212>  PRT
<213>  Alternaria alternata

<400>  805

Val Pro Leu Gly Tyr Lys Thr Ala Phe Ser Met Leu Ala Asn Leu
1               5                   10                  15


<210>  806
<211>  14
<212>  PRT
<213>  Alternaria alternata

<400>  806

Val Pro Leu Gly Tyr Lys Thr Ala Phe Ser Met Leu Ala Asn
1               5                   10


<210>  807
<211>  13
<212>  PRT
<213>  Alternaria alternata

<400>  807

Val Pro Leu Gly Tyr Lys Thr Ala Phe Ser Met Leu Ala
1               5                   10


<210>  808
<211>  14
<212>  PRT
<213>  Alternaria alternata

<400>  808

Pro Leu Gly Tyr Lys Thr Ala Phe Ser Met Leu Ala Asn Leu
1               5                   10

<210>  809
<211>  13
<212>  PRT
<213>  Alternaria alternata

<400>  809

Pro Leu Gly Tyr Lys Thr Ala Phe Ser Met Leu Ala Asn
1               5                   10


<210>  810
<211>  12
<212>  PRT
<213>  Alternaria alternata

<400>  810

Pro Leu Gly Tyr Lys Thr Ala Phe Ser Met Leu Ala
1               5                   10


<210>  811
<211>  13
<212>  PRT
<213>  Alternaria alternata

<400>  811

Leu Gly Tyr Lys Thr Ala Phe Ser Met Leu Ala Asn Leu
1               5                   10


<210>  812
<211>  12
<212>  PRT
<213>  Alternaria alternata

<400>  812

Leu Gly Tyr Lys Thr Ala Phe Ser Met Leu Ala Asn
1               5                   10


<210>  813
<211>  11
<212>  PRT
<213>  Alternaria alternata

<400>  813

Leu Gly Tyr Lys Thr Ala Phe Ser Met Leu Ala
1               5                   10


<210>  814
<211>  12
<212>  PRT
<213>  Artificial sequence

<220>

<223>  Synthetic sequence: Peptide derivative

<400>  814

Val Pro Leu Gly Tyr Lys Thr Ala Phe Ser Leu Leu
1               5                   10


<210>  815
<211>  11
<212>  PRT
<213>  Artificial sequence

<220>
<223>  Synthetic sequence: Peptide derivative

<400>  815

Pro Leu Gly Tyr Lys Thr Ala Phe Ser Leu Leu
1               5                   10


<210>  816
<211>  10
<212>  PRT
<213>  Artificial sequence

<220>
<223>  Synthetic sequence: Peptide derivative

<400>  816

Leu Gly Tyr Lys Thr Ala Phe Ser Leu Leu
1               5                   10


<210>  817
<211>  12
<212>  PRT
<213>  Artificial sequence

<220>
<223>  Synthetic sequence: Peptide derivative

<400>  817

Gly Tyr Lys Thr Ala Phe Ser Leu Leu Ala Asn Leu
1               5                   10


<210>  818
<211>  11
<212>  PRT
<213>  Artificial sequence

<220>
<223>  Synthetic sequence: Peptide derivative

<400>  818

Gly Tyr Lys Thr Ala Phe Ser Leu Leu Ala Asn
1               5                   10

<210>  819
<211>  10
<212>  PRT
<213>  Artificial sequence

<220>
<223>  Synthetic sequence: Peptide derivative

<400>  819

Gly Tyr Lys Thr Ala Phe Ser Leu Leu Ala
1               5                   10


<210>  820
<211>  15
<212>  PRT
<213>  Artificial sequence

<220>
<223>  Synthetic sequence: Peptide derivative

<400>  820

Val Pro Leu Gly Tyr Lys Thr Ala Phe Ser Leu Leu Ala Asn Leu
1               5                   10                  15


<210>  821
<211>  14
<212>  PRT
<213>  Artificial sequence

<220>
<223>  Synthetic sequence: Peptide derivative

<400>  821

Val Pro Leu Gly Tyr Lys Thr Ala Phe Ser Leu Leu Ala Asn
1               5                   10


<210>  822
<211>  13
<212>  PRT
<213>  Artificial sequence

<220>
<223>  Synthetic sequence: Peptide derivative

<400>  822

Val Pro Leu Gly Tyr Lys Thr Ala Phe Ser Leu Leu Ala
1               5                   10


<210>  823
<211>  14
<212>  PRT
<213>  Artificial sequence

<220>

<223>  Synthetic sequence: Peptide derivative

<400>  823

Pro Leu Gly Tyr Lys Thr Ala Phe Ser Leu Leu Ala Asn Leu
1               5                   10


<210>  824
<211>  13
<212>  PRT
<213>  Artificial sequence

<220>
<223>  Synthetic sequence: Peptide derivative

<400>  824

Pro Leu Gly Tyr Lys Thr Ala Phe Ser Leu Leu Ala Asn
1               5                   10


<210>  825
<211>  12
<212>  PRT
<213>  Artificial sequence

<220>
<223>  Synthetic sequence: Peptide derivative

<400>  825

Pro Leu Gly Tyr Lys Thr Ala Phe Ser Leu Leu Ala
1               5                   10


<210>  826
<211>  13
<212>  PRT
<213>  Artificial sequence

<220>
<223>  Synthetic sequence: Peptide derivative

<400>  826

Leu Gly Tyr Lys Thr Ala Phe Ser Leu Leu Ala Asn Leu
1               5                   10


<210>  827
<211>  12
<212>  PRT
<213>  Artificial sequence

<220>
<223>  Synthetic sequence: Peptide derivative

<400>  827

Leu Gly Tyr Lys Thr Ala Phe Ser Leu Leu Ala Asn
1               5                   10

<210>  828
<211>  11
<212>  PRT
<213>  Artificial sequence

<220>
<223>  Synthetic sequence: Peptide derivative

<400>  828

Leu Gly Tyr Lys Thr Ala Phe Ser Leu Leu Ala
1               5                   10


<210>  829
<211>  12
<212>  PRT
<213>  Alternaria alternata

<400>  829

Leu Ser Asp Phe Val Pro Gln Asp Ile Gln Lys Leu
1               5                   10


<210>  830
<211>  11
<212>  PRT
<213>  Alternaria alternata

<400>  830

Ser Asp Phe Val Pro Gln Asp Ile Gln Lys Leu
1               5                   10


<210>  831
<211>  10
<212>  PRT
<213>  Alternaria alternata

<400>  831

Asp Phe Val Pro Gln Asp Ile Gln Lys Leu
1               5                   10


<210>  832
<211>  12
<212>  PRT
<213>  Alternaria alternata

<400>  832

Phe Val Pro Gln Asp Ile Gln Lys Leu Trp His Ser
1               5                   10


<210>  833
<211>  11
<212>  PRT
<213>  Alternaria alternata

<400>  833

Phe Val Pro Gln Asp Ile Gln Lys Leu Trp His
1               5                   10


<210>  834
<211>  10
<212>  PRT
<213>  Alternaria alternata

<400>  834

Phe Val Pro Gln Asp Ile Gln Lys Leu Trp
1               5                   10


<210>  835
<211>  15
<212>  PRT
<213>  Alternaria alternata

<400>  835

Leu Ser Asp Phe Val Pro Gln Asp Ile Gln Lys Leu Trp His Ser
1               5                   10                  15


<210>  836
<211>  14
<212>  PRT
<213>  Alternaria alternata

<400>  836

Leu Ser Asp Phe Val Pro Gln Asp Ile Gln Lys Leu Trp His
1               5                   10


<210>  837
<211>  13
<212>  PRT
<213>  Alternaria alternata

<400>  837

Leu Ser Asp Phe Val Pro Gln Asp Ile Gln Lys Leu Trp
1               5                   10


<210>  838
<211>  14
<212>  PRT
<213>  Alternaria alternata

<400>  838

Ser Asp Phe Val Pro Gln Asp Ile Gln Lys Leu Trp His Ser
1               5                   10


<210>  839

<211>  13
<212>  PRT
<213>  Alternaria alternata

<400>  839

Ser Asp Phe Val Pro Gln Asp Ile Gln Lys Leu Trp His
1               5                   10


<210>  840
<211>  12
<212>  PRT
<213>  Alternaria alternata

<400>  840

Ser Asp Phe Val Pro Gln Asp Ile Gln Lys Leu Trp
1               5                   10


<210>  841
<211>  13
<212>  PRT
<213>  Alternaria alternata

<400>  841

Asp Phe Val Pro Gln Asp Ile Gln Lys Leu Trp His Ser
1               5                   10


<210>  842
<211>  12
<212>  PRT
<213>  Alternaria alternata

<400>  842

Asp Phe Val Pro Gln Asp Ile Gln Lys Leu Trp His
1               5                   10


<210>  843
<211>  11
<212>  PRT
<213>  Alternaria alternata

<400>  843

Asp Phe Val Pro Gln Asp Ile Gln Lys Leu Trp
1               5                   10


<210>  844
<211>  12
<212>  PRT
<213>  Alternaria alternata

<400>  844

Lys Gly Ala Tyr Val Tyr Phe Ala Ser Asp Ala Ser
1               5                   10

<210>  845
<211>  11
<212>  PRT
<213>  Alternaria alternata

<400>  845

Gly Ala Tyr Val Tyr Phe Ala Ser Asp Ala Ser
1               5                   10


<210>  846
<211>  10
<212>  PRT
<213>  Alternaria alternata

<400>  846

Ala Tyr Val Tyr Phe Ala Ser Asp Ala Ser
1               5                   10


<210>  847
<211>  12
<212>  PRT
<213>  Artificial sequence

<220>
<223>  Synthetic sequence: Peptide derivative

<400>  847

Tyr Val Tyr Phe Ala Ser Asp Ala Ser Ser Tyr Val
1               5                   10


<210>  848
<211>  11
<212>  PRT
<213>  Alternaria alternata

<400>  848

Tyr Val Tyr Phe Ala Ser Asp Ala Ser Ser Tyr
1               5                   10


<210>  849
<211>  10
<212>  PRT
<213>  Alternaria alternata

<400>  849

Tyr Val Tyr Phe Ala Ser Asp Ala Ser Ser
1               5                   10


<210>  850
<211>  15
<212>  PRT

<213>  Artificial sequence

<220>
<223>  Synthetic sequence: Peptide derivative

<400>  850

Lys Gly Ala Tyr Val Tyr Phe Ala Ser Asp Ala Ser Ser Tyr Val
1               5                   10                  15


<210>  851
<211>  14
<212>  PRT
<213>  Alternaria alternata

<400>  851

Lys Gly Ala Tyr Val Tyr Phe Ala Ser Asp Ala Ser Ser Tyr
1               5                   10


<210>  852
<211>  13
<212>  PRT
<213>  Alternaria alternata

<400>  852

Lys Gly Ala Tyr Val Tyr Phe Ala Ser Asp Ala Ser Ser
1               5                   10


<210>  853
<211>  14
<212>  PRT
<213>  Artificial sequence

<220>
<223>  Synthetic sequence: Peptide derivative

<400>  853

Gly Ala Tyr Val Tyr Phe Ala Ser Asp Ala Ser Ser Tyr Val
1               5                   10


<210>  854
<211>  13
<212>  PRT
<213>  Alternaria alternata

<400>  854

Gly Ala Tyr Val Tyr Phe Ala Ser Asp Ala Ser Ser Tyr
1               5                   10


<210>  855
<211>  12
<212>  PRT
<213>  Alternaria alternata

<400>  855

Gly Ala Tyr Val Tyr Phe Ala Ser Asp Ala Ser Ser
1               5                   10


<210>  856
<211>  13
<212>  PRT
<213>  Artificial sequence

<220>
<223>  Synthetic sequence: Peptide derivative

<400>  856

Ala Tyr Val Tyr Phe Ala Ser Asp Ala Ser Ser Tyr Val
1               5                   10


<210>  857
<211>  12
<212>  PRT
<213>  Alternaria alternata

<400>  857

Ala Tyr Val Tyr Phe Ala Ser Asp Ala Ser Ser Tyr
1               5                   10


<210>  858
<211>  11
<212>  PRT
<213>  Alternaria alternata

<400>  858

Ala Tyr Val Tyr Phe Ala Ser Asp Ala Ser Ser
1               5                   10


<210>  859
<211>  12
<212>  PRT
<213>  Alternaria alternata

<400>  859

Tyr Val Tyr Phe Ala Ser Asp Ala Ser Ser Tyr Cys
1               5                   10


<210>  860
<211>  15
<212>  PRT
<213>  Alternaria alternata

<400>  860

Lys Gly Ala Tyr Val Tyr Phe Ala Ser Asp Ala Ser Ser Tyr Cys
1               5                   10                  15

<210>  861
<211>  14
<212>  PRT
<213>  Alternaria alternata

<400>  861

Gly Ala Tyr Val Tyr Phe Ala Ser Asp Ala Ser Ser Tyr Cys
1               5                   10


<210>  862
<211>  13
<212>  PRT
<213>  Alternaria alternata

<400>  862

Ala Tyr Val Tyr Phe Ala Ser Asp Ala Ser Ser Tyr Cys
1               5                   10


<210>  863
<211>  12
<212>  PRT
<213>  Alternaria alternata

<400>  863

Asp Lys Gly Tyr Phe Ile Glu Pro Thr Ile Phe Ser
1               5                   10


<210>  864
<211>  11
<212>  PRT
<213>  Alternaria alternata

<400>  864

Lys Gly Tyr Phe Ile Glu Pro Thr Ile Phe Ser
1               5                   10


<210>  865
<211>  10
<212>  PRT
<213>  Alternaria alternata

<400>  865

Gly Tyr Phe Ile Glu Pro Thr Ile Phe Ser
1               5                   10


<210>  866
<211>  12
<212>  PRT
<213>  Alternaria alternata

<400>  866

Tyr Phe Ile Glu Pro Thr Ile Phe Ser Asn Val Thr
1               5                   10

<210>  867
<211>  11
<212>  PRT
<213>  Alternaria alternata

<400>  867

Tyr Phe Ile Glu Pro Thr Ile Phe Ser Asn Val
1               5                   10

<210>  868
<211>  10
<212>  PRT
<213>  Alternaria alternata

<400>  868

Tyr Phe Ile Glu Pro Thr Ile Phe Ser Asn
1               5                   10

<210>  869
<211>  15
<212>  PRT
<213>  Alternaria alternata

<400>  869

Asp Lys Gly Tyr Phe Ile Glu Pro Thr Ile Phe Ser Asn Val Thr
1               5                   10                  15

<210>  870
<211>  14
<212>  PRT
<213>  Alternaria alternata

<400>  870

Asp Lys Gly Tyr Phe Ile Glu Pro Thr Ile Phe Ser Asn Val
1               5                   10

<210>  871
<211>  13
<212>  PRT
<213>  Alternaria alternata

<400>  871

Asp Lys Gly Tyr Phe Ile Glu Pro Thr Ile Phe Ser Asn
1               5                   10

<210>  872
<211>  14
<212>  PRT
<213>  Alternaria alternata

<400>  872

Lys Gly Tyr Phe Ile Glu Pro Thr Ile Phe Ser Asn Val Thr
1               5                   10


<210>  873
<211>  13
<212>  PRT
<213>  Alternaria alternata

<400>  873

Lys Gly Tyr Phe Ile Glu Pro Thr Ile Phe Ser Asn Val
1               5                   10


<210>  874
<211>  12
<212>  PRT
<213>  Alternaria alternata

<400>  874

Lys Gly Tyr Phe Ile Glu Pro Thr Ile Phe Ser Asn
1               5                   10


<210>  875
<211>  13
<212>  PRT
<213>  Alternaria alternata

<400>  875

Gly Tyr Phe Ile Glu Pro Thr Ile Phe Ser Asn Val Thr
1               5                   10


<210>  876
<211>  12
<212>  PRT
<213>  Alternaria alternata

<400>  876

Gly Tyr Phe Ile Glu Pro Thr Ile Phe Ser Asn Val
1               5                   10


<210>  877
<211>  11
<212>  PRT
<213>  Alternaria alternata

<400>  877

Gly Tyr Phe Ile Glu Pro Thr Ile Phe Ser Asn
1               5                   10


<210>  878

<211>  12
<212>  PRT
<213>  Alternaria alternata

<400>  878

Leu Asp Asn Tyr Ile Gln Thr Lys Thr Val Ser Ile
1               5                   10


<210>  879
<211>  11
<212>  PRT
<213>  Alternaria alternata

<400>  879

Asp Asn Tyr Ile Gln Thr Lys Thr Val Ser Ile
1               5                   10


<210>  880
<211>  10
<212>  PRT
<213>  Alternaria alternata

<400>  880

Asn Tyr Ile Gln Thr Lys Thr Val Ser Ile
1               5                   10


<210>  881
<211>  12
<212>  PRT
<213>  Alternaria alternata

<400>  881

Tyr Ile Gln Thr Lys Thr Val Ser Ile Arg Leu Gly
1               5                   10


<210>  882
<211>  11
<212>  PRT
<213>  Alternaria alternata

<400>  882

Tyr Ile Gln Thr Lys Thr Val Ser Ile Arg Leu
1               5                   10


<210>  883
<211>  10
<212>  PRT
<213>  Alternaria alternata

<400>  883

Tyr Ile Gln Thr Lys Thr Val Ser Ile Arg
1               5                   10

<210>  884
<211>  15
<212>  PRT
<213>  Alternaria alternata

<400>  884

Leu Asp Asn Tyr Ile Gln Thr Lys Thr Val Ser Ile Arg Leu Gly
1               5                   10                  15


<210>  885
<211>  14
<212>  PRT
<213>  Alternaria alternata

<400>  885

Leu Asp Asn Tyr Ile Gln Thr Lys Thr Val Ser Ile Arg Leu
1               5                   10


<210>  886
<211>  13
<212>  PRT
<213>  Alternaria alternata

<400>  886

Leu Asp Asn Tyr Ile Gln Thr Lys Thr Val Ser Ile Arg
1               5                   10


<210>  887
<211>  14
<212>  PRT
<213>  Alternaria alternata

<400>  887

Asp Asn Tyr Ile Gln Thr Lys Thr Val Ser Ile Arg Leu Gly
1               5                   10


<210>  888
<211>  13
<212>  PRT
<213>  Alternaria alternata

<400>  888

Asp Asn Tyr Ile Gln Thr Lys Thr Val Ser Ile Arg Leu
1               5                   10


<210>  889
<211>  12
<212>  PRT
<213>  Alternaria alternata

<400>  889

Asp Asn Tyr Ile Gln Thr Lys Thr Val Ser Ile Arg
1               5                   10

<210>  890
<211>  13
<212>  PRT
<213>  Alternaria alternata

<400>  890

Asn Tyr Ile Gln Thr Lys Thr Val Ser Ile Arg Leu Gly
1               5                   10

<210>  891
<211>  12
<212>  PRT
<213>  Alternaria alternata

<400>  891

Asn Tyr Ile Gln Thr Lys Thr Val Ser Ile Arg Leu
1               5                   10

<210>  892
<211>  11
<212>  PRT
<213>  Alternaria alternata

<400>  892

Asn Tyr Ile Gln Thr Lys Thr Val Ser Ile Arg
1               5                   10

<210>  893
<211>  15
<212>  PRT
<213>  Alternaria alternata

<400>  893

Gln Thr Lys Thr Val Ser Ile Arg Leu Gly Asp Val Leu Phe Gly
1               5                   10                  15

<210>  894
<211>  14
<212>  PRT
<213>  Alternaria alternata

<400>  894

Thr Lys Thr Val Ser Ile Arg Leu Gly Asp Val Leu Phe Gly
1               5                   10

<210>  895
<211>  13
<212>  PRT

<213>  Alternaria alternata

<400>  895

Lys Thr Val Ser Ile Arg Leu Gly Asp Val Leu Phe Gly
1               5                   10


<210>  896
<211>  12
<212>  PRT
<213>  Alternaria alternata

<400>  896

Thr Val Ser Ile Arg Leu Gly Asp Val Leu Phe Gly
1               5                   10


<210>  897
<211>  11
<212>  PRT
<213>  Alternaria alternata

<400>  897

Val Ser Ile Arg Leu Gly Asp Val Leu Phe Gly
1               5                   10


<210>  898
<211>  10
<212>  PRT
<213>  Alternaria alternata

<400>  898

Ser Ile Arg Leu Gly Asp Val Leu Phe Gly
1               5                   10


<210>  899
<211>  12
<212>  PRT
<213>  Alternaria alternata

<400>  899

Gly Thr Val Trp Val Asn Ser Tyr Asn Thr Leu His
1               5                   10


<210>  900
<211>  11
<212>  PRT
<213>  Alternaria alternata

<400>  900

Thr Val Trp Val Asn Ser Tyr Asn Thr Leu His
1               5                   10

<210>  901
<211>  10
<212>  PRT
<213>  Alternaria alternata

<400>  901

Val Trp Val Asn Ser Tyr Asn Thr Leu His
1               5                   10


<210>  902
<211>  12
<212>  PRT
<213>  Alternaria alternata

<400>  902

Trp Val Asn Ser Tyr Asn Thr Leu His Trp Gln Leu
1               5                   10


<210>  903
<211>  11
<212>  PRT
<213>  Alternaria alternata

<400>  903

Trp Val Asn Ser Tyr Asn Thr Leu His Trp Gln
1               5                   10


<210>  904
<211>  10
<212>  PRT
<213>  Alternaria alternata

<400>  904

Trp Val Asn Ser Tyr Asn Thr Leu His Trp
1               5                   10


<210>  905
<211>  15
<212>  PRT
<213>  Alternaria alternata

<400>  905

Gly Thr Val Trp Val Asn Ser Tyr Asn Thr Leu His Trp Gln Leu
1               5                   10                  15


<210>  906
<211>  14
<212>  PRT
<213>  Alternaria alternata

<400>  906

Gly Thr Val Trp Val Asn Ser Tyr Asn Thr Leu His Trp Gln

1               5                   10


<210>  907
<211>  13
<212>  PRT
<213>  Alternaria alternata

<400>  907

Gly Thr Val Trp Val Asn Ser Tyr Asn Thr Leu His Trp
1               5                   10


<210>  908
<211>  14
<212>  PRT
<213>  Alternaria alternata

<400>  908

Thr Val Trp Val Asn Ser Tyr Asn Thr Leu His Trp Gln Leu
1               5                   10


<210>  909
<211>  13
<212>  PRT
<213>  Alternaria alternata

<400>  909

Thr Val Trp Val Asn Ser Tyr Asn Thr Leu His Trp Gln
1               5                   10


<210>  910
<211>  12
<212>  PRT
<213>  Alternaria alternata

<400>  910

Thr Val Trp Val Asn Ser Tyr Asn Thr Leu His Trp
1               5                   10


<210>  911
<211>  13
<212>  PRT
<213>  Alternaria alternata

<400>  911

Val Trp Val Asn Ser Tyr Asn Thr Leu His Trp Gln Leu
1               5                   10


<210>  912
<211>  12
<212>  PRT
<213>  Alternaria alternata

<400>  912

Val Trp Val Asn Ser Tyr Asn Thr Leu His Trp Gln
1               5                   10


<210>  913
<211>  11
<212>  PRT
<213>  Alternaria alternata

<400>  913

Val Trp Val Asn Ser Tyr Asn Thr Leu His Trp
1               5                   10


<210>  914
<211>  9
<212>  PRT
<213>  Alternaria alternata

<400>  914

Val Ala Thr Ala Thr Leu Pro Asn Tyr
1               5


<210>  915
<211>  9
<212>  PRT
<213>  Alternaria alternata

<400>  915

Val Tyr Gln Lys Leu Lys Ala Leu Ala
1               5


<210>  916
<211>  9
<212>  PRT
<213>  Alternaria alternata

<400>  916

Lys Leu Lys Ala Leu Ala Lys Lys Thr
1               5


<210>  917
<211>  9
<212>  PRT
<213>  Alternaria alternata

<400>  917

Leu Lys Ala Leu Ala Lys Lys Thr Tyr
1               5


<210>  918
<211>  9

<212>  PRT
<213>  Alternaria alternata

<400>  918

Phe Gly Ala Gly Trp Gly Val Met Val
1               5


<210>  919
<211>  9
<212>  PRT
<213>  Alternaria alternata

<400>  919

Gly Val Met Val Ser His Arg Ser Gly
1               5


<210>  920
<211>  9
<212>  PRT
<213>  Alternaria alternata

<400>  920

Val Met Val Ser His Arg Ser Gly Glu
1               5


<210>  921
<211>  9
<212>  PRT
<213>  Alternaria alternata

<400>  921

Met Val Ser His Arg Ser Gly Glu Thr
1               5


<210>  922
<211>  9
<212>  PRT
<213>  Alternaria alternata

<400>  922

Tyr Val Trp Lys Ile Ser Glu Phe Tyr
1               5


<210>  923
<211>  9
<212>  PRT
<213>  Alternaria alternata

<400>  923

Leu Leu Leu Lys Gln Lys Val Ser Asp
1               5

<210>  924
<211>  9
<212>  PRT
<213>  Alternaria alternata

<400>  924

Leu Leu Lys Gln Lys Val Ser Asp Asp
1               5


<210>  925
<211>  9
<212>  PRT
<213>  Alternaria alternata

<400>  925

Val Val Leu Val Ala Tyr Phe Ala Ala
1               5


<210>  926
<211>  9
<212>  PRT
<213>  Alternaria alternata

<400>  926

Val Val Gly Arg Gln Ile Leu Lys Ser
1               5


<210>  927
<211>  9
<212>  PRT
<213>  Alternaria alternata

<400>  927

Val Val Gly Arg Gln Ile Met Lys Ser
1               5


<210>  928
<211>  15
<212>  PRT
<213>  Alternaria alternata

<400>  928

Glu Gly Asp Tyr Val Trp Lys Ile Ser Glu Phe Tyr Gly Arg Lys
1               5                   10                  15


<210>  929
<211>  15
<212>  PRT
<213>  Alternaria alternata

<400>  929

Arg Ser Gly Leu Leu Leu Lys Gln Lys Val Ser Asp Asp Ile Thr
1               5                   10                  15


<210>  930
<211>  15
<212>  PRT
<213>  Alternaria alternata

<400>  930

Ser Gly Leu Leu Leu Lys Gln Lys Val Ser Asp Asp Ile Thr Tyr
1               5                   10                  15


<210>  931
<211>  15
<212>  PRT
<213>  Alternaria alternata

<400>  931

Ala Asp Lys Val Val Leu Val Ala Tyr Phe Ala Ala Asp Asp Lys
1               5                   10                  15


<210>  932
<211>  15
<212>  PRT
<213>  Artificial sequence

<220>
<223>  Synthetic sequence: Peptide derivative

<400>  932

Gly Ser Thr Val Val Gly Arg Gln Ile Leu Lys Ser Ala Ala Gly
1               5                   10                  15


<210>  933
<211>  9
<212>  PRT
<213>  Dermatophagoides pteronyssinus

<400>  933

Leu Glu Ser Val Lys Tyr Val Gln Ser
1               5


<210>  934
<211>  9
<212>  PRT
<213>  Dermatophagoides pteronyssinus

<400>  934

Glu Ser Val Lys Tyr Val Gln Ser Asn
1               5


<210>  935

<211>  9
<212>  PRT
<213>  Dermatophagoides pteronyssinus

<400>  935

Ser Val Lys Tyr Val Gln Ser Asn Gly
1               5


<210>  936
<211>  9
<212>  PRT
<213>  Dermatophagoides pteronyssinus

<400>  936

Val Lys Tyr Val Gln Ser Asn Gly Gly
1               5


<210>  937
<211>  9
<212>  PRT
<213>  Dermatophagoides pteronyssinus

<400>  937

Lys Tyr Val Gln Ser Asn Gly Gly Ala
1               5


<210>  938
<211>  9
<212>  PRT
<213>  Dermatophagoides pteronyssinus

<400>  938

Tyr Val Gln Ser Asn Gly Gly Ala Ile
1               5


<210>  939
<211>  9
<212>  PRT
<213>  Dermatophagoides pteronyssinus

<400>  939

Val Gln Ser Asn Gly Gly Ala Ile Asn
1               5


<210>  940
<211>  9
<212>  PRT
<213>  Dermatophagoides pteronyssinus

<400>  940

Gln Ser Asn Gly Gly Ala Ile Asn His
1               5

<210>  941
<211>  9
<212>  PRT
<213>  Dermatophagoides pteronyssinus

<400>  941

Leu Asp Glu Phe Lys Asn Arg Phe Leu
1               5


<210>  942
<211>  9
<212>  PRT
<213>  Dermatophagoides pteronyssinus

<400>  942

Asp Glu Phe Lys Asn Arg Phe Leu Met
1               5


<210>  943
<211>  9
<212>  PRT
<213>  Dermatophagoides pteronyssinus

<400>  943

Glu Phe Lys Asn Arg Phe Leu Met Ser
1               5


<210>  944
<211>  9
<212>  PRT
<213>  Dermatophagoides pteronyssinus

<400>  944

Phe Lys Asn Arg Phe Leu Met Ser Ala
1               5


<210>  945
<211>  9
<212>  PRT
<213>  Dermatophagoides pteronyssinus

<400>  945

Lys Asn Arg Phe Leu Met Ser Ala Glu
1               5


<210>  946
<211>  9
<212>  PRT
<213>  Dermatophagoides pteronyssinus

<400>  946

Asn Arg Phe Leu Met Ser Ala Glu Ala
1               5

<210>  947
<211>  9
<212>  PRT
<213>  Dermatophagoides pteronyssinus

<400>  947

Arg Phe Leu Met Ser Ala Glu Ala Phe
1               5

<210>  948
<211>  9
<212>  PRT
<213>  Dermatophagoides pteronyssinus

<400>  948

Phe Leu Met Ser Ala Glu Ala Phe Glu
1               5

<210>  949
<211>  9
<212>  PRT
<213>  Dermatophagoides pteronyssinus

<400>  949

Leu Arg Gln Met Arg Thr Val Thr Pro
1               5

<210>  950
<211>  9
<212>  PRT
<213>  Dermatophagoides pteronyssinus

<400>  950

Arg Gln Met Arg Thr Val Thr Pro Ile
1               5

<210>  951
<211>  9
<212>  PRT
<213>  Dermatophagoides pteronyssinus

<400>  951

Gln Met Arg Thr Val Thr Pro Ile Arg
1               5

<210>  952
<211>  9
<212>  PRT

<213>  Dermatophagoides pteronyssinus

<400>  952

Met Arg Thr Val Thr Pro Ile Arg Met
1               5


<210>  953
<211>  9
<212>  PRT
<213>  Dermatophagoides pteronyssinus

<400>  953

Arg Thr Val Thr Pro Ile Arg Met Gln
1               5


<210>  954
<211>  9
<212>  PRT
<213>  Dermatophagoides pteronyssinus

<400>  954

Thr Val Thr Pro Ile Arg Met Gln Gly
1               5


<210>  955
<211>  9
<212>  PRT
<213>  Dermatophagoides pteronyssinus

<400>  955

Val Thr Pro Ile Arg Met Gln Gly Gly
1               5


<210>  956
<211>  9
<212>  PRT
<213>  Dermatophagoides pteronyssinus

<400>  956

Thr Pro Ile Arg Met Gln Gly Gly Cys
1               5


<210>  957
<211>  9
<212>  PRT
<213>  Dermatophagoides pteronyssinus

<400>  957

Pro Ile Arg Met Gln Gly Gly Cys Gly
1               5

<210> 958
<211> 9
<212> PRT
<213> Dermatophagoides pteronyssinus

<400> 958

Ile Arg Met Gln Gly Gly Cys Gly Ser
1               5


<210> 959
<211> 9
<212> PRT
<213> Dermatophagoides pteronyssinus

<400> 959

Arg Met Gln Gly Gly Cys Gly Ser Cys
1               5


<210> 960
<211> 9
<212> PRT
<213> Dermatophagoides pteronyssinus

<400> 960

Ala Tyr Leu Ala Tyr Arg Asn Gln Ser
1               5


<210> 961
<211> 9
<212> PRT
<213> Dermatophagoides pteronyssinus

<400> 961

Tyr Leu Ala Tyr Arg Asn Gln Ser Leu
1               5


<210> 962
<211> 9
<212> PRT
<213> Dermatophagoides pteronyssinus

<400> 962

Leu Ala Tyr Arg Asn Gln Ser Leu Asp
1               5


<210> 963
<211> 9
<212> PRT
<213> Dermatophagoides pteronyssinus

<400> 963

Ala Tyr Arg Asn Gln Ser Leu Asp Leu

1               5


<210>  964
<211>  9
<212>  PRT
<213>  Dermatophagoides pteronyssinus

<400>  964

Tyr Arg Asn Gln Ser Leu Asp Leu Ala
1               5


<210>  965
<211>  9
<212>  PRT
<213>  Dermatophagoides pteronyssinus

<400>  965

Arg Asn Gln Ser Leu Asp Leu Ala Glu
1               5


<210>  966
<211>  9
<212>  PRT
<213>  Dermatophagoides pteronyssinus

<400>  966

Ser Tyr Tyr Arg Tyr Val Ala Arg Glu
1               5


<210>  967
<211>  9
<212>  PRT
<213>  Dermatophagoides pteronyssinus

<400>  967

Tyr Tyr Arg Tyr Val Ala Arg Glu Gln
1               5


<210>  968
<211>  9
<212>  PRT
<213>  Dermatophagoides pteronyssinus

<400>  968

Tyr Arg Tyr Val Ala Arg Glu Gln Ser
1               5


<210>  969
<211>  9
<212>  PRT
<213>  Dermatophagoides pteronyssinus

<400>  969

Arg Tyr Val Ala Arg Glu Gln Ser Cys
1               5


<210>  970
<211>  9
<212>  PRT
<213>  Dermatophagoides pteronyssinus

<400>  970

Tyr Val Ala Arg Glu Gln Ser Cys Arg
1               5


<210>  971
<211>  9
<212>  PRT
<213>  Dermatophagoides pteronyssinus

<400>  971

Val Ala Arg Glu Gln Ser Cys Arg Arg
1               5


<210>  972
<211>  9
<212>  PRT
<213>  Dermatophagoides pteronyssinus

<400>  972

Ala Arg Glu Gln Ser Cys Arg Arg Pro
1               5


<210>  973
<211>  9
<212>  PRT
<213>  Dermatophagoides pteronyssinus

<400>  973

Arg Glu Gln Ser Cys Arg Arg Pro Asn
1               5


<210>  974
<211>  9
<212>  PRT
<213>  Dermatophagoides pteronyssinus

<400>  974

Glu Gln Ser Cys Arg Arg Pro Asn Ala
1               5


<210>  975
<211>  9

<212>  PRT
<213>  Dermatophagoides pteronyssinus

<400>  975

Gln Ser Cys Arg Arg Pro Asn Ala Gln
1               5


<210>  976
<211>  9
<212>  PRT
<213>  Dermatophagoides pteronyssinus

<400>  976

Gly Tyr Gly Tyr Phe Ala Ala Asn Ile
1               5


<210>  977
<211>  9
<212>  PRT
<213>  Dermatophagoides pteronyssinus

<400>  977

Tyr Gly Tyr Phe Ala Ala Asn Ile Asp
1               5


<210>  978
<211>  9
<212>  PRT
<213>  Dermatophagoides pteronyssinus

<400>  978

Gly Tyr Phe Ala Ala Asn Ile Asp Leu
1               5


<210>  979
<211>  9
<212>  PRT
<213>  Dermatophagoides pteronyssinus

<400>  979

Tyr Phe Ala Ala Asn Ile Asp Leu Met
1               5


<210>  980
<211>  9
<212>  PRT
<213>  Dermatophagoides pteronyssinus

<400>  980

Phe Ala Ala Asn Ile Asp Leu Met Met
1               5

<210>  981
<211>  9
<212>  PRT
<213>  Dermatophagoides pteronyssinus

<400>  981

Ala Ala Asn Ile Asp Leu Met Met Ile
1               5


<210>  982
<211>  9
<212>  PRT
<213>  Dermatophagoides pteronyssinus

<400>  982

Ala Asn Ile Asp Leu Met Met Ile Glu
1               5


<210>  983
<211>  9
<212>  PRT
<213>  Betula pendula

<400>  983

Pro Ala Ala Arg Met Phe Lys Ala Phe
1               5


<210>  984
<211>  9
<212>  PRT
<213>  Betula pendula

<400>  984

Ala Ala Arg Met Phe Lys Ala Phe Ile
1               5


<210>  985
<211>  9
<212>  PRT
<213>  Betula pendula

<400>  985

Ala Arg Met Phe Lys Ala Phe Ile Leu
1               5


<210>  986
<211>  9
<212>  PRT
<213>  Betula pendula

<400>  986

Arg Met Phe Lys Ala Phe Ile Leu Asp
1               5


<210>  987
<211>  9
<212>  PRT
<213>  Betula pendula

<400>  987

Val Phe Asn Tyr Glu Ile Gly Ala Thr
1               5


<210>  988
<211>  9
<212>  PRT
<213>  Betula pendula

<400>  988

Phe Asn Tyr Glu Ile Gly Ala Thr Ser
1               5


<210>  989
<211>  9
<212>  PRT
<213>  Betula pendula

<400>  989

Asn Tyr Glu Ile Gly Ala Thr Ser Val
1               5


<210>  990
<211>  9
<212>  PRT
<213>  Betula pendula

<400>  990

Tyr Glu Ile Gly Ala Thr Ser Val Ile
1               5


<210>  991
<211>  9
<212>  PRT
<213>  Betula pendula

<400>  991

Glu Ile Gly Ala Thr Ser Val Ile Pro
1               5


<210>  992
<211>  9
<212>  PRT
<213>  Betula pendula

<400>  992

Ile Gly Ala Thr Ser Val Ile Pro Ala
1               5


<210>  993
<211>  9
<212>  PRT
<213>  Betula pendula

<400>  993

Gly Ala Thr Ser Val Ile Pro Ala Ala
1               5


<210>  994
<211>  9
<212>  PRT
<213>  Betula pendula

<400>  994

Ser Pro Phe Lys Tyr Val Lys Glu Arg
1               5


<210>  995
<211>  9
<212>  PRT
<213>  Betula pendula

<400>  995

Pro Phe Lys Tyr Val Lys Glu Arg Val
1               5


<210>  996
<211>  9
<212>  PRT
<213>  Betula pendula

<400>  996

Phe Lys Tyr Val Lys Glu Arg Val Asp
1               5


<210>  997
<211>  9
<212>  PRT
<213>  Betula pendula

<400>  997

Lys Tyr Val Lys Glu Arg Val Asp Glu
1               5


<210>  998

<211>  9
<212>  PRT
<213>  Betula pendula

<400>  998

Tyr Val Lys Glu Arg Val Asp Glu Val
1               5


<210>  999
<211>  9
<212>  PRT
<213>  Betula pendula

<400>  999

Val Lys Glu Arg Val Asp Glu Val Asp
1               5


<210>  1000
<211>  9
<212>  PRT
<213>  Betula pendula

<400>  1000

Lys Glu Arg Val Asp Glu Val Asp His
1               5


<210>  1001
<211>  9
<212>  PRT
<213>  Betula pendula

<400>  1001

Asn Phe Lys Tyr Ser Tyr Ser Met Ile
1               5


<210>  1002
<211>  9
<212>  PRT
<213>  Betula pendula

<400>  1002

Phe Lys Tyr Ser Tyr Ser Met Ile Glu
1               5


<210>  1003
<211>  9
<212>  PRT
<213>  Betula pendula

<400>  1003

Lys Tyr Ser Tyr Ser Met Ile Glu Gly
1               5

<210>  1004
<211>  9
<212>  PRT
<213>  Betula pendula

<400>  1004

Tyr Ser Tyr Ser Met Ile Glu Gly Gly
1               5


<210>  1005
<211>  9
<212>  PRT
<213>  Betula pendula

<400>  1005

Ser Tyr Ser Met Ile Glu Gly Gly Ala
1               5


<210>  1006
<211>  9
<212>  PRT
<213>  Betula pendula

<400>  1006

Tyr Ser Met Ile Glu Gly Gly Ala Leu
1               5


<210>  1007
<211>  9
<212>  PRT
<213>  Betula pendula

<400>  1007

Ser Met Ile Glu Gly Gly Ala Leu Gly
1               5


<210>  1008
<211>  9
<212>  PRT
<213>  Betula pendula

<400>  1008

Ala Leu Leu Arg Ala Val Glu Ser Tyr
1               5


<210>  1009
<211>  9
<212>  PRT
<213>  Betula pendula

<400>  1009

Leu Leu Arg Ala Val Glu Ser Tyr Leu
1               5

<210>  1010
<211>  9
<212>  PRT
<213>  Betula pendula

<400>  1010

Leu Arg Ala Val Glu Ser Tyr Leu Leu
1               5

<210>  1011
<211>  9
<212>  PRT
<213>  Betula pendula

<400>  1011

Arg Ala Val Glu Ser Tyr Leu Leu Ala
1               5

<210>  1012
<211>  9
<212>  PRT
<213>  Betula pendula

<400>  1012

Ala Val Glu Ser Tyr Leu Leu Ala His
1               5

<210>  1013
<211>  9
<212>  PRT
<213>  Betula pendula

<400>  1013

Val Glu Ser Tyr Leu Leu Ala His Ser
1               5

<210>  1014
<211>  9
<212>  PRT
<213>  Betula pendula

<400>  1014

Glu Ser Tyr Leu Leu Ala His Ser Asp
1               5

<210>  1015
<211>  9
<212>  PRT

<213>  Betula pendula

<400>  1015

Ser Tyr Leu Leu Ala His Ser Asp Ala
1               5


<210>  1016
<211>  9
<212>  PRT
<213>  Betula pendula

<400>  1016

Tyr Leu Leu Ala His Ser Asp Ala Tyr
1               5


<210>  1017
<211>  9
<212>  PRT
<213>  Phleum pratense

<400>  1017

Gly Asp Glu Gln Lys Leu Arg Ser Ala
1               5


<210>  1018
<211>  9
<212>  PRT
<213>  Phleum pratense

<400>  1018

Asp Glu Gln Lys Leu Arg Ser Ala Gly
1               5


<210>  1019
<211>  9
<212>  PRT
<213>  Phleum pratense

<400>  1019

Glu Gln Lys Leu Arg Ser Ala Gly Glu
1               5


<210>  1020
<211>  9
<212>  PRT
<213>  Phleum pratense

<400>  1020

Gln Lys Leu Arg Ser Ala Gly Glu Leu
1               5

<210>  1021
<211>  9
<212>  PRT
<213>  Phleum pratense

<400>  1021

Lys Leu Arg Ser Ala Gly Glu Leu Glu
1               5


<210>  1022
<211>  9
<212>  PRT
<213>  Phleum pratense

<400>  1022

Leu Arg Ser Ala Gly Glu Leu Glu Leu
1               5


<210>  1023
<211>  9
<212>  PRT
<213>  Phleum pratense

<400>  1023

Arg Ser Ala Gly Glu Leu Glu Leu Gln
1               5


<210>  1024
<211>  9
<212>  PRT
<213>  Phleum pratense

<400>  1024

Asn Tyr Leu Ala Leu Leu Val Lys Tyr
1               5


<210>  1025
<211>  9
<212>  PRT
<213>  Phleum pratense

<400>  1025

Tyr Leu Ala Leu Leu Val Lys Tyr Val
1               5


<210>  1026
<211>  9
<212>  PRT
<213>  Phleum pratense

<400>  1026

Leu Ala Leu Leu Val Lys Tyr Val Asn

1               5


<210>  1027
<211>  9
<212>  PRT
<213>  Phleum pratense

<400>  1027

Ala Leu Leu Val Lys Tyr Val Asn Gly
1               5


<210>  1028
<211>  9
<212>  PRT
<213>  Phleum pratense

<400>  1028

Ser Trp Gly Ala Ile Trp Arg Ile Asp
1               5


<210>  1029
<211>  9
<212>  PRT
<213>  Phleum pratense

<400>  1029

Trp Gly Ala Ile Trp Arg Ile Asp Thr
1               5


<210>  1030
<211>  9
<212>  PRT
<213>  Phleum pratense

<400>  1030

Gly Ala Ile Trp Arg Ile Asp Thr Pro
1               5


<210>  1031
<211>  9
<212>  PRT
<213>  Phleum pratense

<400>  1031

Ala Ile Trp Arg Ile Asp Thr Pro Asp
1               5


<210>  1032
<211>  9
<212>  PRT
<213>  Phleum pratense

<400>  1032

Ile Trp Arg Ile Asp Thr Pro Asp Lys
1               5


<210>  1033
<211>  9
<212>  PRT
<213>  Phleum pratense

<400>  1033

Trp Arg Ile Asp Thr Pro Asp Lys Leu
1               5


<210>  1034
<211>  9
<212>  PRT
<213>  Phleum pratense

<400>  1034

Arg Ile Asp Thr Pro Asp Lys Leu Thr
1               5


<210>  1035
<211>  9
<212>  PRT
<213>  Phleum pratense

<400>  1035

Gly Asp Glu Gln Lys Leu Arg Ser Ala
1               5


<210>  1036
<211>  9
<212>  PRT
<213>  Phleum pratense

<400>  1036

Asp Glu Gln Lys Leu Arg Ser Ala Gly
1               5


<210>  1037
<211>  9
<212>  PRT
<213>  Phleum pratense

<400>  1037

Glu Gln Lys Leu Arg Ser Ala Gly Glu
1               5


<210>  1038
<211>  9

<212>  PRT
<213>  Phleum pratense

<400>  1038

Gln Lys Leu Arg Ser Ala Gly Glu Leu
1               5


<210>  1039
<211>  9
<212>  PRT
<213>  Phleum pratense

<400>  1039

Lys Leu Arg Ser Ala Gly Glu Leu Glu
1               5


<210>  1040
<211>  9
<212>  PRT
<213>  Phleum pratense

<400>  1040

Leu Arg Ser Ala Gly Glu Leu Glu Leu
1               5


<210>  1041
<211>  9
<212>  PRT
<213>  Phleum pratense

<400>  1041

Lys Ile Asn Ala Gly Phe Lys Ala Ala
1               5


<210>  1042
<211>  9
<212>  PRT
<213>  Phleum pratense

<400>  1042

Ile Asn Ala Gly Phe Lys Ala Ala Leu
1               5


<210>  1043
<211>  9
<212>  PRT
<213>  Phleum pratense

<400>  1043

Asn Ala Gly Phe Lys Ala Ala Leu Ala
1               5

<210>  1044
<211>  9
<212>  PRT
<213>  Phleum pratense

<400>  1044

Ala Gly Phe Lys Ala Ala Leu Ala Ala
1               5


<210>  1045
<211>  9
<212>  PRT
<213>  Phleum pratense

<400>  1045

Gly Phe Lys Ala Ala Leu Ala Ala Ala
1               5


<210>  1046
<211>  9
<212>  PRT
<213>  Phleum pratense

<400>  1046

Phe Lys Ala Ala Leu Ala Ala Ala Ala
1               5


<210>  1047
<211>  9
<212>  PRT
<213>  Phleum pratense

<400>  1047

Lys Ala Ala Leu Ala Ala Ala Ala Gly
1               5


<210>  1048
<211>  9
<212>  PRT
<213>  Phleum pratense

<400>  1048

Ala Ala Leu Ala Ala Ala Ala Gly Val
1               5


<210>  1049
<211>  9
<212>  PRT
<213>  Phleum pratense

<400>  1049

Ala Leu Ala Ala Ala Ala Gly Val Gln
1               5


<210>  1050
<211>  9
<212>  PRT
<213>  Phleum pratense

<400>  1050

Lys Tyr Arg Thr Phe Val Ala Thr Phe
1               5


<210>  1051
<211>  9
<212>  PRT
<213>  Phleum pratense

<400>  1051

Tyr Arg Thr Phe Val Ala Thr Phe Gly
1               5


<210>  1052
<211>  9
<212>  PRT
<213>  Phleum pratense

<400>  1052

Arg Thr Phe Val Ala Thr Phe Gly Ala
1               5


<210>  1053
<211>  9
<212>  PRT
<213>  Phleum pratense

<400>  1053

Thr Phe Val Ala Thr Phe Gly Ala Ala
1               5


<210>  1054
<211>  9
<212>  PRT
<213>  Phleum pratense

<400>  1054

Phe Val Ala Thr Phe Gly Ala Ala Ser
1               5


<210>  1055
<211>  9
<212>  PRT
<213>  Phleum pratense

<400>  1055

Val Ala Thr Phe Gly Ala Ala Ser Asn
1               5


<210>  1056
<211>  9
<212>  PRT
<213>  Phleum pratense

<400>  1056

Thr Ser Lys Leu Asp Ala Ala Tyr Lys
1               5


<210>  1057
<211>  9
<212>  PRT
<213>  Phleum pratense

<400>  1057

Ser Lys Leu Asp Ala Ala Tyr Lys Leu
1               5


<210>  1058
<211>  9
<212>  PRT
<213>  Phleum pratense

<400>  1058

Lys Leu Asp Ala Ala Tyr Lys Leu Ala
1               5


<210>  1059
<211>  9
<212>  PRT
<213>  Phleum pratense

<400>  1059

Leu Asp Ala Ala Tyr Lys Leu Ala Tyr
1               5


<210>  1060
<211>  9
<212>  PRT
<213>  Phleum pratense

<400>  1060

Asp Ala Ala Tyr Lys Leu Ala Tyr Lys
1               5


<210>  1061

<211>  9
<212>  PRT
<213>  Phleum pratense

<400>  1061

Ala Ala Tyr Lys Leu Ala Tyr Lys Thr
1               5


<210>  1062
<211>  9
<212>  PRT
<213>  Phleum pratense

<400>  1062

Ala Tyr Lys Leu Ala Tyr Lys Thr Ala
1               5


<210>  1063
<211>  9
<212>  PRT
<213>  Phleum pratense

<400>  1063

Tyr Lys Leu Ala Tyr Lys Thr Ala Glu
1               5


<210>  1064
<211>  9
<212>  PRT
<213>  Phleum pratense

<400>  1064

Lys Leu Ala Tyr Lys Thr Ala Glu Gly
1               5


<210>  1065
<211>  9
<212>  PRT
<213>  Phleum pratense

<400>  1065

Leu Ala Tyr Lys Thr Ala Glu Gly Ala
1               5


<210>  1066
<211>  9
<212>  PRT
<213>  Phleum pratense

<400>  1066

Lys Val Asp Ala Ala Phe Lys Val Ala
1               5

<210>  1067
<211>  9
<212>  PRT
<213>  Phleum pratense

<400>  1067

Val Asp Ala Ala Phe Lys Val Ala Ala
1               5


<210>  1068
<211>  9
<212>  PRT
<213>  Phleum pratense

<400>  1068

Asp Ala Ala Phe Lys Val Ala Ala Thr
1               5


<210>  1069
<211>  9
<212>  PRT
<213>  Phleum pratense

<400>  1069

Ala Ala Phe Lys Val Ala Ala Thr Ala
1               5


<210>  1070
<211>  9
<212>  PRT
<213>  Phleum pratense

<400>  1070

Ala Phe Lys Val Ala Ala Thr Ala Ala
1               5


<210>  1071
<211>  9
<212>  PRT
<213>  Phleum pratense

<400>  1071

Phe Lys Val Ala Ala Thr Ala Ala Asn
1               5


<210>  1072
<211>  8
<212>  PRT
<213>  Phleum pratense

<400>  1072

Lys Val Ala Ala Thr Ala Ala Asn
1               5


<210>  1073
<211>  9
<212>  PRT
<213>  Phleum pratense

<400>  1073

Val Ala Ala Thr Ala Ala Asn Ala Ala
1               5


<210>  1074
<211>  9
<212>  PRT
<213>  Phleum pratense

<400>  1074

Ala Ala Thr Ala Ala Asn Ala Ala Pro
1               5


<210>  1075
<211>  9
<212>  PRT
<213>  Phleum pratense

<400>  1075

Ser Tyr Lys Phe Ile Pro Ala Leu Glu
1               5


<210>  1076
<211>  9
<212>  PRT
<213>  Phleum pratense

<400>  1076

Tyr Lys Phe Ile Pro Ala Leu Glu Ala
1               5


<210>  1077
<211>  9
<212>  PRT
<213>  Phleum pratense

<400>  1077

Lys Phe Ile Pro Ala Leu Glu Ala Ala
1               5


<210>  1078
<211>  9
<212>  PRT

<213>  Phleum pratense

<400>  1078

Phe Ile Pro Ala Leu Glu Ala Ala Val
1               5


<210>  1079
<211>  9
<212>  PRT
<213>  Phleum pratense

<400>  1079

Ile Pro Ala Leu Glu Ala Ala Val Lys
1               5


<210>  1080
<211>  9
<212>  PRT
<213>  Phleum pratense

<400>  1080

Pro Ala Leu Glu Ala Ala Val Lys Gln
1               5


<210>  1081
<211>  9
<212>  PRT
<213>  Phleum pratense

<400>  1081

Ala Leu Glu Ala Ala Val Lys Gln Ala
1               5


<210>  1082
<211>  9
<212>  PRT
<213>  Phleum pratense

<400>  1082

Leu Glu Ala Ala Val Lys Gln Ala Tyr
1               5


<210>  1083
<211>  9
<212>  PRT
<213>  Phleum pratense

<400>  1083

Glu Ala Ala Val Lys Gln Ala Tyr Ala
1               5

<210>  1084
<211>  9
<212>  PRT
<213>  Phleum pratense

<400>  1084

Lys Tyr Thr Val Phe Glu Thr Ala Leu
1               5


<210>  1085
<211>  9
<212>  PRT
<213>  Phleum pratense

<400>  1085

Tyr Thr Val Phe Glu Thr Ala Leu Lys
1               5


<210>  1086
<211>  9
<212>  PRT
<213>  Phleum pratense

<400>  1086

Thr Val Phe Glu Thr Ala Leu Lys Lys
1               5


<210>  1087
<211>  9
<212>  PRT
<213>  Phleum pratense

<400>  1087

Val Phe Glu Thr Ala Leu Lys Lys Ala
1               5


<210>  1088
<211>  9
<212>  PRT
<213>  Phleum pratense

<400>  1088

Phe Glu Thr Ala Leu Lys Lys Ala Ile
1               5


<210>  1089
<211>  9
<212>  PRT
<213>  Phleum pratense

<400>  1089

Glu Thr Ala Leu Lys Lys Ala Ile Thr

1               5


<210>  1090
<211>  9
<212>  PRT
<213>  Phleum pratense

<400>  1090

Thr Ala Leu Lys Lys Ala Ile Thr Ala
1               5


<210>  1091
<211>  9
<212>  PRT
<213>  Phleum pratense

<400>  1091

Ala Leu Lys Lys Ala Ile Thr Ala Met
1               5


<210>  1092
<211>  9
<212>  PRT
<213>  Phleum pratense

<400>  1092

Leu Lys Lys Ala Ile Thr Ala Met Ser
1               5


<210>  1093
<211>  9
<212>  PRT
<213>  Phleum pratense

<400>  1093

Lys Lys Ala Ile Thr Ala Met Ser Glu
1               5


<210>  1094
<211>  9
<212>  PRT
<213>  Alternaria alternata

<400>  1094

Gln Leu Leu Met Leu Ser Ala Lys Arg
1               5


<210>  1095
<211>  9
<212>  PRT
<213>  Alternaria alternata

<400>  1095

Leu Leu Met Leu Ser Ala Lys Arg Met
1               5


<210>  1096
<211>  9
<212>  PRT
<213>  Alternaria alternata

<400>  1096

Leu Met Leu Ser Ala Lys Arg Met Lys
1               5


<210>  1097
<211>  9
<212>  PRT
<213>  Alternaria alternata

<400>  1097

Met Leu Ser Ala Lys Arg Met Lys Val
1               5


<210>  1098
<211>  9
<212>  PRT
<213>  Alternaria alternata

<400>  1098

Leu Ser Ala Lys Arg Met Lys Val Ala
1               5


<210>  1099
<211>  9
<212>  PRT
<213>  Alternaria alternata

<400>  1099

Ser Ala Lys Arg Met Lys Val Ala Phe
1               5


<210>  1100
<211>  9
<212>  PRT
<213>  Alternaria alternata

<400>  1100

Ala Lys Arg Met Lys Val Ala Phe Lys
1               5


<210>  1101
<211>  9

<212>  PRT
<213>  Alternaria alternata

<400>  1101

Lys Arg Met Lys Val Ala Phe Lys Leu
1               5


<210>  1102
<211>  9
<212>  PRT
<213>  Alternaria alternata

<400>  1102

Arg Met Lys Val Ala Phe Lys Leu Asp
1               5


<210>  1103
<211>  9
<212>  PRT
<213>  Alternaria alternata

<400>  1103

Met Lys Val Ala Phe Lys Leu Asp Ile
1               5


<210>  1104
<211>  9
<212>  PRT
<213>  Alternaria alternata

<400>  1104

Lys Val Ala Phe Lys Leu Asp Ile Glu
1               5


<210>  1105
<211>  9
<212>  PRT
<213>  Alternaria alternata

<400>  1105

Gly Phe Lys Arg Cys Leu Gln Phe Thr
1               5


<210>  1106
<211>  9
<212>  PRT
<213>  Alternaria alternata

<400>  1106

Phe Lys Arg Cys Leu Gln Phe Thr Leu
1               5

<210>  1107
<211>  9
<212>  PRT
<213>  Alternaria alternata

<400>  1107

Lys Arg Cys Leu Gln Phe Thr Leu Tyr
1               5


<210>  1108
<211>  9
<212>  PRT
<213>  Alternaria alternata

<400>  1108

Arg Cys Leu Gln Phe Thr Leu Tyr Arg
1               5


<210>  1109
<211>  9
<212>  PRT
<213>  Alternaria alternata

<400>  1109

Cys Leu Gln Phe Thr Leu Tyr Arg Pro
1               5


<210>  1110
<211>  9
<212>  PRT
<213>  Alternaria alternata

<400>  1110

Leu Gln Phe Thr Leu Tyr Arg Pro Arg
1               5


<210>  1111
<211>  9
<212>  PRT
<213>  Alternaria alternata

<400>  1111

Gln Phe Thr Leu Tyr Arg Pro Arg Asp
1               5


<210>  1112
<211>  9
<212>  PRT
<213>  Alternaria alternata

<400>  1112

Phe Thr Leu Tyr Arg Pro Arg Asp Leu
1               5


<210>  1113
<211>  9
<212>  PRT
<213>  Alternaria alternata

<400>  1113

Thr Leu Tyr Arg Pro Arg Asp Leu Leu
1               5


<210>  1114
<211>  9
<212>  PRT
<213>  Alternaria alternata

<400>  1114

Leu Tyr Arg Pro Arg Asp Leu Leu Ser
1               5


<210>  1115
<211>  9
<212>  PRT
<213>  Alternaria alternata

<400>  1115

Tyr Arg Pro Arg Asp Leu Leu Ser Leu
1               5


<210>  1116
<211>  9
<212>  PRT
<213>  Alternaria alternata

<400>  1116

Arg Pro Arg Asp Leu Leu Ser Leu Leu
1               5


<210>  1117
<211>  9
<212>  PRT
<213>  Alternaria alternata

<400>  1117

Thr Tyr Tyr Asn Ser Leu Gly Phe Asn
1               5


<210>  1118
<211>  9
<212>  PRT
<213>  Alternaria alternata

<400>  1118

Tyr Tyr Asn Ser Leu Gly Phe Asn Ile
1               5


<210>  1119
<211>  9
<212>  PRT
<213>  Alternaria alternata

<400>  1119

Tyr Asn Ser Leu Gly Phe Asn Ile Lys
1               5


<210>  1120
<211>  9
<212>  PRT
<213>  Alternaria alternata

<400>  1120

Asn Ser Leu Gly Phe Asn Ile Lys Ala
1               5


<210>  1121
<211>  9
<212>  PRT
<213>  Alternaria alternata

<400>  1121

Ser Leu Gly Phe Asn Ile Lys Ala Thr
1               5


<210>  1122
<211>  9
<212>  PRT
<213>  Alternaria alternata

<400>  1122

Leu Gly Phe Asn Ile Lys Ala Thr Asn
1               5


<210>  1123
<211>  9
<212>  PRT
<213>  Alternaria alternata

<400>  1123

Gly Phe Asn Ile Lys Ala Thr Asn Gly
1               5


<210>  1124

<211>  9
<212>  PRT
<213>  Alternaria alternata

<400>  1124

Phe Asn Ile Lys Ala Thr Asn Gly Gly
1               5


<210>  1125
<211>  9
<212>  PRT
<213>  Alternaria alternata

<400>  1125

Asn Ile Lys Ala Thr Asn Gly Gly Thr
1               5


<210>  1126
<211>  9
<212>  PRT
<213>  Alternaria alternata

<400>  1126

Ile Lys Ala Thr Asn Gly Gly Thr Leu
1               5


<210>  1127
<211>  9
<212>  PRT
<213>  Alternaria alternata

<400>  1127

Lys Ala Thr Asn Gly Gly Thr Leu Asp
1               5


<210>  1128
<211>  9
<212>  PRT
<213>  Alternaria alternata

<400>  1128

Asp Ile Thr Tyr Val Ala Thr Ala Thr
1               5


<210>  1129
<211>  9
<212>  PRT
<213>  Alternaria alternata

<400>  1129

Ile Thr Tyr Val Ala Thr Ala Thr Leu
1               5

<210>  1130
<211>  9
<212>  PRT
<213>  Alternaria alternata

<400>  1130

Thr Tyr Val Ala Thr Ala Thr Leu Pro
1               5


<210>  1131
<211>  9
<212>  PRT
<213>  Alternaria alternata

<400>  1131

Tyr Val Ala Thr Ala Thr Leu Pro Asn
1               5


<210>  1132
<211>  9
<212>  PRT
<213>  Alternaria alternata

<400>  1132

Val Ala Thr Ala Thr Leu Pro Asn Tyr
1               5


<210>  1133
<211>  9
<212>  PRT
<213>  Alternaria alternata

<400>  1133

Ala Thr Ala Thr Leu Pro Asn Tyr Cys
1               5


<210>  1134
<211>  9
<212>  PRT
<213>  Alternaria alternata

<400>  1134

Ala Tyr Ile Thr Leu Val Thr Leu Pro
1               5


<210>  1135
<211>  9
<212>  PRT
<213>  Alternaria alternata

<400>  1135

Tyr Ile Thr Leu Val Thr Leu Pro Lys
1               5

<210>  1136
<211>  9
<212>  PRT
<213>  Alternaria alternata

<400>  1136

Ile Thr Leu Val Thr Leu Pro Lys Ser
1               5

<210>  1137
<211>  9
<212>  PRT
<213>  Alternaria alternata

<400>  1137

Thr Leu Val Thr Leu Pro Lys Ser Ser
1               5

<210>  1138
<211>  9
<212>  PRT
<213>  Alternaria alternata

<400>  1138

Glu Val Tyr Gln Lys Leu Lys Ala Leu
1               5

<210>  1139
<211>  9
<212>  PRT
<213>  Alternaria alternata

<400>  1139

Val Tyr Gln Lys Leu Lys Ala Leu Ala
1               5

<210>  1140
<211>  9
<212>  PRT
<213>  Alternaria alternata

<400>  1140

Tyr Gln Lys Leu Lys Ala Leu Ala Lys
1               5

<210>  1141
<211>  9
<212>  PRT

<213>  Alternaria alternata

<400>  1141

Gln Lys Leu Lys Ala Leu Ala Lys Lys
1               5


<210>  1142
<211>  9
<212>  PRT
<213>  Alternaria alternata

<400>  1142

Lys Leu Lys Ala Leu Ala Lys Lys Thr
1               5


<210>  1143
<211>  9
<212>  PRT
<213>  Alternaria alternata

<400>  1143

Leu Lys Ala Leu Ala Lys Lys Thr Tyr
1               5


<210>  1144
<211>  9
<212>  PRT
<213>  Alternaria alternata

<400>  1144

Lys Ala Leu Ala Lys Lys Thr Tyr Gly
1               5


<210>  1145
<211>  9
<212>  PRT
<213>  Alternaria alternata

<400>  1145

Gly Tyr Thr Gly Lys Ile Lys Ile Ala
1               5


<210>  1146
<211>  9
<212>  PRT
<213>  Alternaria alternata

<400>  1146

Tyr Thr Gly Lys Ile Lys Ile Ala Met
1               5

<210>  1147
<211>  9
<212>  PRT
<213>  Alternaria alternata

<400>  1147

Thr Gly Lys Ile Lys Ile Ala Met Asp
1               5


<210>  1148
<211>  9
<212>  PRT
<213>  Alternaria alternata

<400>  1148

Gly Lys Ile Lys Ile Ala Met Asp Val
1               5


<210>  1149
<211>  9
<212>  PRT
<213>  Alternaria alternata

<400>  1149

Lys Ile Lys Ile Ala Met Asp Val Ala
1               5


<210>  1150
<211>  9
<212>  PRT
<213>  Alternaria alternata

<400>  1150

Ile Lys Ile Ala Met Asp Val Ala Ser
1               5


<210>  1151
<211>  9
<212>  PRT
<213>  Alternaria alternata

<400>  1151

Lys Ile Ala Met Asp Val Ala Ser Ser
1               5


<210>  1152
<211>  9
<212>  PRT
<213>  Alternaria alternata

<400>  1152

Ile Ala Met Asp Val Ala Ser Ser Glu

1               5


<210>  1153
<211>  9
<212>  PRT
<213>  Alternaria alternata

<400>  1153

Ala Met Asp Val Ala Ser Ser Glu Phe
1               5


<210>  1154
<211>  9
<212>  PRT
<213>  Alternaria alternata

<400>  1154

Ala Phe Gly Ala Gly Trp Gly Val Met
1               5


<210>  1155
<211>  9
<212>  PRT
<213>  Alternaria alternata

<400>  1155

Phe Gly Ala Gly Trp Gly Val Met Val
1               5


<210>  1156
<211>  9
<212>  PRT
<213>  Alternaria alternata

<400>  1156

Gly Ala Gly Trp Gly Val Met Val Ser
1               5


<210>  1157
<211>  9
<212>  PRT
<213>  Alternaria alternata

<400>  1157

Ala Gly Trp Gly Val Met Val Ser His
1               5


<210>  1158
<211>  9
<212>  PRT
<213>  Alternaria alternata

<400>  1158

Gly Trp Gly Val Met Val Ser His Arg
1               5


<210>  1159
<211>  9
<212>  PRT
<213>  Alternaria alternata

<400>  1159

Trp Gly Val Met Val Ser His Arg Ser
1               5


<210>  1160
<211>  9
<212>  PRT
<213>  Alternaria alternata

<400>  1160

Gly Val Met Val Ser His Arg Ser Gly
1               5


<210>  1161
<211>  9
<212>  PRT
<213>  Alternaria alternata

<400>  1161

Val Met Val Ser His Arg Ser Gly Glu
1               5


<210>  1162
<211>  9
<212>  PRT
<213>  Alternaria alternata

<400>  1162

Met Val Ser His Arg Ser Gly Glu Thr
1               5


<210>  1163
<211>  9
<212>  PRT
<213>  Alternaria alternata

<400>  1163

Val Ser His Arg Ser Gly Glu Thr Glu
1               5

## Description

### Background to the Invention

*Aspergillus* and other infectious airborne fungi such as species of *Alternaria* (e.g. *Alternaria alternata*) are major causes of fungal allergy disease. For example, hypersensitivity to *Aspergillus* allergens may result in IgE-mediated asthma (2), allergic sinusitis or rhinitis or, in severe situations, ABPA. The allergens may be fungal proteins, but what determines which proteins are allergens is not known. Existing treatment of fungal allergy is through use of steroids or small-molecule anti-fungal agents. These are aimed at either relieving the symptoms of the allergy, e.g. allergic rhinitis, difficulty breathing, swelling, itchiness, or at reducing or controlling the extent of fungal infection. Fungal allergens can be grouped into several categories including proteases, glycosidases, components of protein production, oxidative stress response proteins and enzymes involved in gluconeogenesis or the pentose phosphate shunt (1).

In addition to acting as an allergen fungi such as *Aspergillus* and *Alternaria* are capable of active germination in the host leading to infection. This is in contrast to other allergens such as house dust mites, cat dander or grass pollen. Thus, fungal proteins can act as allergens and also cause airway damage through infection. They have also been reported to induce a 'bystander-effect' by enhancing the allergenic potential of other proteins (1).

### SAFS

Fungal infection of the lungs can lead to an allergic condition known as Severe Asthma with Fungal Sensitisation (SAFS) (1). This term was proposed by Denning et al (1) for patients having persistent severe or brittle asthma (despite treatment) and evidence of fungal sensitisation, as defined by positive prick testing, or fungus or fungal antigen-specific blood IgE testing, and who do not meet the criteria for ABPA.

Patients with SAFS have acute asthma with impaired pulmonary function. SAFS patients may present with nasal symptoms such as runny nose, sneezing and hay fever-like symptoms but do not produce plugs of sputum as seen in ABPA patients. CT scans may show mucous in the airways and eosinophilia is common.

SAFS diagnosis criteria are: (i) severe asthma (British Thoracic Society step 4 or worse), (ii) exclusion of ABPA (total IgE <1000IU/mL), (iii) evidence of sensitisation to one or more fungi, by skin prick test or RAST test.

Some patients are sensitised to many fungi, but most only react to one or two fungi. Common fungi that SAFS patients are sensitised to include: *Aspergillus fumigatus, Candida albicans, Alternaria alternata, Trichopyton* spp, *Cladosporium herbarum, Penicillium chrysogenum* and *Botrytis cinerea.*

Existing modes of treatment for patients with SAFS usually consist of multiple medication programmes. Inhaled and oral corticosteroids are used for long term control of the most severe symptoms but are associated with adverse events over the longer term, e.g. osteoporosis and weight gain. Short or long-acting beta-2 agonists or leukotriene antagonists may be used with some benefits.

Treatment with the anti-fungal itraconazole has some beneficial effect on pulmonary and nasal symptoms. Drug monitoring is required to optimise drug exposure and this may require switching between drug formulations and changes in the dosage amount.

### ABPA

Allergic Bronchopulmonary Aspergillosis (ABPA) is an allergic condition produced in response to *Aspergillus.* It is common in asthmatics. Symptoms are similar to those of asthma with intermittent episodes of coughing and wheezing. Some patients cough up brown plugs of mucus. Diagnosis can be made by X-ray or by sputum, skin and blood tests. In the longer term, and particularly if untreated, ABPA can lead to permanent lung damage through fibrosis.

Existing treatment is through use of steroids such as prednisolone by aerosol or oral administration but this has side-effects over the longer term as described above in respect of the use of steroids in SAFS treatment. Itraconazole can be used to reduce the amount of steroid required.

### Immunotherapy

Conventional immunotherapy for allergic disease has contemplated administration of whole protein allergens, but this often requires years of injections for successful desensitization and can lead to severe side-effects caused by IgE mediated allergic reactions, including anaphylaxis. Peptide based immunotherapy has been proposed as a vaccine treatment for cat allergy (3, 13). Failure to account for peptide MHC restriction has led to limited success (4).

A principal feature of MHC molecules is their allelic polymorphism, at least 707 class II molecules are known. MHC alleles have arisen under evolutionary pressure resulting in geographical diversity. Any poly-epitope vaccine targeting the whole population would need to bind a range of HLA molecules. MHC polymorphism thus greatly complicates epitope-based vaccine development, particularly in regard to population coverage (15).

Peptide-based allergy vaccines may be safer because they do not contain IgE-binding epitopes capable of crosslinking IgE. However, designing an effective peptide vaccine for human immunization is complicated by the polymorphism of the human MHC molecules and the antigenic complexity of allergens. The diversity of immunodominant sequences recognized in the context of the widely polymorphic HLA genotypes in man represents an obstacle for the development of synthetic peptides as potential diagnostic tools or vaccines (14). This points towards the need for a peptide cocktail in order to provide allergy protection for a majority of human patients (13).

### Summary of the Invention

The inventors have identified peptides proposed to be useful in immunotherapy.

The peptides are preferably T-cell epitopes capable of binding human or animal HLA-DR molecules and stimulating an immune response. The peptides are preferably epitopes of fungal proteins. In particular, the peptides are preferably T-cell epitopes identified from *Alternaria alternata* proteins.

Modified peptides are also provided in which the wild type fungal peptide epitope amino acid sequence has been modified but still retains its ability to stimulate an immune response.

Accordingly, the present invention provides therapeutic compositions and methods for treating disease conditions in humans and animals associated with an antigen specific immune response by the human or animal to an antigen such as a protein antigen.

In accordance with the above, in one aspect of the present invention a peptide is provided, the peptide being chosen from one of:

| **SEQ ID NO:** | **Peptide** | **SEQ ID NO:** | **Peptide** |
|---|---|---|---|
| 1-3 | F T T I A S L F A | 30 | LRIINEPTA |
| | MQFTTIASL | | |
| | IASLFAAAG | | |
| 4 | LAAAAPLES | 31 | LRRLRTACE |
| 5 | WKISEFYGR | 32 | IQGFPTIKL |
| 6 | YYNSLGFNI | 33 | VVGLRSGQI |
| 7 | LGFNIKATN | 34 | FVSTGTLGG |
| 8 | FNIKATNGG | 35 | VVIVTGASG |
| 9 | IKATNGGTL | 36 | LVITSSMSG |
| 10 | ITYVATATL | 37 | LVITSSLSG (*) |
| 11 | YVATATLPN | 38 | IVLLSLCQL |
| 12 | LPNYCRAGG | 39 | IVLLSLVQL (*) |
| 13 | LPNYVRAGG (*) | 40 | FRDDRIEII |
| 14 | FVCQGVADA | 41 | VAMNPVNTV |
| 15 | FVVQGVADA (*) | 42 | VALNPVNTV (*) |
| 16 | YITLVTLPK | 43 | LVTIAKVDA |
| 17 | ITLVTLPKS | 44 | MKHLAAYLL |
| 18 | LFAAAGLAA | 45 | LKHLAAYLL(*) |
| 19 | FAAAGLAAA | 46 | YQKLKALAK |
| 20 | YNSLGFNIK | 47 | LKSCNALLL |
| 21 | YCRAGGNGP | 48 | LKSVNALLL (*) |
| 22 | YVRAGGNGP (*) | 49 | WGVMVSHRS |
| 23 | LDFTCSAQA | 50 | WGVLVSHRS (*) |
| 24 | LDFTVSAQA (*) | 51 | GYKTAFSML |
| 25 | WYSCGENSF | 52 | GYKTAFSLL (*) |
| 26 | WYSVGENSF (*) | 53 | FVPQDIQKL |
| 27 | VSDDITYVA | 54 | YVYFASDAS |
| 28 | LVNHFTNEF | 55 | YFIEPTIFS |
| 29 | ILLLDVAPL | 56 | YIQTKTVSI |
| | | 57 | IRLGDVLFG |
| | | 58 | WVNSYNTLH |

In the above, SEQ ID NOS: 13, 15, 22, 24, 26, 37, 39, 42, 45, 48, 50 and 52 (indicated by (*)) are Cys/Val and/or Met/Leu substitution variants of wild type SEQ ID NOS: 12, 14, 21, 23, 25, 36, 38, 41, 44, 47, 49 and 51, respectively. In some aspects, SEQ ID NOs: 13, 15, 22, 24, 26, 37, 39, 42, 45, 48, 50 and 52 are preferred compared to the respective corresponding sequence selected from one of SEQ ID NOs: 12, 14, 21, 23, 25, 36, 38, 41, 44, 47, 49 and 51.

In another aspect of the present invention a peptide is provided which peptide consists of or comprises the sequence of one of SEQ ID NOs: 1-58. The amino acid sequence of the selected SEQ ID NO is preferably included in the peptide as a contiguous amino acid sequence.

In another aspect of the present invention a peptide is provided having at least 60% amino acid sequence identity to one of SEQ ID NOs:1-58. More preferably, the degree of sequence identity is one of 65%, 70%, 75%, 80%, 85%, 87%, 90%, 91%, 92%, 93%, 94%, 95%, 96%, 97%, 98%, 99% or 100% identity.

The minimum epitope for HLA DR recognition may be any of 7-11 amino acids in length and is typically a 9-mer epitope. Improved binding may be afforded by including at least one, two or three amino acids at one or both ends of the minimum epitope. Accordingly, peptides are provided as part of the present invention having a core amino acid sequence of any one of SEQ ID NOs:1-58 as well as an additional one, two, three, four, five, six (or more) amino acids of any type or combination at the N-terminal end, C-terminal end or at both the N- and C- terminal ends of the sequence. For example, a peptide may have a core amino acid sequence of any one of SEQ ID NOS:1-58 as well as an additional 1, 2, 3, 4, 5, 6, 7, 8, 9, 10, 11, 12, 13, 14, 15, amino acids at the N-terminal end, C-terminal end or at both the N- and C-terminal ends of the sequence.

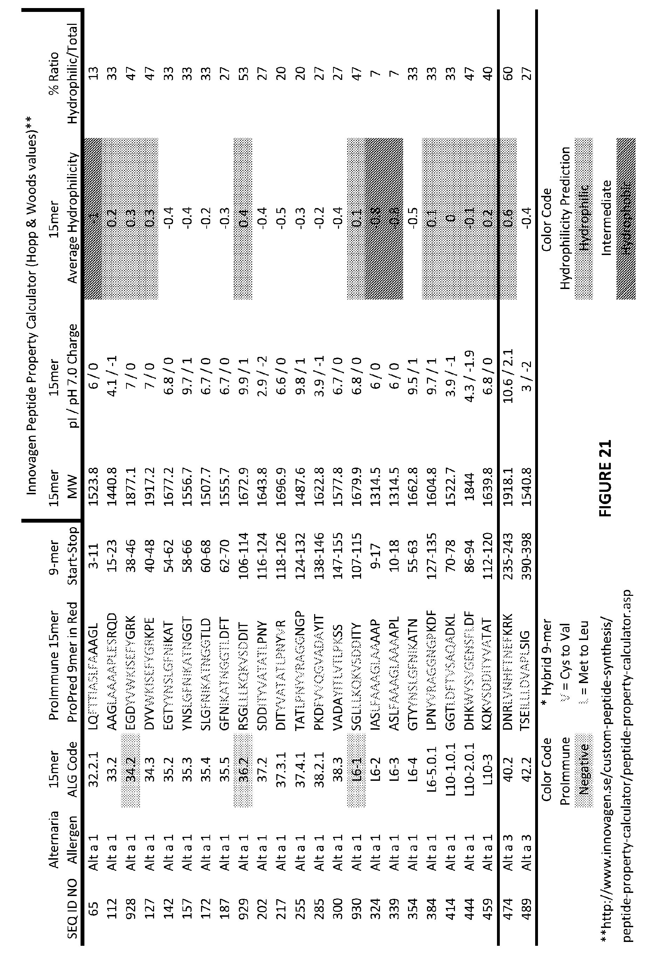

The additional amino acids preferably correspond to amino acids from the parent protein amino acid sequence from which the peptide is derived, i.e. the wild-type amino acid sequence of the protein. For example, SEQ ID NOs:65, 112, 127, 142, 157, 172, 187, 202, 217, 255, 285, 300, 324, 359, 354, 384, 414, 444 and 459 are from the Alt a 1 protein (the position of the peptide in the Alt a 1 polypeptide is indicated in Figure 21). In the parent protein (Alt a 1) the N-terminal Leucine of SEQ ID NO:112 is preceded by amino acids AAG and the C-terminal Serine is followed by amino acids RQD.

In some instances, addition of amino acids corresponding to those in the parent protein sequence in this way results in an unstable amino acid, e.g. cysteine (C), occurring at the N- and/or the C-terminal end of the peptide. In such cases, an unstable amino acid may be substituted by a more stable amino acid. For example, a C/V and/or M/L substitution may be made (see, for example, SEQ ID NOs:217, 255, 285, 384, 414, 444, 609, 639, 654, 688, 718, 760, 790, 820, 850).

In one aspect of the present invention a peptide is provided, the peptide consisting of or comprising the amino acid sequence of one of SEQ ID NOs:1-913 or a peptide having a contiguous amino acid sequence having at least 70% sequence identity to the amino acid sequence of one of SEQ ID NOs: 1-913, wherein the peptide has an amino acid length of from 8 to 50 amino acids.

The degree of sequence identity may be chosen from one of 80%, 85%, 90% or 95%. The peptide may have a maximum length of 30 amino acids and a minimum length of 9 amino acids, or a maximum length of 20 amino acids and a minimum length of 11 amino acids, or a maximum length of 15 amino acids and a minimum length of 9 amino acids, or a maximum length of 11 amino acids and a minimum length of 8 amino acids, or a length of 9 or 15 amino acids. The peptide is preferably capable of stimulating an immune response.

In one aspect of the present invention a peptide is provided comprising the amino acid sequence of one of SEQ ID NOs:1-58 or a peptide having a contiguous amino acid sequence having at least 80% sequence identity to the amino acid sequence of one of SEQ ID NOs:1-58, wherein the peptide has an amino acid length of from 8 to 50 amino acids.

In one aspect of the present invention a peptide is provided, being chosen from one of:
(i) SEQ ID NO: 1, SEQ ID NOs: 59-80; or
(ii) SEQ ID NO: 2, SEQ ID NOs: 81-92; or
(iii) SEQ ID NO: 3, SEQ ID NOs: 93-105; or
(iv) SEQ ID NO: 4, SEQ ID NOs: 106-120; or
(v) SEQ ID NO: 5, SEQ ID NOs: 121-135; or
(vi) SEQ ID NO: 6, SEQ ID NOs: 136-150; or
(vii) SEQ ID NO: 7, SEQ ID NOs: 151-165; or
(viii) SEQ ID NO: 8, SEQ ID NOs: 166-180; or
(ix) SEQ ID NO: 9, SEQ ID NOs: 181-195; or
(x) SEQ ID NO: 10, SEQ ID NOs: 196-210; or
(xi) SEQ ID NO: 11, SEQ ID NOs: 211-233; or
(xii) SEQ ID NO: 12, SEQ ID NOs: 234-248; or
(xiii) SEQ ID NO: 13, SEQ ID NOs: 249-263; or
(xiv) SEQ ID NO: 14, SEQ ID NOs: 264-278; or
(xv) SEQ ID NO: 15, SEQ ID NOs: 279-293; or
(xvi) SEQ ID NO: 16, SEQ ID NOs: 294-307; or
(xvii) SEQ ID NO: 17, SEQ ID NOs: 308-317 ;or
(xviii) SEQ ID NO: 18, SEQ ID NOs: 318-332 ;or
(xix) SEQ ID NO: 19, SEQ ID NOs: 333-347 ;or
(xx) SEQ ID NO: 20, SEQ ID NOs: 348-362 ;or
(xxi) SEQ ID NO: 21, SEQ ID NOs: 363-377 ;or
(xxii) SEQ ID NO: 22, SEQ ID NOs: 378-392 ;or
(xxiii) SEQ ID NO: 23, SEQ ID NOs: 393-407 ;or
(xxiv) SEQ ID NO: 24, SEQ ID NOs: 408-422 ;or
(xxv) SEQ ID NO: 25, SEQ ID NOs: 423-437 ;or
(xxvi) SEQ ID NO: 26, SEQ ID NOs: 438-452 ;or
(xxvii) SEQ ID NO: 27, SEQ ID NOs: 453-467 ;or
(xxviii) SEQ ID NO: 28, SEQ ID NOs: 468-482 ;or
(xxix) SEQ ID NO: 29, SEQ ID NOs: 483-497 ;or
(xxx) SEQ ID NO: 30, SEQ ID NOs: 498-512 ;or
(xxxi) SEQ ID NO: 31, SEQ ID NOs: 513-527 ;or
(xxxii) SEQ ID NO: 32, SEQ ID NOs: 528-542 ;or
(xxxiii) SEQ ID NO: 33, SEQ ID NOs: 543-557 ;or
(xxxiv) SEQ ID NO: 34, SEQ ID NOs: 558-572 ;or
(xxxv) SEQ ID NO: 35, SEQ ID NOs: 573-587;or
(xxxvi) SEQ ID NO: 36, SEQ ID NOs: 588-602 ;or
(xxxvii) SEQ ID NO: 37, SEQ ID NOs: 603-617;or
(xxxviii) SEQ ID NO: 38, SEQ ID NOs: 618-632 ;or
(xxxix) SEQ ID NO: 39, SEQ ID NOs: 633-647 ;or
(xl) SEQ ID NO: 40, SEQ ID NOs: 648-662 ;or
(xli) SEQ ID NO: 41, SEQ ID NOs: 667-681 ;or
(xlii) SEQ ID NO: 42, SEQ ID NOs: 682-696 ;or
(xliii) SEQ ID NO: 43, SEQ ID NOs: 697-711 ;or
(xliv) SEQ ID NO: 44, SEQ ID NOs: 712-717 ;or
(xlv) SEQ ID NO: 45, SEQ ID NOs: 718-723 ;or
(xlvi) SEQ ID NO: 46, SEQ ID NOs: 724-738;or
(xlvii) SEQ ID NO: 47, SEQ ID NOs: 739-753 ;or
(xlviii) SEQ ID NO: 48, SEQ ID NOs: 754-768 ;or
(xlix) SEQ ID NO: 49, SEQ ID NOs: 769-783 ;or
(I) SEQ ID NO: 50, SEQ ID NOs: 784-798 ;or
(Ii) SEQ ID NO: 51, SEQ ID NOs: 799-813 ;or
(Iii) SEQ ID NO: 52, SEQ ID NOs: 814-828 ;or
(Iiii) SEQ ID NO: 53, SEQ ID NOs: 829-843 ;or
(liv) SEQ ID NO: 54, SEQ ID NOs: 844-862 ;or
(Iv) SEQ ID NO: 55, SEQ ID NOs: 863-877 ;or
(Ivi) SEQ ID NO: 56, SEQ ID NOs: 878-892 ;or
(Ivii) SEQ ID NO: 57, SEQ ID NOs: 893-898 ;or
(Iviii) SEQ ID NO: 58, SEQ ID NOs: 899-913.

In one aspect of the present invention a pharmaceutical composition is provided, the pharmaceutical composition comprising a peptide according to any of the aspects and embodiments described herein. The pharmaceutical composition may further comprise a pharmaceutically acceptable carrier, adjuvant or diluent. The pharmaceutical composition may be a vaccine.

In some aspects of the present invention the peptides and/or pharmaceutical compositions are provided for use in the prevention or treatment of disease. The disease may be an allergic disease. The disease may be chosen from fungal allergy, fungal asthma, fungal infection, SAFS, ABPA, or Aspergillosis. The disease may be an allergic disease caused by an *Alternaria alternata* protein allergen or by infection of tissue by *Alternaria alternata.*

In another aspect of the present invention a method of treating or preventing disease in a patient in need of treatment thereof is provided, the method comprising administering to the patient a therapeutically effective amount of a peptide or pharmaceutical composition according to any one of the aspects and embodiments described herein.

In another aspect of the present invention a method for the production of a pharmaceutical composition is provided, the method comprising providing a peptide according to any one of the aspects and embodiments described herein, and mixing the peptide with a pharmaceutically acceptable carrier, adjuvant or diluent.

In another aspect of the present invention a nucleic acid, preferably an isolated and/or purified nucleic acid, encoding a peptide according to any one of the aspects and embodiments described herein is provided. A cell is also provided, having integrated in its genome a nucleic acid encoding a peptide according to any one of the aspects and embodiments described herein operably linked to a transcription control nucleic acid sequence. A nucleic acid expression vector is also provided having a nucleic acid encoding a peptide according to any one of the aspects and embodiments described herein operably linked to a transcription control nucleic acid sequence, wherein the vector is configured for expression of a peptide according to any one of the aspects and embodiments described herein when transfected into a suitable cell. Accordingly, a cell transfected with the nucleic acid expression vector is also provided.

In another aspect of the present invention a method of identifying a peptide that is capable of stimulating an immune response is provided, the method comprising the steps of:
(i) providing a candidate peptide having a contiguous amino acid sequence having at least 70% sequence identity to the amino acid sequence of one of SEQ ID NOs:1-913, wherein the peptide has an amino acid length of from 8 to 50 amino acids, and
(ii) testing the ability of the candidate peptide to induce an immune response.

Step (i) may comprise providing a peptide having the sequence of one of SEQ ID NOs:1-913 and chemically modifying the structure of the peptide to provide the candidate peptide. Step (ii) may comprise contacting the candidate peptide with a population of T cells in vitro and assaying T cell proliferation. Step (ii) may comprise or further comprise monitoring for production of IL-4 and/or IFNγ.

### Description of Preferred Embodiments

In aspects and embodiments of the present invention a peptide is provided, the peptide comprising or consisting of one of SEQ ID NOs:59-913 set out below. In the sequences shown below, the 9mer peptide of the corresponding sequence selected from one of SEQ ID NOs:1-58 is shown in bold.

| Peptide | SEQ ID NO: |
|---|---|
| LQ**FTTIASLFA** | 59 |
| Q**FTTIASLFA** | 60 |
| **FTTIASLFA**AAGL | 61 |
| **FTTIASLFA**AAG | 62 |
| **FTTIASLFA**AA | 63 |
| **FTTIASLFA**A | 64 |
| LQ**FTTIASLFA**AAGL | 65 |
| Q**FTTIASLFA**AAGL | 66 |
| Q**FTTIASLFA**AAG | 67 |
| Q**FTTIASLFA**AA | 68 |
| Q**FTTIASLFA**A | 69 |
| LQ**FTTIASLFA**AAG | 70 |
| Q**FTTIASLFA**AAG | 71 |
| LQ**FTTIASLFA**AA | 72 |
| Q**FTTIASLFA**AA | 73 |
| LQ**FTTIASLFA**A | 74 |
| Q**FTTIASLFA**A | 75 |
| | |
| MQ**FTTIASLFA** | 76 |
| MQ**FTTIASLFA**AAGL | 77 |
| MQ**FTTIASLFA**AAG | 78 |
| MQ**FTTIASLFA**AA | 79 |
| MQ**FTTIASLFA**A | 80 |

SEQ ID NOs:59-80 correspond to SEQ ID NO:1 in which one, two or three additional contiguous amino acids from the Alt a 1 protein sequence are optionally incorporated at the N- terminus, C-terminus and both N- and C-terminus. An M/L substitution has been made in SEQ ID NOs:59, 65, 70, 73, 74.

| Peptide | SEQ ID NO: |
|---|---|
| **MQFTTIASL**FAAAGL | 81 |
| **MQFTTIASL**FAAAG | 82 |
| **MQFTTIASL**FAAA | 83 |
| **MQFTTIASL**FAA | 84 |
| **MQFTTIASL**FA | 85 |
| **MQFTTIASL**F | 86 |
| | |
| **LQFTTIASL**FAAAGL | 87 |
| **LQFTTIASL**FAAAG | 88 |
| **LQFTTIASL**FAAA | 89 |
| **LQFTTIASL**FAA | 90 |
| **LQFTTIASL**FA | 91 |
| **LQFTTIASL**F | 92 |

SEQ ID NOs:81-92 correspond to SEQ ID NO:2 in which one, two, three, four, five or six additional contiguous amino acids from the Alt a 1 protein sequence are incorporated at the C-terminus. An M/L substitution has been made in SEQ ID NOs:87-92.

| Peptide | SEQ ID NO: |
|---|---|
| LQFTT**IASLFAAAG**L | 93 |
| LQFTT**IASLFAAAG** | 94 |
| QFTT**IASLFAAAG** | 95 |
| FTT**IASLFAAAG** | 96 |
| TT**IASLFAAAG** | 97 |
| T**IASLFAAAG** | 98 |
| QFTT**IASLFAAAG**L | 99 |
| FTT**IASLFAAAG**L | 100 |
| TT**IASLFAAAG**L | 101 |
| T**IASLFAAAG**L | 102 |
| **IASLFAAAG**L | 103 |
| | |
| MQFTT**IASLFAAAG**L | 104 |
| MQFTT**IASLFAAAG** | 105 |

SEQ ID NOs:93-105 correspond to SEQ ID NO:3 in which one, two, three, four or five additional contiguous amino acids from the Alt a 1 protein sequence are optionally incorporated at the N- terminus, and a single amino acid are optionally incorporated C-terminus and optionally at both the N- and C-terminus.

| Peptide | SEQ ID NO: |
|---|---|
| AAG**LAAAAPLES** | 106 |
| AG**LAAAAPLES** | 107 |
| G**LAAAAPLES** | 108 |
| L**AAAAPLES**RQD | 109 |
| **LAAAAPLES**RQ | 110 |
| **LAAAAPLES**R | 111 |
| AAG**LAAAAPLES**RQD | 112 |
| AG**LAAAAPLES**RQD | 113 |
| G**LAAAAPLES**RQD | 114 |
| AAG**LAAAAPLES**RQ | 115 |
| AG**LAAAAPLES**RQ | 116 |
| G**LAAAAPLES**RQ | 117 |
| AAG**LAAAAPLES**R | 118 |
| AG**LAAAAPLES**R | 119 |
| G**LAAAAPLES**R | 120 |

SEQ ID NOs:106-120 correspond to SEQ ID NO:4 in which one, two or three additional contiguous amino acids from the Alt a 1 protein sequence are optionally incorporated at the N- terminus, C-terminus and both N- and C-terminus.

| Peptide | SEQ ID NO: |
|---|---|
| DYV**WKISEFYGR** | 121 |
| YV**WKISEFYGR** | 122 |
| VW**KISEFYGR** | 123 |
| **WKISEFYGR**KPE | 124 |
| **WKISEFYGR**KP | 125 |
| **WKISEFYGR**K | 126 |
| DYV**WKISEFYGR**KPE | 127 |
| DYV**WKISEFYGR**KP | 128 |
| DYV**WKISEFYGR**K | 129 |
| YV**WKISEFYGR**KPE | 130 |
| YV**WKISEFYGR**KP | 131 |
| YV**WKISEFYGR**K | 132 |
| V**WKISEFYGR**KPE | 133 |
| V**WKISEFYGR**KP | 134 |
| V**WKISEFYGR**K | 135 |

SEQ ID NOs:121-135 correspond to SEQ ID NO:5 in which one, two or three additional contiguous amino acids from the Alt a 1 protein sequence are optionally incorporated at the N- terminus, C-terminus and both N- and C-terminus.

| Peptide | SEQ ID NO: |
|---|---|
| EGT**YYNSLGFNI** | 136 |
| GT**YYNSLGFNI** | 137 |
| T**YYNSLGFNI** | 138 |
| **YYNSLGFNI**KAT | 139 |
| **YYNSLGFNI**KA | 140 |
| **YYNSLGFNI**K | 141 |
| EGT**YYNSLGFNI**KAT | 142 |
| EGT**YYNSLGFNI**KA | 143 |
| EGT**YYNSLGFNI**K | 144 |
| GT**YYNSLGFNI**KAT | 145 |
| GT**YYNSLGFNI**KA | 146 |
| GT**YYNSLGFNI**K | 147 |
| T**YYNSLGFNI**KAT | 148 |
| T**YYNSLGFNI**KA | 149 |
| T**YYNSLGFNI**K | 150 |

SEQ ID NOs:136-150 correspond to SEQ ID NO:6 in which one, two or three additional contiguous amino acids from the Alt a 1 protein sequence are optionally incorporated at the N- terminus, C-terminus and both N- and C-terminus.

| Peptide | SEQ ID NO: |
|---|---|
| YNS**LGFNIKATN** | 151 |
| NS**LGFNIKATN** | 152 |
| S**LGFNIKATN** | 153 |
| **LGFNIKATN**GGT | 154 |
| **LGFNIKATN**GG | 155 |
| **LGFNIKATN**G | 156 |
| YNS**LGFNIKATN**GGT | 157 |
| YNS**LGFNIKATN**GG | 158 |
| YNS**LGFNIKATN**G | 159 |
| NS**LGFNIKATN**GGT | 160 |
| NS**LGFNIKATN**GG | 161 |
| NS**LGFNIKATN**G | 162 |
| S**LGFNIKATN**GGT | 163 |
| S**LGFNIKATN**GG | 164 |
| S**LGFNIKATN**G | 165 |

SEQ ID NOs:151-165 correspond to SEQ ID NO:7 in which one, two or three additional contiguous amino acids from the Alt a 1 protein sequence are optionally incorporated at the N- terminus, C-terminus and both N- and C-terminus.

| Peptide | SEQ ID NO: |
|---|---|
| SLG**FNIKATNGG** | 166 |
| LG**FNIKATNGG** | 167 |
| G**FNIKATNGG** | 168 |
| **FNIKATNGGT**LD | 169 |
| **FNIKATNGGT**L | 170 |
| **FNIKATNGGT** | 171 |
| SLG**FNIKATNGG**TLD | 172 |
| SLG**FNIKATNGG**TL | 173 |
| SLG**FNIKATNGG**T | 174 |
| LGF**NIKATNGG**TLD | 175 |
| LGF**NIKATNGG**TL | 176 |
| LGF**NIKATNGG**T | 177 |
| GF**NIKATNGG**TLD | 178 |
| GF**NIKATNGG**TL | 179 |
| GF**NIKATNGG**T | 180 |

SEQ ID NOs:166-180 correspond to SEQ ID NO:8 in which one, two or three additional contiguous amino acids from the Alt a 1 protein sequence are optionally incorporated at the N- terminus, C-terminus and both N- and C-terminus.

| Peptide | SEQ ID NO: |
|---|---|
| GFN**IKATNGG**TL | 181 |
| FN**IKATNGGTL** | 182 |
| N**IKATNGGTL** | 183 |
| **IKATNGGTL**DFT | 184 |
| **IKATNGGTL**DF | 185 |
| **IKATNGGTL**D | 186 |
| GFN**IKATNGGTL**DFT | 187 |
| GFN**IKATNGGTL**DF | 188 |
| GFN**IKATNGGTL**D | 189 |
| FN**IKATNGGTL**DFT | 190 |
| FN**IKATNGGTL**DF | 191 |
| FN**IKATNGGTL**D | 192 |
| N**IKATNGGTL**DFT | 193 |
| N**IKATNGGTL**DF | 194 |
| N**IKATNGGTL**D | 195 |

SEQ ID NOs:181-195 correspond to SEQ ID NO:9 in which one, two or three additional contiguous amino acids from the Alt a 1 protein sequence are optionally incorporated at the N- terminus, C-terminus and both N- and C-terminus.

| Peptide | **SEQ ID NO:** |
|---|---|
| SDD**ITYVATATL** | 196 |
| DD**ITYVATATL** | 197 |
| D**ITYVATATL** | 198 |
| **ITYVATATL**PNY | 199 |
| **ITYVATATL**PN | 200 |
| **ITYVATATL**P | 201 |
| SDD**ITYVATATL**PNY | 202 |
| SDD**ITYVATATL**PN | 203 |
| SDD**ITYVATATL**P | 204 |
| DD**ITYVATATL**PNY | 205 |
| DD**ITYVATATL**PN | 206 |
| DD**ITYVATATL**P | 207 |
| D**ITYVATATL**PNY | 208 |
| D**ITYVATATL**PN | 209 |
| D**ITYVATATL**P | 210 |

SEQ ID NOs:196-210 correspond to SEQ ID NO:10 in which one, two or three additional contiguous amino acids from the Alt a 1 protein sequence are optionally incorporated at the N- terminus, C-terminus and both N- and C-terminus.

| Peptide | SEQ ID NO: |
|---|---|
| DIT**YVATATLPN** | 211 |
| IT**YVATATLPN** | 212 |
| T**YVATATLPN** | 213 |
| **YVATATLPN**YVR | 214 |
| **YVATATLPN**YV | 215 |
| **YVATATLPN**Y | 216 |
| DIT**YVATATLPN**YVR | 217 |
| DIT**YVATATLPN**YV | 218 |
| DIT**YVATATLPN**Y | 219 |
| IT**YVATATLPN**YVR | 220 |
| IT**YVATATLPN**YV | 221 |
| IT**YVATATLPN**Y | 222 |
| T**YVATATLPN**YVR | 223 |
| T**YVATATLPN**YV | 224 |
| T**YVATATLPN**Y | 225 |
| **YVATATLPN**YCR | 226 |
| **YVATATLPN**YC | 227 |
| DIT**YVATATLPN**YCR | 228 |
| DIT**YVATATLPN**YC | 229 |
| IT**YVATATLPN**YCR | 230 |
| IT**YVATATLPN**YC | 231 |
| T**YVATATLPN**YCR | 232 |
| T**YVATATLPN**YC | 233 |

SEQ ID NOs:211-233 correspond to SEQ ID NO:11 in which one, two or three additional contiguous amino acids from the Alt a 1 protein sequence are optionally incorporated at the N- terminus, C-terminus and both N- and C-terminus. A C/V substitution has been made in SEQ ID NOs:214, 215, 217, 218, 220, 221, 223, 224.

| Peptide | SEQ ID NO: |
|---|---|
| TAT**LPNYCRAGG** | 234 |
| AT**LPNYCRAGG** | 235 |
| T**LPNYCRAGG** | 236 |
| **LPNYCRAGG**NGP | 237 |
| **LPNYCRAGG**NG | 238 |
| **LPNYCRAGG**N | 239 |
| TAT**LPNYCRAGG**NGP | 240 |
| TAT**LPNYCRAGG**NG | 241 |
| TAT**LPNYCRAGG**N | 242 |
| AT**LPNYCRAGG**NGP | 243 |
| AT**LPNYCRAGG**NG | 244 |
| AT**LPNYCRAGG**N | 245 |
| T**LPNYCRAGG**NGP | 246 |
| T**LPNYCRAGG**NG | 247 |
| T**LPNYCRAGG**N | 248 |

SEQ ID NOs:234-248 correspond to SEQ ID NO:12 in which one, two or three additional contiguous amino acids from the Alt a 1 protein sequence are optionally incorporated at the N- terminus, C-terminus and both N- and C-terminus.

| Peptide | SEQ ID NO: |
|---|---|
| TAT**LPNYVRAGG** | 249 |
| AT**LPNYVRAGG** | 250 |
| T**LPNYVRAGG** | 251 |
| **LPNYVRAGG**NGP | 252 |
| **LPNYVRAGG**NG | 253 |
| **LPNYVRAGG**N | 254 |
| TAT**LPNYVRAGG**NGP | 255 |
| TAT**LPNYVRAGG**NG | 256 |
| TAT**LPNYVRAGG**N | 257 |
| AT**LPNYVRAGG**NGP | 258 |
| AT**LPNYVRAGG**NG | 259 |
| AT**LPNYVRAGG**N | 260 |
| T**LPNYVRAGG**NGP | 261 |
| T**LPNYVRAGG**NG | 262 |
| T**LPNYVRAGG**N | 263 |

SEQ ID NOs:249-263 correspond to SEQ ID NO:13 in which one, two or three additional contiguous amino acids from the Alt a 1 protein sequence are optionally incorporated at the N- terminus, C-terminus and both N- and C-terminus.

| Peptide | SEQ ID NO: |
|---|---|
| PKD**FVCQGVADA** | 264 |
| KD**FVCQGVADA** | 265 |
| D**FVCQGVADA** | 266 |
| **FVCQGVADA**YIT | 267 |
| **FVCQGVADA**YI | 268 |
| **FVCQGVADA**Y | 269 |
| PKD**FVCQGVADA**YIT | 270 |
| PKD**FVCQGVADA**YI | 271 |
| PKD**FVCQGVADA**Y | 272 |
| KD**FVCQGVADA**YIT | 273 |
| KD**FVCQGVADA**YI | 274 |
| KD**FVCQGVADA**Y | 275 |
| D**FVCQGVADA**YIT | 276 |
| D**FVCQGVADA**YI | 277 |
| D**FVCQGVADA**Y | 278 |

SEQ ID NOs:264-278 correspond to SEQ ID NO:14 in which one, two or three additional contiguous amino acids from the Alt a 1 protein sequence are optionally incorporated at the N- terminus, C-terminus and both N- and C-terminus.

| Peptide | SEQ ID NO: |
|---|---|
| PKD**FVCQGVADA** | 279 |
| KD**FVCQGVADA** | 280 |
| D**FVCQGVADA** | 281 |
| **FVVQGVADA**YIT | 282 |
| **FVVQGVADA**YI | 283 |
| **FVVQGVADA**Y | 284 |
| PKD**FVVQGVADA**YIT | 285 |
| PKD**FVVQGVADA**YI | 286 |
| PKD**FVVQGVADA**Y | 287 |
| KD**FVVQGVADA**YIT | 288 |
| KD**FVVQGVADA**YI | 289 |
| KD**FVVQGVADA**Y | 290 |
| D**FVVQGVADA**YIT | 291 |
| D**FVVQGVADA**YI | 292 |
| D**FVVQGVADA**Y | 293 |

SEQ ID NOs:279-293 correspond to SEQ ID NO:15 in which one, two or three additional contiguous amino acids from the Alt a 1 protein sequence are optionally incorporated at the N- terminus, C-terminus and both N- and C-terminus.

| Peptide | SEQ ID NO: |
|---|---|
| VADA**YITLVTLPK** | 294 |
| ADA**YITLVTLPK** | 295 |
| DA**YITLVTLPK** | 296 |
| A**YITLVTLPK** | 297 |
| Y**ITLVTLPK**SS | 298 |
| Y**ITLVTLPK**S | 299 |
| VADA**YITLVTLPK**SS | 300 |
| VADA**YITLVTLPK**S | 301 |
| ADA**YITLVTLPK**SS | 302 |
| ADA**YITLVTLPK**S | 303 |
| DA**YITLVTLPK**SS | 304 |
| DA**YITLVTLPK**S | 305 |
| A**YITLVTLPK**SS | 306 |
| A**YITLVTLPK**S | 307 |

SEQ ID NOs:294-307 correspond to SEQ ID NO:16 in which one, two or three additional contiguous amino acids from the Alt a 1 protein sequence are optionally incorporated at the N- terminus, C-terminus and both N- and C-terminus.

| Peptide | SEQ ID NO: |
|---|---|
| VADAYI**TLVTLPKS** | 308 |
| ADAY**ITLVTLPKS** | 309 |
| DAY**ITLVTLPKS** | 310 |
| AY**ITLVTLPKS** | 311 |
| Y**ITLVTLPKS** | 312 |
| VADAY**ITLVTLPKSS** | 313 |
| ADAY**ITLVTLPKSS** | 314 |
| DAY**ITLVTLPKSS** | 315 |
| AY**ITLVTLPKSS** | 316 |
| Y**ITLVTLPKSS** | 317 |

SEQ ID NOs:308-317 correspond to SEQ ID NO:17 in which one, two, three, four or five additional contiguous amino acids from the Alt a 1 protein sequence are optionally incorporated at the N- terminus, and a single amino acid is optionally incorporated at the C-terminus.

| Peptide | SEQ ID NO: |
|---|---|
| IAS**LFAAAGLAA** | 318 |
| AS**LFAAAGLAA** | 319 |
| S**LFAAAGLAA** | 320 |
| **LFAAAGLAA**AAP | 321 |
| **LFAAAGLAA**AA | 322 |
| **LFAAAGLAA**A | 323 |
| IAS**LFAAAGLAA**AAP | 324 |
| IAS**LFAAAGLAA**AA | 325 |
| IAS**LFAAAGLAA**A | 326 |
| AS**LFAAAGLAA**AAP | 327 |
| AS**LFAAAGLAA**AA | 328 |
| AS**LFAAAGLAA**A | 329 |
| S**LFAAAGLAA**AAP | 330 |
| S**LFAAAGLAA**AA | 331 |
| S**LFAAAGLAA**A | 332 |

SEQ ID NOs:318-332 correspond to SEQ ID NO:18 in which one, two or three additional contiguous amino acids from the Alt a 1 protein sequence are optionally incorporated at the N- terminus, C-terminus and both N- and C-terminus.

| Peptide | SEQ ID NO: |
|---|---|
| ASL**FAAAGLAAA** | 333 |
| SL**FAAAGLAAA** | 334 |
| L**FAAAGLAAA** | 335 |
| **FAAAGLAAA**APL | 336 |
| **FAAAGLAAA**AP | 337 |
| **FAAAGLAAA**A | 338 |
| ASL**FAAAGLAAA**APL | 339 |
| ASL**FAAAGLAAA**AP | 340 |
| ASL**FAAAGLAAA**A | 341 |
| SL**FAAAGLAAA**APL | 342 |
| SL**FAAAGLAAA**AP | 343 |
| SL**FAAAGLAAA**A | 344 |
| L**FAAAGLAAA**APL | 345 |
| L**FAAAGLAAA**AP | 346 |
| L**FAAAGLAAA**A | 347 |

SEQ ID NOs:333-347 correspond to SEQ ID NO:19 in which one, two or three additional contiguous amino acids from the Alt a 1 protein sequence are optionally incorporated at the N- terminus, C-terminus and both N- and C-terminus.

| Peptide | SEQ ID NO: |
|---|---|
| GTY**YNSLGFNIK** | 348 |
| TY**YNSLGFNIK** | 349 |
| Y**YNSLGFNIK** | 350 |
| **YNSLGFNIK**ATN | 351 |
| **YNSLGFNIK**AT | 352 |
| **YNSLGFNIK**A | 353 |
| GTY**YNSLGFNIK**ATN | 354 |
| GTY**YNSLGFNIK**AT | 355 |
| GTY**YNSLGFNIK**A | 356 |
| TY**YNSLGFNIK**ATN | 357 |
| TY**YNSLGFNIK**AT | 358 |
| TY**YNSLGFNIK**A | 359 |
| Y**YNSLGFNIK**ATN | 360 |
| Y**YNSLGFNIK**AT | 361 |
| Y**YNSLGFNIK**A | 362 |

SEQ ID NOs:348-362 correspond to SEQ ID NO:20 in which one, two or three additional contiguous amino acids from the Alt a 1 protein sequence are optionally incorporated at the N- terminus, C-terminus and both N- and C-terminus.

| Peptide | SEQ ID NO: |
|---|---|
| LPN**YCRAGGNGP** | 363 |
| PN**YCRAGGNGP** | 364 |
| N**YCRAGGNGP** | 365 |
| **YCRAGGNGP**KDF | 366 |
| **YCRAGGNGP**KD | 367 |
| **YCRAGGNGP**K | 368 |
| LPN**YCRAGGNGP**KDF | 369 |
| LPN**YCRAGGNGP**KD | 370 |
| LPN**YCRAGGNGP**K | 371 |
| PN**YCRAGGNGP**KDF | 372 |
| PN**YCRAGGNGP**KD | 373 |
| PN**YCRAGGNGP**K | 374 |
| N**YCRAGGNGP**KDF | 375 |
| N**YCRAGGNGP**KD | 376 |
| N**YCRAGGNGP**K | 377 |

SEQ ID NOs:363-377 correspond to SEQ ID NO:21 in which one, two or three additional contiguous amino acids from the Alt a 1 protein sequence are optionally incorporated at the N- terminus, C-terminus and both N- and C-terminus.

| Peptide | SEQ ID NO: |
|---|---|
| LPN**YVRAGGNGP** | 378 |
| PN**YVRAGGNGP** | 379 |
| N**YVRAGGNGP** | 380 |
| **YVRAGGNGP**KDF | 381 |
| **YVRAGGNGP**KD | 382 |
| **YVRAGGNGP**K | 383 |
| LPN**YVRAGGNGP**KDF | 384 |
| LPN**YVRAGGNGP**KD | 385 |
| LPN**YVRAGGNGP**K | 386 |
| PN**YVRAGGNGP**KDF | 387 |
| PN**YVRAGGNGP**KD | 388 |
| PN**YVRAGGNGP**K | 389 |
| N**YVRAGGNGP**KDF | 390 |
| N**YVRAGGNGP**KD | 391 |
| N**YVRAGGNGP**K | 392 |

SEQ ID NOs:378-392 correspond to SEQ ID NO:22 in which one, two or three additional contiguous amino acids from the Alt a 1 protein sequence are optionally incorporated at the N- terminus, C-terminus and both N- and C-terminus.

| Peptide | SEQ ID NO: |
|---|---|
| GGT**LDFTCSAQA** | 393 |
| GT**LDFTVCSAQA** | 394 |
| T**LDFTCSAQA** | 395 |
| **LDFTCSAQA**DKL | 396 |
| **LDFTCSAQA**DK | 397 |
| **LDFTCSAQA**D | 398 |
| GGT**LDFTCSAQA**DKL | 399 |
| GGT**LDFTCSAQA**DK | 400 |
| GGT**LDFTCSAQA**D | 401 |
| GT**LDFTCSAQA**DKL | 402 |
| GT**LDFTCSAQA**DK | 403 |
| GT**LDFTCSAQA**D | 404 |
| T**LDFTCSAQA**DKL | 405 |
| T**LDFTCSAQA**DK | 406 |
| T**LDFTCSAQA**D | 407 |

SEQ ID NOs:393-407 correspond to SEQ ID NO:23 in which one, two or three additional contiguous amino acids from the Alt a 1 protein sequence are optionally incorporated at the N- terminus, C-terminus and both N- and C-terminus.

| Peptide | SEQ ID NO: |
|---|---|
| GGT**LDFTVSAQA** | 408 |
| GT**LDFTVSAQA** | 409 |
| T**LDFTVSAQ**A | 410 |
| **LDFTVSAQA**DKL | 411 |
| **LDFTVSAQA**DK | 412 |
| **LDFTVSAQA**D | 413 |
| GGT**LDFTVSAQA**DKL | 414 |
| GGT**LDFTVSAQA**DK | 415 |
| GGT**LDFTVSAQA**D | 416 |
| GT**LDFTVSAQA**DKL | 417 |
| GT**LDFTVSAQA**DK | 418 |
| GT**LDFTVSAQA**D | 419 |
| T**LDFTVSAQA**DKL | 420 |
| T**LDFTVSAQA**DK | 421 |
| T**LDFTVSAQA**D | 422 |

SEQ ID NOs:408-422 correspond to SEQ ID NO:24 in which one, two or three additional contiguous amino acids from the Alt a 1 protein sequence are optionally incorporated at the N- terminus, C-terminus and both N- and C-terminus.

| Peptide | SEQ ID NO: |
|---|---|
| DHK**WYSCGENSF** | 423 |
| HK**WYSCGENSF** | 424 |
| K**WYSCGENSF** | 425 |
| **WYSCGENSF**LDF | 426 |
| **WYSCGENSF**LD | 427 |
| **WYSCGENSF**L | 428 |
| DHK**WYSCGENSF**LDF | 429 |
| DHK**WYSCGENSF**LD | 430 |
| DHK**WYSCGENSF**L | 431 |
| HK**WYSCGENSFL**DF | 432 |
| HK**WYSCGENSF**LD | 433 |
| HK**WYSCGENSFL** | 434 |
| K**WYSCGENSF**LDF | 435 |
| K**WYSCGENSF**LD | 436 |
| K**WYSCGENSF**L | 437 |

SEQ ID NOs:423-437 correspond to SEQ ID NO:25 in which one, two or three additional contiguous amino acids from the Alt a 1 protein sequence are optionally incorporated at the N- terminus, C-terminus and both N- and C-terminus.

| Peptide | SEQ ID NO: |
|---|---|
| DHK**WYSVGENSF** | 438 |
| HK**WYSVGENSF** | 439 |
| K**WYSVGENSF** | 440 |
| **WYSVGENSF**LDF | 441 |
| **WYSVGENSF**LD | 442 |
| **WYSVGENSF**L | 443 |
| DHK**WYSVGENSF**LDF | 444 |
| DHK**WYSVGENSF**LD | 445 |
| DHK**WYSVGENSF**L | 446 |
| HK**WYSVGENSF**LDF | 447 |
| HK**WYSVGENSF**LD | 448 |
| HK**WYSVGENSF**L | 449 |
| K**WYSVGENSF**LDF | 450 |
| K**WYSVGENSF**LD | 451 |
| K**WYSVGENSF**L | 452 |

SEQ ID NOs:438-452 correspond to SEQ ID NO:26 in which one, two or three additional contiguous amino acids from the Alt a 1 protein sequence are optionally incorporated at the N- terminus, C-terminus and both N- and C-terminus.

| Peptide | SEQ ID NO: |
|---|---|
| KQK**VSDDITYVA** | 453 |
| QK**VSDDITYVA** | 454 |
| K**VSDDITYVA** | 455 |
| **VSDDITYVA**TAT | 456 |
| **VSDDITYVA**TA | 457 |
| **VSDDITYVA**T | 458 |
| KQK**VSDDITYVA**TAT | 459 |
| KQK**VSDDITYVA**TA | 460 |
| KQK**VSDDITYVA**T | 461 |
| QK**VSDDITYVAT**AT | 462 |
| QK**VSDDITYVA**TA | 463 |
| QK**VSDDITYVA**T | 464 |
| K**VSDDITYVA**TAT | 465 |
| K**VSDDITYVA**TA | 466 |
| K**VSDDITYVA**T | 467 |

SEQ ID NOs:453-467 correspond to SEQ ID NO:27 in which one, two or three additional contiguous amino acids from the Alt a 1 protein sequence are optionally incorporated at the N- terminus, C-terminus and both N- and C-terminus.

| Peptide | SEQ ID NO: |
|---|---|
| DNR**LVNHFTNEF** | 468 |
| NR**LVNHFTNEF** | 469 |
| R**LVNHFTNEF** | 470 |
| **LVNHFTNEF**KRK | 471 |
| **LVNHFTNEF**KR | 472 |
| **LVNHFTNEF**K | 473 |
| DNR**LVNHFTNEF**KRK | 474 |
| DNR**LVNHFTNEF**KR | 475 |
| DNR**LVNHFTNEF**K | 476 |
| NR**LVNHFTNEF**KRK | 477 |
| NR**LVNHFTNEF**KR | 478 |
| NR**LVNHFTNEF**K | 479 |
| R**LVNHFTNEF**KRK | 480 |
| R**LVNHFTNEF**KR | 481 |
| R**LVNHFTNEF**K | 482 |

SEQ ID NOs:468-482 correspond to SEQ ID NO:28 in which one, two or three additional contiguous amino acids from the Alt a 3 protein sequence are optionally incorporated at the N- terminus, C-terminus and both N- and C-terminus.

| Peptide | SEQ ID NO: |
|---|---|
| TSE**ILLLDVAPL** | 483 |
| SE**ILLLDVAPL** | 484 |
| E**ILLLDVAPL** | 485 |
| **ILLLDVAPL**SIG | 486 |
| **ILLLDVAPL**SI | 487 |
| **ILLLDVAPL**S | 488 |
| TSE**ILLLDVAPL**SIG | 489 |
| TSE**ILLLDVAPL**SI | 490 |
| TSE**ILLLDVAPL**S | 491 |
| SE**ILLLDVAPL**SIG | 492 |
| SE**ILLLDVAPL**SI | 493 |
| SE**ILLLDVAPL**S | 494 |
| E**ILLLDVAPL**SIG | 495 |
| E**ILLLDVAPL**SI | 496 |
| E**ILLLDVAPL**S | 497 |

SEQ ID NOs:483-497 correspond to SEQ ID NO:29 in which one, two or three additional contiguous amino acids from the Alt a 3 protein sequence are optionally incorporated at the N- terminus, C-terminus and both N- and C-terminus.

| Peptide | SEQ ID NO: |
|---|---|
| LNV**LRIINEPTA** | 498 |
| NV**LRIINEPTA** | 499 |
| V**LRIINEPTA** | 500 |
| **LRIINEPTA**AAI | 501 |
| **LRIINEPTA**AA | 502 |
| **LRIINEPTA**A | 503 |
| LNV**LRIINEPTA**AAI | 504 |
| LNV**LRIINEPTA**AA | 505 |
| LNV**LRIINEPTA**A | 506 |
| NV**LRIINEPTA**AAI | 507 |
| NV**LRIINEPT**AAA | 508 |
| NV**LRIINEPTA**A | 509 |
| V**LRIINEPTA**AAI | 510 |
| V**LRIINEPTA**AA | 511 |
| V**LRIINEPTA**A | 512 |

SEQ ID NOs:498-512 correspond to SEQ ID NO: 30 in which one, two or three additional contiguous amino acids from the Alt a 3 protein sequence are optionally incorporated at the N- terminus, C-terminus and both N- and C-terminus.

| Peptide | SEQ ID NO: |
|---|---|
| ARA**LRRLRTACE** | 513 |
| RA**LRRLRTACE** | 514 |
| A**LRRLRTACE** | 515 |
| **LRRLRTACE**RAK | 516 |
| **LRRLRTACE**RA | 517 |
| **LRRLRTACE**R | 518 |
| ARA**LRRLRTACE**RAK | 519 |
| ARA**LRRLRTACE**RA | 520 |
| ARA**LRRLRTACE**R | 521 |
| RA**LRRLRTACE**RAK | 522 |
| RA**LRRLRTACE**RA | 523 |
| RA**LRRLRTACE**R | 524 |
| A**LRRLRTACE**RAK | 525 |
| A**LRRLRTACE**RA | 526 |
| A**LRRLRTACE**R | 527 |

SEQ ID NOs:513-527 correspond to SEQ ID NO:31 in which one, two or three additional contiguous amino acids from the Alt a 3 protein sequence are optionally incorporated at the N- terminus, C-terminus and both N- and C-terminus.

| Peptide | SEQ ID NO: |
|---|---|
| PDE**IQGFPTIKL** | 528 |
| DE**IQGFPTIKL** | 529 |
| E**IQGFPTIKL** | 530 |
| **IQGFPTIKL**FPA | 531 |
| **IQGFPTIKL**FP | 532 |
| **IQGFPTIKL**F | 533 |
| PDE**IQGFPTIKL**FPA | 534 |
| PDE**IQGFPTIKL**FP | 535 |
| PDE**IQGFPTIKL**F | 536 |
| DE**IQGFPTIKL**FPA | 537 |
| DE**IQGFPTIKL**FP | 538 |
| DE**IQGFPTIKL**F | 539 |
| E**IQGFPTIKL**FPA | 540 |
| E**IQGFPTIKL**FP | 541 |
| E**IQGFPTIKL**F | 542 |

SEQ ID NOs:528-542 correspond to SEQ ID NO:32 in which one, two or three additional contiguous amino acids from the Alt a 4 protein sequence are optionally incorporated at the N- terminus, C-terminus and both N- and C-terminus.

| Peptide | SEQ ID NO: |
|---|---|
| AD**IVVGLRSGQI** | 543 |
| DI**VVGLRSGQI** | 544 |
| I**VVGLRSGQI** | 545 |
| **VVGLRSGQI**KTG | 546 |
| **VVGLRSGQI**KT | 547 |
| **VVGLRSGQI**K | 548 |
| ADI**VVGLRSGQI**KTG | 549 |
| ADI**VVGLRSGQI**KT | 550 |
| ADI**VVGLRSGQI**K | 551 |
| DI**VVGLRSGQI**KTG | 552 |
| DI**VVGLRSGQI**KT | 553 |
| DI**VVGLRSGQI**K | 554 |
| I**VVGLRSGQI**KTG | 555 |
| I**VVGLRSGQI**KT | 556 |
| I**VVGLRSGQI**K | 557 |

SEQ ID NOs:543-557 correspond to SEQ ID NO:33 in which one, two or three additional contiguous amino acids from the Alt a 6 protein sequence are optionally incorporated at the N- terminus, C-terminus and both N- and C-terminus.

| Peptide | SEQ ID NO: |
|---|---|
| AGV**FVSTGTLGG** | 558 |
| GV**FVSTGTLGG** | 559 |
| V**FVSTGTLGG** | 560 |
| **FVSTGTLGG**GQE | 561 |
| **FVSTGTLGG**GQ | 562 |
| **FVSTGTLGG**G | 563 |
| AGV**FVSTGTLGG**GQE | 564 |
| AGV**FVSTGTLGG**GQ | 565 |
| AGV**FVSTGTLGG**G | 566 |
| GV**FVSTGTLGG**GQE | 567 |
| GV**FVSTGTLGG**GQ | 568 |
| GV**FVSTGTLGG**G | 569 |
| V**FVSTGTLGG**GQE | 570 |
| V**FVSTGTLGG**GQ | 571 |
| V**FVSTGTLGG**G | 572 |

SEQ ID NOs:558-572 correspond to SEQ ID NO:34 in which one, two or three additional contiguous amino acids from the Alt a 7 protein sequence are optionally incorporated at the N- terminus, C-terminus and both N- and C-terminus.

| Peptide | SEQ ID NO: |
|---|---|
| KGK**VVIVTGASG** | 573 |
| GK**VVIVTGASG** | 574 |
| K**VVIVTGASG** | 575 |
| **VVIVTGASG**PTG | 576 |
| **VVIVTGASG**PT | 577 |
| **VVIVTGASG**P | 578 |
| KGK**VVIVTGASG**PTG | 579 |
| KGK**VVIVTGASG**PT | 580 |
| KGK**VVIVTGASG**P | 581 |
| GK**VVIVTGASG**PTG | 582 |
| GK**VVIVTGASG**PT | 583 |
| GK**VVIVTGASG**P | 584 |
| K**VVIVTGASG**PTG | 585 |
| K**VVIVTGASG**PT | 586 |
| K**VVIVTGASG**P | 587 |

SEQ ID NOs:573-587 correspond to SEQ ID NO:35 in which one, two or three additional contiguous amino acids from the Alt a 8 protein sequence are optionally incorporated at the N- terminus, C-terminus and both N- and C-terminus.

| Peptide | SEQ ID NO: |
|---|---|
| TGS**LVITSSMSG** | 588 |
| GS**LVITSSMSG** | 589 |
| S**LVITSSMSG** | 590 |
| **LVITSSMSG**HIA | 591 |
| **LVITSSMSG**HI | 592 |
| **LVITSSMSG**H | 593 |
| TGS**LVITSSMSG**HIA | 594 |
| TGS**LVITSSMSG**HI | 595 |
| TGS**LVITSSMSG**H | 596 |
| GS**LVITSSMSG**HIA | 597 |
| GS**LVITSSMSG**HI | 598 |
| GS**LVITSSMSG**H | 599 |
| S**LVITSSMSG**HIA | 600 |
| S**LVITSSMSG**HI | 601 |
| S**LVITSSMSG**H | 602 |

SEQ ID NOs:588-602 correspond to SEQ ID NO:36 in which one, two or three additional contiguous amino acids from the Alt a 8 protein sequence are optionally incorporated at the N- terminus C-terminus and both N- and C-terminus.

| Peptide | SEQ ID NO: |
|---|---|
| TGS**LVITSSLSG** | 603 |
| GS**LVITSSLSG** | 604 |
| S**LVITSSLSG** | 605 |
| **LVITSSLSG**HIA | 606 |
| **LVITSSLSG**HI | 607 |
| **LVITSSLSG**H | 608 |
| TGS**LVITSSLSGH**IA | 609 |
| TGS**LVITSSLSG**HI | 610 |
| TGS**LVITSSLSG**H | 611 |
| GS**LVITSSLSG**HIA | 612 |
| GS**LVITSSLSG**HI | 613 |
| GS**LVITSSLSG**H | 614 |
| S**LVITSSLSG**HIA | 615 |
| S**LVITSSLSG**HI | 616 |
| S**LVITSSLSG**H | 617 |

SEQ ID NOs:603-617 correspond to SEQ ID NO:37 in which one, two or three additional contiguous amino acids from the Alt a 8 protein sequence are optionally incorporated at the N- terminus, C-terminus and both N- and C-terminus.

| Peptide | SEQ ID NO: |
|---|---|
| VWA**IVLLSLCQL** | 618 |
| WA**IVLLSLCQL** | 619 |
| A**IVLLSLCQL** | 620 |
| **IVLLSLCQL**TTP | 621 |
| **IVLLSLCQL**TT | 622 |
| **IVLLSLCQL**T | 623 |
| VWA**IVLLSLCQL**TTP | 624 |
| VWA**IVLLSLCQL**TT | 625 |
| VWA**IVLLSLCQL**T | 626 |
| WA**IVLLSLCQL**TTP | 627 |
| WA**IVLLSLCQL**TT | 628 |
| WA**IVLLSLCQL**T | 629 |
| A**IVLLSLCQL**TTP | 630 |
| A**IVLLSLCQL**TT | 631 |
| A**IVLLSLCQL**T | 632 |

SEQ ID NOs:618-632 correspond to SEQ ID NO:38 in which one, two or three additional contiguous amino acids from the Alt a 10 protein sequence are optionally incorporated at the N- terminus, C-terminus and both N- and C-terminus.

| Peptide | SEQ ID NO: |
|---|---|
| VWA**IVLLSLVQL** | 633 |
| WA**IVLLSLVQL** | 634 |
| A**IVLLSLVQL** | 635 |
| **IVLLSLVQL**TTP | 636 |
| **IVLLSLVQL**TT | 637 |
| **IVLLSLVQL**T | 638 |
| VWA**IVLLSLVQL**TTP | 639 |
| VWA**IVLLSLVQL**TT | 640 |
| VWA**IVLLSLVQL**T | 641 |
| WA**IVLLSLVQL**TTP | 642 |
| WA**IVLLSLVQL**TT | 643 |
| WA**IVLLSLVQL**T | 644 |
| A**IVLLSLVQL**TTP | 645 |
| A**IVLLSLVQL**TT | 646 |
| A**IVLLSLVQL**T | 647 |

SEQ ID NOs:633-647 correspond to SEQ ID NO: 39 in which one, two or three additional contiguous amino acids from the Alt a 10 protein sequence are optionally incorporated at the N- terminus, C-terminus and both N- and C-terminus.

| Peptide | SEQ ID NO: |
|---|---|
| VGI**FRDDRIEII** | 648 |
| GI**FRDDRIEII** | 649 |
| I**FRDDRIEII** | 650 |
| **FRDDRIEII**AND | 651 |
| **FRDDRIEII**AN | 652 |
| **FRDDRIEII**A | 653 |
| VGI**FRDDRIEII**AND | 654 |
| VGI**FRDDRIEII**AN | 655 |
| VGI**FRDDRIEII**A | 656 |
| GI**FRDDRIEII**AND | 657 |
| GI**FRDDRIEII**AN | 658 |
| GI**FRDDRIEII**A | 659 |
| IF**RDDRIEII**AND | 660 |
| IF**RDDRIEII**AN | 661 |
| I**FRDDRIEII**A | 662 |

SEQ ID NOs:648-662 correspond to SEQ ID NO:40 in which one, two or three additional contiguous amino acids from the Alt a 3 protein sequence are optionally incorporated at the N- terminus, C-terminus and both N- and C-terminus.

| Peptide | SEQ ID NO: |
|---|---|
| KNQ**VAMNPVNTV** | 667 |
| NQ**VAMNPVNTV** | 668 |
| Q**VAMNPVNTV** | 669 |
| **VAMNPVNTV**FDA | 670 |
| **VAMNPVNTV**FD | 671 |
| **VAMNPVNTV**F | 672 |
| KNQ**VAMNPVNTVF**DA | 673 |
| KNQ**VAMNPVNTV**FD | 674 |
| KNQ**VAMNPVNTV**F | 675 |
| NQ**VAMNPVNTV**FDA | 676 |
| NQ**VAMNPVNTV**FD | 677 |
| NQ**VAMNPVNTV**F | 678 |
| Q**VAMNPVNTV**FDA | 679 |
| Q**VAMNPVNTV**FD | 680 |
| Q**VAMNPVNTV**F | 681 |

SEQ ID NOs:667-681 correspond to SEQ ID NO:41 in which one, two or three additional contiguous amino acids from the Alt a 3 protein sequence are optionally incorporated at the N- terminus, C-terminus and both N- and C-terminus.

| Peptide | SEQ ID NO: |
|---|---|
| KNQ**VALNPVNTV** | 682 |
| NQ**VALNPVNTV** | 683 |
| Q**VALNPVNTV** | 684 |
| **VALNPVNTV**FDA | 685 |
| **VALNPVNTV**FD | 686 |
| **VALNPVNTVF** | 687 |
| KNQ**VALNPVNTV**FDA | 688 |
| KNQ**VALNPVNTV**FD | 689 |
| KNQ**VALNPVNTV**F | 690 |
| NQ**VALNPVNTVF**DA | 691 |
| NQ**VALNPVNTV**FD | 692 |
| NQ**VALNPVNTV**F | 693 |
| Q**VALNPVNTV**FDA | 694 |
| Q**VALNPVNTV**FD | 695 |
| Q**VALNPVNTVF** | 696 |

SEQ ID NOs:682-696 correspond to SEQ ID NO:42 in which one, two or three additional contiguous amino acids from the Alt a 3 protein sequence are optionally incorporated at the N- terminus, C-terminus and both N- and C-terminus.

| Peptide | SEQ ID NO: |
|---|---|
| LSK**LVTIAKVDA** | 697 |
| SK**LVTIAKVDA** | 698 |
| K**LVTIAKVDA** | 699 |
| L**VTIAKVDA**TLN | 700 |
| **LVTIAKVDA**TL | 701 |
| **LVTIAKVDA**T | 702 |
| LSK**LVTIAKVDA**TLN | 703 |
| LSK**LVTIAKVDA**TL | 704 |
| LSK**LVTIAKVDA**T | 705 |
| SK**LVTIAKVDA**TLN | 706 |
| SK**LVTIAKVDA**TL | 707 |
| SK**LVTIAKVDA**T | 708 |
| K**LVTIAKVDA**TLN | 709 |
| K**LVTIAKVDA**TL | 710 |
| K**LVTIAKVDA**T | 711 |

SEQ ID NOs:697-711 correspond to SEQ ID NO: 43 in which one, two or three additional contiguous amino acids from the Alt a 4 protein sequence are optionally incorporated at the N- terminus, C-terminus and both N- and C-terminus.

| Peptide | SEQ ID NO: |
|---|---|
| **MKHLAAYLL**LGLGGN | 712 |
| **MKHLAAYLL**LGLGG | 713 |
| **MKHLAAYLL**LGLG | 714 |
| **MKHLAAYL**LLGL | 715 |
| **MKHLAAYLL**LG | 716 |
| **MKHLAAYLL**L | 717 |

SEQ ID NOs:712-717 correspond to SEQ ID NO:44 in which one, two, three, four, five or six additional contiguous amino acids from the Alt a 5 protein sequence are optionally incorporated at the C-terminus.

| Peptide | SEQ ID NO: |
|---|---|
| **LKHLAAYLL**LGLGGN | 718 |
| **LKHLAAYLL**LGLGG | 719 |
| **LKHLAAYLL**LGLG | 720 |
| **LKHLAAYLL**LGL | 721 |
| **LKHLAAYLL**LG | 722 |
| **LKHLAAYLL**L | 723 |

SEQ ID NOs:718-723 correspond to SEQ ID NO:45 in which one, two, three, four, five or six additional contiguous amino acids from the Alt a 5 protein sequence are optionally incorporated at the C-terminus.

| Peptide | SEQ ID NO: |
|---|---|
| AEV**YQKLKALAK** | 724 |
| EV**YQKLKALAK** | 725 |
| V**YQKLKALAK** | 726 |
| **YQKLKALAKK**TY | 727 |
| **YQKLKALAK**KT | 728 |
| **YQKLKALAK**K | 729 |
| AEV**YQKLKALAK**KTY | 730 |
| AEV**YQKLKALAK**KT | 731 |
| AEV**YQKLKALAK**K | 732 |
| EV**YQKLKALAK**KTY | 733 |
| EV**YQKLKALAK**KT | 734 |
| EV**YQKLKALAK**K | 735 |
| V**YQKLKALAK**KTY | 736 |
| V**YQKLKALAK**KT | 737 |
| V**YQKLKALAK**K | 738 |

SEQ ID NOs:724-738 correspond to SEQ ID NO:46 in which one, two or three additional contiguous amino acids from the Alt a 6 protein sequence are optionally incorporated at the N- terminus, C-terminus and both N- and C-terminus.

| Peptide | SEQ ID NO: |
|---|---|
| AIE**LKSCNALLL** | 739 |
| IE**LKSCNALLL** | 740 |
| E**LKSCNALLL** | 741 |
| **LKSCNALLL**KVN | 742 |
| **LKSCNALLL**KV | 743 |
| **LKSCNALLL**K | 744 |
| AIE**LKSCNALLL**KVN | 745 |
| AIE**LKSCNALLL**KV | 746 |
| AIE**LKSCNALLL**K | 747 |
| IE**LKSCNALLL**KVN | 748 |
| IE**LKSCNALLL**KV | 749 |
| IE**LKSCNALLL**K | 750 |
| E**LKSCNALLL**KVN | 751 |
| E**LKSCNALLL**KV | 752 |
| E**LKSCNALLL**K | 753 |

SEQ ID NOs:739-753 correspond to SEQ ID NO:47 in which one, two or three additional contiguous amino acids from the Alt a 6 protein sequence are optionally incorporated at the N- terminus, C-terminus and both N- and C-terminus.

| Peptide | SEQ ID NO: |
|---|---|
| AIE**LKSVNALLL** | 754 |
| IE**LKSVNALLL** | 755 |
| E**LKSVNALLL** | 756 |
| **LKSVNALLL**KVN | 757 |
| **LKSVNALLL**KV | 758 |
| **LKSVNALLL**K | 759 |
| AIE**LKSVNALLL**KVN | 760 |
| AIE**LKSVNALLL**KV | 761 |
| AIE**LKSVNALLL**K | 762 |
| IE**LKSVNALLL**KVN | 763 |
| IE**LKSVNALLL**KV | 764 |
| IE**LKSVNALLL**K | 765 |
| E**LKSVNALLL**KVN | 766 |
| E**LKSVNALLL**KV | 767 |
| E**LKSVNALLL**K | 768 |

SEQ ID NOs:754-768 correspond to SEQ ID NO:48 in which one, two or three additional contiguous amino acids from the Alt a 6 protein sequence are optionally incorporated at the N- terminus, C-terminus and both N- and C-terminus.

| Peptide | SEQ ID NO: |
|---|---|
| GAG**WGVMVSHRS** | 769 |
| AG**WGVMVSHRS** | 770 |
| G**WGVMVSHRS** | 771 |
| **WGVMVSHRS**GET | 772 |
| **WGVMVSHRS**GE | 773 |
| **WGVMVSHRS**G | 774 |
| GAG**WGVMVSHRS**GET | 775 |
| GAG**WGVMVSHRS**GE | 776 |
| GAG**WGVMVSHRS**G | 777 |
| AG**WGVMVSHRS**GET | 778 |
| AG**WGVMVSHRS**GE | 779 |
| AG**WGVMVSHRS**G | 780 |
| G**WGVMVSHRS**GET | 781 |
| G**WGVMVSHRS**GE | 782 |
| G**WGVMVSHRS**G | 783 |

SEQ ID NOs:769-783 correspond to SEQ ID NO:49 in which one, two or three additional contiguous amino acids from the Alt a 6 protein sequence are optionally incorporated at the N- terminus, C-terminus and both N- and C-terminus.

| Peptide | SEQ ID NO: |
|---|---|
| GAG**WGVLVSHRS** | 784 |
| AG**WGVLVSHRS** | 785 |
| G**WGVLVSHRS** | 786 |
| **WGVLVSHRS**GET | 787 |
| **WGVLVSHRS**GE | 788 |
| **WGVLVSHRS**G | 789 |
| GAG**WGVLVSHRS**GET | 790 |
| GAG**WGVLVSHRS**GE | 791 |
| GAG**WGVLVSHRS**G | 792 |
| AG**WGVLVSHRS**GET | 793 |
| AG**WGVLVSHRS**GE | 794 |
| AG**WGVLVSHRS**G | 795 |
| G**WGVLVSHRS**GET | 796 |
| G**WGVLVSHRS**GE | 797 |
| G**WGVLVSHRS**G | 798 |

SEQ ID NOs:784-798 correspond to SEQ ID NO:50 in which one, two or three additional contiguous amino acids from the Alt a 6 protein sequence are optionally incorporated at the N- terminus, C-terminus and both N- and C-terminus.

| Peptide | SEQ ID NO: |
|---|---|
| VPL**GYKTAFSML** | 799 |
| PL**GYKTAFSML** | 800 |
| L**GYKTAFSML** | 801 |
| **GYKTAFSML**ANL | 802 |
| **GYKTAFSML**AN | 803 |
| **GYKTAFSML**A | 804 |
| VPL**GYKTAFSML**ANL | 805 |
| VPL**GYKTAFSML**AN | 806 |
| VPL**GYKTAFSML**A | 807 |
| PL**GYKTAFSML**ANL | 808 |
| PL**GYKTAFSML**AN | 809 |
| PL**GYKTAFSML**A | 810 |
| L**GYKTAFSML**ANL | 811 |
| L**GYKTAFSML**AN | 812 |
| L**GYKTAFSML**A | 813 |

SEQ ID NOs:799-813 correspond to SEQ ID NO:51 in which one, two or three additional contiguous amino acids from the Alt a 7 protein sequence are optionally incorporated at the N- terminus, C-terminus and both N- and C-terminus.

| Peptide | SEQ ID NO: |
|---|---|
| VPL**GYKTAFSLL** | 814 |
| PL**GYKTAFSLL** | 815 |
| L**GYKTAFSLL** | 816 |
| **GYKTAFSLL**ANL | 817 |
| **GYKTAFSLL**AN | 818 |
| **GYKTAFSLL**A | 819 |
| VPL**GYKTAFSLL**ANL | 820 |
| VPL**GYKTAFSLL**AN | 821 |
| VPL**GYKTAFSLL**A | 822 |
| PL**GYKTAFSLL**ANL | 823 |
| PL**GYKTAFSLL**AN | 824 |
| PL**GYKTAFSLL**A | 825 |
| L**GYKTAFSLL**ANL | 826 |
| L**GYKTAFSLL**AN | 827 |
| L**GYKTAFSLL**A | 828 |

SEQ ID NOs:814-828 correspond to SEQ ID NO:52 in which one, two or three additional contiguous amino acids from the Alt a 7 protein sequence are optionally incorporated at the N- terminus, C-terminus and both N- and C-terminus.

| Peptide | SEQ ID NO: |
|---|---|
| LSD**FVPQDIQKL** | 829 |
| SD**FVPQDIQKL** | 830 |
| D**FVPQDIQKL** | 831 |
| **FVPQDIQKL**WHS | 832 |
| **FVPQDIQKL**WH | 833 |
| **FVPQDIQKL**W | 834 |
| LSD**FVPQDIQKL**WHS | 835 |
| LSD**FVPQDIQKL**WH | 836 |
| LSD**FVPQDIQKL**W | 837 |
| SD**FVPQDIQKL**WHS | 838 |
| SD**FVPQDIQKL**WH | 839 |
| SD**FVPQDIQKL**W | 840 |
| D**FVPQDIQKL**WHS | 841 |
| D**FVPQDIQKL**WH | 842 |
| D**FVPQDIQKL**W | 843 |

SEQ ID NOs:829-843 correspond to SEQ ID NO:53 in which one, two or three additional contiguous amino acids from the Alt a 8 protein sequence are optionally incorporated at the N- terminus, C-terminus and both N- and C-terminus.

| Peptide | SEQ ID NO: |
|---|---|
| KGA**YVYFASDAS** | 844 |
| GA**YVYFASDAS** | 845 |
| A**YVYFASDAS** | 846 |
| **YVYFASDAS**SYV | 847 |
| **YVYFASDAS**SY | 848 |
| **YVYFASDAS**S | 849 |
| KGA**YVYFASDAS**SYV | 850 |
| KGA**YVYFASDAS**SY | 851 |
| KGA**YVYFASDAS**S | 852 |
| GA**YVYFASDAS**SYV | 853 |
| GA**YVYFASDAS**SY | 854 |
| GA**YVYFASDAS**S | 855 |
| A**YVYFASDAS**SYV | 856 |
| A**YVYFASDAS**SY | 857 |
| A**YVYFASDAS**S | 858 |
| Y**VYFASDAS**SYC | 859 |
| KGA**YVYFASDAS**SYC | 860 |
| GA**YVYFASDAS**SYC | 861 |
| A**YVYFASDAS**SYC | 862 |

SEQ ID NOs:844-862 correspond to SEQ ID NO:54 in which one, two or three additional contiguous amino acids from the Alt a 8 protein sequence are optionally incorporated at the N- terminus, C-terminus and both N- and C-terminus. A C/V substitution has been made in SEQ ID NOs:847, 850, 853, 856.

| Peptide | SEQ ID NO: |
|---|---|
| DKG**YFIEPTIFS** | 863 |
| KG**YFIEPTIFS** | 864 |
| G**YFIEPTIFS** | 865 |
| **YFIEPTIFS**NVT | 866 |
| **YFIEPTIFS**NV | 867 |
| **YFIEPTIFS**N | 868 |
| DKG**YFIEPTIFS**NVT | 869 |
| DKG**YFIEPTIFS**NV | 870 |
| DKG**YFIEPTIFS**N | 871 |
| KG**YFIEPTIFS**NVT | 872 |
| KG**YFIEPTIFS**NV | 873 |
| KG**YFIEPTIFS**N | 874 |
| G**YFIEPTIFS**NVT | 875 |
| G**YFIEPTIFS**NV | 876 |
| G**YFIEPTIFS**N | 877 |

SEQ ID NOs:863-877 correspond to SEQ ID NO:55 in which one, two or three additional contiguous amino acids from the Alt a 10 protein sequence are optionally incorporated at the N- terminus, C-terminus and both N- and C-terminus.

| Peptide | SEQ ID NO: |
|---|---|
| LDN**YIQTKTVSI** | 878 |
| DN**YIQTKTVSI** | 879 |
| N**YIQTKTVSI** | 880 |
| **YIQTKTVSI**RLG | 881 |
| **YIQTKTVSI**RL | 882 |
| **YIQTKTVSI**R | 883 |
| LDN**YIQTKTVSI**RLG | 884 |
| LDN**YIQTKTVSI**RL | 885 |
| LDN**YIQTKTVSI**R | 886 |
| DN**YIQTKTVSI**RLG | 887 |
| DN**YIQTKTVSI**RL | 888 |
| DN**YIQTKTVSI**R | 889 |
| N**YIQTKTVSI**RLG | 890 |
| N**YIQTKTVSI**RL | 891 |
| N**YIQTKTVSI**R | 892 |

SEQ ID NOs:878-892 correspond to SEQ ID NO:56 in which one, two or three additional contiguous amino acids from the Alt a 10 protein sequence are optionally incorporated at the N- terminus, C-terminus and both N- and C-terminus.

| Peptide | SEQ ID NO: |
|---|---|
| QTKTVS**IRLGDVLFG** | 893 |
| TKTVS**IRLGDVLFG** | 894 |
| KTVS**IRLGDVLFG** | 895 |
| TVS**IRLGDVLFG** | 896 |
| VS**IRLGDVLFG** | 897 |
| S**IRLGDVLFG** | 898 |

SEQ ID NOs:893-898 correspond to SEQ ID NO:57 in which one, two, three, four, five or six additional contiguous amino acids from the Alt a 10 protein sequence are optionally incorporated at the N-terminus.

| Peptide | SEQ ID NO: |
|---|---|
| GTV**WVNSYNTLH** | 899 |
| TV**WVNSYNTLH** | 900 |
| V**WVNSYNTLH** | 901 |
| **WVNSYNTLH**WQL | 902 |
| **WVNSYNTLH**WQ | 903 |
| **WVNSYNTLH**W | 904 |
| GTV**WVNSYNTLH**WQL | 905 |
| GTV**WVNSYNTLH**WQ | 906 |
| GTV**WVNSYNTLH**W | 907 |
| TV**WVNSYNTLH**WQL | 908 |
| TV**WVNSYNTLH**WQ | 909 |
| TV**WVNSYNTLH**W | 910 |
| V**WVNSYNTLH**WQL | 911 |
| V**WVNSYNTLH**WQ | 912 |
| V**WVNSYNTLH**W | 913 |

SEQ ID NOs:899-913 correspond to SEQ ID NO:58 in which one, two or three additional contiguous amino acids from the Alt a 10 protein sequence are optionally incorporated at the N- terminus, C-terminus and both N- and C-terminus.

The invention may optionally exclude peptides comprising one or more of the following sequences, or peptides having a contiguous sequence of 7, 8 or 9 amino acids that has one of 90%, 91%, 92%, 93%, 94%, 95%, 96%, 97%, 98%, 99% or 100% sequence identity to one or more of the following sequences:
▪ YYNSLGFNI (e.g. SEQ ID NOs:6 and 136-150)
▪ LGFNIKATN (e.g. SEQ ID NOs:7 and 151-165)
▪ FNIKATNGG (e.g. SEQ ID NOs:8 and 166-180)
▪ IKATNGGTL (e.g. SEQ ID NOs:9 and 181-195)
▪ ITYVATATL (e.g. SEQ ID NOs:10 and 196-210)
▪ VATATLPNY [SEQ ID NO:914 ] (e.g. SEQ ID NOs:199, 202, 205, 208)
▪ YVATATLPN (e.g. SEQ ID NOs:11 and 211-233)
▪ YITLVTLPK (e.g. SEQ ID NOs:16 and 294-307)
▪ ITLVTLPKS (e.g. SEQ ID NOs:17 and 308-317)
▪ VYQKLKALA [SEQ ID NO:915] (e.g. SEQ ID NOs:724-726, 730-738)
▪ YQKLKALAK (e.g. SEQ ID NOs:46 and 724-738)
▪ KLKALAKKT [SEQ ID NO:916] (e.g. SEQ ID NOs:727, 728, 730, 731, 733, 732, 736, 737
▪ LKALAKKTY [SEQ ID NO:917 ] (e.g. SEQ ID NOs:727, 730, 733, 736
▪ FGAGWGVMV [SEQ ID NO:918]
▪ WGVMVSHRS (e.g. SEQ ID NOs:49 and 769-783)
▪ WGVLVSHRS (e.g. SEQ ID NOs:50 and 784-798)
▪ GVMVSHRSG [SEQ ID NO:919] (e.g. SEQ ID NOs:
▪ VMVSHRSGE [SEQ ID NO:920] (e.g. SEQ ID NOs:772, 773, 775, 776, 778, 779, 781, 782)
▪ MVSHRSGET [SEQ ID NO:921] (e.g. SEQ ID NOs:772, 775, 778, 781)
▪ YVWKISEFY [SEQ ID NO:922]
▪ LLLKQKVSD [SEQ ID NO:923]
▪ LLKQKVSDD [SEQ ID NO:924]
▪ VVLVAYFAA [SEQ ID NO:925]
▪ VVGRQILKS [SEQ ID NO:926]
▪ VVGRQIMKS [SEQ ID NO:927]

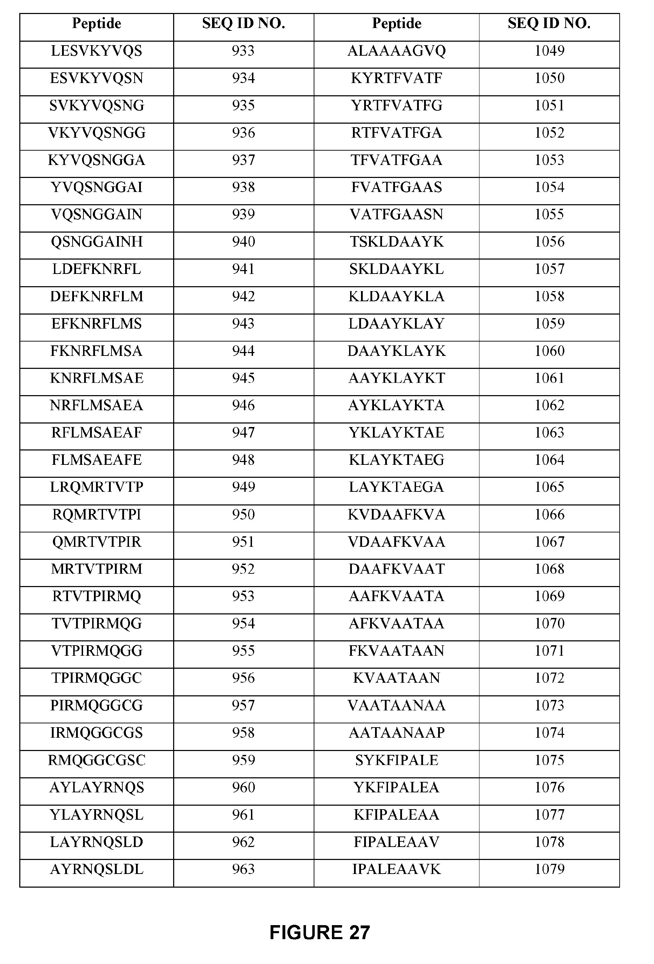

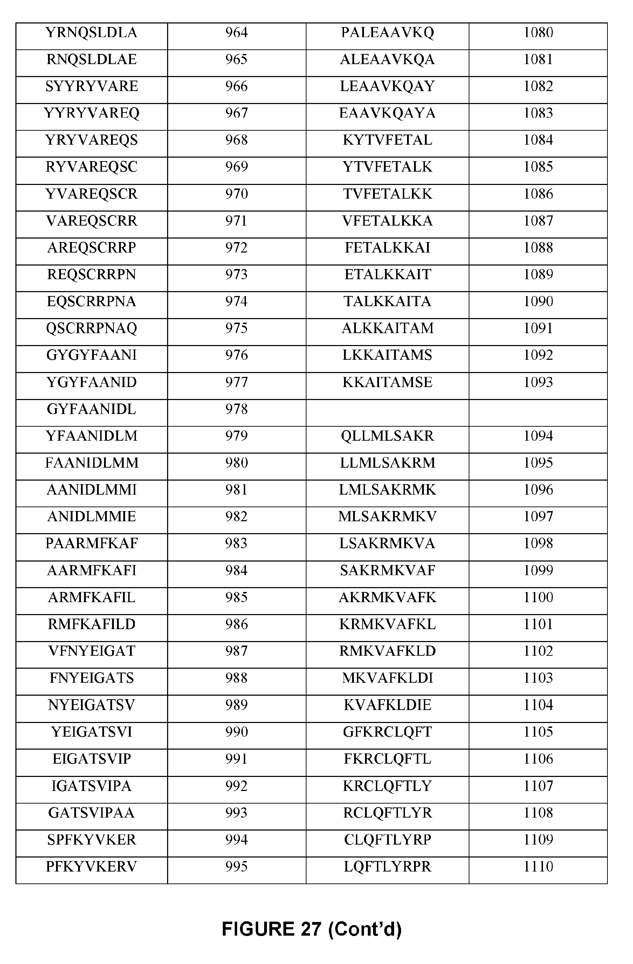

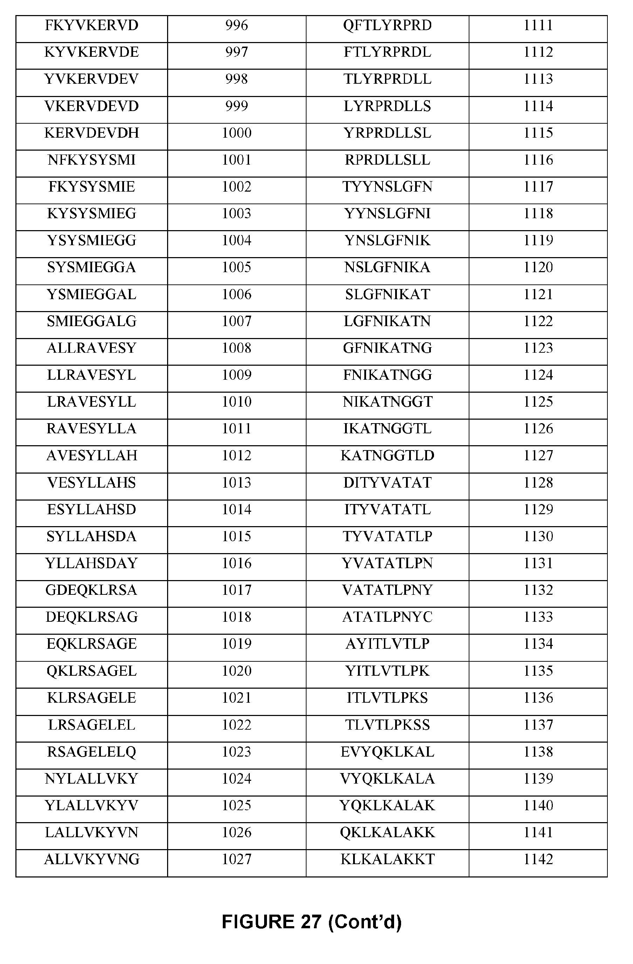

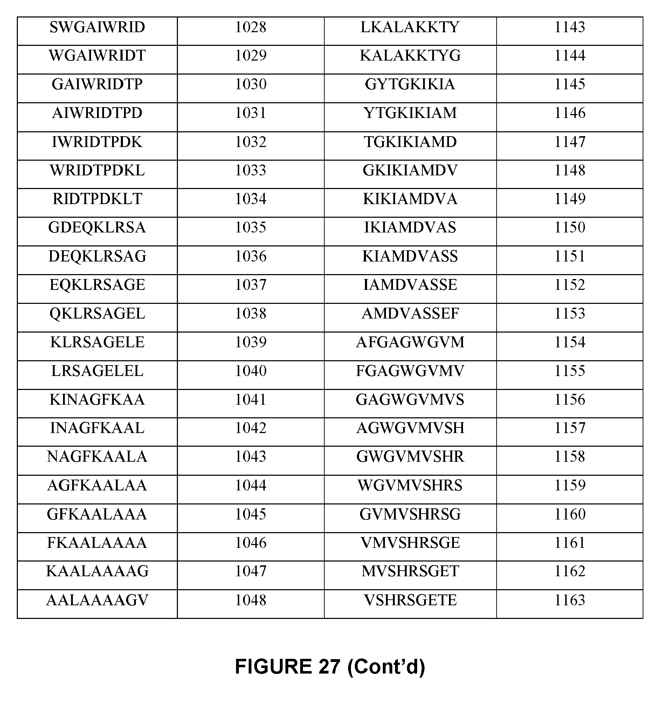

The invention may optionally exclude peptides comprising one or more of SEQ ID NOs:933-1163 (Figure 27), or peptides having a contiguous sequence of 7, 8 or 9 amino acids that has one of 90%, 91%, 92%, 93%, 94%, 95%, 96%, 97%, 98%, 99% or 100% sequence identity to one or more of SEQ ID Nos:933-1163 (Figure 27).

In another aspect of the present invention a peptide is provided wherein the peptide comprises the sequence of one of SEQ ID NOs:1-913. The amino acid sequence of the selected SEQ ID NO is preferably included in the peptide as a contiguous amino acid sequence.

In another aspect of the present invention a peptide is provided having at least 60% amino acid sequence identity to one of SEQ ID NOs:1-913. More preferably, the degree of sequence identity is one of 65%, 70%, 75%, 80%, 85%, 87%, 90%, 91%, 92%, 93%, 94%, 95%, 96%, 97%, 98%, 99% or 100% identity.

A peptide according to the present invention may have a maximum length of 50 amino acids and less than the full length of the corresponding protein allergen. More preferably the maximum peptide length is 40 amino acids, still more preferably 30 amino acids, still more preferably the maximum length is chosen from one of 29, 28, 27, 26, 25, 24, 23, 22, 21, 20, 19, 18, 17, 16, 15, 14, 13, 12, 11, 10 or 9 amino acids. For example, a peptide may have a maximum length of 20 amino acids or 15 amino acids.

A peptide according to the present invention may have a minimum length of 7 amino acids. More preferably the minimum length is chosen from one of 7, 8, 9, 10, 11, 12, 13, 14, 15, 16, 17, 18, 19 or 20 amino acids.

A peptide according to the present invention may have any length between said minimum and maximum. Thus, for example, a peptide may have a length of from 8 to 30, 10 to 25, 12 to 20, 9 to 15 amino acids, 8 to 11 amino acids, 9 to 11 amino acids, 9 to 13 amino acids or 9 to 14 amino acids. In particular, the peptide may have an amino acid length chosen from one of 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 amino acids, such as 9 or 15 amino acids.

The present invention incorporates peptide derivatives and peptide mimetics of any one of SEQ ID NO.s:1-913.

Peptide derivatives include variants of a given SEQ ID NO and may include naturally occurring allelic variants and synthetic variants which have substantial amino acid sequence identity to the peptide sequence as identified in the wild type full length protein allergen.

Peptide derivatives may include those peptides having at least 60% amino acid sequence identity to one of SEQ ID NOs:1-913 and which are capable of stimulating an immune response.

Typically a peptide derivative shows similar or improved MHC binding compared to the parent sequence, e.g. one of SEQ ID NOS:1-913. Preferably a peptide derivative shows promiscuous binding to MHC Class II molecules.

Peptide derivatives may include peptides having at least one amino acid modification (e.g. addition, substitution, and/or deletion of one or more amino acids) compared to one of SEQ ID NOs:1-913.

Peptide derivatives preferably differ from one of SEQ ID NOS:1-913 by less than 5 amino acids. More preferably, the number of different amino acids is 4 amino acids or less, 3 amino acids or less, 2 amino acids or less or only 1 amino acid.

Peptide derivatives may arise through natural variations or polymorphisms which may exist between the members of a protein allergen family from which the peptide is derived. All such derivatives are included within the scope of the invention.

Peptide derivatives may result from natural or non-natural (e.g. synthetic) interventions leading to addition, replacement, deletion or modification of the amino acid sequence of one of SEQ ID NOs:1-913.

Conservative replacements and modifications which may be found in such polymorphisms may be between amino acids within the following groups:
(i) alanine, serine, threonine;
(ii) glutamic acid and aspartic acid;
(iii) arginine and leucine;
(iv) asparagine and glutamine;
(v) isoleucine, leucine and valine;
(vi) phenylalanine, tyrosine and tryptophan;
(vii) methionine and leucine;
(viii) cysteine and valine.

Peptide derivatives may be peptide truncates of one or more of SEQ ID NOs:1-913, e.g. one or more of SEQ ID NOs:59-913. A peptide truncate has the same amino acid sequence as one of SEQ ID NOs:1-913 except for the deletion of one or more amino acids. 1, 2, 3, 4, or 5 amino acids may be deleted to provide a peptide truncate. A set of peptide truncates may be prepared in which 1, 2, 3, 4 or 5 amino acids are absent from either the C- or N- terminus of one of SEQ ID Nos:1-913, e.g. one of SEQ ID NOs:59-913 to provide a set of up to 10 peptide truncates. Whilst peptide truncates may be prepared by removing the required number of amino acids from the C- or N- terminus of one of SEQ ID NOs:1-913 it is preferred to directly synthesise the required shorter peptide in accordance with the amino acid sequence of the desired peptide truncate.

Peptide truncates can also be synthesised to have a sequence that corresponds to one of SEQ ID NOs:1-913, e.g. one of SEQ ID NOs:59-913 , where 1, 2, 3, 4 or 5 amino acids in internal positions in the peptide are deleted.

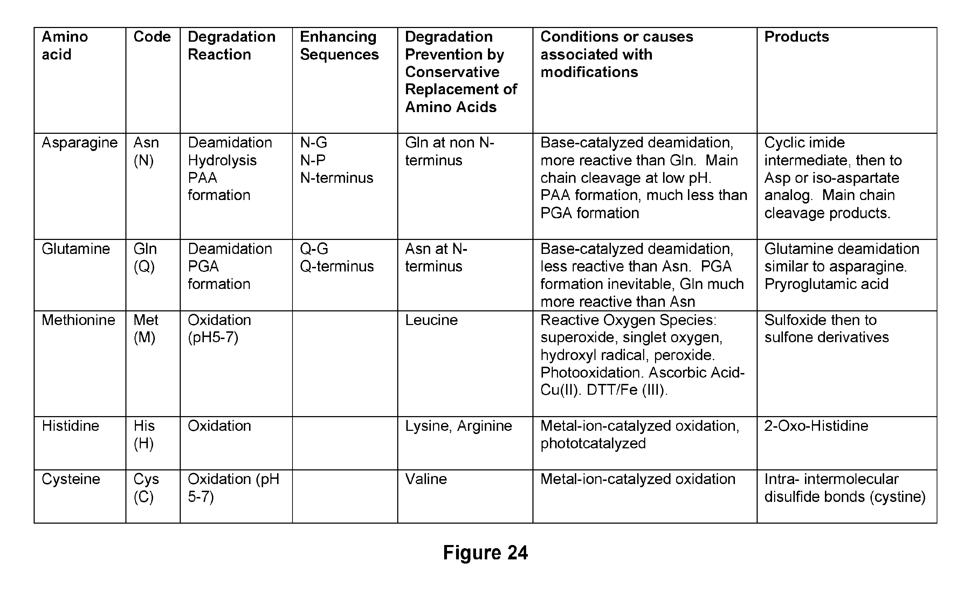

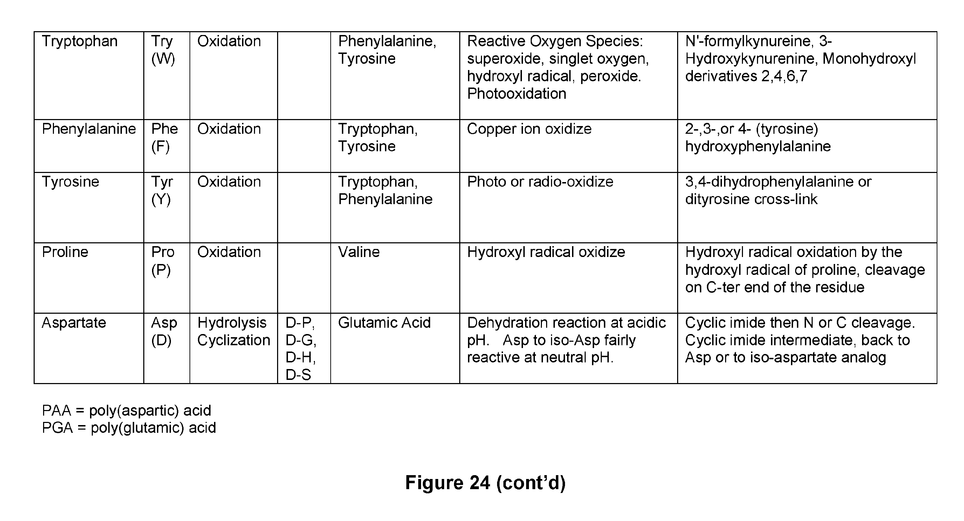

Peptide derivatives may also be provided by modifying one of SEQ ID NO.s:1-913 to resist degradation of the peptide. Figure 24 summarises modifications that may be made to the peptides to help resist peptide degradation and enhance peptide half-life in vitro and in vivo. These modifications may improve in vitro peptide stability and long-term storage. Figure 24 also indicates enhancing sequences that may increase the rate of reaction of an adjacent or nearby amino acid.

Peptide derivatives may be provided by modifying one of SEQ ID NO.s:1-913 for protease resistance, for example by inclusion of chemical blocks for exoproteases.

SEQ ID NOs:444, 609, 688, 718, 790, 820 are derivatives in that each peptide comprises an M/L substitution compared to the corresponding parent allergen sequence.

Similarly, SEQ ID NOs:217, 255, 285, 384, 414, 444, 639, 760, 850 are derivatives in that each peptide comprises a C/V substitution compared to the parent allergen sequence. Peptide derivatives may also be provided by modifying one of SEQ ID NOs:1-913, e.g. one of SEQ ID NOs:59-913, to alter the immunomodulatory properties of the peptide. These derivatives are sometimes referred to as altered peptide ligands (APLs) (25). APLs typically produce an altered immune response compared to the unaltered (e.g. wild type) peptide. For example, an APL may induce increased or decreased T cell activation, altered cytokine profile in activated T cells, and/or altered MHC binding compared to the unaltered peptide. Preferably an APL displays promiscuous binding of MHC molecules as described herein.

Peptide derivatives may be assayed for their ability to induce an immune response, e.g. T cell proliferation and/or cytokine production in a T cell population, in order to identify a peptide pharmacophore representing the minimal or optimised peptide epitope capable of stimulating an immune response and that may be useful in therapy. The immune response induced by a peptide may be one or more of:
(i) in vitro T cell proliferation, e.g. as measured by peptide stimulation of bromodeoxyuridine or ³H-thymidine incorporation in in vitro cultured PBMC, and/or
(ii) secretion of cytokines, e.g. IFNγ and/or IL-4, by in vitro cultured PBMC or T cells, e.g. T helper cells, and/or
(iii) a Th1 or Th2 response (e.g. as measured by secretion of cytokines such as IFNγ or I L-4 respectively).

Peptide derivatives such as APLs may be screened for MHC binding, in particular for binding to HLA Class II molecules.

The invention includes a method of identifying a peptide derivative of one of SEQ ID NOs:1-913 that is capable of stimulating an immune response. The method comprises the steps of (i) providing a candidate peptide derivative and (ii) testing the ability of the candidate peptide derivative to induce an immune response.

Part (i) may comprise synthesising the candidate peptide derivative, which may be a peptide mimetic or APL. Alternatively, part (i) may comprise chemically modifying the structure of one of SEQ ID NOs:1-913 so as to produce a candidate peptide derivative. Part (i) may comprise synthesis of peptide truncates or derivatives. The candidate peptide derivative will preferably have at least 60% sequence identity to one of SEQ ID NOs:1-913.

Chemical modification of one of SEQ ID NOs:1-913 may, for example, comprise deletion of one or more amino acids, addition of one or more amino acids or chemical modification of one or more amino acid side chains.

Part (ii) may comprise screening a candidate peptide derivative for MHC binding, in particular for binding to HLA Class II molecules. Especially, part (ii) may comprise testing a candidate peptide derivative for promiscuous binding to MHC Class II molecules. In silico screening may be carried out using virtual HLA Class II matrices, such as the ProPred software described herein. An in vitro binding assay may be used to assess binding to HLA Class II molecules, such as the Prolmmune Reveal® assay described herein.

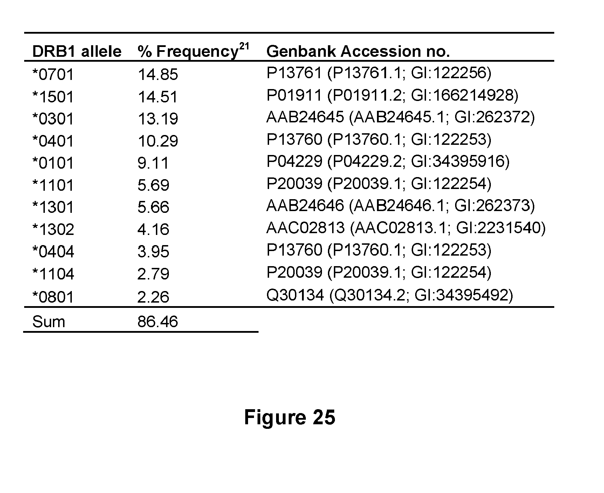

Preferably a peptide derivative, e.g. an APL, is a promiscuous binder of MHC Class II alleles. Typically a promiscuous binding epitope binds over 50%, for example, at least 60% or at least 70%, of the HLA-DR alleles expressed by European Americans. The 11 most common alleles expressed by European Americans are shown in Figure 25. Preferably a promiscuous binding epitope binds one of at least 2, 3, 4, 5, 6, 7, 8, 9, 10 or all 11 of the HLA-DR alleles in Figure 25. In one aspect a peptide derivative may bind at least 2, 3, 4, 5, 6, 7, 8, 9, 10 or 11 of the alleles in Figure 25 and also the HLA-DR allele *1401. The method may therefore comprise selecting a peptide that binds promiscuously.

Part (ii) may comprise contacting the candidate peptide derivative with a population of T cells and assaying T cell proliferation. Additionally, or alternatively, part (ii) may comprise contacting the candidate peptide derivative with a population of T cells and monitoring cytokine production, such as production of IFNγ and/or IL-4. The T cells are preferably T helper cells. The T cells may be provided as an in vitro culture of PBMC.

The method may further comprise the step of selecting one or more candidate peptide derivatives that stimulate T cell proliferation and detecting the production of cytokines in order to determine the induction of a Th1 or Th2 response. Preferably, the method comprises detection of IFNγ and/or IL-4. The method may further comprise selecting a peptide that induces a Th1 or Th2 response.

Methods according to the present invention may be performed in vitro or in vivo. The term "in vitro" is intended to encompass experiments with cells in culture whereas the term "in vivo" is intended to encompass experiments with intact multi-cellular organisms. Where the method is performed in vitro it may comprise a high throughput screening assay. Test compounds used in the method may be obtained from a synthetic combinatorial peptide library, or may be synthetic peptides or peptide mimetic molecules. Method steps (i) and (ii) are preferably performed in vitro, e.g. in cultured cells. Cells may be of any suitable cell type, e.g. mammalian, bacterial or fungal. Host cell(s) may be non-human, e.g. rabbit, guinea pig, rat, mouse or other rodent (including cells from any animal in the order Rodentia), cat, dog, pig, sheep, goat, cattle, horse, non-human primate or other non-human vertebrate organism; and/or non-human mammalian; and/or human. Suitable cells, e.g. PBMCs, may be obtained by taking a blood sample.

Part (ii) of the method may additionally comprise testing a candidate peptide derivative in animal models or patient populations for therapeutic effects on fungal allergy or fungal infection.

Peptides according to the present invention may be useful in the prevention or treatment of disease. In particular, peptides according to the present invention may be used to prepare pharmaceutical compositions. The pharmaceutical compositions may comprise medicaments or vaccines.

A pharmaceutical composition may be provided comprising a predetermined quantity of one or more peptides according to the present invention. Pharmaceutical compositions according to the present invention may be formulated for clinical use and may comprise a pharmaceutically acceptable carrier, diluent or adjuvant.

Pharmaceutical compositions of the invention are purified reproducible preparations which are suitable for human therapy. Preferred compositions of the invention comprise at least one isolated, purified peptide, free from all other polypeptides or contaminants, the peptide having a defined sequence of amino acid residues which comprises at least one T cell epitope of an antigen of interest.

As used herein, the term "isolated" refers to a peptide which is free of all other polypeptides, contaminants, starting reagents or other materials, and which is not conjugated to any other molecule.

A pharmaceutical composition of the invention is capable of down regulating an antigen specific immune response to an antigen of interest in a population of humans or animals subject to the antigen specific immune response such that disease symptoms are reduced or eliminated, and/or the onset or progression of disease symptoms is prevented or slowed.

Compositions and methods of the invention may be used to treat sensitivity to protein allergens in humans such as allergies to fungi, particularly to *Alternaria* spp.

Accordingly, in a further aspect of the invention a peptide according to the present invention is provided for use in the prevention or treatment of disease.

In another aspect of the present invention a peptide according to the present invention is provided for use in a method of medical treatment. The medical treatment may comprise treatment of a disease, e.g. allergic disease.

In another aspect of the present invention the use of a peptide according to the present invention in the manufacture of a medicament for the prevention or treatment of disease is provided.

In another aspect of the present invention a method is provided for preventing or treating disease in a patient in need of treatment, the method comprising administering to the patient a therapeutically effective amount of a peptide or pharmaceutical composition according to the present invention.

In accordance with the present invention methods are also provided for the production of pharmaceutically useful compositions, which may be based on a peptide or peptide derivative according to the present invention. In addition to the steps of the methods described herein, such methods of production may further comprise one or more steps selected from:
(a) identifying and/or characterising the structure of a selected peptide or peptide derivative;
(b) obtaining the peptide or peptide derivative;
(c) mixing the selected peptide or peptide derivative with a pharmaceutically acceptable carrier, adjuvant or diluent.

For example, a further aspect of the present invention relates to a method of formulating or producing a pharmaceutical composition for use in the treatment of disease, the method comprising identifying a peptide or peptide derivative in accordance with one or more of the methods described herein, and further comprising one or more of the steps of:
(i) identifying the peptide or peptide derivative; and/or
(ii) formulating a pharmaceutical composition by mixing the selected peptide or peptide derivative, with a pharmaceutically acceptable carrier, adjuvant or diluent.

As such, the method may comprise providing a peptide which peptide comprises the sequence of one of SEQ ID NOs:1-913, and formulating a pharmaceutical composition by mixing the selected peptide or peptide derivative with a pharmaceutically acceptable carrier, adjuvant or diluent.

The peptide or peptide derivative may be present in the pharmaceutical composition in the form of a physiologically acceptable salt.

Methods of medical treatment may involve administering more than one peptide according to the invention to the patient. Administering two, three or more peptides derived from a single allergen, or derived from multiple allergens, may be used to ensure that peptide epitopes that bind to a large number of HLA alleles are provided. For example, one may wish to ensure that the treatment includes administration of peptide epitopes derived from a given allergen that collectively bind to all 11 alleles of Figure 25. Administration of multiple peptides may be simultaneous, separate or sequential and may form part of a combination therapy.

Accordingly, a pharmaceutical composition or medicament according to the invention may comprise more than one peptide epitope of the invention and may be active against one or more allergens. The one or more allergens may be derived from one or more species, e.g. fungal species such as *Alternaria* spp. Such compositions and medicaments may contain more than one peptide and/or peptide derivative and/or peptide mimetic according to the invention, for example, 1, 2, 3, 4, 5, 6, 7, 8, 9, 10, 11, 12, 13, 14, 15, 16, 17, 18, 19, 20 or more peptides, peptide derivatives and/or peptide mimetics. These may be derived from the same or different protein allergen(s).

For example, in some embodiments a pharmaceutical composition may comprise 2, 3, 4, 5, 6, 7, 8, 9, 10, 11, 12, 13, 14, 15, 16, 17, 18, 19, 20, 21, 22, 23, 24, 25, 26, or 27 peptides selected from the following list but wherein no more than three (preferably no more than two or one) peptide(s) from each of the following numbered groups is present:
(i) SEQ ID NO: 1, SEQ ID NOs: 59-80
(ii) SEQ ID NO: 2, SEQ ID NOs: 81-92
(iii) SEQ ID NO: 3, SEQ ID NOs: 93-105
(iv) SEQ ID NO: 4, SEQ ID NOs: 106-120
(v) SEQ ID NO: 5, SEQ ID NOs: 121-135
(vi) SEQ ID NO: 6, SEQ ID NOs: 136-150
(vii) SEQ ID NO: 7, SEQ ID NOs: 151-165
(viii) SEQ ID NO: 8, SEQ ID NOs: 166-180
(ix) SEQ ID NO: 9, SEQ ID NOs: 181-195
(x) SEQ ID NO: 10, SEQ ID NOs: 196-210
(xi) SEQ ID NO: 11, SEQ ID NOs: 211-233
(xii) SEQ ID NO: 12, SEQ ID NOs: 234-248
(xiii) SEQ ID NO: 13, SEQ ID NOs: 249-263
(xiv) SEQ ID NO: 14, SEQ ID NOs: 264-278
(xv) SEQ ID NO: 15, SEQ ID NOs: 279-293
(xvi) SEQ ID NO: 16, SEQ ID NOs: 294-307
(xvii) SEQ ID NO: 17, SEQ ID NOs: 308-317
(xviii) SEQ ID NO: 18, SEQ ID NOs: 318-332
(xix) SEQ ID NO: 19, SEQ ID NOs: 333-347
(xx) SEQ ID NO: 20, SEQ ID NOs: 348-362
(xxi) SEQ ID NO: 21, SEQ ID NOs: 363-377
(xxii) SEQ ID NO: 22, SEQ ID NOs: 378-392
(xxiii) SEQ ID NO: 23, SEQ ID NOs: 393-407
(xxiv) SEQ ID NO: 24, SEQ ID NOs: 408-422
(xxv) SEQ ID NO: 25, SEQ ID NOs: 423-437
(xxvi) SEQ ID NO: 26, SEQ ID NOs: 438-452
(xxvii) SEQ ID NO: 27, SEQ ID NOs: 453-467.

In some embodiments a pharmaceutical composition may comprise 2, 3, 4, 5, 6, 7, 8, 9, or 10 peptides selected from the following list but wherein no more than three (preferably no more than two or one) peptides from each of the following numbered groups is present:
(i) SEQ ID NO: 4, SEQ ID NOs: 106-120
(ii) SEQ ID NO: 5, SEQ ID NOs: 121-135
(iii) SEQ ID NO: 8, SEQ ID NOs: 166-180
(iv) SEQ ID NO: 9, SEQ ID NOs: 181-195
(v) SEQ ID NO: 11, SEQ ID NOs: 211-233
(vi) SEQ ID NO: 13, SEQ ID NOs: 249-263
(vii) SEQ ID NO: 22, SEQ ID NOs: 378-392
(viii) SEQ ID NO: 24, SEQ ID NOs: 408-422
(ix) SEQ ID NO: 26, SEQ ID NOs: 438-452
(x) SEQ ID NO: 27, SEQ ID NOs: 453-467.

In some embodiments a pharmaceutical composition may comprise 2, 3, 4, 5, 6, 7, 8 or 9 peptides selected from the following list but wherein no more than three (preferably no more than two or one) peptides from each of the following numbered groups is present:
(i) SEQ ID NO: 1, SEQ ID NOs: 59-80
(ii) SEQ ID NO: 6, SEQ ID NOs: 136-150
(iii) SEQ ID NO: 7, SEQ ID NOs: 151-165
(iv) SEQ ID NO: 10, SEQ ID NOs: 196-210
(v) SEQ ID NO: 15, SEQ ID NOs: 279-293
(vi) SEQ ID NO: 16, SEQ ID NOs: 294-307
(vii) SEQ ID NO: 18, SEQ ID NOs: 318-332
(viii) SEQ ID NO: 19, SEQ ID NOs: 333-347
(ix) SEQ ID NO: 20, SEQ ID NOs: 348-362.

In some embodiments a pharmaceutical composition may comprise 2, 3, 4, 5, 6, 7, 8, 9, 10, 11, or 12 peptides selected from the following list but wherein no more than three (preferably no more than two or one) peptides from each of the following numbered groups is present:
(i) SEQ ID NO: 28, SEQ ID NOs: 468-482
(ii) SEQ ID NO: 31, SEQ ID NOs: 513-527
(iii) SEQ ID NO: 32, SEQ ID NOs: 528-542
(iv) SEQ ID NO: 33, SEQ ID NOs: 543-557
(v) SEQ ID NO: 35, SEQ ID NOs: 573-587
(vi) SEQ ID NO: 37, SEQ ID NOs: 603-617
(vii) SEQ ID NO: 43, SEQ ID NOs: 697-711
(viii) SEQ ID NO: 45, SEQ ID NOs: 718-723
(ix) SEQ ID NO: 46, SEQ ID NOs: 724-738
(x) SEQ ID NO: 50, SEQ ID NOs: 784-798
(xi) SEQ ID NO: 53, SEQ ID NOs: 829-843
(xii) SEQ ID NO: 57, SEQ ID NOs: 893-898.

In some embodiments a pharmaceutical composition may comprise 2, 3, 4, 5, 6, 7, 8, 9, 10, 11, or 12 peptides selected from the following list but wherein no more than three (preferably no more than two or one) peptides from each of the following numbered groups is present:
(i) SEQ ID NO: 29, SEQ ID NOs: 483-497
(ii) SEQ ID NO: 30, SEQ ID NOs: 498-512
(iii) SEQ ID NO: 34, SEQ ID NOs: 558-572
(iv) SEQ ID NO: 39, SEQ ID NOs: 633-647
(v) SEQ ID NO: 40, SEQ ID NOs: 648-662
(vi) SEQ ID NO: 42, SEQ ID NOs: 682-696
(vii) SEQ ID NO: 48, SEQ ID NOs: 754-768
(viii) SEQ ID NO: 52, SEQ ID NOs: 814-828
(ix) SEQ ID NO: 54, SEQ ID NOs: 844-862
(x) SEQ ID NO: 55, SEQ ID NOs: 863-877
(xi) SEQ ID NO: 56, SEQ ID NOs: 878-892
(xii) SEQ ID NO: 58, SEQ ID NOs: 899-913.

In yet a further aspect of the present invention nucleic acids encoding peptides according to the present invention are provided, together with their complementary sequences. The nucleic acid may have a maximum length of 1000 nucleotides, more preferably one of 200, 190, 180, 170, 160, 150, 140, 130, 120, 110, 100, 90, 80, 70, 60, 50, 40, 30, 25 nucleotides. The nucleic acid may have a minimum length of 24 nucleotides, more preferably one of 27, 30, 35, 40, 45, 50, 55 or 60 nucleotides.

A nucleic acid vector having nucleic acid encoding a peptide of the present invention is also provided. The vector may be an expression vector, e.g. a plasmid, in which a nucleic acid sequence encoding a peptide of the present invention is operably linked to a suitable promoter and/or other regulatory sequence. A host cell transfected with such a vector is also provided.

In this specification the term "operably linked" may include the situation where a selected nucleotide sequence and regulatory or control nucleotide sequence are covalently linked in such a way as to place the expression of a nucleotide sequence under the influence or control of the regulatory sequence. Thus a regulatory or control sequence is operably linked to a selected nucleotide sequence if the regulatory sequence is capable of effecting transcription of a nucleotide sequence which forms part or all of the selected nucleotide sequence. Where appropriate, the resulting transcript may then be translated into a desired peptide.

The vector may be configured to enable transcription of mRNA encoding the peptide upon transfection into a suitable cell. Transcribed mRNA may then be translated by the cell such that the cell expresses the peptide.

A cell having a nucleic acid sequence encoding a peptide of the present invention operably linked to a suitable promoter and/or other transcription regulatory element or control sequence integrated in the genome of the cell is also provided.

Nucleic acids according to the invention may be single or double stranded and may be DNA or RNA.

Diseases or conditions that may be prevented or treated include allergic disease. Examples of allergic disease include asthma, allergic asthma, fungal asthma, SAFS, ABPA, allergic bronchopulmonary mycoses, allergic sinusitis, rhinitis, allergic rhinitis, hypersensitivity pneumonitis, atopic eczema. Other diseases or conditions that may be prevented or treated include fungal infection, Aspergillosis (e.g. invasive, non-invasive, chronic pulmonary, aspergilloma).

Peptide therapy may comprise the use of peptides according to the invention in the prevention/prophylaxis of disease or in the treatment of disease. As such, therapy may comprise relief or reduction of symptoms such as airway inflammation, difficulty in breathing, swelling, itchiness, allergic rhinitis, allergic sinusitis, eosinophilia, hypersensitivity to fungal allergens and/or spores. A reduction in asthmatic symptoms may be measured by conventional techniques, such as measuring peak flow, white blood cell count, patch testing.

Peptides according to the present invention may be useful as prophylactics for the prevention of allergy responses to fungal allergens, particularly allergens from *Alternaria alternata.*

Patients to be treated may be any animal or human. The patient may be a non-human mammal, but is more preferably a human. Subjects, individuals or patients to be treated may be male or female. In one aspect, patients are of a selected ethnicity, which may include one or more of (by birth or residence): (i) European, (ii) from a Member State of the European Union, (iii) North American, e.g. from USA and/or Canada. Patients to be treated may be European American and/or Caucasian.

Medicaments and pharmaceutical compositions according to aspects of the present invention may be formulated for administration by a number of routes, including intravenous, intradermal, intramuscular, oral and nasal. The medicaments and compositions may be formulated in fluid or solid form. Fluid formulations may be formulated for administration by injection to a selected region of the human or animal body. Pharmaceutical compositions may comprise peptides encapsulated in liposomes, e.g. formed from polyglycerol esters.

Administration of peptides or pharmaceutical compositions for therapeutic purposes is preferably in a "therapeutically effective amount", this being sufficient to show benefit to the individual. The actual amount administered, and rate and time-course of administration, will depend on the nature and severity of the disease being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 20th Edition, 2000, pub. Lippincott, Williams & Wilkins.

A composition may be administered alone or in combination with other treatments, either simultaneously or sequentially, dependent upon the condition to be treated.

Efficacious peptide immunotherapy may require the repeat administration of a pharmaceutical composition according to the present invention. For example, a dosage regime comprising a series of injections of the pharmaceutical composition may be required in order to treat existing allergic disease symptoms and to provide a vaccination effect against future allergic disease caused by fungal allergens.

Peptides comprising or consisting of SEQ ID NOS:1-913 are disclosed along with variants and derivatives thereof, including peptides having conservative alterations. These peptides are each proposed for use in the treatment of fungal allergy, preferably allergic disease caused by *A.alternata.*

The peptides identified may be synthesised by standard techniques (e.g. using commercially available peptide synthesis services such as that provided by Invitrogen, Carlsbad, CA, USA) and tested for use as a therapeutic or vaccine against fungal infection or fungal allergy.

Various methods of chemically synthesizing peptides are known in the art such as solid phase synthesis which has been fully or semi- automated on commercially available peptide synthesizers. Synthetically produced peptides may then be purified to homogeneity (i.e. at least 90%, more preferably at least 95% and even more preferably at least 97% purity), free from all other polypeptides and contaminants.

Peptide compositions may then be characterized by a variety of techniques well known to those of ordinary skill in the art such as mass spectroscopy, amino acid analysis and sequencing and HPLC.

Peptides useful in the methods of the present invention may also be produced using recombinant DNA techniques in a host cell transformed with a nucleic acid sequence coding for such peptide. When produced by recombinant techniques, host cells transformed with nucleic acid encoding the desired peptide are cultured in a medium suitable for the cells and isolated peptides can be purified from cell culture medium, host cells, or both using techniques known in the art for purifying peptides and proteins including ion-exchange chromatography, ultra filtration, electrophoresis or immunopurification with antibodies specific for the desired peptide. Peptides produced recombinantly may be isolated and purified to homogeneity, free of cellular material, other polypeptides or culture medium for use in accordance with the methods described above.

Pharmaceutical compositions of the invention should be sterile, stable under conditions of manufacture, storage, distribution and use and should be preserved against the contaminating action of microorganisms such as bacteria and fungi. A preferred means for manufacturing a pharmaceutical composition of the invention in order to maintain the integrity of the composition is to prepare the formulation of peptide and pharmaceutically acceptable carrier(s) such that the composition may be in the form of a lyophilized powder which is reconstituted in a pharmaceutically acceptable carrier, such as sterile water, just prior to use.

Biodegradable poly(D,L-lactic-co-glycolic) acid (PGLA) particles has been suggested for delivery of peptides for treatment of allergy (Scholl et al. Immunol. Allergy Clin. N. Am. 2006. 26:349-364.).

T-cell epitope validation can be performed by assaying peptide-induced proliferation of peripheral blood mononuclear cells (PBMC) obtained from subjects having fungal allergy or fungal infection and from control subjects not having fungal allergy or fungal infection. HLA-DR typing of subject PBMCs may also be performed to confirm the promiscuous binding nature of the peptides.

The status of the proliferated T helper cells may also be determined and used to assist in validation of peptides as therapeutic or vaccine candidates. Th1 cells participate in cell-mediated immunological responses. Th2 cells participate in antibody mediated immunity.

Th1/Th2 status can be determined by examining the cytokine profile of the proliferated cells (27). Production of interferon γ (IFNγ) and optionally one or more of interleukin 2 (IL-2), tumor necrosis factor β (TNFβ) and granulocyte-macrophage colony stimulating factor (GM-CSF) is indicative of Th1 status. Typically this indicates a non-allergic cellular immune response. Production of interleukin 4 (IL-4) and optionally one or more of interleukin 3 (IL-3), interleukin 5 (IL-5), interleukin 6 (IL-6), interleukin 10 (IL-10) and interleukin 13 (IL-13) is indicative of Th2 status. Often this is associated with an allergic Th2 response. Production of both IFNγ and IL-4 is indicative of Th0 status. Production of IL-10 is associated with a Treg non-allergic response. (27)

Th2 cells play an important role in the immunological processes of allergic asthma (11) and Th2 associated cytokines such as IL-4, IL-5, IL-9 and IL-13 are implicated in the development of allergen specific Th2 cells, IgE production, airway eosinophilia and airway hyper-responsiveness. Inhibition or suppression of allergen-specific Th2 cells and their cytokines provides a strategy for intervention.

Such inhibition or suppression may be achieved by selecting Th1 stimulating peptides leading to suppression of the Th2 response (11). Alternatively, Th2 stimulating peptides administered via different routes (oral, lymph node injection or intravenous) and by specific dose variation may be used to suppress an allergen induced Th2 response through a bystander effect. The bystander effect is defined as an influence on the immune response to a particular antigen(s) of interest by the immune response to other unrelated antigens, usually mediated by a local cytokine and cellular environment. The bystander effect can result in an amplification of an immune response (22) or a suppression of a response (23).

Low-dose T-cell epitope peptides from allergen proteins are proposed to cause antigen specific hypo-responsiveness associated with the induction of a suppressive population of CD4+ T cells, together with up regulation of surface CD5 levels on antigen-specific T cells (12). Intravenous injection of a single peptide induces a bystander suppression and thus can provide protection against a multicomponent allergen trigger (13).

Accordingly, in addition to assaying for T cell proliferation (e.g. based on Bromodeoxyuridine (BRdU) or ³H thymidine incorporation), cytokine assays' may be performed to detect secretion of one or more of IFNγ, IL-2, TNFβ, GM-CSF, IL-4, IL-3, IL-5, IL-6, IL-10 and IL-13. Further assays to detect the presence of an IgE response and/or eosinophilia may also be performed.

Human T cell stimulating activity can be tested by culturing T cells obtained from an individual sensitive to a predetermined protein antigen with a peptide derived from the antigen and determining whether proliferation of T cells occurs in response to the peptide as measured, e.g., by cellular uptake of ³H thymidine. Stimulation indices for responses by T cells to peptides can be calculated as the maximum counts per minute (CPM) in response to a peptide divided by the control CPM. A T cell stimulation index (S.I.) equal to or greater than two times the background level is considered "positive". Positive results are used to calculate the mean stimulation index for each peptide for the group of peptides tested.

Preferred peptides have a mean T cell stimulation index of greater than or equal to 2.0. A peptide having a T cell stimulation index of greater than or equal to 2.0 is considered useful as a therapeutic agent. Preferred peptides have a mean T cell stimulation index of at least 2.5, more preferably at least 3.5, even more preferably at least 4.0, and most preferably at least 5.0.

The positivity index (P.I.) for a peptide is determined by multiplying the mean T cell stimulation index by the percent of individuals, in a population of individuals tested, sensitive to the antigen being tested (e.g., preferably at least 9 individuals, more preferably at least 16 individuals or more, more preferably at least 20 individuals or more, or even more preferably at least 30 individuals or more), who have T cells that respond to the peptide. The positivity index represents the strength of a T cell response to a peptide (S.I.) and the frequency of a T cell response to a peptide in a population of individuals sensitive to the antigen being tested. Preferred peptides may also have a positivity index (P.I.) of at least about 100, more preferably at least 150, even more preferably at least about 200 and most preferably at least about 250.

Cytokine production may be analysed using any of the methods described herein. One such method employs an Enzyme-linked ImmunoSpot (ELISPOT) assay. The ELISPOT assay will allow the analysis of cells at the single cell level for cytokine production, and thus provides a method for determining the number of individual T cells secreting a cytokine after stimulation with a specific antigen or peptide (28). The ELISPOT assay typically uses two high-affinity cytokine-specific antibodies directed against different epitopes on the same cytokine molecule. Spots are generated with a colorimetric reaction in which soluble substrate is cleaved, leaving an insoluble precipitate at the site of the reaction. The spot represents a foot-print of the original cytokine producing cell. The number of spots is a direct measurement of the frequency of cytokine-producing T cells.

The production of cytokines by T-cells in PMBC cell cultures in response to allergen indicates that stimulation has occurred and identification of the cytokine pattern allows a comparison of the type of cellular response.

Peptides selected through in vitro validation assays such as those described above may be tested in animal models or patient populations for therapeutic effects on fungal allergy or fungal infection, e.g. as described in Kheradmand et al (24). For example, a mouse model may be used, such as BALB/c(H2^{d}) mice. Patients or animals may receive a series of peptide formulations, e.g. by injection, and fungal infection or allergy symptoms and characteristics monitored. Such symptoms and characteristics may include airway inflammation, eosinophilia, rhinitis, cytokine secretion, Th1 or Th2 response status. Suitably, a control patient population receiving placebo formulations may be used to assess efficacy of the peptide formulation.

### Peptide Mimetics

The designing of mimetics to a known pharmaceutically active compound is a known approach to the development of pharmaceuticals based on a "lead" compound. This might be desirable where the active compound is difficult or expensive to synthesise or where it is unsuitable for a particular method of administration, e.g. some peptides may be unsuitable active agents for oral compositions as they tend to be quickly degraded by proteases in the alimentary canal. Mimetic design, synthesis and testing is generally used to avoid randomly screening large numbers of molecules for a target property.

There are several steps commonly taken in the design of a mimetic from a compound having a given target property. Firstly, the particular parts of the compound that are critical and/or important in determining the target property are determined. In the case of a peptide, this can be done by systematically varying the amino acid residues in the peptide, e.g. by substituting each residue in turn. These parts or residues constituting the active region of the compound are known as its "pharmacophore".

Once the pharmacophore has been found, its structure is modelled according to its physical properties, e.g. stereochemistry, bonding, size and/or charge, using data from a range of sources, e.g. spectroscopic techniques, X-ray diffraction data and NMR. Computational analysis, similarity mapping (which models the charge and/or volume of a pharmacophore, rather than the bonding between atoms) and other techniques can be used in this modelling process.

In a variant of this approach, the three-dimensional structure of the ligand and its binding partner are modelled. This can be especially useful where the ligand and/or binding partner change conformation on binding, allowing the model to take account of this in the design of the mimetic.

A template molecule is then selected onto which chemical groups which mimic the pharmacophore can be grafted. The template molecule and the chemical groups grafted on to it can conveniently be selected so that the mimetic is easy to synthesise, is likely to be pharmacologically acceptable, and does not degrade *in vivo,* while retaining the biological activity of the lead compound. The mimetic or mimetics found by this approach can then be screened to see whether they have the target property, or to what extent they exhibit it. Further optimisation or modification can then be carried out to arrive at one or more final mimetics for *in vivo* or clinical testing.

With regard to the present invention, a peptide mimetic is one form of peptide derivative. A method of identifying a peptide derivative capable of stimulating an immune response may comprise the step of modifying the peptide structure to produce a peptide mimetic. This peptide mimetic may optionally be subject to testing in a T cell proliferation assay, and/or in cytokine secretion assays (e.g. assaying for IFN γ or IL-4 production). This process of modification of the peptide or peptide mimetic and testing may be repeated a number of times, as desired, until a peptide having the desired effect, or level of effect, on T cell proliferation and/or cytokine secretion is identified.

The modification steps employed may comprise truncating the peptide or peptide mimetic length (this may involve synthesising a peptide or peptide mimetic of shorter length), substitution of one or more amino acid residues or chemical groups, and/or chemically modifying the peptide or peptide mimetic to increase stability, resistance to degradation, transport across cell membranes and/or resistance to clearance from the body.

### Altered Peptide Ligands (APLs)

Altered peptide ligands (APLs) are modified versions of peptide epitopes, with altered immunomodulatory properties (25).

A Th1-skewing APL has been reported, having a single 336N/A substitution compared to the wild type peptide epitope (implicated in allergic asthma) and which inhibits the allergic Th2 response in a mouse model of allergic asthma (11).

An APL of an immunodominant epitope of lipocalin allergen Bos d2 has also been reported which produces a Th1/Th0 response in vitro compared to the Th2/Th0 response induced by the wild type epitope (29). The T cell population induced by the APL are cross-reactive with the wild type epitope (29).

Changes in the residues flanking the core epitope of the immunodominant myelin basic protein (MBP) peptide 84-102 have been reported to alter both MHC binding and T cell activation, the latter independently of MHC binding (30). It is suggested that C-terminal basic residues may enhance processing and presentation of an epitope.

With regard to the present invention, an APL is one form of peptide derivative.

An APL typically induces an altered immune response compared to the unaltered (usually wild type) peptide.

Immunomodulatory properties that may be altered include one or more of:

### T cell activation

T cell activation in response to the APL may be increased or decreased compared to the unmodified peptide. Activation may occur at a higher or lower dose of peptide. Some APLs are unable to originate T cell signalling and lead to an impairment of T cell activation (antagonist APLs). Some APLs elicit some but not all of the signals for full T cell activation (partial agonist APLs) (25).

### Cytokine profile

T cells activated by the peptide may secrete a different pattern of cytokines than T cells activated by the unmodified peptide. Thus, a modified peptide may induce a different type of T cell response, e.g. Th1 in place of Th2, Treg in place of Th2, or Th1 in place of Treg.

### MHC binding

An APL may show altered MHC binding compared to the unmodified peptide. In the present case it is preferred that an APL shows similar or improved MHC binding compared to the unaltered peptide. In particular it is preferred that an APL is a promiscuous binder of MHC Class II alleles.

The T cells activated by the APL may be cross reactive with the unmodified or wild type epitope.

A method of identifying a peptide derivative capable of stimulating an immune response as described herein may comprise the step of modifying the peptide structure to produce an APL with altered immunomodulatory properties as described herein.

Modifying the peptide may comprise modifying, substituting, adding or deleting one or more amino acids. Modifications which may be found in peptide derivatives are described herein.

For example, modifying a peptide may comprise systematically altering one or more amino acids in the peptide, e.g. substituting each amino acid in turn. For example, an initial screen may use an alanine scan to prepare a set of peptide derivatives from a starting peptide, each derivative being substituted with an alanine at a single position (11).

Modifying a peptide may comprise adding 1, 2, or 3 (or more) amino acids at the N-terminal end, the C-terminal end, or at both N-terminal and the C-terminal end.

Modification may be at an amino acid within any of SEQ ID NOS:1-913. Alternatively, modification may be at an amino acid in a region flanking any of these sequences, such as the N-terminal and/or C-terminal 1, 2, 3, 4, 5 or 6 amino acids. For example, one or more additional amino acids may be added, substituted or chemically modified at the N-terminal and/or C-terminal region of an epitope. Preferably one or more basic amino acids is included at the C-terminal end of a peptide.

Binding core 9-mers of class II DR epitopes have a general pattern of amino acid side chains important in binding to the MHC and important for binding of the MHC/peptide complex to the T-cell receptor. For a typical peptide epitope, alterations of residues P1, P4, P6 or P9 can alter peptide binding strength to MHC alleles while alterations of P2, P3, P5, P7 and P8 can alter the interactions of MHC/peptide complex with T-cell receptors. Altering the strength of binding of the MHC/peptide complex to the T-cell receptor is known to have the ability to change the fate of the original T-cell receptor clone as to cytokine polarization and/or interact with structurally related T-cell receptor clones not induced by the original peptide.

Candidate APL(s) may be assessed for binding to MHC Class II molecules, in particular HLA Class II molecules such as HLA-DR alleles. Typically an APL is tested for binding to HLA DR alleles which occur at a frequency of at least 40% in the European-American population, for example at least 50%, 60%, 70%, 80% or 90% in the population. Preferably an APL is tested for binding to at least 2, 3, 4, 5, 6, 7, 8, 9, 10, or 11 of the alleles in Figure 25 (and optionally also to the HLA DR *1401 allele).

Preferably an APL exhibits substantially similar or improved binding compared to the unaltered peptide. Preferably an APL shows promiscuous binding to HLA Class II molecules as described herein.

MHC binding may be assessed using *in silico* screening. Typically *in silico* screening, such as the ProPred software described herein, comprises use of virtual HLA Class II matrices. Additionally or alternatively, MHC binding may be assessed using an in vitro binding assay, such as the Prolmmune REVEAL® assay described herein.

Candidate APL(s) may be subject to testing in a T cell proliferation assay, and/or in cytokine secretion assays (e.g. assaying for IFN γ or IL-4 production) to determine the nature of the T cell response to the APL. For example, epitope specific T-cell lines and clones can be isolated from sensitized allergic donors. An APL modified from the native sequence may cross-react with the original clones induced by the native peptide and/or it may induce new T-cell receptor clones. Using an original line or clone induced by the native epitope for testing with APLs allows precise characterization of proliferation/cytokine pattern changes on the original population of clones due to amino acid changes in the peptide. Specific APLs that exhibit the desired properties can be tested for effects on whole TCR populations from the targeted patient population.

APLs selected through in vitro validation assays such as those described above may be tested in animal models or patient populations for therapeutic effects on fungal allergy or fungal infection as described herein.

This process of modification of the peptide and testing may be repeated a number of times, as desired, until a peptide having the desired effect, or level of effect, on T cell proliferation and/or cytokine secretion is identified.

In one aspect a peptide derivative herein refers to an APL of any one of SEQ ID NOs:1-913.

### Peptide Solubility

For some applications it is desirable for the peptide to be soluble in a liquid, e.g. water, saline solution or another pharmaceutically acceptable liquid carrier. Some hydrophobic peptides may first be dissolved in DMSO or other solvents and diluted into aqueous solution. Where the hydrophobic character of the peptide prevents such an approach the peptide may be modified to improve solubility. Modification of the peptide may be achieved in several ways well known to one of skill in the art, including the following.

One type of modification involves alteration of the peptide amino acid sequence to provide a peptide derivative in which one or more hydrophobic amino acids are substituted with amino acids of moderate or low hydrophobicity or with charged or uncharged polar amino acids.

Another type of modification involves modification of the N- and/or C-terminal ends of the peptide. Peptide derivatives may be provided in which the N-terminus is free and charged (NH₂-) or blocked with an acetyl group (AC-) or with Biotin. The C-terminus may also be free and charged (-COOH) or blocked (-CONH₂).

Another type of modification involves addition of one, two or three amino acids to the N-and/or C-terminus of the peptide to provide a longer peptide derivative. The additional amino acids may be any amino acids. In preferred embodiments the additional amino acids are chosen from the amino acids adjacent the N- or C-terminus of the peptide sequence as found in the protein amino acid sequence from which the peptide is derived. However, these may be modified to increase solubility.

Following modification to provide a peptide derivative the peptide derivative would be tested for retention of biological activity and for improvement in solubility.

### Sequence identity

Aspects of the invention concern compounds which are isolated peptides/polypeptides comprising an amino acid sequence having a sequence identity of at least 60% with a given sequence. Alternatively, this identity may be any one of 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100% sequence identity.

Percentage (%) sequence identity is defined as the percentage of amino acid residues in a candidate sequence that are identical with residues in the given listed sequence (referred to by the SEQ ID NO.) after aligning the sequences and introducing gaps if necessary, to achieve the maximum sequence identity, and not considering any conservative substitutions as part of the sequence identity. Sequence identity is preferably calculated over the entire length of the respective sequences.

Alignment for purposes of determining percent amino acid sequence identity can be achieved in various ways known to a person of skill in the art, for instance, using publicly available computer software such as ClustalW 1.82. T-coffee or Megalign (DNASTAR) software. When using such software, the default parameters, e.g. for gap penalty and extension penalty, are preferably used. The default parameters of ClustalW 1.82 are: Protein Gap Open Penalty = 10.0, Protein Gap Extension Penalty = 0.2, Protein matrix = Gonnet, Protein/DNA ENDGAP = -1, Protein/DNA GAPDIST = 4.

Identity of nucleic acid sequences may be determined in a similar manner involving aligning the sequences and introducing gaps if necessary, to achieve the maximum sequence identity, and calculating sequence identity over the entire length of the respective sequences.

The invention includes the combination of the aspects and preferred features described except where such a combination is clearly impermissible or expressly avoided.

The section headings used herein are for organizational purposes only and are not to be construed as limiting the subject matter described.

Aspects and embodiments of the present invention will now be illustrated, by way of example, with reference to the accompanying figures. Further aspects and embodiments will be apparent to those skilled in the art. All documents mentioned in this text are incorporated herein by reference.

### Brief Description of the Figures

Embodiments and experiments illustrating the principles of the invention will now be discussed with reference to the accompanying figures in which:
**Figure 1****.** Table of REVEAL® scores and stability indices for SEQ ID NOs:65, 112, 928, 127, 142, 157, 172, 187, 929, 202, 217, 255, 285, 300, 930, 324, 339, 354, 384, 414, 444, 459, 474, 489 against DRB*0101.
**Figure 2****.** Table of REVEAL® scores and stability indices for SEQ ID NOs:65, 112, 928, 127, 142, 157, 172, 187, 929, 202, 217, 255, 285, 300, 930, 324, 339, 354, 384, 414, 444, 459, 474, 489 against DRB*1501.
**Figure 3****.** Table of REVEAL® scores and stability indices for SEQ ID NOs:65, 112, 928, 127, 142, 157, 172, 187, 929, 202, 217, 255, 285, 300, 930, 324, 339, 354, 384, 414, 444, 459, 474, 489 against DRB*0301.
**Figure 4****.** Table of REVEAL® scores and stability indices for SEQ ID NOs:65, 112, 928, 127, 142, 157, 172, 187, 929, 202, 217, 255, 285, 300, 930, 324, 339, 354, 384, 414, 444, 459, 474, 489 against DRB*0401.
**Figure 5****.** Table of REVEAL® scores and stability indices for SEQ ID NOs:65, 112, 928, 127, 142, 157, 172, 187, 929, 202, 217, 255, 285, 300, 930, 324, 339, 354, 384, 414, 444, 459, 474, 489 against DRB*1101.
**Figure 6****.** Table of REVEAL® scores and stability indices for SEQ ID NOs:65, 112, 928, 127, 142, 157, 172, 187, 929, 202, 217, 255, 285, 300, 930, 324, 339, 354, 384, 414, 444, 459, 474, 489 against DRB*1301.
**Figure 7****.** Table of REVEAL® scores and stability indices for SEQ ID NOs:65, 112, 928, 127, 142, 157, 172, 187, 929, 202, 217, 255, 285, 300, 930, 324, 339, 354, 384, 414, 444, 459, 474, 489 against DRB*0701.
**Figure 8****.** Table of Pan-Allele REVEAL® binding assay data for SEQ ID NOs:65, 112, 928, 127, 142, 157, 172, 187, 929, 202, 217, 255, 285, 300, 930, 324, 339, 354, 384, 414, 444, 459, 474, 489 (shown in table as Peptide ID Nos1-24).
**Figure 9****.** Graph showing Pan-Allele REVEAL® binding assay data for SEQ ID NOs:65, 112, 928, 127, 142, 157, 172, 187, 929, 202, 217, 255, 285, 300, 930, 324, 339, 354, 384, 414, 444, 459, 474, 489 (shown in table as Peptide Sequences 1-24).
**Figure 10****.** Table of Pan-Allele REVEAL® Stability Index data for SEQ ID NOs:65, 112, 928, 127, 142, 157, 172, 187, 929, 202, 217, 255, 285, 300, 930, 324, 339, 354, 384, 414, 444, 459, 474, 489 (shown in table as Peptide ID Nos1-24).
**Figure 11****.** Graph showing Pan-Allele REVEAL® Stability data for SEQ ID NOs:65, 112, 928, 127, 142, 157, 172, 187, 929, 202, 217, 255, 285, 300, 930, 324, 339, 354, 384, 414, 444, 459, 474, 489 (shown in table as Peptide Sequences 1-24).
**Figure 12****.** Table of REVEAL® scores and stability indices for SEQ ID NOs:504, 519, 534, 931, 549, 564, 579, 609, 639, 932, 654, 688, 703, 718, 730, 760, 790, 820, 835, 850, 869, 884, 893, 905 against DRB*0101.
**Figure 13****.** Table of REVEAL® scores and stability indices for SEQ ID NOs:504, 519, 534, 931, 549, 564, 579, 609, 639, 932, 654, 688, 703, 718, 730, 760, 790, 820, 835, 850, 869, 884, 893, 905 against DRB*1501.
**Figure 14****.** Table of REVEAL® scores and stability indices for SEQ ID NOs:504, 519, 534, 931, 549, 564, 579, 609, 639, 932, 654, 688, 703, 718, 730, 760, 790, 820, 835, 850, 869, 884, 893, 905 against DRB*0301.
**Figure 15****.** Table of REVEAL® scores and stability indices for SEQ ID NOs:504, 519, 534, 931, 549, 564, 579, 609, 639, 932, 654, 688, 703, 718, 730, 760, 790, 820, 835, 850, 869, 884, 893, 905 against DRB*0401.
**Figure 16****.** Table of REVEAL® scores and stability indices for SEQ ID NOs:504, 519, 534, 931, 549, 564, 579, 609, 639, 932, 654, 688, 703, 718, 730, 760, 790, 820, 835, 850, 869, 884, 893, 905 against DRB*0701.
**Figure 17****.** Table of Pan-Allele REVEAL® binding assay data for SEQ ID NOs:504, 519, 534, 931, 549, 564, 579, 609, 639, 932, 654, 688, 703, 718, 730, 760, 790, 820, 835, 850, 869, 884, 893, 905 (shown in table as Peptide ID Nos1-24).
**Figure 18****.** Graph showing Pan-Allele REVEAL® binding assay data for SEQ ID NOs:504, 519, 534, 931, 549, 564, 579, 609, 639, 932, 654, 688, 703, 718, 730, 760, 790, 820, 835, 850, 869, 884, 893, 905 (shown in table as Peptide Sequences 1-24).
**Figure 19****.** Table of Pan-Allele REVEAL® Stability Index data for SEQ ID NOs:504, 519, 534, 931, 549, 564, 579, 609, 639, 932, 654, 688, 703, 718, 730, 760, 790, 820, 835, 850, 869, 884, 893, 905 (shown in table as Peptide ID Nos1-24).
**Figure 20****.** Graph showing Pan-Allele REVEAL® Stability data for SEQ ID NOs:504, 519, 534, 931, 549, 564, 579, 609, 639, 932, 654, 688, 703, 718, 730, 760, 790, 820, 835, 850, 869, 884, 893, 905 (shown in table as Peptide Sequences 1-24).
**Figure 21****.** Table showing peptides subjected to Prolmmune REVEAL® assay (SEQ ID NOs:65, 112, 928, 127, 142, 157, 172, 187, 929, 202, 217, 255, 285, 300, 930, 324, 339, 354, 384, 414, 444, 459, 474, 489). Position of the 9mer epitope derived from ProPred in the 15mer tested shown in red, M/L and C/V substitutions are highlighted in light blue. The parent allergen polypeptide is indicated, e.g. Alt a 1, and the start and stop positions of the 9mer in the parent allergen polypeptide sequence is also indicated. Physicial properties of the 15mer (molecular weight, net charge, average hydrophilicity) is provided. Negative binders in the Prolmmune REVEAL® assay are shaded in the "15mer ALG Code" column (34.2, 36.2 and L6-1 were negative). Note that the 15mer of SEQ ID NO:65 starts at the Alt a 1 N-terminus and the 15mer of SEQ ID NO:300 starts at the Alt a 1 C-terminus.

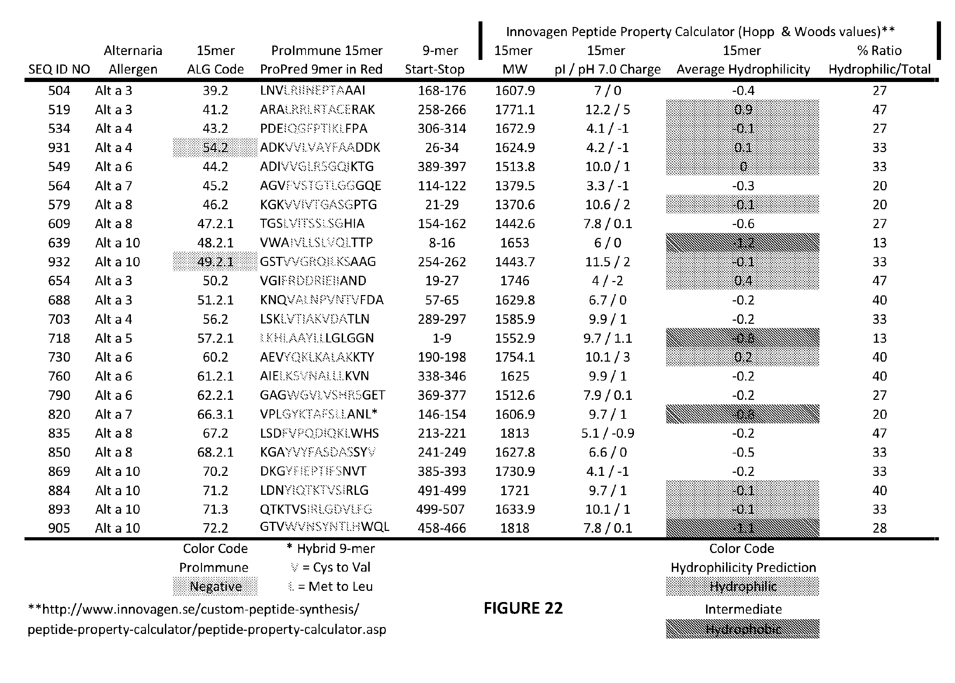

**Figure 22****.** Table showing peptides subjected to Prolmmune REVEAL® assay (SEQ ID NOs:504, 519, 534, 931, 549, 564, 579, 609, 639, 932, 654, 688, 703, 718, 730, 760, 790, 820, 835, 850, 869, 884, 893, 905). Position of the 9mer epitope derived from ProPred in the 15mer tested shown in red, M/L and C/V substitutions are highlighted in light blue. The parent allergen polypeptide is indicated, e.g. Alt a 3, and the start and stop positions of the 9mer in the parent allergen polypeptide sequence is also indicated. Physicial properties of the 15mer (molecular weight, net charge, average hydrophilicity) is provided. Negative binders in the Prolmmune REVEAL® assay are shaded in the "15mer ALG Code" column (54.2, and 49.2.1 were negative). Note that the 15mer and 9mer of SEQ ID NO:730 starts at the Alt a 5 N-terminus and the 15mer and 9mer of SEQ ID NO:893 starts at the Alt a 10 C-terminus.
**Figure 23****.** Table showing 9mer epitope sequences identified by ProPred analysis in Alt a 1.
**Figure 24****.** Table of conservative amino acid modifications indicating amino acid modifications that may be made to peptides of the invention in order to increase peptide resistance to degradation.
**Figure 25****.** Table of top 11 DRB1 alleles used in ProPred search. Alleles are shown by percentage population frequency present in European Americans.

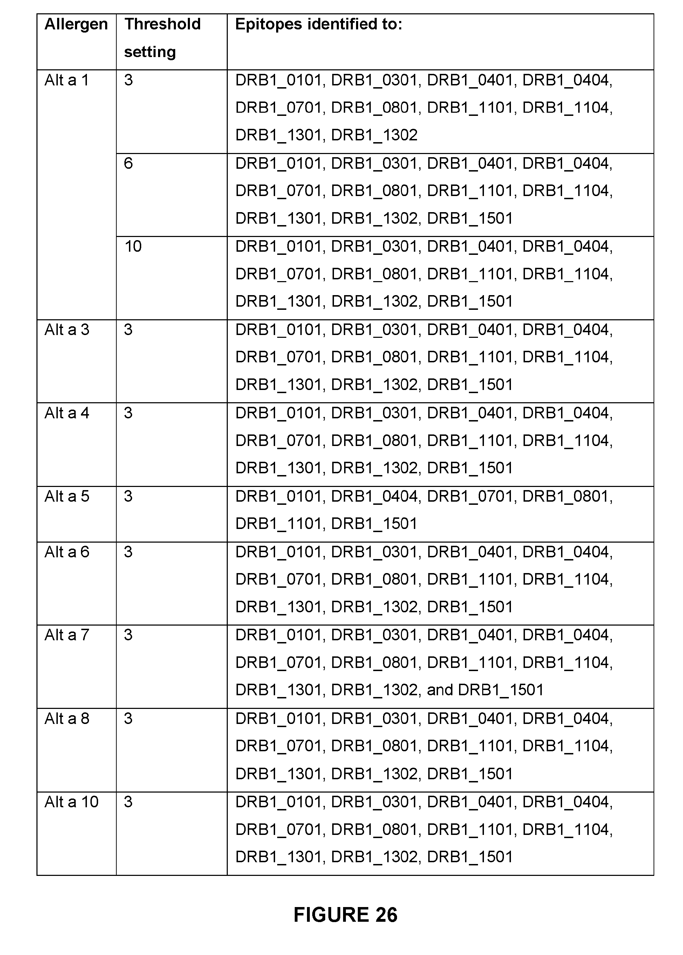

**Figure 26****.** Table showing alleles for which 9mer epitopes were identified by ProPred analysis for each of Alt a 1, Alt a 3, Alt a 4, Alt a 5, Alt a 6, Alt a 7, Alt a 8, and Alt a 10.
**Figure 27****.** Table showing SEQ ID NOs:933-1163.
**Figure 28****.** Table showing ProPred predicted 9mer sequences and predicted DRB allele binding selected under threshold setting 3 (high stringency).
**Figure 29****.** Graph showing IL-4 induction in individual patients in response to individual Alt a 1 soluble peptides and the Alt a 8 peptide 47.2.1.
**Figure 30****.** Graph showing IL-4 induction in individual patients in response to individual Alt a 1 DMSO peptides and the Alt a 8 peptide 47.2.1.
**Figure 31****.** Graph showing IL-4 induction in individual patients in response to individual Alt a 3, Alt a 4, Alt a 6, Alt a 7, Alt a 8, and Alt a 10 peptides.
**Figure 32****.** Graph showing IL-4 induction in individual patients in response to combination preparations Alt a 1 Sol, Alt a 1 DMSO, Alt a Oth Sol, Alt a Oth DMSO.

### Detailed Description of the Invention

The details of one or more embodiments of the invention are set forth in the accompanying description below including specific details of the best mode contemplated by the inventors for carrying out the invention, by way of example. It will be apparent to one skilled in the art that the present invention may be practiced without limitation to these specific details.

Treatment of allergic disease with peptide epitopes derived from or corresponding to peptide fragments or protein allergens offers substantial advantages over treatment with full length allergen protein molecules because of the reduced potential for cross-linking IgE bound to the surface of mast cells and basophils (12).

MHC class II genes encode cell surface glycoproteins having structural similarity to MHC Class I molecules but expressed only on Antigen Presenting Cells (APC). In conjunction with antigenic protein allergen fragments MHC class II proteins are recognised by T-helper (CD4+) cells. Therefore MHC class II molecules are involved in nearly all antigen responses. In humans MHC molecules are referred to as Human Leukocyte Antigens (HLA) encoded by genes HLA-DM, HLA-DO, HLA-DP, HLA-DQ and HLA-DR.

A T cell epitope is the basic unit recognised by a T cell receptor. T cell epitopes are believed to be involved in the initiation and perpetuation of the immune response to an antigen such as a protein allergen which is responsible for the clinical symptoms of allergy. These T cell epitopes are thought to trigger early immune response events at the level of the T helper cell by binding to an appropriate HLA molecule on the surface of an antigen presenting cell and stimulating the relevant T cell subpopulation. These events lead to T cell proliferation, cytokine secretion, local inflammatory reactions, recruitment of additional immune cells to the site, and activation of the B cell cascade leading to production of antibodies.

A therapeutic/prophylactic treatment regimen in accordance with the invention (which results in prevention of, or delay in, the onset of disease symptoms caused by an offending antigen or results in reduction, slowed progression, or alleviation of symptoms caused by an offending antigen i.e. down regulation of an antigen specific immune response) comprises administration, in non-immunogenic form (e.g. without adjuvant) of a pharmaceutical composition of the invention comprising at least one isolated peptide which may be derived from a protein antigen responsible for the disease condition being treated. While not intending to be limited to any theory, it is believed that administration of a therapeutic composition of the invention may: (i) cause T cell non responsiveness of appropriate T cell subpopulations such that they become unresponsive to the offending antigen and do not participate in stimulating an immune response upon exposure to the offending protein antigen (i.e. via anergy or apoptosis); (ii) modify the cytokine secretion profile as compared with exposure to the naturally occurring offending antigen (e.g. result in a decrease of IL-4 and/or an increase in INF-y); (iii) cause T cell subpopulations which normally participate in the response to the offending antigen to be drawn away from the sites of normal exposure (e.g. nasal mucosa, skin and lung for allergy) towards the sites of administration of the composition (this redistribution of T cell subpopulations may ameliorate or reduce the ability of an individual's immune system to stimulate the usual immune response at the site of normal exposure to the offending antigen, resulting in diminution in allergic symptoms); or (iv) cause induction of T suppressor cells, e.g. induction of allergen specific IL-10 producing Treg CD+ T-cells.

It is preferred that peptides according to the present invention do not bind immunoglobulin E (IgE) or bind IgE to a substantially lesser extent (e.g. at least 100-fold less binding and more preferably at least 1,000-fold less binding) than the protein allergen from which the peptide has been identified. Major complications of standard immunotherapy include IgE-mediated responses such as anaphylaxis. Thus for example, a method of identifying a peptide derivative may comprise assaying for binding to IgE, and selecting a peptide with the required binding as above.

HLA-DR molecules have a wide range of structures owing to the HLA-DR molecule being encoded by several loci with several genes at each locus. This leads to a large number and diversity of HLA-DR alleles and variants with a wide range of allele frequency between ethnic and geographical populations. Additionally the HLA-DR allele population is constantly evolving.

Some allergen fragments are only bound by one or two HLA-DR molecules and hence are considered MHC restricted. Use of such fragments as vaccines can only be expected to benefit those patients expressing the required allele and can therefore severely limit the use of the vaccine to treat the majority of a patient population that has a wide diversity of HLA-DR alleles. Some allergen fragments are bound by many HLA-DR alleles and therefore may provide the basis of vaccines capable of treating a much larger patient population.

Genomic analysis of allergen genes in *Alternaria alternata* has identified a number of protein allergens and potential allergens via identity to allergens from other fungal species (6-7) including Alt a 1, Alt a 3, Alt a 4, Alt a 5, Alt a 6, Alt a 7, Alt a 8, Alt a 10. These sequences were subject to analysis for epitopes exhibiting promiscuous binding to HLA-DR alleles.

The inventors first identified peptides from these protein allergens predicted to be T cell epitopes to one or more of the alleles listed in Figure 25.
This first stage in the identification of peptides as candidate T cell epitopes for in vivo validation as vaccines for fungal allergy was performed by use of virtual HLA class II matrices. In such a system a matrix-based algorithm is used to extract all possible peptide frames from a given protein allergen sequence. Subsequently, the corresponding position- and amino acid- specific matrix values are assigned to each residue of these peptide frames. Finally, the sum of these matrix values is determined for each frame. The resulting numerical values (peptide scores) correlate with the binding affinity of HLA-DR ligands (16). One such method of predicting T cell epitopes is provided by the TEPITOPE software (9, 10, 16).

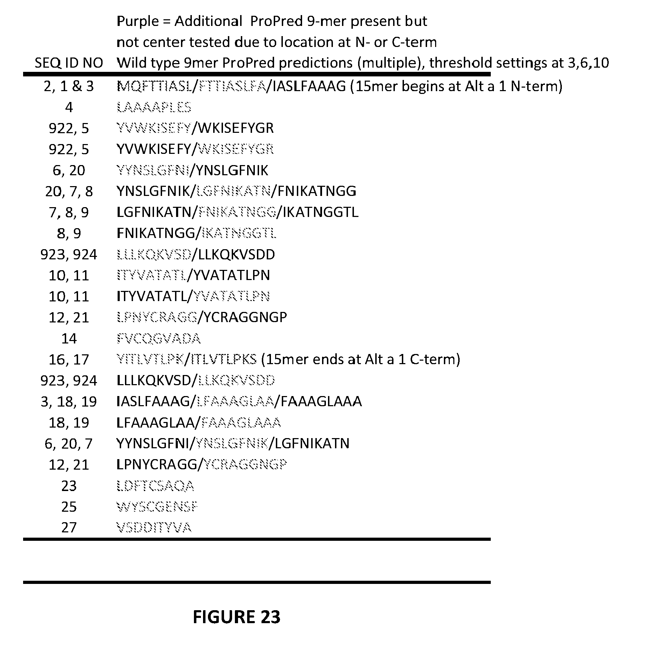

In the embodiments described here the inventors used the ProPred epitope prediction software (8) [http://www.imtech.res.in/raghava/propred/index.html], running experiments at levels 3, 6 and 10 (3 is the most stringent). This computes the binding strength to each HLA-DR allele of all peptide frames in the selected allergen sequence. The ProPred software contains 51 HLA-DR alleles. Potential promiscuous binders were selected from fungal protein allergens by identifying 9mer epitope sequences predicted by ProPred to bind more than one of the HLA-DR alleles listed in Figure 25. The peptide epitopes identified using the ProPred software are shown in Figure 23.

Some of the epitopes identified contain methionine or cysteine residues. When synthesising peptides for in vitro testing peptide derivatives were prepared in which Methionine residues were replaced with Leucine and Cysteine residues with Valine (M/L and C/V derivatives).

The promiscuous epitopes identified using the ProPred software were used to synthesise 15mer peptides comprising the core 9mer sequence. The additional flanking amino acids normally correspond to the amino acids flanking the 9mer sequence in the wild type fungal protein sequence from which the 9mer sequence is derived. For example, most 15mers were prepared by synthesising a 15 amino acid sequence of the wild type allergen in which the 9mer is centred in the synthesised sequence, being flanked by 3 amino acids at the N-terminal end, and 3 additional amino acids at the C-terminal end. In some cases the 9mer epitope sequence occurs close to the N- or C-terminal end of the wild type allergen polypeptide. In these cases, the 9mer was not centred in the synthesised 15mer. The 15mers are shown in Figures 21 and 22. In some 15mers, the synthesised peptide differs from the amino acid sequence of the polypeptide allergen in that Methionine residues were replaced with Leucine and Cysteine residues with Valine (M/L and C/V derivatives).

The 15mer peptides were tested for binding to class II HLA alleles using the Prolmmune REVEAL® Class II HLA-Peptide Binding Assay (http://www.proimmune.com/ecommerce/page.php?page=reveal class2) (26).
Potential T-cell epitopes predicted by software can be screened by the experimental determination of their binding to specific MHC molecules (Panigada et al. Infec. Immun. 2002. 70:79-85). A commercial fee-for-service to measure the binding of peptides to MHC molecules has been developed by Prolmmune Ltd (Oxford UK) and has been used to predict epitopes to MHC Class I (Westrop et al. J. Immunol. Methods. 2009. 341:76-85) and MHC Class II DR (Muixi et al. J. Immunol. 2008. 181:795-807) alleles.

The Prolmmune Class II REVEAL® rapid epitope discovery assay is a cell-free in vitro assay that determines binding of a candidate peptide to one or more HLA class II DRB1 alleles compared with a positive and an intermediate control peptide. The assay is a measure of the ability of each peptide to stabilise the MHC-peptide complex. Detection is based on the presence or absence of the native conformation of this MHC-peptide complex. For the standard REVEAL assay the peptides to be tested are first synthesized as 15mers (Module 1: PEPscreen peptide synthesis) followed by incubation with the appropriate MHC protein at two dilutions for 7 days at 10°C followed by a single "on" point measurement (Module 2: REVEAL® MHC binding assay). Each peptide is given a score relative to the positive control peptide which is a known T cell epitope. Results for an intermediate control peptide are also shown. The intermediate control is a known T cell epitope, or in the case of certain class II alleles, a known epitope for the beta-chain in the allele alpha and beta pair. The score is reported quantitatively as a percentage of the signal generated by the test peptide relative to the positive control peptide. Proimmmune considers peptides that bind ≥15% relative to the control as being "passed". To further confirm a peptide as an epitope, after the initial "on" point determination the reaction temperature is raised to 37°C followed by stability measurements at 0 and 24 hrs from which a stability index is calculated by multiplying the estimated half-life by the module 2 REVEAL score (Module 4: Quick check stability assay). The higher the stability index the slower the off-rate and the more stable the epitope.

Of the alleles available for the assay, the inventors selected HLA-DRB1*0101, DRB1*1501, DRB1*0301, DRB1*0401, DRB1*0701, DRB1*1101 and DRB1*1301 for testing of SEQ ID NOs:65, 112, 928, 127, 142, 157, 172, 187, 929, 202, 217, 255, 285, 300, 930, 324, 339, 354, 384, 414, 444, 459, 474, 489 and HLA-DRB1*0101, DRB1*1501, DRB1*0301, DRB1*0401 and DRB1*0701 for testing of SEQ ID NOs:504, 519, 534, 931, 549, 564, 579, 609, 639, 932, 654, 688, 703, 718, 730, 760, 790, 820, 835, 850, 869, 884, 893, 905.

DRB1*1501, DRB1*0301, and DRB1*0701 are the three DRB1 alleles having the highest frequency of occurrence in the European/American population, and together have a total frequency of 42.55% in the population (see Figure 25).

Thus, as set out in the following Examples the inventors carried out an *in silico* screen for epitopes amongst known and/or suspected fungal allergens from *Alternaria alternata,* and arranged *in vitro* analysis of synthetic peptides containing the identified epitopes to confirm their promiscuous binding character.

### Examples

### Example 1 - Identification of Predicted Promiscuous T-cell Epitopes

Predicted T cell epitopes were identified from *Alternaria alternata* protein allergens Alt a 1, Alt a 3, Alt a 4, Alt a 5, Alt a 6, Alt a 7, Alt a 8, Alt a 10.

The amino acid sequences of the polypeptides were first subject to analysis using the ProPred software (http://www.imtech.res.in/raghava/propred/index.html, ref 8) in order to identify predicted HLA-DR binding peptide epitopes within each protein allergen. In total, ProPred includes 51 HLA-DR alleles against which peptide binding may be assessed. Peptide containing epitopes were selected on the basis of predicted binding to the following alleles: DRB1_0101, DRB1_0301, DRB1_0401, DRB1_0404, DRB1_0701, DRB1_0801, DRB1_1101, DRB1_1104, DRB1_1301, DRB1_1302, and DRB1_1501.

The alleles listed above represent 11 of the 12 most common HLA-DR alleles occurring in European Americans (1401 is not present in the ProPred database). The 11 alleles listed occur in 86.46% of the population (21). Therefore epitopes predicted to bind to 10 or 11 of these alleles can be considered extremely promiscuous HLA-DR binders.

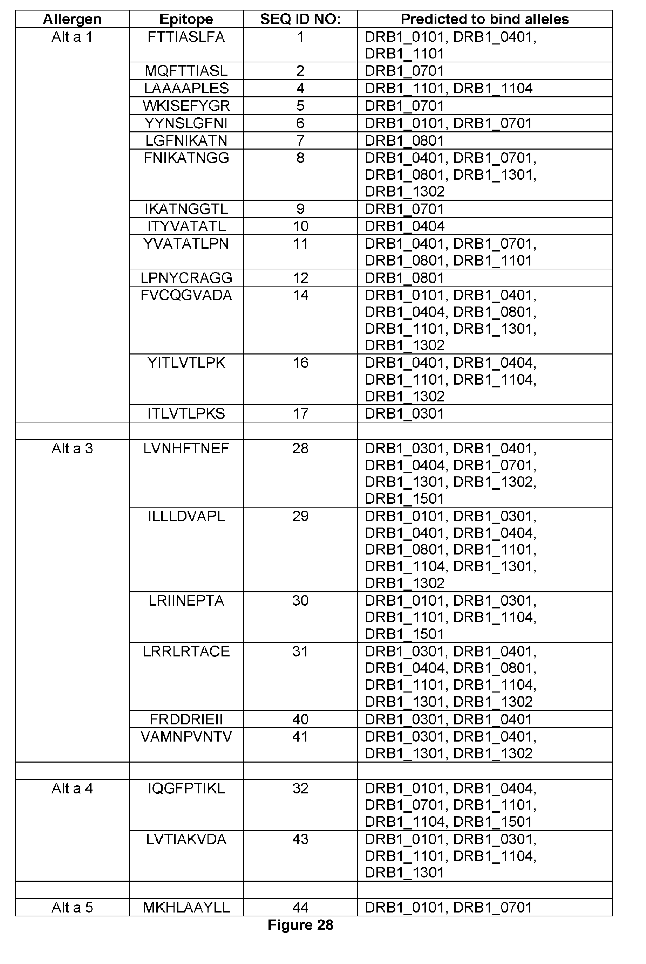

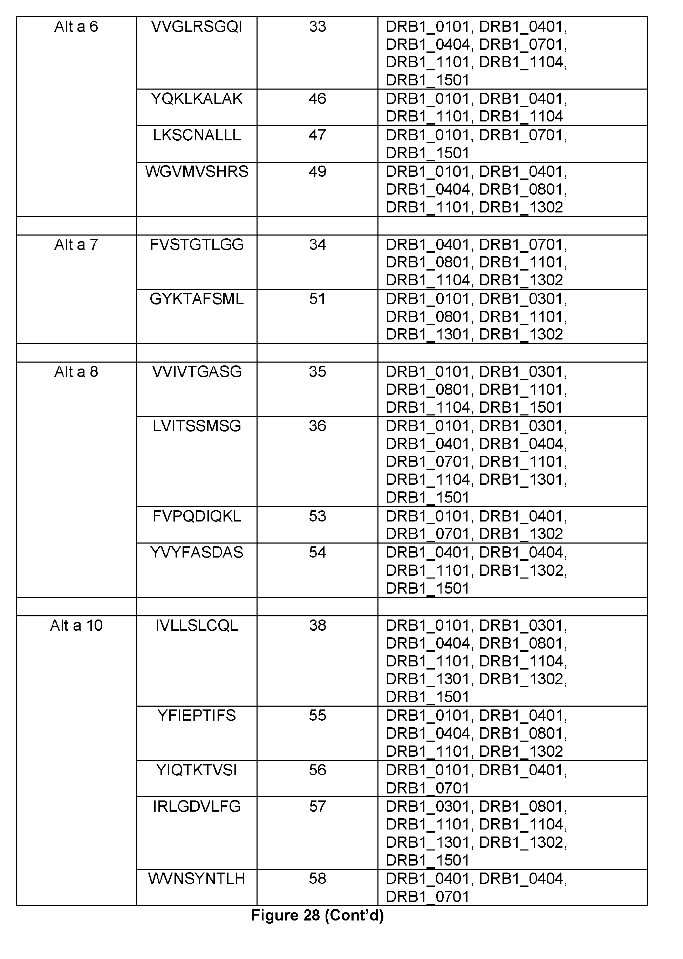

ProPred predictions for all allergens tested were obtained using threshold setting 3 (high stringency). For Alt a 1 additional predictions were obtained using threshold settings 6 and 10. Figure 28 shows predicted 9mer sequences selected using threshold setting 3 and indicates the DRB alleles predicted to be bound by the 9mer.

Several of the 9mers predicted to be promiscuous epitopes using the ProPred software contained a Methionine residue (M). Methionine is an oxidation-sensitive residue, and is sometimes substituted with a leucine (L) residue, which is less oxidation sensitive. Accordingly, the inventors also devised 9mer derivatives having M/L substitution(s).

Several of the 9mers predicted to be promiscuous epitopes using the ProPred software contained a Cysteine residue (C). The presence of Cysteine residues can lead to low solubility which is undesirable for a candidate therapeutic. Therefore, the inventors also devised 9mer derivatives having C/V substitution(s).

Accordingly, using the ProPred data, the inventors identified predicted promiscuous 9mer epitopes from each allergen tested.

### Example 2 - Peptide Design

Peptides containing predicted promiscuous 9mer epitopes identified in Example 1 were synthesised and assayed for T cell binding using the Prolmmune REVEAL® assay.

Each synthesised peptide comprised a 15 amino acid (15mer) peptide. In most cases the 9mer epitope identified in Example 1 was centred in the 15mer, such that the 9mer is flanked by 3 amino acids at each of the N- and C-terminal ends. The 9mer epitope and flanking sequences were designed starting from the wild type amino acid sequence of the corresponding 9mer and corresponding flanking sequences in the parent allergen polypeptide such that the peptide has 100% sequence identity with a 15 amino acid sequence within the parent allergen polypeptide. In some cases, the 9mer epitope is located close to the N- or C- terminus of the parent allergen polypeptide amino acid sequence such that less than 3 flanking amino acids exist in the parent allergen polypeptide amino acid sequence. In these cases, e.g. SEQ ID NOs:65, 718 and 893 the 9mer is not centred in the 15mer. Finally, the 15mer sequence was reviewed for the presence of Methionine or Cysteine residues, and M/L and/or C/V substitution made.

The peptides so designed and synthesised are listed in Figures 21 and 22 as SEQ ID NOs:65, 112, 928, 127, 142, 157, 172, 187, 929, 202, 217, 255, 285, 300, 930, 324, 339, 354, 384, 414, 444, 459, 474, 489 and SEQ ID NOs:504, 519, 534, 931, 549, 564, 579, 609, 639, 932, 654, 688, 703, 718, 730, 760, 790, 820, 835, 850, 869, 884, 893, 905, respectively

The hydrophilicity and net charge was calculated for each candidate peptide using the Innovagen Peptide Property Calculator (Innovagen, Sweden) and are shown in Figures 21 and 22. Average hydrophilicty of >-0.1 indicates high peptide solubility in physiological buffers.

The synthesised 15mer sequences were submitted to the Prolmmune REVEAL® Class II HLA-Peptide Binding Assay
(http://www.proimmune.com/ecommerce/page.php?page=reveal class2) (ref 26).

### Example 3 - In Vitro Analysis of Promiscuous Binding to HLA DR Alleles

The REVEAL® Rapid Epitope Discovery System consists of several modules:

### Module 1: Custom Peptide Synthesis

The peptides are synthesized in 0.5-2mg quantities with high average purity. Quality control by MALDI-TOF Mass Spectrometry is carried out on 100% of samples.

### Module 2: REVEAL® MHC-Peptide Binding Assay

The high-throughput REVEAL® binding assay determines the ability of each candidate peptide to bind to one or more MHC class II alleles and stabilize the MHC-peptide complex. By comparing the binding to that of high and intermediate affinity T-cell epitopes, immunogenic peptides can be identified. Detection is based on the presence or absence of the native conformation of the MHC-peptide complex.

Each peptide is given a score relative to a positive control peptide, which is a known T-cell epitope, or in the case of certain class II alleles, a known epitope for the beta-chain in the allele alpha and beta pair. The score of the test peptide is given quantitatively as a percentage of the signal generated by the positive control peptide. Assay performance is confirmed by including an intermediate control peptide that is known to bind with weaker affinity to the allele under investigation.

The REVEAL® assay can identify peptides that bind a wide range of DR alleles.

### Module 4: REVEAL® Rate Assays

REVEAL® rate assays assess the on- and off-rates for peptide binding to MHC class II molecules. By indicating how long individual epitopes are presented to T-cells they can help identify which candidate sequences can be presented long enough to be good T cell epitopes.

### Quick Check Stability Assay

This assay measures the amount of peptide bound to the allele at time zero and time 24 hours, at 37°C, and gives an indication of the stability of the HLA-peptide complex. Each peptide is given a 'stability index', allowing comparison of the relative stability of the different peptide epitopes. The REVEAL® MHC-peptide binding assay reflects the on-rate properties of a peptide more strongly than the stability of the assembled complex. However, when the outcomes of the stability assay and the binding assay are combined, the subsequent data provide more complete information as to whether the peptide could be presented long enough for it to be a good T-cell epitope. This index provides a method for comparing the results of different peptides across the allele being measured.

The synthesised 15mer sequences from Example 2 were submitted to the Prolmmune REVEAL® Class II HLA-Peptide Binding Assay
(http://www.proimmune.com/ecommerce/page.php?page=reveal class2) (ref 26).

Of the alleles available for the assay, the inventors selected HLA-DRB1*0101, DRB1*1501, DRB1*0301, DRB1*0401, DRB1*0701, DRB1*1101 and DRB1*1301 for testing of SEQ ID NOs:65, 112, 928, 127, 142, 157, 172, 187, 929, 202, 217, 255, 285, 300, 930, 324, 339, 354, 384, 414, 444, 459, 474, 489 and HLA-DRB1*0101, DRB1*1501, DRB1*0301, DRB1*0401 and DRB1*0701 for testing of SEQ ID NOs:504, 519, 534, 931, 549, 564, 579, 609, 639, 932, 654, 688, 703, 718, 730, 760, 790, 820, 835, 850, 869, 884, 893, 905.

DRB1*1501, DRB1*0301, and DRB1*0701 are the three DRB1 alleles having the highest frequency of occurrence in the European/American population, and together have a total frequency of 42.55% in the population (see Figure 25).

Binding to MHC molecules was compared to that of two known T-cell epitopes: a positive control peptide and an intermediate control peptide with very strong and weaker binding properties, respectively.

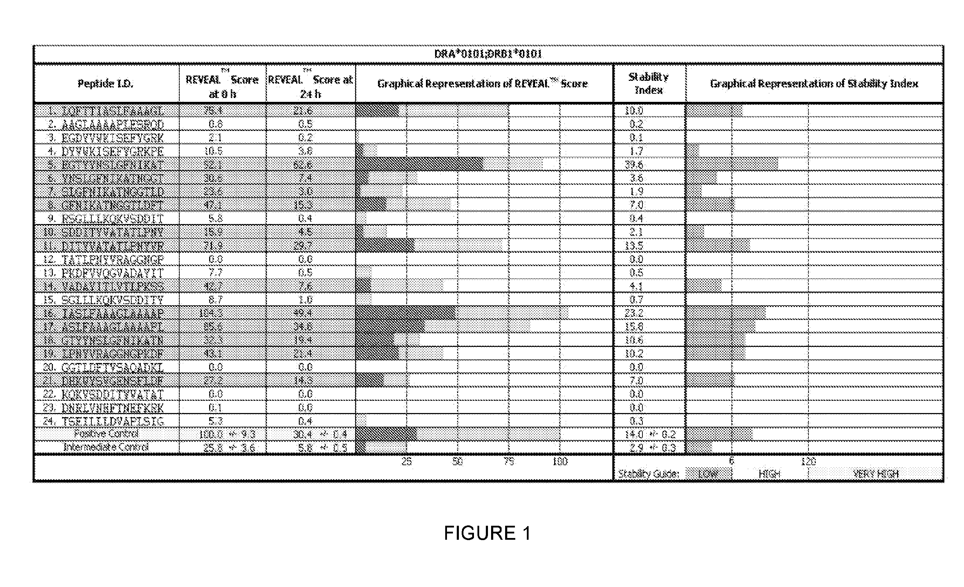

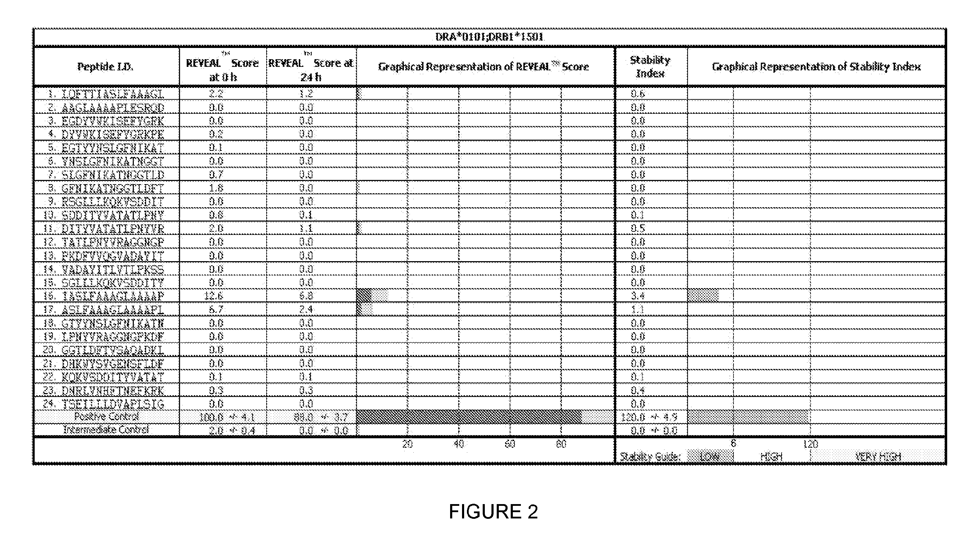

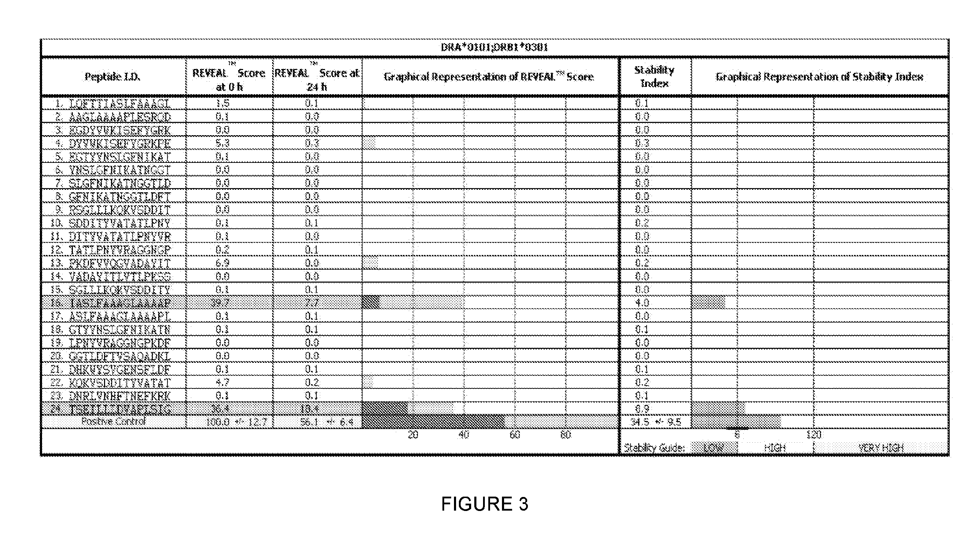

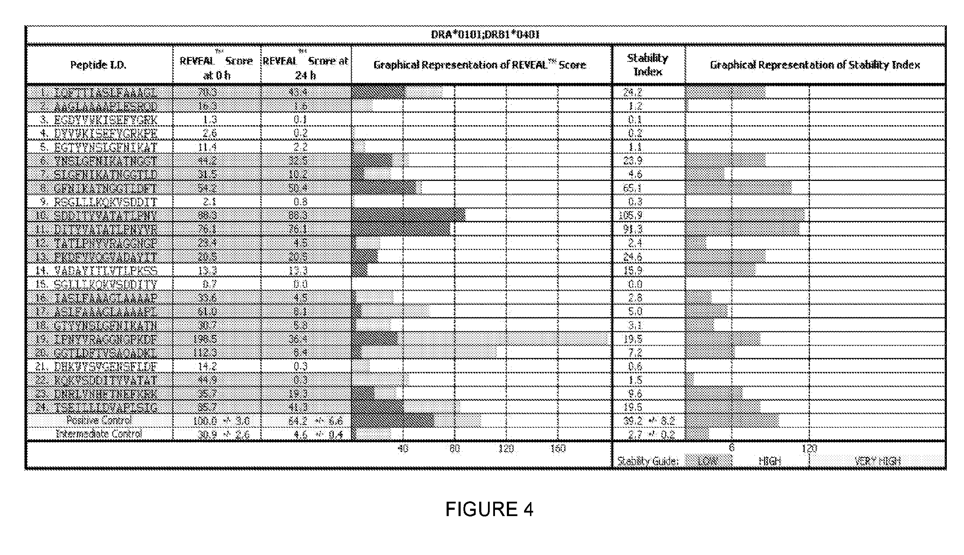

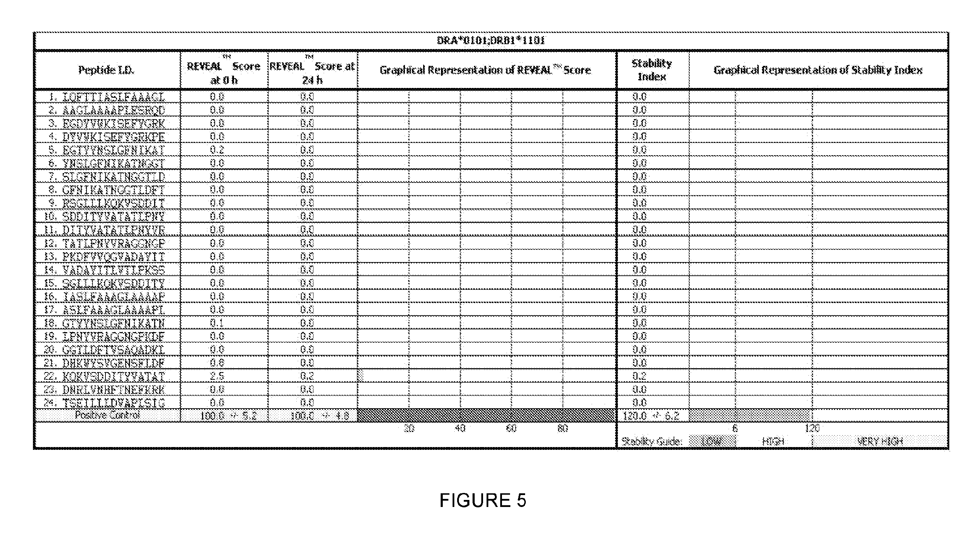

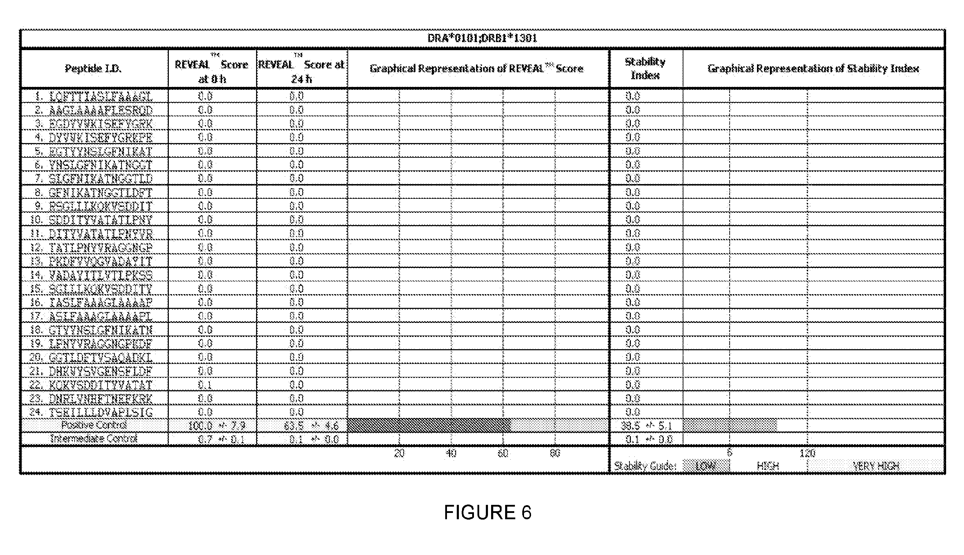

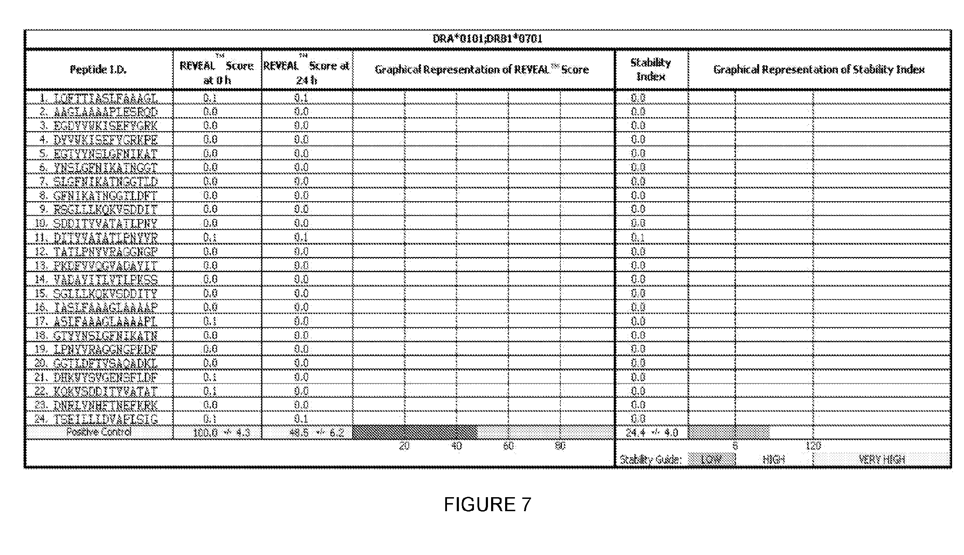

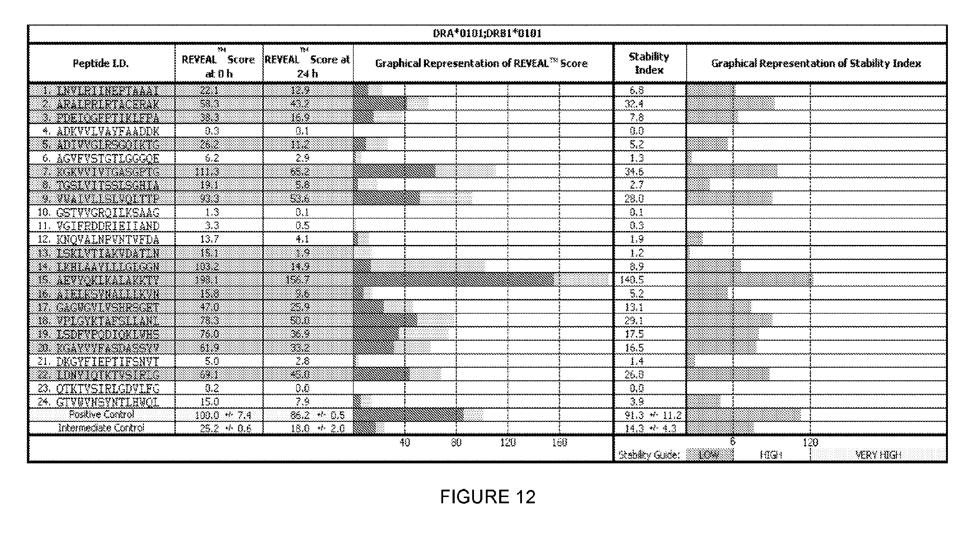

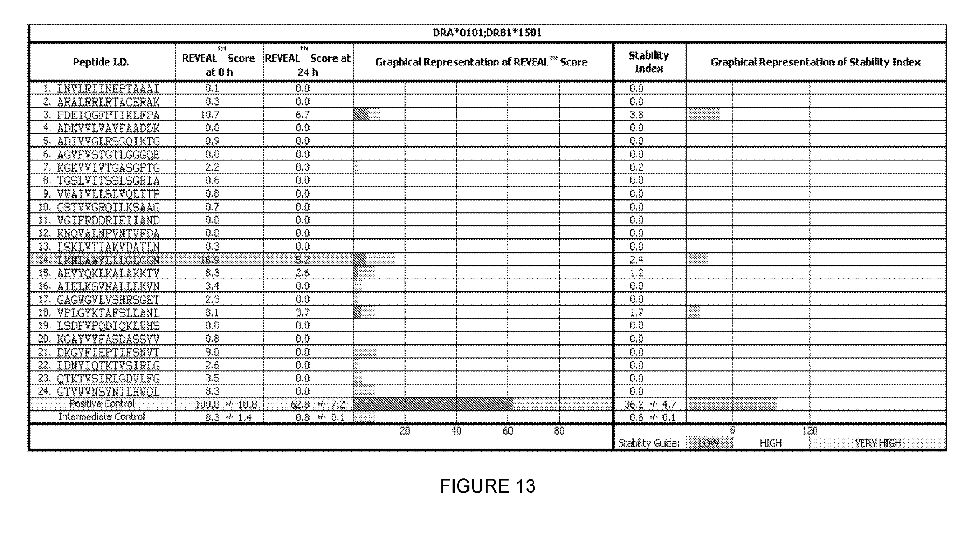

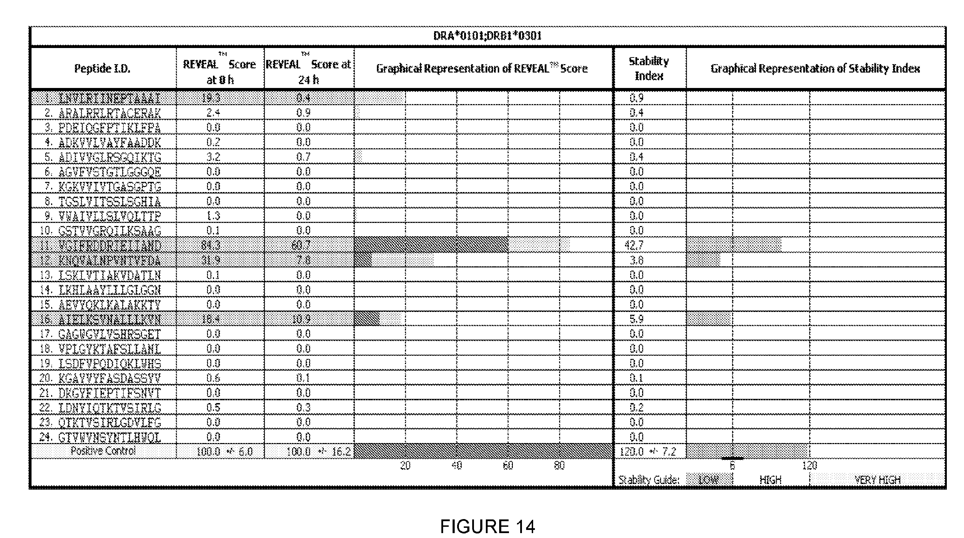

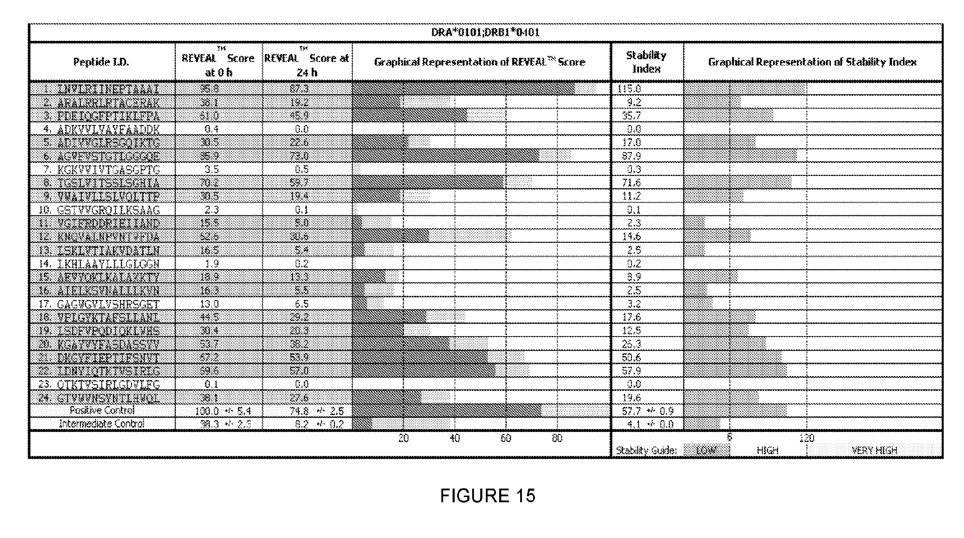

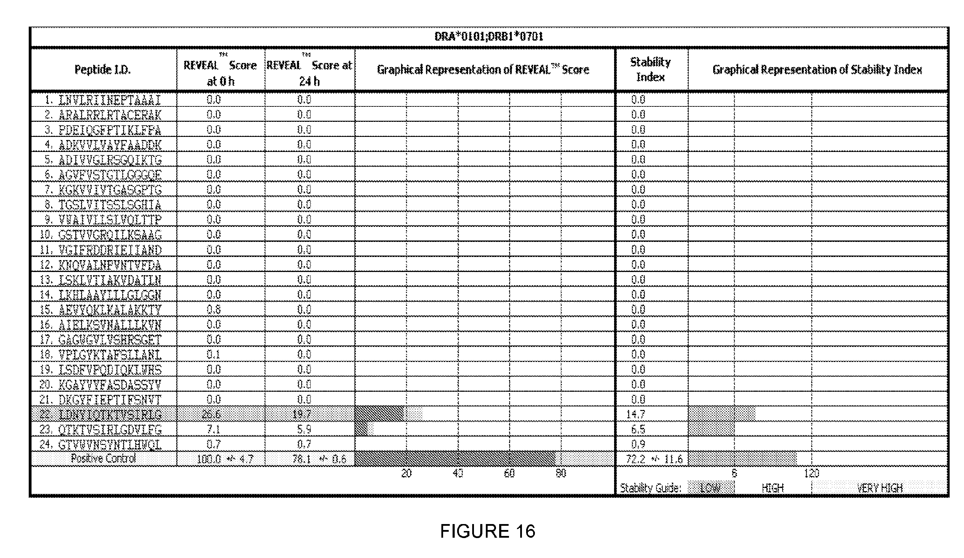

The REVEAL® binding score for each peptide-MHC complex is calculated at 0 and 24 h by comparison to the binding of the relevant positive control at 0 h and indicated numerically in Figures 1 to 7 and 12 to 16. These figures also show the REVEAL® score at the two time points where the score at 24 h (red cross-hatched bar) overlaps the score at 0 h (green bar) and indicates the proportion of assembled complex that has remained after the 24 h incubation.

Peptides considered to be good binders are those peptides with scores ≥15% of the positive control. These peptides are referred to as passed epitopes and are highlighted in blue. Strong T-cell epitopes tend to be identified as clear positive responses in this assay. Binding of the intermediate control is shown, where possible, so that a comparison can be made with another T-cell epitope with weaker binding characteristics.

Experimental standard error was obtained by triplicate positive and intermediate control binding experiments. The standard error for these controls is reported below and can be assumed to be representative of the degree of error that would be present for all samples.

### Module 4: Quick Check Stability Assay

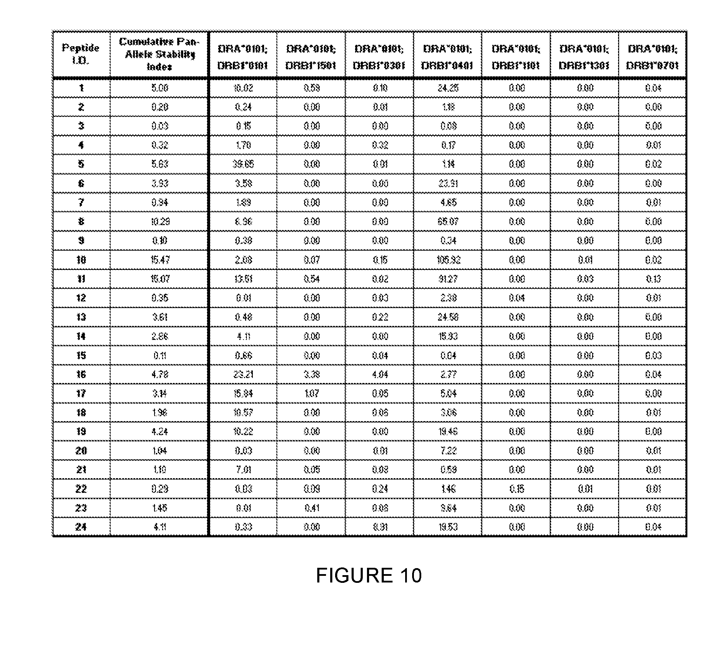

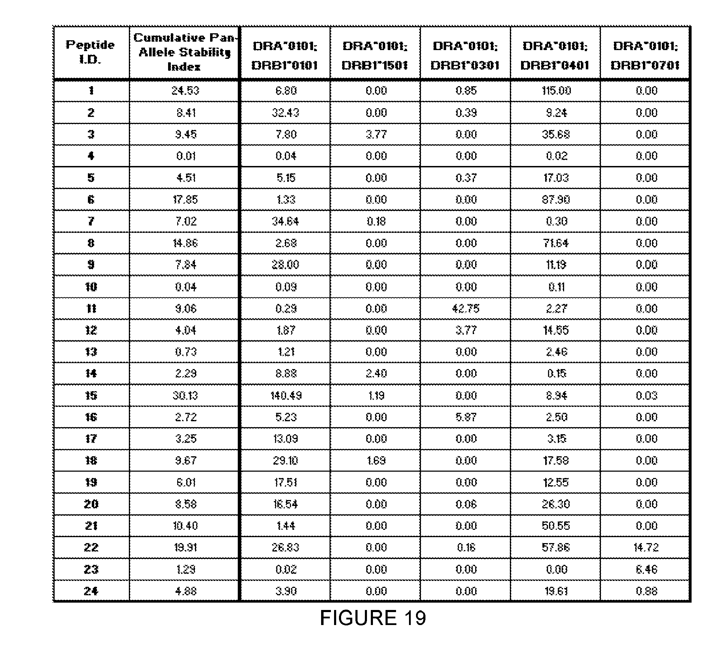

The stability of the MHC-peptide complexes from the REVEAL® assay was determined by taking measurements at 0 and 24 h during incubation at 37°C. The signals at these time points were used to estimate a half-life based on a one-phase dissociation equation. The stability index was then calculated by multiplying the half-life by the REVEAL® score from the binding assay to weight the value so that very weakly binding peptides would not be over represented. For samples with half-lives longer than 120 h, a maximum half-life of 120 h was used in the stability index calculation since a measurement period of 24 h is not long enough to estimate values above this level accurately. Further, the stability index was divided by 100 to bring the scale of values back into a more easily interpretable range. The stability indices are presented numerically in Figures 10 and 19 where a higher stability index is indicative of a more stable MHC-peptide complex and a better epitope. The graphical representation of stability index in these figures is presented on a logarithmic scale where the two gradations represent the three distinct levels of stability:
▪ Low stability (red): the range in which a sample with a REVEAL® score of 100 would be stable for ≤ 6 h
▪ High stability (yellow): the range in which a sample with a REVEAL® score of 100 would be stable for between 6 and 120 h
▪ Very high stability (green): the range in which a sample with a REVEAL® score of 100 would be stable for ≥ 120 h

The stability indices of the test peptides was compared to those of two known T-cell epitopes from the binding assay (the positive and intermediate controls). Experimental standard error was obtained by analyzing triplicate controls and can be assumed to be representative of the degree of error that would be present for all samples.

### Pan-Allele Binding Analysis Based on REVEAL™ Score

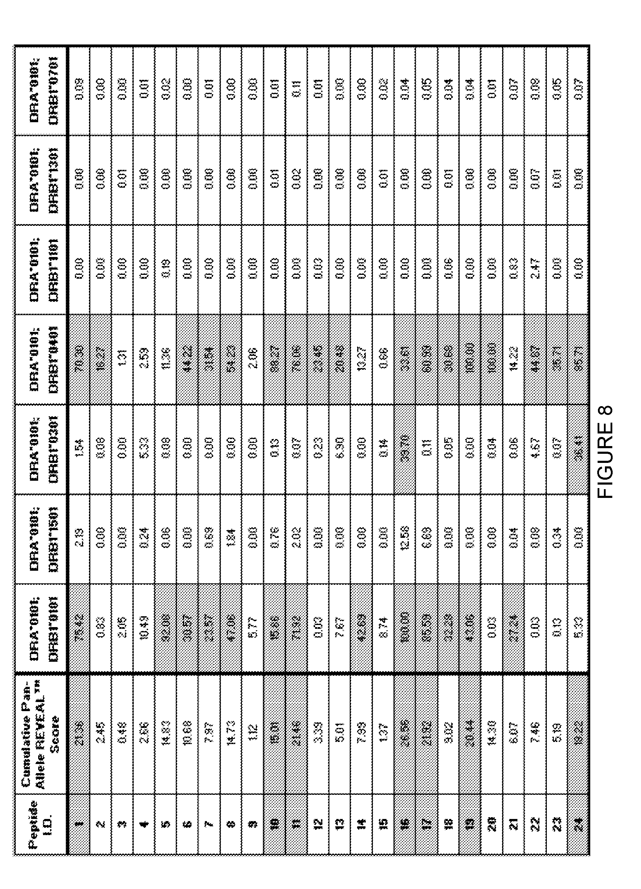

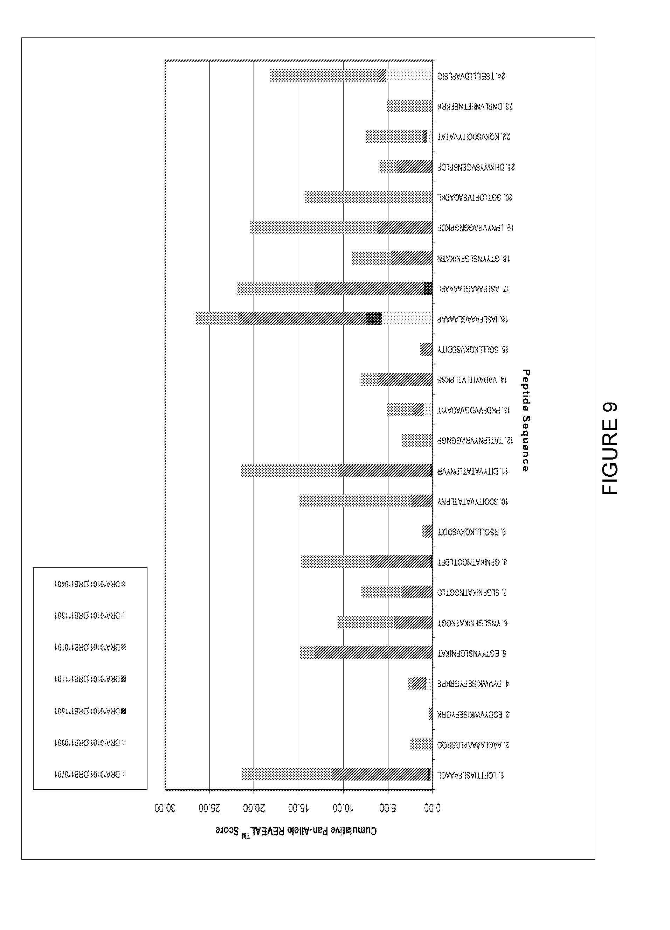

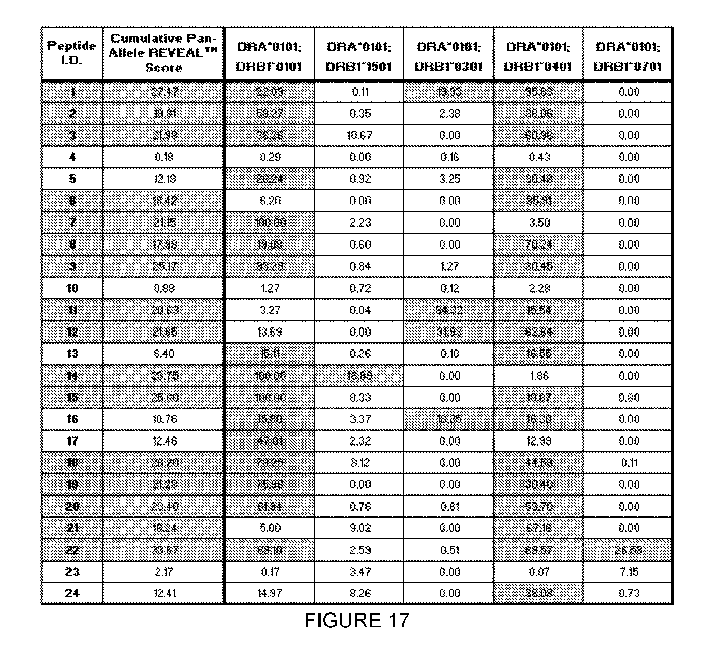

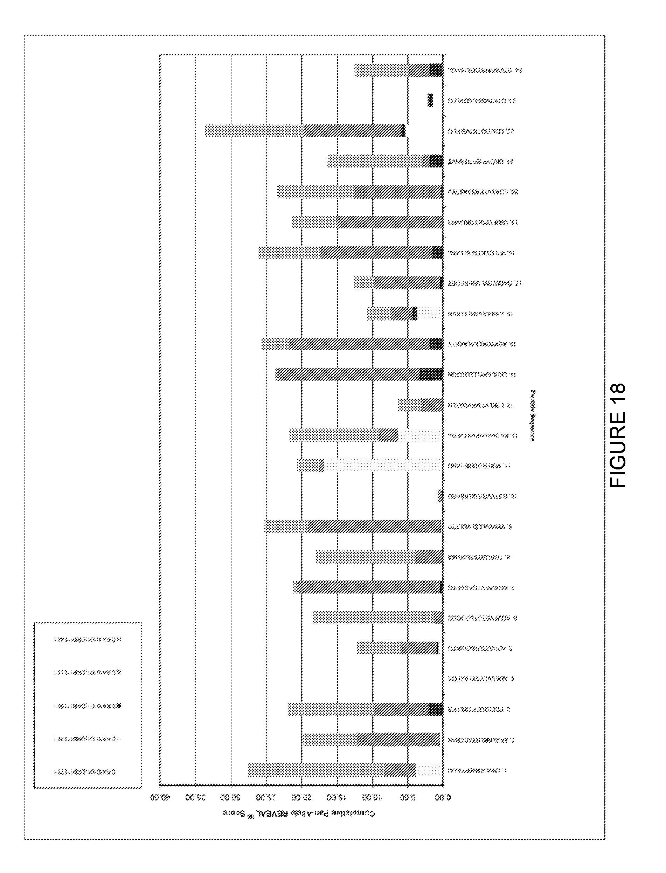

The Pan-allele binding analysis was carried out to show the cumulative binding data for each peptide across the multiple alleles studied in the REVEAL® binding assay. Those peptides whose REVEAL® scores are >100% of the positive control were standardized to a fixed value of 100% to prevent individual alleles from being over-represented in this comparison due to exceptionally high levels of binding. The standardized cumulative total of the REVEAL® scores for each peptide across all the alleles is then determined by the sum of each allele's standardized REVEAL® scores divided by the number of alleles in question. Peptides with standardized cumulative REVEAL® binding assay scores ≥15 are highlighted in blue in the tabulated data (Figures 8 and 17) and can be referred to as pan-allele binding epitopes. The standardized cumulative REVEAL™ scores are also presented graphically in Figures 9 and 18, where the total bar height is made up of the individual contributions from each allele.

### Pan-Allele Stability Index Analysis

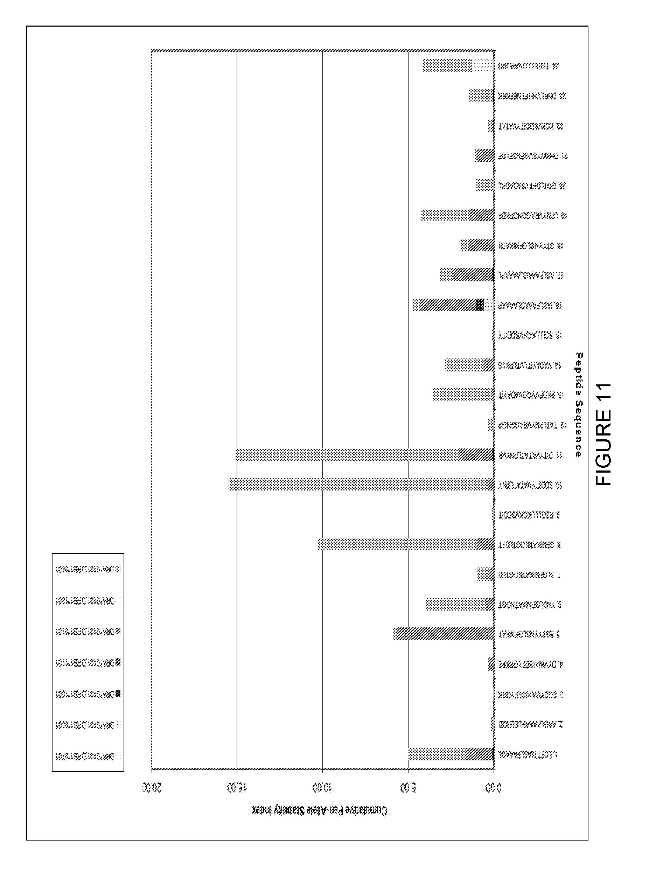

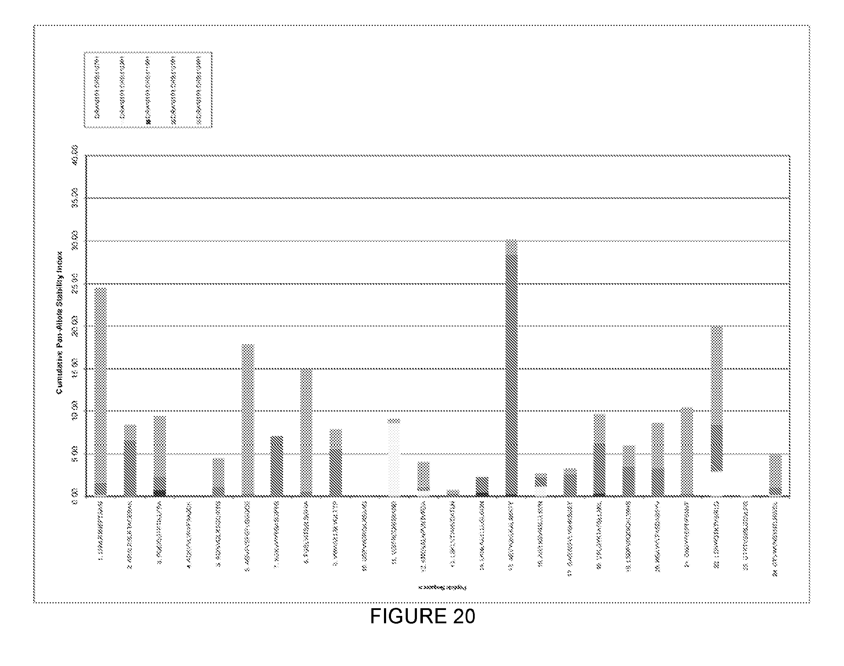

The Pan-Allele stability analysis was carried out to show the cumulative stability data for each peptide across the multiple alleles studied in the rate assays. The cumulative total of the stability index for each peptide across all the alleles is then determined by the sum of each allele's stability index divided by the number of alleles with which that peptide was tested. Each peptide's cumulative stability index is shown in the tabulated data (Figures 10 and 19). The standardized cumulative stability indices are also presented graphically in Figures 11 and 20 as the total bar height made up of the individual contributions from each allele. The positive and intermediate controls are left out of this analysis because different sets of controls are used for each allele.

### Example 4 - T-Cell Epitope Validation Assay

### Assay Description and Use

A T-cell epitope validation assay is provided to test the ability of a peptide or peptide derived from antigen presenting cell (APC) processed whole protein to be presented via a MHC molecule on an APC to form a significant affinity binding reaction with a T-cell receptor present on CD-4⁺ T-lymphocytes (along with other co-stimulatory/binding molecules). This binding reaction results in CD-4+ T-cell activation which can be measured in a number of ways.

One measure of response for activation is proliferation of the lymphocytes detected by new DNA synthesis. Background proliferation can vary considerably and therefore results are expressed as a stimulation index (SI) calculated as proliferation of cultures plus antigen (peptide/protein) divided by proliferation of cultures without antigen (peptide/protein). An SI above 2 or 3 is indicative of induced proliferation and that a T-cell epitope(s) is present.

A second method to measure activation is to measure gene expression via production of specific proteins such as cytokines. Often a single cytokine such as INF-γ or IL-4 can be used as a marker for activation. The enzyme-linked immunospot (ELISPOT) assay can provide sensitive detection of cytokine synthesis by individual cells of activated clonal populations. In this assay the number of peptide activated cells is compared to the number of background cells producing cytokine with no peptide addition. Results are reported in spot forming cells/10⁶ PMBCs.

These assays can be used to 1) define T-cell epitopes, 2) determine "exposure" of a population to antigens, 3) diagnose recognized diseases and conditions, and 4) determine the immune response to epitopes.

### Source of Cells

The immunological status of donor cells is an important variable in the assay. Typically cells from donors who are known to be sensitive/primed to the antigen (epitope) give the greatest responses in the assay. In studies investigating epitopes from allergens, donors who are classified as sensitive or allergic to the corresponding protein on the basis of skin tests or serum antibody levels will show significant responses to epitopes in the cellular assay (17). Donors who test negative for allergy to particular allergens will usually be unreactive in the stimulation assay with an SI less than 2. This type of negative control can be used to set a lower threshold for acceptance of proliferation or number of cytokine producing cells when using known sensitized donors to define an epitope. Pooled cells from many donors can be used for assay development to conserve patient samples.

Another variable is the MHC Class II (DR, DQ, and DP) type present on the donor antigen presenting cells. Epitopes in the form of peptides are MHC restricted and must have affinity for the particular Class II molecules for presentation to CD4+ lymphocytes. Donor cells may therefore be typed for Class II DR, DQ and DP. The ability of test peptides selected by ProPred and/or Prolmmune REVEAL® analysis on the basis that they are Class II DR epitopes can then be compared against their ability to stimulate a response in the T-cell validation assay. This information can be used to validate the accuracy of the ProPred prediction and the results of Prolmmune REVEAL® assay(s).

### Donor Cell Preparation and Assay

Cells for the assay can be prepared from anti-coagulated whole blood using density gradients. The fraction known as peripheral blood mononuclear cells (PBMC) is harvested, and consists of a variety of cell types including monocytes and different lymphocyte classes. Cells are grown from 2-7 days with and without the peptide/protein being tested. Positive proliferation controls may be used, typically using mitogens such as phytohemagglutinin (PHA) or antigens such as tetanus toxoid (TET).

After the incubation phase a proliferation detection method is used, such as [³H] thymidine incorporation for approx 6 hrs and then counts are measured or cytokine production from individual cells is detected using ELISPOT. This system is sufficient for stimulation of "sensitized/primed" cells. The T-cell epitope assay may also use an "unprimed/unsensitized" population of cells. To map epitopes in such cases (including antigens not normally in the patients environment), the assay can be modified to use specific isolated cell populations such as adherent monocytes (dendritic cells) and purified CD4+ lymphocytes (18). Similarly, cloned T cells and purified APC populations can be used to increase the sensitivity of detection.

PBMC fractions may be prepared as follows:
(i) Blood samples are withdrawn from patients with fungal infection or allergy, e.g. SAFS, and optionally from patients without fungal infection or allergy (to act as control samples). The allergy status of patients may be determined for example by skin prick tests with allergen extracts.
(ii) Whole anticoagulated blood is separated on a Ficoll gradient and the PBMC layer is withdrawn and frozen in HSA/DMSO and stored in liquid Nitrogen.
(iii) Cells are defrosted as required, counted, plated at 10⁶ cells/ml, 200 µl/well 2.0x10⁵ cells/well, 96 well round bottom plate, 37°C, 5% CO₂. Culture media: AIM V (BD), contains 0.25% HAS, 50 µg/ml Streptomycin, 10 µg/ml Gentamycin and L-glutamine.

For positive controls: TET stock is diluted and applied 24 hours after plating out cells at a concentration 0.5µg/ml.

Control populations may be analysed by including PBMC from a group of patients who have allergy but do not have evidence of fungal sensitization and a group of "normal" volunteers without allergies or other diseases. Such experiments serve to define the T-cell reactivity of the populations to fungal epitopes for comparison. The "normal" volunteers also provide a negative control for the assay.

### Form of Epitopes

Stimulation of T-cells has been reported using whole proteins and defined peptides of varying lengths. It is assumed that whole proteins are internally processed and peptides are presented by APC's while epitopes added as peptides bind directly to empty (and possibly occupied) MHC molecules on the cell surface. Class II MHC molecule binding pockets will contact and hold a 9 amino acid epitope core. The MHC Class II pocket is open at both ends so varying lengths of peptide can over hang. Peptides up to a total length of 20-30 amino acids have been found to bind Class II molecules. Stimulation experiments typically use chemically synthesized peptides between 10-25 amino acids and obtain positive results. One strategy is to produce a "PepSet" or "Epitope Scan" consisting of a set of peptides between 15-25 amino acids in length which are offset by 3-10 amino acids and span the entire length of a protein.

### Example 5 - Cytokine Analysis

Cytokine analysis can greatly enhance the information content of the stimulation assay. With the discovery of the polarization of T-cells into distinct cytokine producing populations (Th0, Th1 and Th2) the assay has been used to study the effects of epitopes on T-cell differentiation (19).

Cell culture supernatants can be assayed for cytokines using a number of standard assay formats. The use of FACS analysis to evaluate the cells and molecules in the stimulation assay increases the flexibility of analytical analysis. A FACS method can differentiate cell types, measure proliferation, and measure intracellular cytokine levels (20). An ELISPOT assay allows analysis of cells at the single cell level for multiple cytokine production (28)

### Proliferation and Cytokine Detection Methods

### 1. FACS Based FastImmune™ Anti-BrdU Proliferation Assay:

Bromodeoxyuridine (BrdU) is a uridine derivative that can be incorporated specifically into DNA in place of thymidine. Anti-BrdU identifies BrdU (but not thymidine) in single-stranded DNA, free BrdU or BrdU coupled to a protein carrier. DNase-I cleaves each strand of DNA at random and permits the binding of Anti-BrdU antibody to its antigen (BrdU).

Cells can be pulse-labelled with BrdU, and those cells that are synthesising DNA will incorporate BrdU into the DNA. Anti-BrdU can then be used to identify cells that undergo DNA synthesis during exposure to BrdU.

Cells are bromodeoxyuridine (BrdU) labelled and stained at 7 days post application of antigen. BrdU is added to wells to give a final concentration of 0.01 mM, 5 hours before staining.

Cells are fixed, permeabilized and stained. Staining antibody BD anti-BrdU-FITC, supplied as a solution already containing DNase (Becton Dickinson Fastlmmune Catalog Number 340649). Solutions used are PBS 1% BSA, BD FACS Perm 2 solution and PBS 1% Paraformaldehyde.

Cells fixed and stained as above are stored at 4°C overnight before running on a FACS machine the following day.

### 2. FACS Based Fastimmune™ CD4 intracellular cytokine detection

FACS based Fastimmune™ intracellular cytokine detection kits (Becton Dickinson Catalog No.s 337185, 337187, 337189) are used to detect CD4+, CD69+ and Interferon γ (INF-y) status of the T-cell population.

### 3. Th1/Th2 status determination

A Cytometric Bead Array (CBA) Human Th1/Th2 Cytokine Kit (Becton Dickinson Catalog Number 550749) is used to detect the Th1/Th2 status of the T-cell population.

This assay kit enables one to detect production of IL-2, IL-4, IL-5, IL-10, THF, and IFN-γ in liquid samples and determine the Th1 or Th2 status of a T cell population.

### 4. ELISPOT assay for cytokine detection

The ELISPOT assay uses two high affinity cytokine specific antibodies, directed against two different epitopes on the same cytokine molecule (28).

96 well PVDF membrane ELISPOT plates are coated with capture antibody in coating buffer and incubated O/N at 4°C. Plates are washed and then blocked with complete RPMI-1640 media at RT for 1 h. Cells, peptides, allergens and controls in RPMI-1640 media will be aliquoted to wells and incubated at 37°C, 5% CO₂ in a humidified incubator for 24-48 h. Cells and medium are decanted and the plates are washed 3x with wash buffer. Biotinylated detection antibody in assay diluent is added to the plates are incubated at RT for 2 h. Plates are then washed 4x and aviden-horseradish peroxidase reagent is added and the plates are incubated at RT for 45 min. Plates are washed 3x with wash buffer and 2x with PBS. AEC substrate is added for 10-60 min at RT and spot development is followed and the reaction is stopped with diH₂O. The plates are air dried and the spots are counted with an automated ELISPOT plate reader.

### Example 6

Twenty (20) patients sensitised to *Alternaria alternata* and exhibiting symptoms of allergic rhino sinusitis will be identified. With the appropriate consent, a blood sample will be collected from each patient. Patients will be evaluated at the start of the trial by monitoring blood levels of antibodies, respiratory testing, HLA typing and skin-prick tests. Peptides identified from Prolmmune REVEAL® analysis as promiscuous and strong HLA DR allele binders will be validated in vitro against patient blood samples using an IL-4 ELISPOT assay.

In particular, 10 patients will not receive Alternaria extract and 10 patients will be given Alternaria extract intranasally. After two weeks, blood samples will be taken and ELISPOT IL-4 cytokine assays performed. All samples will be challenged in vitro with the candidate peptide(s) and the effect of the peptide(s) on IL-4 cytokine production assessed by ELISPOT assay.

### Example 7 - Measurement of IL-4 Response Following Peptide Stimulation of Blood Samples from Human Patients

Forty three (43) 15mer peptides identified from Prolmmune REVEAL® analysis as promiscuous and strong HLA DRB1 allele binders were validated by *in vitro* analysis of patient blood and control group blood samples using an IL-4 ELISPOT assay.

The 43 peptides are predicted to be positive binders selected from the group of 48 peptides listed in Figures 21 and 22. The five peptides excluded from the analysis were 34.2, 36.2, L6-1 (shaded in the "15mer ALG Code" column in Figure 21) and 54.2 and 49.2.1 (shaded in the "15mer ALG Code" column in Figure 22).

Patients from the Allergy Unit, Hospital Clinic, University of Barcelona (Barcelona, Spain) were selected and divided into two groups:
1. Nineteen (19) test patients sensitised to *Alternaria alternata:*
   ▪ All 19 test patients had allergic rhinitis and conjunctivitis and a majority of test patients had asthma.
   ▪ All 19 test patients were skin prick test positive to *Alternaria* extract and positive for nasal challenge with *Alternaria* extract.
   ▪ 17 of 19 of the test patients were tested for Basophil activation by rAlt a 1 and were found to be positive.
   ▪ Test patients were positive for IgE response to *Alternaria* extract.
2. Fifteen (15) control patients
   ▪ All control patients were negative for skin prick test to *Alternaria* extract and negative for specific nasal challenge with *Alternaria* extract.

Response to nasal challenge with *Alternaria* was measured by acoustic rinometry, where challenge was considered positive if the nasal challenge with diluent was negative and the volume between 2nd and 5th cm into the nose closed > 25% after the *Alternaria* challenge (as described in Allergen-specific nasal provocation testing: review by the rhinoconjunctivitis committee of the Spanish Society of Allergy and Clinical Immunology. Dordal MT, Lluch-Bernal M, Sánchez MC, Rondón C, Navarro A, Montoro J, Matheu V, Ibáñez MD, Fernández-Parra B, Dávila I, Conde J, Antón E, Colás C, Valero A; SEAIC Rhinoconjunctivitis Committee. J Investig Allergol Clin Immunol. 2011;21(1):1-12).

The basophil activation test (Basotest™ Glycotope Biotechnology GmbH, Heidelberg, Germany) in response to rAlt a 1 was considered positive if the percentage of Basophil activation was greater than 20% with a stimulation index (SI: test value/background value) higher than 2.

With the appropriate consent, blood samples were collected from each patient for analysis at the start and during the trial.

Patients were evaluated at the start of the trial by monitoring blood levels of antibodies, respiratory testing, HLA typing and skin-prick tests. In particular, serum total IgE and specific IgE response to *Alternaria* and Alt a1 was measured (ImmunoCAP Phadia and Microarray ISAC Phadia).

PBMC were isolated from heparinised whole blood by centrifugation in Ficoll-Paque, washed and put into serum free freezing media containing DMSO for storage in liquid N₂. All cell-culture media is serum-free. Thawed PBMC were used at 350,000 cells per well with or without peptide. The final concentration of each peptide is 10 µg/ml per assay well either individually or as a member of a pool. Incubation is at 37°C and 5% CO₂ for 48 hours for the IL-4 assay, after which time plates are processed for the detection of IL-4 production.

PBMC samples from test and control patients were prepared from blood samples, challenged with candidate peptide and subjected to ELISPOT assay to determine IL-4 response to peptide stimulation.

For challenge, peptides were administered as follows:
- Individual peptides (soluble). Individual water soluble peptides were dissolved as 10, 5 or 2.5 mg/ml stock solutions in sterile distilled water. Before use, each peptide was diluted in water to 2 mg/ml and 10 µl of this 2 mg/ml mix was added to 990 µl of media. Then 100 µl of this peptide/media mix was added to 100 µl of media containing 350,000 PBMC in an ELISPOT anti-IL-4 coated cell culture plate well for a final peptide concentration of 10 µg/ml. Identically diluted media only was added to the cells and used for the no peptide media control wells (media only).
- Individual peptides (DMSO). Individual water insoluble peptides were dissolved in 100% DMSO to a stock concentration of 50 mg/ml. Before use each peptide was diluted in water to 2 mg/ml and then 10 µl of the 2 mg/ml mix was added to 990 µl of cell-culture media. Then 100 µl of this peptide/media mix was added to 100 µl of media containing 350,000 PBMC in an ELISPOT anti-IL-4 coated cell culture plate well for a final peptide concentration of 10 µg/ml. Identically diluted media only was added to the cells and used for the no peptide media control wells (media only).
- A combination preparation containing Alt a 1 soluble peptides (33.2, 34.3, 35.4, 35.5, 37.3.1, 37.4.1, L6-5.01, L10-1.01, L10-2.01, L10-3), called Alt a 1 Sol. Each peptide was diluted from water stock to 2mg/ml in sterile distilled water. Then 10 µl of each peptide required for the combined preparation was mixed and brought up to 1 ml in media. Then 100 µl of this peptide/media mix was added to 100 µl of media containing 350,000 PBMC in an ELISPOT anti-IL-4 coated cell culture plate well for a final concentration of each peptide of 10 µg/ml. Identically diluted media only was used for the no peptide control wells (media only).
- A combination preparation containing Alt a 1 DMSO peptides (32.2.1, 38.2.1, 35.2, 35.3, L6-2, L6-3, L6-4, 38.3, 37.2), called Alt a 1 DMSO. Each peptide was diluted from DMSO stock to 2mg/ml in sterile distilled water. Then 10 µl of each peptide required for the combined preparation was mixed and brought up to 1 ml in media. Then 100 µl of this peptide/media mix is added to 100 µl of media containing 350,000 PBMC in an ELISPOT anti-IL-4 coated cell culture plate well for a final concentration of each peptide of 10 µg/ml. Identically diluted media only was used for the no peptide control wells (media only).
- A combination preparation containing non-Alt a 1 soluble peptides (40.2, 41.2, 43.2, 44.2, 46.2, 47.2.1, 56.2, 57.2.1, 60.2, 62.2.1, 67.2, 71.3), called Alt a Other Sol. Each peptide was diluted from water stock to 2mg/ml in sterile distilled water. Then 10 µl of each peptide required for the combined preparation was mixed and brought up to 1 ml in media, Then 100 µl of this peptide/media mix was added to 100 µl of media containing 350,000 PBMC in an ELISPOT anti-IL-4 coated cell culture plate well for a final concentration of each peptide at 10 µg/ml. Identically diluted media only was used for the no peptide control wells (media only).
- A combination preparation containing non-Alt a 1 DMSO peptides (39.2, 42.2, 45.2, 48.2.1, 50.2, 51.2.1, 61.2.1, 66.3.1, 68.2.1, 70.2, 71.2, 72.2), called Alt a Other DMSO. Each peptide was diluted from DMSO stock to 2mg/ml in sterile distilled water. Then 10 ul of each peptide required for the combined preparation was mixed and brought up to 1 ml in media, Then 100 µl of this peptide/media mix was added to 100 µl of media containing 350,000 PBMC in an ELISPOT anti-IL-4 coated cell culture plate well for a final concentration of each peptide at 10 µg/ml. Identically diluted media only was used for the no peptide control wells (media only).

### Results

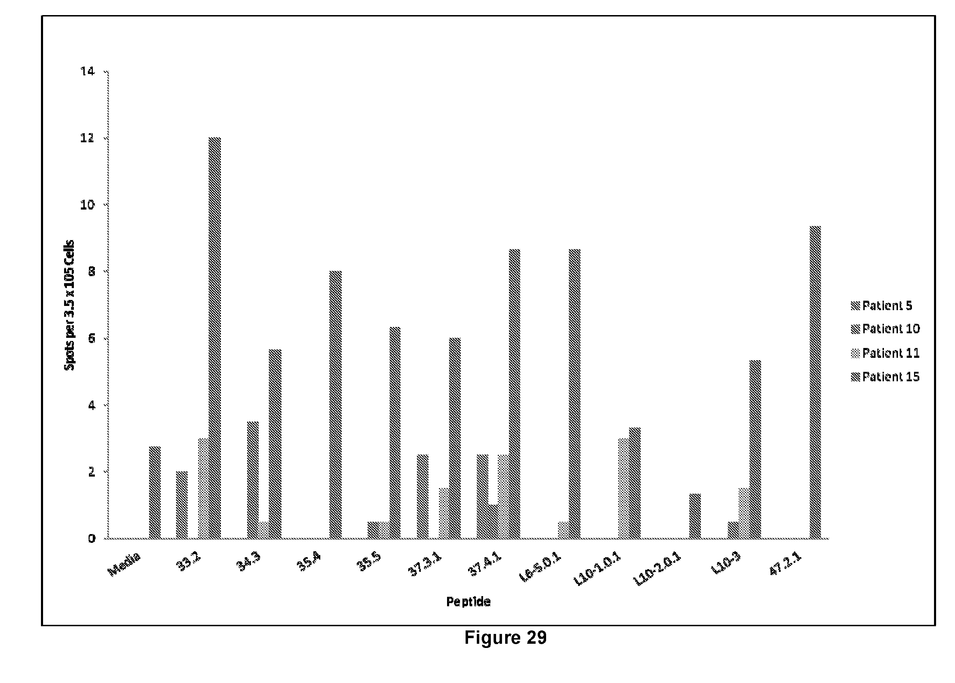

Results are summarised in Figures 29 to 32.

A large subset of the peptides tested stimulated IL-4 counts in test patient PBMC over no peptide background (media only) and control patient PBMC. Most of the positive peptides caused stimulation of multiple patients.

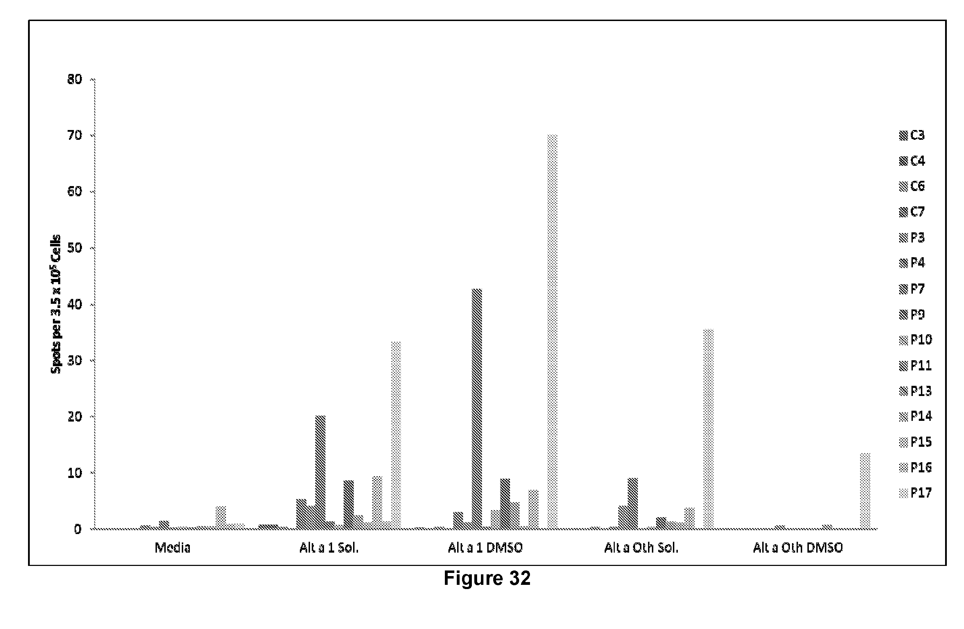

For the combined preparations, data was obtained from all of the control patients and test patients listed in the legend to Figure 32 for Alt a 1 Sol, Alt a 1 DMSO, and Alt a 1 Other Sol. Figure 32 indicates that the control patients were negative for IL-4 stimulation compared to media. For the Alt a Other DMSO preparation, only 5 patients were tested: P3, P4, P7, P13, P17.

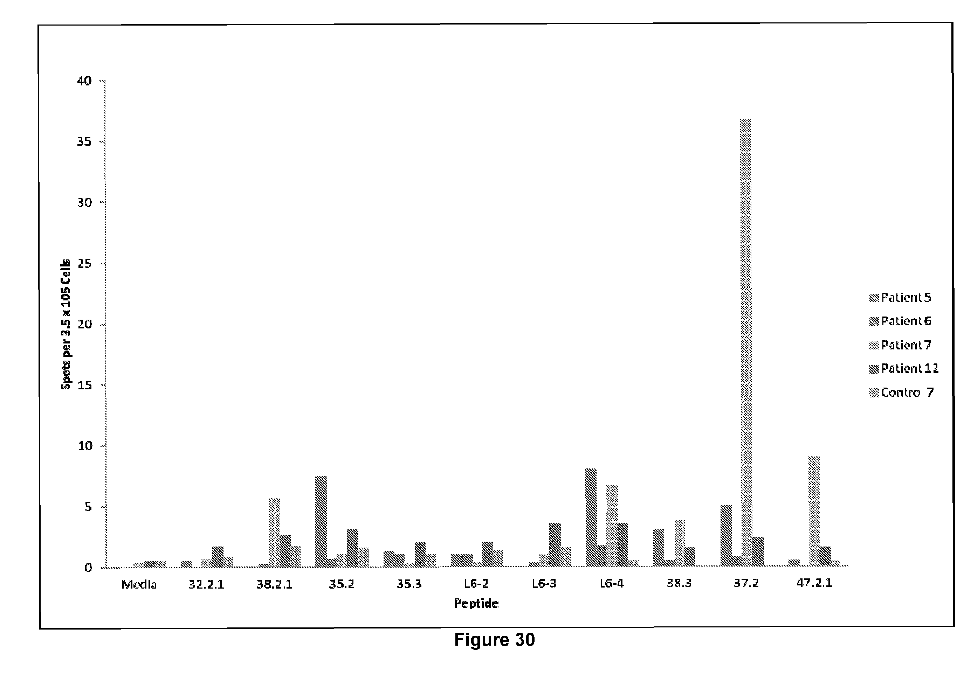

Figures 29 and 30 show results of IL-4 stimulation by individual; Alt a 1 peptides as well as the Alt a 8 peptide 47.2.1.

Figures 29 and 30 show positive stimulation of IL-4 induction in response to a range of individual Alt a 1 peptides compared to media only control.

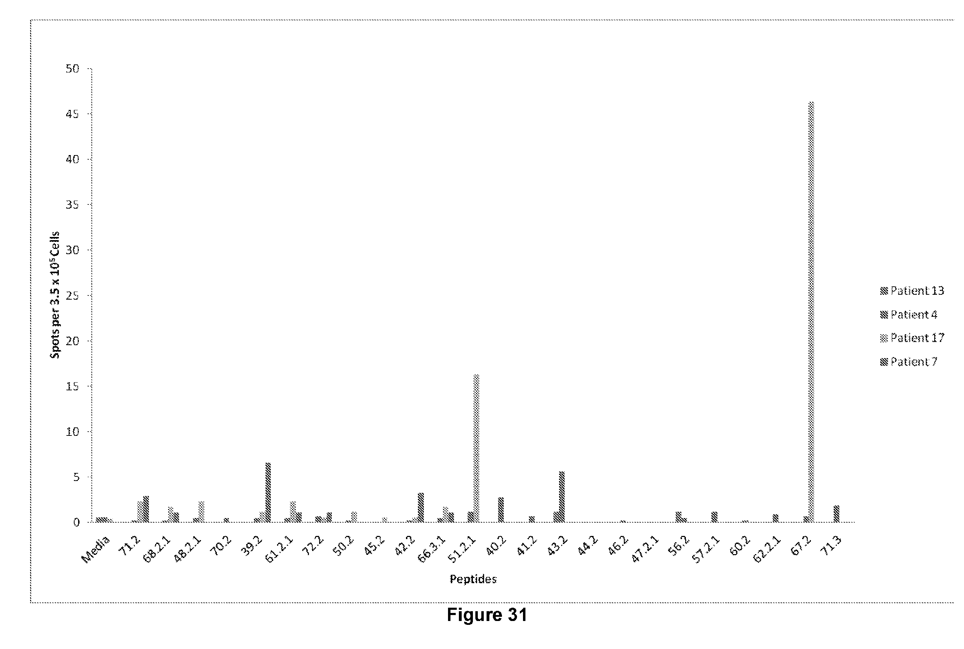

Figure 31 shows positive stimulation of IL-4 induction in response to a range of individual Alt a 3, Alt a 4, Alt a 6, Alt a 7, Alt a 8, and Alt a 10 peptides compared to media only control.

Figure 32 shows positive stimulation of IL-4 induction of combined peptide preparations compared to media only control and to control patients.

### Discussion

Induction of IL-4 cytokine in response to peptide stimulation is indicative of the peptide containing a T-cell epitope, and the results obtained in this study confirm the presence of T-cell epitopes in selected peptides tested.

Induction of an IL-4 response is indicative of an allergic response to a peptide independent of a diagnosis. Where a Th2 (IL-4+) population of CD4+ T-cells responds to selected peptides, those peptides can be used to induce tolerance and downregulate the Th2 allergic response to those allergens from where the peptides are derived.

Thus, the data obtained from this study validates selected peptides as useful in the prevention and/or treatment of allergic disease mediated by the corresponding protein allergen.

### References:

1. Denning et al. Eur. Respir. J. 2006. 27: 615-626.
2. Bowyer et al. Med. Mycol. 2006. 44 S23-S28.
3. Maguire et al. Clin. Immunol. 1999. 93: 222-231.
4. Haselden et al. J. Exp. Med. 1999. 189:1885-1894.
5. Bowyer and Denning. Med. Mycol. 2007. 45:17-26.
6. Schneider et al. Clin. Exp. Allergy. 2006. 36:1513-1524.
7. Shankar et al. Mol. Immunol. 2006. 43:1927-1932.
8. Singh and Raghava. Bioinformatics 2001. 17:1236-1237.
9. Bian and Hammer. Methods 2004. 34:468-475.
10. Mustafa and Shaban. Tuberculosis 2006. 86:115-124.
11. Janssen et al. J. Immunol. 2000. 164:580-588.
12. Verhoef et al. PLoS Med. 2005. 2:253-261.
13. Zuleger et al. Vaccine 2005.23:3181-3186.
14. Caccamo et al. Clin. Exp. Immunol. 2003. 133:260-266.
15. Doytchinova and Flower. J. Immunol. 2005. 174:7085-7095.
16. Sturniolo et al. Nat. Biotechnol. 1999. 17:555-561.
17. Rawle et al. J. Immunol. 1984. 133:195-201.
18. Stickler et al. J. Immunother. 2000. 23:654-660.
19. Rogers and Croft. J. Immunol. 1999. 163:1205-1213.
20. Mehta and Maino. J. Immunol. Methods 1997. 208:49-59.
21. Klitz et al. Tissue Antigens 2003. 62:296-307.
22. Rudin et al. Allergy 2001.56:1042-1048.
23. Marsland et al. Proc. Natl. Acad. Sci. U.S.A. 2004. 101:6116-6121.
24. Kheradmand et al. J. Immunol. 2002. 169:5904-5911.
25. De Palma et al, Allergy, 2000. 55: Suppl 61:56-59.
26. Schwabe N & A Turner, Genetic Engineering & Biotechnology News, 2008. Vol 28, No.4
27. Larche, Chest, 2007. 132: 1007-1014
28. Smith et al., Clin. Diagn. Lab Immunol. 2001. 8: 871-879
29. Kinnunen et al. J. Allergy Clin. Immunol. 2007. 119:965 -972.
30. Godkin et al. J. Immunol. 2001. 166:6720-6727

The following numbered paragraphs (paras.) contain statements of broad combinations of the inventive technical features herein disclosed:-
1. A peptide consisting of or comprising the amino acid sequence of one of SEQ ID NOs:10, 196-210, or one of SEQ ID NOs:11, 211-233, or one of SEQ ID NOs:1-9, 12-195, 234-913 or a peptide having a contiguous amino acid sequence having at least 70% sequence identity to the amino acid sequence of one of SEQ ID NOs:10, 196-210, or one of SEQ ID NOs:11, 211-233, or one of SEQ ID NOs:1-9, 12-195, 234-913, wherein the peptide has an amino acid length of from 8 to 50 amino acids.
2. A peptide consisting of or comprising the amino acid sequence of one of SEQ ID NOs:10, 196-210 or one of SEQ ID NOs:11, 211-233 or a peptide having a contiguous amino acid sequence having at least 70% sequence identity to the amino acid sequence of one of SEQ ID NOs:10, 196-210 or one of SEQ ID NOs:11, 211-233, wherein the peptide has an amino acid length of from 8 to 50 amino acids.
3. A peptide according to paragraph 1 or 2 wherein the degree of sequence identity is chosen from one of 80%, 85%, 90% or 95%.
4. A peptide according to paragraph 1 or 2 having a maximum length of 30 amino acids and a minimum length of 9 amino acids.
5. A peptide according to paragraph 1 or 2 having a maximum length of 20 amino acids and a minimum length of 11 amino acids.
6. A peptide according to any one of paragraphs 1 to 5 wherein the peptide is capable of stimulating an immune response.
7. A peptide comprising the amino acid sequence of one of SEQ ID NOs:10, 11, 1-9, 12-58 or a peptide having a contiguous amino acid sequence having at least 80% sequence identity to the amino acid sequence of one of SEQ ID NOs:10, 11, 1-9, 12-58, wherein the peptide has an amino acid length of from 8 to 50 amino acids.
8. A peptide comprising the amino acid sequence of SEQ ID NO:10 or 11 or a peptide having a contiguous amino acid sequence having at least 80% sequence identity to the amino acid sequence of SEQ ID NO:10 or 11, wherein the peptide has an amino acid length of from 8 to 50 amino acids.
9. A peptide chosen from one of:
   (i) SEQ ID NO: 10, SEQ ID NOs: 196-210; or
   (ii) SEQ ID NO: 11, SEQ ID NOs: 211-233; or
   (iii) SEQ ID NO: 1, SEQ ID NOs: 59-80; or
   (iv) SEQ ID NO: 2, SEQ ID NOs: 81-92; or
   (v) SEQ ID NO: 3, SEQ ID NOs: 93-105; or
   (vi) SEQ ID NO: 4, SEQ ID NOs: 106-120; or
   (vii) SEQ ID NO: 5, SEQ ID NOs: 121-135; or
   (viii) SEQ ID NO: 6, SEQ ID NOs: 136-150; or
   (ix) SEQ ID NO: 7, SEQ ID NOs: 151-165; or
   (x) SEQ ID NO: 8, SEQ ID NOs: 166-180; or
   (xi) SEQ ID NO: 9, SEQ ID NOs: 181-195; or
   (xii) SEQ ID NO: 12, SEQ ID NOs: 234-248; or
   (xiii) SEQ ID NO: 13, SEQ ID NOs: 249-263; or
   (xiv) SEQ ID NO: 14, SEQ ID NOs: 264-278; or
   (xv) SEQ ID NO: 15, SEQ ID NOs: 279-293; or
   (xvi) SEQ ID NO: 16, SEQ ID NOs: 294-307; or
   (xvii) SEQ ID NO: 17, SEQ ID NOs: 308-317 ;or
   (xviii) SEQ ID NO: 18, SEQ ID NOs: 318-332 ;or
   (xix) SEQ ID NO: 19, SEQ ID NOs: 333-347 ;or
   (xx) SEQ ID NO: 20, SEQ ID NOs: 348-362 ;or
   (xxi) SEQ ID NO: 21, SEQ ID NOs: 363-377 ;or
   (xxii) SEQ ID NO: 22, SEQ ID NOs: 378-392 ;or
   (xxiii) SEQ ID NO: 23, SEQ ID NOs: 393-407 ;or
   (xxiv) SEQ ID NO: 24, SEQ ID NOs: 408-422 ;or
   (xxv) SEQ ID NO: 25, SEQ ID NOs: 423-437 ;or
   (xxvi) SEQ ID NO: 26, SEQ ID NOs: 438-452 ;or
   (xxvii) SEQ ID NO: 27, SEQ ID NOs: 453-467 ;or
   (xxviii) SEQ ID NO: 28, SEQ ID NOs: 468-482 ;or
   (xxix) SEQ ID NO: 29, SEQ ID NOs: 483-497 ;or
   (xxx) SEQ ID NO: 30, SEQ ID NOs: 498-512 ;or
   (xxxi) SEQ ID NO: 31, SEQ ID NOs: 513-527 ;or
   (xxxii) SEQ ID NO: 32, SEQ ID NOs: 528-542 ;or
   (xxxiii) SEQ ID NO: 33, SEQ ID NOs: 543-557 ;or
   (xxxiv) SEQ ID NO: 34, SEQ ID NOs: 558-572 ;or
   (xxxv) SEQ ID NO: 35, SEQ ID NOs: 573-587 ;or
   (xxxvi) SEQ ID NO: 36, SEQ ID NOs: 588-602 ;or
   (xxxvii) SEQ ID NO: 37, SEQ ID NOs: 603-617;or
   (xxxviii) SEQ ID NO: 38, SEQ ID NOs: 618-632 ;or
   (xxxix) SEQ ID NO: 39, SEQ ID NOs: 633-647 ;or
   (xl) SEQ ID NO: 40, SEQ ID NOs: 648-662 ;or
   (xli) SEQ ID NO: 41, SEQ ID NOs: 667-681 ;or
   (xlii) SEQ ID NO: 42, SEQ ID NOs: 682-696 ;or
   (xliii) SEQ ID NO: 43, SEQ ID NOs: 697-711 ;or
   (xliv) SEQ ID NO: 44, SEQ ID NOs: 712-717 ;or
   (xlv) SEQ ID NO: 45, SEQ ID NOs: 718-723 ;or
   (xlvi) SEQ ID NO: 46, SEQ ID NOs: 724-738;or
   (xlvii) SEQ ID NO: 47, SEQ ID NOs: 739-753 ;or
   (xlviii) SEQ ID NO: 48, SEQ ID NOs: 754-768 ;or
   (xlix) SEQ ID NO: 49, SEQ ID NOs: 769-783 ;or
   (I) SEQ ID NO: 50, SEQ ID NOs: 784-798 ;or
   (li) SEQ ID NO: 51, SEQ ID NOs: 799-813 ;or
   (lii) SEQ ID NO: 52, SEQ ID NOs: 814-828 ;or
   (liii) SEQ ID NO: 53, SEQ ID NOs: 829-843 ;or
   (liv) SEQ ID NO: 54, SEQ ID NOs: 844-862 ;or
   (lv) SEQ ID NO: 55, SEQ ID NOs: 863-877 ;or
   (Ivi) SEQ ID NO: 56, SEQ ID NOs: 878-892 ;or
   (Ivii) SEQ ID NO: 57, SEQ ID NOs: 893-898 ;or
   (Iviii) SEQ ID NO: 58, SEQ ID NOs: 899-913.
10. A pharmaceutical composition comprising a peptide according to any one of paragraphs 1 to 9.
11. A pharmaceutical composition according to paragraph 10 further comprising a pharmaceutically acceptable carrier, adjuvant or diluent.
12. The pharmaceutical composition of paragraph 10, wherein the pharmaceutical composition is a vaccine.
13. The peptide or pharmaceutical composition of any one of paragraphs 1 to 12 for use in the prevention or treatment of disease.
14. The peptide or pharmaceutical composition of paragraph 13 wherein the disease is an allergic disease.
15. The peptide or pharmaceutical composition of paragraph 13 wherein the disease is chosen from fungal allergy, fungal asthma, fungal infection, SAFS, ABPA, or Aspergillosis.
16. The peptide or pharmaceutical composition of paragraph 12 or 13 wherein the disease is an allergic disease caused by an *Alternaria alternata* protein allergen or by infection of tissue by *Alternaria alternata.*
17. A method of treating or preventing disease in a patient in need of treatment thereof, the method comprising administering to the patient a therapeutically effective amount of a peptide or pharmaceutical composition according to any one of paragraphs 1 to 12.
18. A method for the production of a pharmaceutical composition, the method comprising providing a peptide according to any one of paragraphs 1 to 9, and mixing the peptide with a pharmaceutically acceptable carrier, adjuvant or diluent.
19. A nucleic acid encoding a peptide of any one of paragraphs 1 to 9.
20. A cell having integrated in its genome a nucleic acid encoding a peptide of any one of paragraphs 1 to 9 operably linked to a transcription control nucleic acid sequence.
21. A nucleic acid expression vector having a nucleic acid according to paragraph 19 operably linked to a transcription control nucleic acid sequence, wherein the vector is configured for expression of a peptide according to any one of paragraphs 1 to 9 when transfected into a suitable cell.
22. A cell transfected with the nucleic acid expression vector of paragraph 21.
23. A method of identifying a peptide that is capable of stimulating an immune response, the method comprising the steps of:
   (i) providing a candidate peptide having a contiguous amino acid sequence having at least 70% sequence identity to the amino acid sequence of one of SEQ ID NOs:10, 196-210, or one of SEQ ID NOs:11, 211-233, or one of SEQ ID NOs:1-9, 12-195, 234-913, wherein the peptide has an amino acid length of from 8 to 50 amino acids, and
   (ii) testing the ability of the candidate peptide to induce an immune response.
24. The method of paragraph 23 wherein step (i) comprises providing a peptide having the sequence of one of SEQ ID NOs:10, 196-210, or one of SEQ ID NOs:11, 211-233, or one of SEQ ID NOs:1-9, 12-195, 234-913 and chemically modifying the structure of the peptide to provide the candidate peptide.
25. The method of paragraph 23 or 24 wherein step (ii) comprises contacting the candidate peptide with a population of T cells in vitro and assaying T cell proliferation.
26. The method of any one of paragraphs 23 to 25 wherein step (ii) comprises monitoring for production of IL-4 and/or IFNγ.

## Claims

1. A peptide consisting of or comprising the amino acid sequence of one of SEQ ID NOs:3, 61-62, 93-105, 318, , 324-326; or one of SEQ ID NOs: 19, 333-347, or one of SEQ ID NOs: 4, 106-120 or a peptide having a contiguous amino acid sequence having at least 70% sequence identity to the amino acid sequence of one SEQ ID NOs:3, 61-62, 93-105, 318, , 324-326; or one of SEQ ID NOs: 19, 333-347, or one of SEQ ID NOs: 4, 106-120, wherein the peptide has an amino acid length of from 8 to 50 amino acids.

2. A peptide according to claim 1 wherein the degree of sequence identity is chosen from one of 80%, 85%, 90% or 95%.

3. A peptide according to claim 1 or 2 having a maximum length of 30 amino acids and a minimum length of 9 amino acids, or a maximum length of 20 amino acids and a minimum length of 11 amino acids.

4. A peptide according to any one of claims 1 to 3 wherein the peptide is capable of stimulating an immune response.

5. A pharmaceutical composition comprising a peptide according to any one of claims 1 to 3 and optionally further comprising a pharmaceutically acceptable carrier, adjuvant or diluent, the composition being optionally formulated as a vaccine.

6. The peptide or pharmaceutical composition of any one of claims 1 to 5 for use in the prevention or treatment of disease.

7. The peptide or pharmaceutical composition of claim 6 wherein the disease is an allergic disease, optionally allergic disease caused by an *Alternaria alternata* protein allergen or by infection of tissue by *Alternaria alternata..*

8. The peptide or pharmaceutical composition of claim 6 wherein the disease is chosen from fungal allergy, fungal asthma, fungal infection, SAFS, ABPA, or Aspergillosis.

9. A method for the production of a pharmaceutical composition, the method comprising providing a peptide according to any one of claims 1 to 4, and mixing the peptide with a pharmaceutically acceptable carrier, adjuvant or diluent.

10. A nucleic acid encoding a peptide of any one of claims 1 to 4.

11. A nucleic acid expression vector having a nucleic acid according to claim 10 operably linked to a transcription control nucleic acid sequence, wherein the vector is configured for expression of a peptide according to any one of claims 1 to 4 when transfected into a suitable cell.

12. A cell transfected with the nucleic acid expression vector of claim 11 or having integrated in its genome a nucleic acid encoding a peptide of any one of claims 1 to 4 operably linked to a transcription control nucleic acid sequence..

13. A method of identifying a peptide that is capable of stimulating an immune response, the method comprising the steps of:
(i) providing a candidate peptide having a contiguous amino acid sequence having at least 70% sequence identity to the amino acid sequence of one of SEQ ID NOs:3, 61-62, 93-105, 318, , 324-326; or one of SEQ ID NOs: 19, 333-347, or one of SEQ ID NOs: 4, 106-120, wherein the peptide has an amino acid length of from 8 to 50 amino acids, and
(ii) testing the ability of the candidate peptide to induce an immune response.

14. The method of claim13 wherein step (i) comprises providing a peptide having the sequence of one of SEQ ID NOs:3, 61-62, 93-105, 318, , 324-326; or one of SEQ ID NOs: 19, 333-347, or one of SEQ ID NOs: 4, 106-120 and chemically modifying the structure of the peptide to provide the candidate peptide.

15. The method of claim 13 or 14 wherein step (ii) comprises contacting the candidate peptide with a population of T cells in vitro and assaying T cell proliferation, and/or monitoring for production of IL-4 and/or IFNγ.
